(19) Europäisches Patentamt
European Patent Office
Office européen des brevets

(11) EP 2 269 993 B1

(12) **EUROPEAN PATENT SPECIFICATION**

(45) Date of publication and mention of the grant of the patent:
**27.02.2013 Bulletin 2013/09**

(21) Application number: **09734846.0**

(22) Date of filing: **23.04.2009**

(51) Int Cl.:
*C07D 239/84* (2006.01) *A61K 31/517* (2006.01)
*A61K 31/5377* (2006.01) *A61P 35/00* (2006.01)
*A61P 35/02* (2006.01) *A61P 43/00* (2006.01)
*C07D 401/04* (2006.01) *C07D 401/12* (2006.01)
*C07D 401/14* (2006.01) *C07D 403/04* (2006.01)
*C07D 403/14* (2006.01) *C07D 405/12* (2006.01)
*C07D 405/14* (2006.01) *C07D 413/04* (2006.01)
*C07D 413/14* (2006.01) *C07D 417/04* (2006.01)

(86) International application number:
**PCT/JP2009/058064**

(87) International publication number:
**WO 2009/131173 (29.10.2009 Gazette 2009/44)**

(54) **2-AMINOQUINAZOLINE DERIVATIVE**

2-AMINOCHINAZOLIN-DERIVAT

DÉRIVÉ DE 2-AMINOQUINAZOLINE

(84) Designated Contracting States:
**AT BE BG CH CY CZ DE DK EE ES FI FR GB GR HR HU IE IS IT LI LT LU LV MC MK MT NL NO PL PT RO SE SI SK TR**

(30) Priority: **23.04.2008 JP 2008113169**

(43) Date of publication of application:
**05.01.2011 Bulletin 2011/01**

(73) Proprietor: **Kyowa Hakko Kirin Co., Ltd.**
**Tokyo 100-8185 (JP)**

(72) Inventors:
- **KASHIMA, Hajime**
  **Shizuoka 411-8731 (JP)**
- **NISHIKAWA, Tomoyuki**
  **Shizuoka 411-8731 (JP)**
- **YAMADA, Yusuke**
  **Shizuoka 411-8731 (JP)**
- **NAKASATO, Yoshisuke**
  **Shizuoka 411-8731 (JP)**
- **SEIKE, Toshihiro**
  **Shizuoka 411-8731 (JP)**
- **UMEHARA, Hiroshi**
  **Shizuoka 411-8731 (JP)**
- **NAKASATO, Yoshisuke**
  **Shizuoka 411-8731 (JP)**

(74) Representative: **von Uexküll - Güldenband, Alexa et al**
**Vossius & Partner**
**Postfach 86 07 67**
**81634 München (DE)**

(56) References cited:
**EP-A1- 1 726 584** **EP-A1- 1 878 727**
**WO-A1-2006/137421** **WO-A1-2008/050808**
**WO-A2-2006/039718**

- **DIMAURO, E.F. ET AL.: 'Structure-Guided Design of Aminopyrimidine Amides as Potent, Selective Inhibitors of Lymphocyte Specific Kinase: Synthesis, Structure-Activity Relationships, and Inhibition of in Vivo T Cell Activation' JOURNAL OF MEDICINAL CHEMISTRY vol. 51, no. 6, March 2008, pages 1681 - 1694, XP008143310**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

### Technical Field

The present invention relates to a 2-aminoquinazoline derivative having Tie-2 kinase inhibitory activity, and the like.

### Background Art

Tie-2 kinase (also referred to as TEK) is a member of the receptor tyrosine kinase (RTK) family, which is mainly expressed in an endothelial cell and early hemopoietic cells, and plays a critical role in the steps of vasculogenesis and angiogenesis. The angiopoietin family of the growth factor regulates Tie-2 kinase activity through a combination of agonistic and antagonistic extracellular ligands. Binding of angiopoietin (Ang)-1 or Ang-4, which is a ligand of Tie-2 kinase, induces autophosphorylation, results in an increase of receptor-dependent signaling, while binding to Ang-2 and Ang-3 results in down-regulation of the receptor activity. Ang-1 signaling through Tie-2 kinase facilitates later stages of vascular development by regulating cell-cell, and cell-matrix interactions, resulting in the survival and stabilization of newly formed blood vessels [Nature Reviews Molecular Cell Biology, vol.2, p.257 (2001)].

Tumor vessel progression requires the recruitment of new blood vessels into the tumor from existing vessels. Tie-2 kinase expression is identified in a wide variety of tumor types including ovarian cancer, breast cancer, renal cancer, prostate cancer, lung cancer, thyroid cancer, myeloid leukemia, hemangiomas, melanomas, astrocytomas, and glioblastomas, and is known to be related to these tumors [International Journal of Cancer, vol.99, p.821 (2002), and the like]. Tie-2 kinase activation is related to venous malformations (VM), the most common form of vascular morphogenesis in humans.

Also, Tie-2 kinase activation is known to be related to psoriasis and pulmonary hypertension.
Furthermore, a pyridinylimidazole derivative which is a Tie-2 kinase inhibitor is known to suppresses tumor growth (see Non-Patent Literature 1).
From the above, a Tie-2 kinase inhibitor is considered to be useful as an agent for treating and/or preventing cancer or a disease related to vasculogenesis and angiogenesis.

On the other hand, antihypertensive agents (see Patent Literature 1), phosphodiesterase (PDE) inhibitors (see Patent Literature 2), PDE-IV inhibitors (see Patent Literature 3), serine/threonine protein kinase regulators (see Patent Literature 4), antifungals (see Patent Literatures 5 and 8), neuropeptide ligands (see Patent Literature 6), developer compositions (see Patent Literature 7), insecticides (see Patent Literature 9), kinase inhibitors (see Patent Literatures 10 and 11), cyclin dependent kinase inhibitors (see Patent Literature 12), therapeutic agents for diabetes (see Patent Literature 13), p38MAP kinase inhibitors (see Patent Literature 14), PDK1 inhibitors (see Patent Literature 15), and the like which contain a 2-aminoquinazoline derivative have been known.

### Prior Art

### Patent Literature

Patent Literature 1: Japanese Published Examined Patent Application No. 25050/1964
Patent Literature 2: WO 93/07124
Patent Literature 3: WO 93/22460
Patent Literature 4: WO 98/50370
Patent Literature 5: U.S. Patent No. 6156758
Patent Literature 6: WO 2003/26667
Patent Literature 7: Japanese Published Unexamined Patent Application No. 324437/1994
Patent Literature 8: WO 2004/098494
Patent Literature 9: WO 2005/087742
Patent Literature 10: WO 2006/015859
Patent Literature 11: WO 2006/039718
Patent Literature 12: WO 01/38315
Patent Literature 13: WO 2006/071095
Patent Literature 14: WO 2006/118256
Patent Literature 15: WO 2007/117607


Non-Patent Literature

Non-Patent Literature 1: Bioorg. Med. Chem. Lett., 2007, vol.17, p.4756-4760

Summary of Invention

Problems to be Solved by Invention

An object of the present invention is to provide a 2-aminoquinazoline derivative or a pharmaceutically acceptable salt thereof having Tie-2 kinase inhibitory activity, and the like.

Means for Solving Problems

The present invention relates to the following (1) to (25).

(1) A 2-aminoquinazoline derivative represented by formula (I)

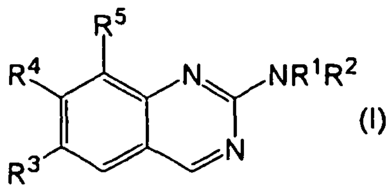

(I)

{wherein in formula (I), $R^1$ represents a hydrogen atom, optionally substituted lower alkyl, optionally substituted lower alkenyl, optionally substituted lower alkynyl, optionally substituted cycloalkyl, optionally substituted cycloalkenyl, optionally substituted lower alkanoyl, optionally substituted cycloalkylcarbonyl, optionally substituted lower alkoxycarbonyl, optionally substituted aryl, an optionally substituted aliphatic heterocyclic group, an optionally substituted aromatic heterocyclic group, or
$-C(=O)NR^6R^7$ (wherein $R^6$ and $R^7$ may be the same or different and each represents a hydrogen atom or optionally substituted lower alkyl),
$R^2$ represents a hydrogen atom,
$R^3$ represents formula (II)

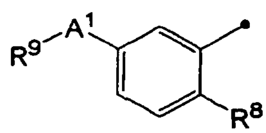

(II)

[wherein in formula (II), $A^1$ represents formula (III)

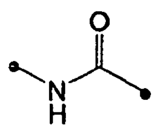

(III)

or formula (IV)

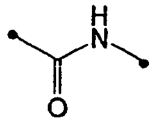

(IV)

,

$R^8$ represents a hydrogen atom or optionally substituted lower alkyl, and $R^9$ represents optionally substituted aryl or an optionally substituted aromatic heterocyclic group],
$R^4$ represents hydroxy, or optionally substituted lower alkoxy, and
$R^5$ represents optionally substituted aryl, or an optionally substituted aromatic heterocyclic group}, or

a pharmaceutically acceptable salt thereof.

(2) The 2-aminoquinazoline derivative or a pharmaceutically acceptable salt thereof according to (1), wherein $R^1$ is a hydrogen atom, optionally substituted lower alkyl, optionally substituted lower alkenyl, optionally substituted lower alkynyl, optionally substituted cycloalkenyl, optionally substituted lower alkanoyl, optionally substituted cycloalkylcarbonyl, optionally substituted lower alkoxycarbonyl or - $C(=O)NR^6R^7$ (wherein $R^6$ and $R^7$ have the same meanings as defined above, respectively).

(3) The 2-aminoquinazoline derivative or a pharmaceutically acceptable salt thereof according to (1), wherein $R^1$ is a hydrogen atom.

(4) The 2-aminoquinazoline derivative or a pharmaceutically acceptable salt thereof according to (1), wherein $R^1$ is optionally substituted lower alkyl, optionally substituted lower alkanoyl, optionally substituted cycloalkylcarbonyl or optionally substituted lower alkoxycarbonyl.

(5) The 2-aminoquinazoline derivative or a pharmaceutically acceptable salt thereof according to (1), wherein $R^1$ is optionally substituted cycloalkyl, optionally substituted aryl, an optionally substituted aliphatic heterocyclic group or an optionally substituted aromatic heterocyclic group.

(6) The 2-aminoquinazoline derivative or a pharmaceutically acceptable salt thereof according to any one of (1) to (5), wherein $R^4$ is hydroxy or methoxy.

(7) The 2-aminoquinazoline derivative or a pharmaceutically acceptable salt thereof according to any one of (1) to (6), wherein $R^8$ is methyl.

(8) The 2-aminoquinazoline derivative or a pharmaceutically acceptable salt thereof according to any one of (1) to (7), wherein $R^9$ is a group represented by formula (V)

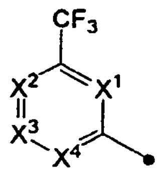

(V)

(wherein $X^1$, $X^2$, $X^3$ , and $X^4$ may be the same or different, and each represents CH, C-$A^2$ {wherein $A^2$ represents halogen, optionally substituted lower alkyl, an optionally substituted aliphatic heterocyclic group, an optionally substituted aromatic heterocyclic group, optionally substituted lower alkoxy, optionally substituted aliphatic heterocycle-oxy, optionally substituted aromatic heterocycle-oxy, optionally substituted aliphatic heterocycle-methyl, optionally substituted aromatic heterocycle-methyl, $CH_2NR^{10}R^{11}$ (wherein $R^{10}$ and $R^{11}$ may be the same or different, and each represents a hydrogen atom, or optionally substituted lower alkyl) or $NR^{12}R^{13}$ [wherein $R^{12}$ represents a hydrogen atom or optionally substituted lower alkyl, and $R^{13}$ represents a hydrogen atom, optionally substituted lower alkyl, an optionally substituted aliphatic heterocyclic group, an optionally substituted aromatic heterocyclic group or -$NR^{10A}R^{11A}$ (wherein $R^{10A}$ and $R^{11A}$ have the same meanings as $R^{10}$ and $R^{11}$ defined above, respectively)]}, or a nitrogen atom).

(9) The 2-aminoquinazoline derivative or a pharmaceutically acceptable salt thereof according to (8), wherein $X^1$, $X^2$, $X^3$, and $X^4$ may be the same or different, and each is CH or a nitrogen atom.

(10) The 2-aminoquinazoline derivative or a pharmaceutically acceptable salt thereof according to (8), wherein $A^2$ is an optionally substituted aliphatic heterocyclic group, $NR^{12}R^{13}$ (wherein $R^{12}$ and $R^{13}$ have the same meanings as defined above, respectively), optionally substituted aliphatic heterocycle-oxy, optionally substituted aliphatic heterocycle-methyl, or $CH_2NR^{10}R^{11}$ (wherein $R^{10}$ and $R^{11}$ have the same meanings as defined above, respectively).

(11) The 2-aminoquinazoline derivative or a pharmaceutically acceptable salt thereof according to (8), wherein $A^2$ is $NR^{12}R^{13}$, wherein said $R^{12}$ is a hydrogen atom, and said $R^{13}$ is optionally substituted lower alkyl.

(12) The 2-aminoquinazoline derivative or a pharmaceutically acceptable salt thereof according to (8), wherein one of $X^1$, $X^2$, $X^3$, and $X^4$ is a nitrogen atom, and the others may be the same or different, and each is CH or C-$A^2$ (wherein $A^2$ has the same meaning as defined above).

(13) The 2-aminoquinazoline derivative or a pharmaceutically acceptable salt thereof according to (8), wherein among $X^1$, $X^2$, $X^3$, and $X^4$, one of $X^1$, $X^2$ and $X^4$ is a nitrogen atom, and the other three may be the same or different, and each is CH or C-$A^2$ (wherein $A^2$ has the same meaning as defined above).

(14) The 2-aminoquinazoline derivative or a pharmaceutically acceptable salt thereof according to (8), wherein $X^1$, $X^2$, and $X^3$ may be the same or different, and each is CH or C-$A^2$ (wherein $A^2$ has the same meaning as defined above), and $X^4$ is a nitrogen atom.

(15) The 2-aminoquinazoline derivative or a pharmaceutically acceptable salt thereof according to (8), wherein among $X^1$, $X^2$, $X^3$, and $X^4$, any two of them are nitrogen atoms, and the other two may be the same or different, and each is CH or C-$A^2$ (wherein $A^2$ has the same meaning as defined above).

(16) The 2-aminoquinazoline derivative or a pharmaceutically acceptable salt thereof according to (8), wherein among $X^1$, $X^2$, $X^3$, and $X^4$, $X^1$ and $X^4$, or $X^2$ and $X^4$, are nitrogen atoms, and the other two may be the same or different, and each is CH or C-$A^2$ (wherein $A^2$ has the same meaning as defined above).
(17) The 2-aminoquinazoline derivative or a pharmaceutically acceptable salt thereof according to any one of (1) to (16), wherein $A^1$ represents formula **(III).**

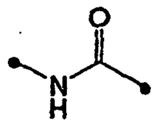
**(III)**

Use of the 2-aminoquinazoline derivative or a pharmaceutically acceptable salt thereof according to any one (1) to (17) for the manufacture of a Tie-2 kinase inhibitor.

The 2-aminoquinazoline derivative or a pharmaceutically acceptable salt thereof according to any one of (1) to (17) for use in the inhibition of Tic-2 kinase.

Effects of Invention

The present invention provides a 2-aminoquinazoline derivative or a pharmaceutically acceptable salt thereof having Tie-2 kinase inhibitory activity, and the like. The 2-aminoquinazoline derivative or a pharmaceutically acceptable salt thereof is useful as an agent for treating and/or preventing a disease associated with Tie-2 kinase (for example, ovarian cancer, breast cancer, renal cancer, prostate cancer, lung cancer, thyroid cancer, myeloid leukemia, hemangiomas, melanomas, astrocytomas, glioblastomas, psoriasis or pulmonary hypertension, or the like).

Modes for Carrying Out Invention

The compound represented by formula (I) is referred to as Compound (I). The same applies to the compounds of other formula numbers.
In the definition of respective groups of formula (I),

(i) examples of the lower alkyl, and the lower alkyl moiety of lower alkoxycarbonyl and lower alkoxy, include linear or branched alkyl having 1 to 10 carbon atoms, and more specifically, methyl, ethyl, propyl, isopropyl, butyl, isobutyl, sec-butyl, *tert*-butyl, pentyl, isopentyl, neopentyl, hexyl, heptyl, octyl, nonyl, decyl, and the like.
(ii) Examples of the lower alkanoyl include linear or branched alkanoyl having 2 to 11 carbon atoms, and more specifically, acetyl, propionyl, pivaloyl, butanoyl, pentanoyl, hexanoyl, heptanoyl, octanoyl, nonanoyl, decanoyl, undecanoyl, and the like.
(iii) Examples of the lower alkenyl include linear or branched alkenyl having 2 to 10 carbon atoms, and more specifically, vinyl, allyl, 1-propenyl, butenyl, pentenyl, hexenyl, heptenyl, octeneyl, nonenyl, decenyl, and the like.
(iv) Examples of the lower alkynyl include linear or branched alkynyl having 2 to 10 carbon atoms, and more specifically, ethynyl, propynyl, butynyl, pentynyl, hexynyl, heptynyl, octynyl, nonynyl, decynyl, and the like.
(v) Examples of the cycloalkyl and the cycloalkyl moiety of cycloalkylcarbonyl include cycloalkyl having 3 to 8 carbon atoms, crosslinking cycloalkyl having 4 to 8 carbon atoms and bicyclic or tricyclic spirocycloalkyl where cylcloalkyl having 3 to 8 carbon atoms is spiro-bonded, and the like, more specifically, cyclopropyl, cyclobutyl, cyclopentyl, cyclohexyl, cycloheptyl, cyclooctyl, adamantyl, noradamantyl, bicyclo[2.2.1]heptyl, spiro[4.5]decanyl, and the like.
(vi) Examples of the cycloalkenyl include cycloalkenyl having 3 to 8 carbon atoms, and more specifically, cyclopropenyl, cyclobutenyl, cyclopentenyl, cyclohexenyl, cycloheptenyl, cyclooctenyl, and the like.
(vii) Examples of the aryl include aryl having 6 to 14 carbon atoms, and more specifically, phenyl, naphthyl, azulenyl, anthryl, and the like.
(viii) Examples of the aliphatic heterocyclic group and the aliphatic heterocyclic group moiety of an aliphatic heterocycle-oxy and aliphatic heterocycle-methyl include a 5- or 6-membered monocyclic aliphatic heterocyclic group containing at least one atom selected from a nitrogen atom, an oxygen atom, and a sulfur atom, a bicyclic or tricyclic condensed aliphatic heterocyclic group in which 3- to 8-membered rings are condensed, containing at least one atom selected from a nitrogen atom, an oxygen atom, and a sulfur atom, and the like. More specifically, examples thereof include aziridinyl, azetidinyl, pyrrolidinyl, piperidino, piperidinyl, azepanyl, 1,2,5,6-tetrahydropyridyl, imidazolidinyl, pyrazolidinyl, piperazinyl, homopiperazinyl, pyrazolinyl, oxyranyl, tetrahydrofuranyl, tetrahydro-2H-pyranyl (tetrahydropyranyl), 5,6-dihydro-2H-pyranyl, tetrahydrothiophenyl, tetrahydro-2H-thiopyranyl, oxazolidinyl, morpholino, morpholinyl, thioxazolidinyl, thiomorpholinyl, 2H-oxazolyl, 2H-thioxazolyl, dihydroindolyl, dihydroisoindolyl,

dihydrobenzofuranyl, benzimidazolidinyl, dihydrobenzoxazolyl, dihydrobenzothioxazolyl, benzodioxolinyl, tetrahydroquinolyl, tetrahydroisoquinolyl, dihydro-2H-chromanyl, dioxepanyl, dihydro-1H-chromanyl, dihydro-2H-thiochromanyl, dihydro-1H -thiochromanyl, tetrahydroquinoxalinyl, tetrahydroquinazolinyl, dihydrobenzodioxanyl, and the like.
(ix) Examples of the aromatic heterocyclic group and the aromatic heterocyclic group moiety of an aromatic heterocyclic group oxy and an aromatic heterocyclic group methyl include a 5- or 6-membered monocyclic aromatic heterocyclic group which contains at least one atom selected from a nitrogen atom, an oxygen atom and a sulfur atom, a bicyclic or tricyclic condensed aromatic heterocyclic group in which 3- to 8-membered rings are condensed and contains at least one atom selected from a nitrogen atom, an oxygen atom and a sulfur atom, and the like. More specific examples thereof include furyl, thienyl, pyrrolyl, imidazolyl, pyrazolyl, oxazolyl, isoxazolyl, oxadiazolyl, thiazolyl, isothiazolyl, thiadiazolyl, triazolyl, tetrazolyl, pyridyl, pyridine 1-oxide-2-yl, pyridine 1-oxide-3-yl, pyridine 1-oxide-4-yl, pyridazinyl, pyrimidinyl, pyrazinyl, triazinyl, benzofuranyl, benzothiophenyl, benzoxazolyl, benzothiazolyl, isoindolyl, indolyl, indazolyl, benzimidazolyl, benzotriazolyl, oxazolopyrimidinyl, thiazolopyrimidinyl, pyrrolopyridinyl pyrrolopyrimidinyl, imidazopyridinyl, purinyl, quinolinyl, isoquinolinyl, cinnolinyl, phthalazinyl, quinazolinyl, quinoxalinyl, naphthyridinyl, and the like.
(x) The halogen means each atom of a fluorine atom, a chlorine atom, a bromine atom, and an iodine atom.
(xi) Examples of the substituent(s) of the optionally substituted lower alkyl, the optionally substituted lower alkenyl, the optionally substituted lower alkynyl, the optionally substituted lower alkanoyl, the optionally substituted lower alkoxy and the optionally substituted lower alkoxycarbonyl, include substituents which may be the same or different and, for example, in number of 1 to 3,selected from the group consisting of
halogen, hydroxy, sulfanyl, nitro, cyano, carboxy, carbamoyl, $C_{3-8}$ cycloalkyl, an aliphatic heterocyclic group, an aromatic heterocyclic group, $C_{1-10}$ alkoxy, $C_{3-8}$ cycloalkoxy, $C_{6-14}$ aryloxy, $C_{7-16}$ aralkyloxy, $C_{2-11}$ alkanoyloxy, $C_{7-15}$ aroyloxy, $C_{1-10}$ alkylsulfanyl, $-NR^XR^Y$ [wherein $R^X$ and $R^Y$ may be the same or different and each represents a hydrogen atom; $C_{1-10}$ alkyl optionally substituted with 1 to 3 substituents selected from hydroxy, $C_{1-10}$ alkoxy, amino, $C_{1-10}$ alkylamino and di($C_{1-10}$ alkyl)amino; $C_{3-8}$ cycloalkyl; $C_{6-14}$ aryl; an aromatic heterocyclic group; aromatic heterocyclic alkyl, aliphatic heterocyclic alkyl; $C_{7-16}$ aralkyl, $C_{2-11}$ alkanoyl; $C_{7-15}$ aroyl; $C_{1-10}$ alkoxycarbonyl or $C_{7-16}$ aralkyloxycarbonyl], $C_{2-11}$ alkanoyl, $C_{7-15}$ aroyl, $C_{1-10}$ alkoxycarbonyl, $C_{6-14}$ aryloxycarbonyl, $C_{1-10}$ alkylcarbamoyl, and di($C_{1-10}$ alkyl)carbamoyl, and the like.
(xii) Examples of the substituents of the optionally substituted aryl, the optionally substituted aromatic heterocyclic group, the optionally substituted aromatic heterocycle-oxy and the optionally substituted aromatic heterocycle-methyl include substituents which may be the same or different and, for example, in number of 1 to 3, selected from the group consisting of
halogen; hydroxy; sulfanyl; nitro; cyano; carboxy; carbamoyl; $C_{1-10}$ alkyl optionally substituted with 1 to 3 substituents selected from hydroxy, $C_{1-10}$ alkoxy, amino, $C_{1-10}$ alkylamino and di($C_{1-10}$ alkyl)amino; trifluoromethyl; $C_{3-8}$ cycloalkyl; $C_{6-14}$ aryl; an optionally substituted aliphatic heterocyclic group; optionally substituted aliphatic heterocyclic alkyl; optionally substituted aliphatic heterocycle-oxy; an optionally substituted aromatic heterocyclic group ; optionally substituted $C_{1-10}$ alkoxy ; $C_{3-8}$ cycloalkoxy; $C_{6-14}$ aryloxy; $C_{7-16}$ aralkyloxy; $C_{2-11}$ alkanoyloxy; $C_{7-15}$ aroyloxy; $C_{1-10}$ alkylsulfanyl; - $NR^{X1}R^{Y1}$ (wherein $R^{X1}$ and $R^{Y1}$ have the same meanings as $R^X$ and $R^Y$ defined above, respectively); $C_{2-11}$ alkanoyl; $C_{7-15}$ aroyl; $C_{1-10}$ alkoxycarbonyl; $C_{6-14}$ aryloxycarbonyl; $C_{1-10}$ alkylcarbamoyl; and di($C_{1-10}$ alkyl) carbamoyl, and the like.

Examples of the substituents (xi-1) of the optionally substituted aliphatic heterocyclic group, the optionally substituted aliphatic heterocyclic alkyl, the optionally substituted aliphatic heterocycle-oxy, and the optionally substituted aromatic heterocyclic group which are described herein, include substituents which may be the same or different and, for example, in number of 1 to 3, selected from the group consisting of
oxo, halogen, hydroxy, sulfanyl, nitro, cyano, carboxy, carbamoyl, $C_{1-10}$ alkyl, hydroxy $C_{1-10}$ alkyl, trifluoromethyl, $C_{3-8}$ cycloalkyl, $C_{6-14}$ aryl, an aliphatic heterocyclic group, an aromatic heterocyclic group, $C_{1-10}$ alkoxy, $C_{3-8}$ cycloalkoxy, $C_{6-14}$ aryloxy, $C_{7-16}$ aralkyloxy, $C_{2-11}$ alkanoyloxy, $C_{7-15}$ aroyloxy, $C_{1-10}$ alkylsulfanyl, $-NR^{X3}R^{Y3}$ (wherein $R^{X3}$ and $R^{Y3}$ have the same meanings as $R^X$ and $R^Y$ defined above, respectively), $C_{2-11}$ alkanoyl, $C_{7-15}$ aroyl, $C_{1-10}$ alkoxycarbonyl, $C_{6-14}$ aryloxycarbonyl, $C_{1-10}$ alkylcarbamoyl, and di($C_{1-10}$ alkyl)carbamoyl, and the like.
Examples of the substituent (xii-2) of the optionally substituted $C_{1-10}$ alkoxy include the group exemplified in the above substituent (xi) of the substituted lower alkyl, and the like.
(xiii) Examples of the substituent of the optionally substituted cycloalkyl, the optionally substituted cycloalkenyl, the optionally substituted cycloalkylcarbonyl, the optionally substituted aliphatic heterocyclic group, the optionally substituted aliphatic heterocycle-oxy, and the optionally substituted aliphatic heterocycle-methyl, include substituents, which may be the same or different and, for example, in number of 1 to 3, selected from the group consisting of
oxo, halogen, hydroxy, sulfanyl, nitro, cyano, carboxy, carbamoyl, $C_{1-10}$ alkyl optionally substituted with l to 2 of hydroxy, trifluoromethyl, $C_{3-8}$ cycloalkyl, $C_{6-14}$ aryl, an aliphatic heterocyclic group, an aromatic heterocyclic group, $C_{1-10}$ alkoxy, $C_{3-8}$ cycloalkoxy, $C_{6-14}$ aryloxy, $C_{7-16}$ aralkyloxy, $C_{2-11}$ alkanoyloxy, $C_{7-15}$ aroyloxy, $C_{1-10}$ alkylsulfanyl, $-NR^{X4}R^{Y4}$ (wherein

$R^{X4}$ and $R^{Y4}$ have the same meanings as $R^X$ and $R^Y$ defined above, respectively), $C_{2-11}$ alkanoyl, $C_{7-15}$ aroyl, $C_{1-10}$ alkoxycarbonyl, $C_{6-14}$ aryloxycarbonyl, $C_{1-10}$ alkylcarbamoyl, and di($C_{1-10}$ alkyl)carbamoyl, and the like.

Examples of the $C_{1-10}$ alkyl and the $C_{1-10}$ alkyl moiety of the $C_{1-10}$ alkoxy, the $C_{2-11}$ alkanoyloxy, the $C_{1-10}$ alkylsulfanyl, the $C_{2-11}$ alkanoyl, the $C_{1-10}$ alkoxycarbonyl, the $C_{1-10}$ alkylcarbamoyl, and the di($C_{1-10}$ alkyl)carbamoyl, which are described in (xi) to (xiii), include groups exemplified in above (i) lower alkyl, and the like. Two $C_{1-10}$ alkyls in the di($C_{1-10}$ alkyl)amino $C_{1-10}$ alkyl, and the di($C_{1-10}$ alkyl)carbamoyl may be the same or different.

Examples of the $C_{3-8}$ cycloalkyl and the cycloalkyl moiety of the $C_{3-8}$ cycloalkoxy include groups exemplified in above (v) cycloalkyl, and the like.

Examples of the $C_{6-14}$ aryl and the aryl moiety of the $C_{6-14}$ aryloxy, the $C_{7-15}$ aroyl, the $C_{7-15}$ aroyloxy, and the $C_{6-14}$ aryloxycarbonyl include groups exemplified in above (vii) aryl, and the like.

Examples of the $C_{7-16}$ aralkyl and the aralkyl moiety of the $C_{7-16}$ aralkyloxy, and the $C_{7-16}$ aralkyloxycarbonyl include aralkyl having 7 to 16 carbon atoms, and more specifically benzyl, phenethyl, phenylpropyl, phenylbutyl, phenylpentyl, phenylhexyl, phenylheptyl, phenyloctyl, phenylnonyl, phenyldecyl, naphthylmethyl, naphthylethyl, naphthylpropyl, naphthylbutyl, naphthylpentyl, naphthylhexyl, anthrylmethyl, anthrylethyl, and the like.

Examples of the aliphatic heterocyclic group and the aliphatic heterocyclic group moiety of the aliphatic heterocyclic alkyl and the aliphatic heterocycle-oxy include groups exemplified in above (viii) aliphatic heterocyclic group, and the like, and examples of the aromatic heterocyclic group and the aromatic heterocyclic group moiety of the aromatic heterocyclic alkyl include groups exemplified in above (ix) aromatic heterocyclic group, and the like. Furthermore, examples of the alkyl moiety of the aliphatic heterocyclic alkyl and the aromatic heterocyclic alkyl include a group which removes a hydrogen atom from the group exemplified in above (i) lower alkyl, and the like. The halogen includes an atom exemplified in (x) halogen. The sulfanyl means -SH.

In respective groups of Compound (I) in addition to (1) to (17),

As the $R^1$, for example, a hydrogen atom, optionally substituted lower alkyl, optionally substituted lower alkenyl, optionally substituted lower alkynyl, optionally substituted cycloalkyl, optionally substituted cycloalkenyl, optionally substituted lower alkanoyl, optionally substituted cycloalkylcarbonyl, optionally substituted lower alkoxycarbonyl, an optionally substituted aliphatic heterocyclic group , or - C(=O)$NR^6R^7$ (wherein $R^6$ and $R^7$ may be the same or different, respectively, and the like are preferred. More preferably, for example, a hydrogen atom, optionally substituted lower alkyl, optionally substituted lower alkanoyl, optionally substituted cycloalkylcarbonyl, optionally substituted lower alkoxycarbonyl, or an optionally substituted aliphatic heterocyclic group is more preferred. For example, a hydrogen atom, optionally substituted lower alkyl, cycloalkylcarbonyl, lower alkoxycarbonyl, and the like are further preferred.

As the $A^1$ of formula (II) in the definition of $R^3$, for example, formula (III) and the like are preferred.

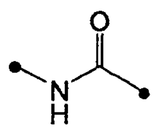

(III)

Further, as the $R^8$, for example, optionally substituted lower alkyl and the like are preferred, methyl and the like are more preferred.

Furthermore, as the $R^9$, for example, substituted aryl, a substituted aromatic heterocyclic group and the like are preferred. As the aryl moiety of said substituted aryl, for example, phenyl and the like are preferred. As the substituent of said substituted aryl, for example, trifluoromethyl and the like are preferred. In other words, as said substituted aryl, for example, m-trifluoromethylphenyl and the like are preferred. Also, as the aromatic heterocyclic group moiety of the substituted aromatic heterocyclic group, for example, pyridyl, pyridine 1-oxide-2-yl, pyridine 1-oxide-3-yl, pyridine 1-oxide-4-yl, pyrimidinyl, pyrazinyl, pyridazinyl, pyrazolyl, thiadiazolyl, oxazolyl, thiazolyl, indolyl, quinolinyl, oxadiazolyl, and the like are preferred, and pyridyl, pyrimidinyl and the like are more preferred. Specifically, in the group defined in formula (V), an aromatic heterocyclic group represented by a group where $X^1$ and $X^4$ are a nitrogen atom, one of $X^2$ and $X^3$ is C-$A^2$, and the other is CH; a group where $X^2$ and $X^4$ are a nitrogen atom, one of $X^1$ and $X^3$ is C-$A^2$, the other is CH; a group where at least one of $X^1$, $X^2$, and $X^3$ is C-$A^2$, others are CH, $X^4$ is a nitrogen atom, and the like are preferred. As the substituent of said substituted aromatic heterocyclic group, among the substituents exemplified in above (xii), halogen; trifluoromethyl; - $NR^{X1}R^{Y1}$ (wherein $R^{X1}$ and $R^{Y1}$ have the same meanings as defined above, respectively); $C_{1-10}$ alkyl optionally substituted with 1 to 3 substituents selected from hydroxy, $C_{1-10}$ alkoxy, amino, $C_{1-10}$ alkylamino and di($C_{1-10}$ alkyl)amino; an optionally substituted aromatic heterocyclic group; an optionally substituted aliphatic heterocyclic group; optionally substituted aliphatic heterocycle-oxy and the like are preferred, trifluoromethyl and the like are more preferred.

As the $R^4$, for example, hydroxy, methoxy and the like are preferred.

As the aryl moiety of the optionally substituted aryl in the $R^5$, for example, phenyl and the like are preferred, as the substituent of said aryl, for example, among the substituents exemplified in the above (xii), halogen; cyano; hydroxy; $C_{1-10}$ alkyl optionally substituted with 1 to 3 substituents selected from $C_{1-10}$ alkoxy, amino, $C_{1-10}$ alkylamino and di($C_{1-10}$ alkyl)amino; optionally substituted $C_{1-10}$ alkoxy; an optionally substituted aliphatic heterocyclic group; $-NR^{X1}R^{Y1}$(wherein $R^{X1}$ and $R^{Y1}$ have the same meanings as defined above, respectively) and the like are preferred. Also, as the aromatic heterocyclic group moiety of the optionally substituted aromatic heterocyclic group, for example, pyridyl, pyrimidinyl, pyrazolyl, pyrazinyl, oxazolyl, thiazolyl, indolyl, quinolyl, 1,2,4-oxadiazolyl and the like are preferred, pyridyl, pyrimidinyl, pyrazolyl, pyrazinyl and the like are more preferred. As the substituent of said optionally substituted aromatic heterocyclic group, for example, among the substituents exemplified in above (xii), halogen; $C_{1-10}$ alkyl optionally substituted with 1 to 3 substituents selected from hydroxy, $C_{1-10}$ alkoxy, amino, $C_{1-10}$ alkylamino and di($C_{1-10}$ alkyl)amino; optionally substituted $C_{1-10}$ alkoxy; an optionally substituted aliphatic heterocyclic group; $-NR^{X1}R^{Y1}$(wherein $R^{X1}$ and $R^{Y1}$ have the same meanings as defined above, respectively) and the like are preferred.

Examples of the pharmaceutically acceptable salts of Compound (I) include pharmaceutically acceptable acid addition salt, pharmaceutically acceptable metal salt, pharmaceutically acceptable ammonium salt, pharmaceutically acceptable organic amine addition salt, pharmaceutically acceptable amino acid addition salt, and the like. Examples of the pharmaceutically acceptable acid addition salts of Compound (1) include inorganic acid salts such as hydrochloride, hydrobromide, nitrate, sulfate, and phosphate, and organic acid salts such as acetate, oxalate, maleate, fumarate, citrate, benzoate, and methanesulfonate. Examples of the pharmaceutically acceptable metal salts include alkaline metal salts such as sodium salt and potassium salt, alkaline earth metal salts such as magnesium salt and calcium salt, aluminum salt, zinc salt, and the like. Examples of the pharmaceutically acceptable ammonium salts include salts of ammonium, tetramethyl ammonium, and the like. Examples of the pharmaceutically acceptable organic amine addition salt include addition salts of morpholine, piperidine, and the like. Examples of the pharmaceutically acceptable amino acid addition salts include addition salts of lysine, glycine, phenylalanine, aspartic acid, glutamic acid, and the like.

Next, the production methods of Compound (I) are described below.

In the following production methods, when the defined group may be changed under the condition of the production methods or the production method is not suitable to perform the production process, a desirable compound can be produced by using introducing and removal process of a protective group generally employed in organic synthetic chemistry and removal process [for example, a process described in Protective Groups in Organic Synthesis, third edition, T. W. Greene, John Wiley & Sons Inc.(1999), and the like], and the like. Also, if necessary, the order of the reaction steps such as introduction of substituents can be changed.

For example, Compound (I) can be produced by Production Processes 1 to 5.

Production Method 1

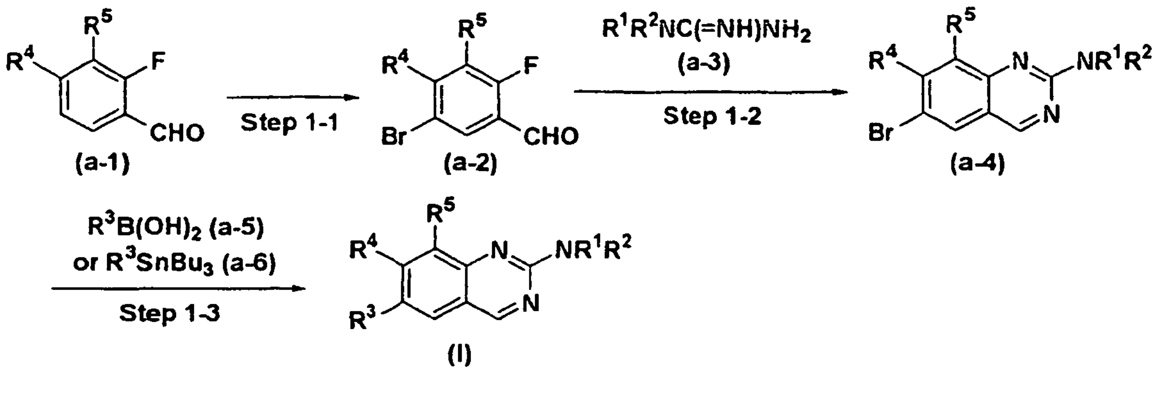

(a-1) (a-2) (a-4) (I)

(wherein $R^1$, $R^2$, $R^3$, $R^4$ and $R^5$ have the same meanings as defined above, respectively, and Bu represents butyl)

Step 1-1

Compound (a-2) can be obtained by reacting Compound (a-1) with 1 to 20 equivalents of bromine in a solvent.

As the solvent, for example, acetic acid, carbon tetrachloride, chloroform, dichloromethane, 1,2-dichloroethane, dioxane, tetrahydrofuran (THF), ethyl acetate, and the like can be used, and acetic acid can be preferably used.

The reaction is completed at a temperature between 0°C and boiling point of the solvent used, preferably 60°C for about 5 minutes to 48 hours.

Instead of bromine, for example, N-bromosuccinicimide, pyrrolidone tribromide, copper bromide (1), pyridinium tribromide and the like can be used. In this case, as the solvent, for example, acetonitrile, methanol, ethanol, dichloromethane, 1,2-dichloroethane, chloroform, dimethoxyethane, N,N-dimethylformamide (DMF), dioxane, THF, diethyl ether, diisopropyl ether, N,N-dimethylimidazolidinone, N-methylpyrrolidone (NMP), sulfolane, and the like can be used, and DMF can be preferably used.

Compound (a-1) can be obtained as a commercially available product, or can be obtained from a fluorobenzene derivative, by known methods {for example, fluorobenzene derivative is subjected to lithiation [for example, see Chemical Reviews, vol. 90, p. 879 (1990), and the like], then formylation [for example, see Jikken Kagaku Koza (Courses in Experimental Chemistry) vol. 21, p. 30 (1991), and the like] or the methods according to those.

Step 1-2

Compound (a-4) can be obtained by reacting Compound (a-2) with 1 to 20 equivalent of Compound (a-3) in a solvent in the presence of 1 to 20 equivalents of a base by known methods [for example, see Journal of Heterocyclic Chemistry, vol. 34, p. 385 (1997)] or the methods according to that.

As the solvent, for example, N,N-dimethylacetamide (DMA), DMF, NMP, dimethylsulfoxide (DMSO) and the like can be used, and DMA can be preferably used.
As the base, for example, potassium carbonate, cesium carbonate, sodium methoxide, potassium *tert*-butoxide and the like can be used, and potassium carbonate or cesium carbonate can be preferably used.

The reaction is completed at a temperature between room temperature and 180°C, preferably 160°C for about 5 minutes to 48 hours.
Compound (a-3) can be obtained as a commercially available product, or can be obtained by known methods [for example, see Journal of Organic Chemistry, vol. 57, p. 2497 (1992)] or the methods according to those.

Step 1-3

Compound (I) can be obtained by reacting Compound (a-4) with 1 to 20 equivalents of Compound (a-5) or (a-6) in a solvent in the presence of 0.1 to 10 equivalents of a base and 0.001 to 1 equivalents of a palladium catalyst.

As the solvent, for example, acetonitrile, methanol, ethanol, dichloromethane, 1,2-dichloroethane, chloroform, DMA, DMF, dioxane, THF, diethyl ether, diisopropyl ether, benzene, toluene, xylene, N,N-dimethylimidazolidinone, NMP, sulfolane and the like can be used, a mixed solvent of at least one solvent selected from these solvents and water at a suitable ratio between 100:1 and 1:100, or the like, can also be used, preferably a mixed solvent of water and dioxane at 1:2 can be used.

As the base, for example, pyridine, triethylamine, N-methylmorpholine, N-methylpiperidine, piperidine, piperazine, potassium acetate, potassium carbonate, cesium carbonate, sodium carbonate, sodium hydrogen carbonate, sodium hydroxide, lithium hydroxide, potassium hydroxide, potassium phosphate, sodium *tert*-butoxide, 1,8-diazabicyclo [5.4.0]-7-undecene (DBU), diisopropylethylamine and the like can be used, and sodium carbonate can be preferably used. In this step, when Compound (a-6) is used, the base may not be used.

Examples of the palladium catalyst include a catalyst consisting of palladium and a suitable ligand. As the palladium source of the palladium catalyst, for example, palladium acetate, palladium trifluoroacetate, tris(dibenzylideneacetone)dipalladium and a chloroform adduct thereof and the like can be used. As the ligand, for example, triphenylphosphine, 1,1'-bis(diphenylphosphino)ferrocene, o-tolylphosphine, 1,2-bis(diphenylphosphino)ethane, 1,3-(bisdiphenylphosphino)propane, 1,4-bis(diphenylphosphino)butane, di-*tert*-butyldiphenylphosphine, 2-(di-*tert*-butylphosphino)biphenyl, 2-(dicyclohexylphosphino)biphenyl and the like can be used. These ligands are preferably used at 1 to 10 equivalents to the palladium. Also, for example, a commercially available reagent in which a ligand (ligands) suitable to carry out the reaction is (are) coordinated to the palladium in advance, such as tetrakis(triphenylphosphine)palladium, 1,1-bis(diphenylphosphino)ferrocenedichloropalladium·dichloromethane 1:1 adduct, and the like, can be used.

The reaction is completed at the temperature between room temperature and the boiling point of the solvent used, preferably 100°C for about 5 minutes to 48 hours.

Compound (a-5) and Compound (a-6) can be obtained as a commercially available product, or can be obtained by known methods [for example, see Jikken Kagaku Koza (Courses in Experimental Chemistry), vol. 24, Japan Society for Analytical Chemistry (1992) and the like] or the methods according to those.

Production Method 2

Among Compound (I), Compound (I-1) where $R^4$ is hydroxy, can be obtained, according to, for example, the following step.

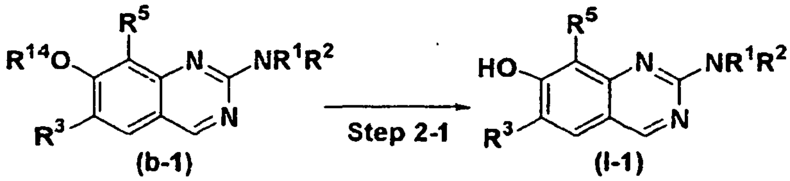


(b-1) (I-1)

(wherein $R^1$, $R^2$, $R^3$ and $R^5$ have the same meanings as defined above, respectively, and $R^{14}$ represents benzyl, methyl, or the like)

Step 2-1

Compound (I-1) can be obtained by treating Compound (b-1) obtained according to Production Methods 1, 3 or 4, or the like, with 1 to 100 equivalents of thiol compound, acid, trimethylsilyl iodide or sodium sulfide in a solvent at the temperature between -30°C and the boiling point of the solvent used for 5 minutes to 72 hours.

As the thiol compound, for example, thiophenol, methanethiol, ethanethiol and the like can be used. Alkaline metal salt thereof, for example, sodium thiophenoxide, sodium thiomethoxide, sodium thioethoxide and the like can also be used.

As the acid, for example, hydrogen bromide/acetic acid, pyridinium chloride, boron trifluoride, boron tribromide, boron trichloride, aluminum bromide, aluminum chloride and the like can be used.

As the solvent, for example, dichloromethane, chloroform, 1,2-dichloroethane, DMF, NMP, diethyl ether, THF and the like can be used, or a mixed solvent thereof can also be used.

When $R^{14}$ of Compound (b-1) is benzyl, Compound (I-1) can be obtained by treating Compound (b-1) in a solvent under a hydrogen atmosphere or in the presence of hydrogen source, in the presence of suitable catalysts at the temperature between -20°C and the boiling point of the solvent used under the normal pressure or the high pressure for 5 minutes to 72 hours.

As the catalyst, for example, palladium-carbon, palladium, palladium hydroxide, palladium acetate, palladium black and the like can be used, and these are preferably used at 0.01 to 50% by weight to Compound (b-1).

As the hydrogen source, for example, formic acid, ammonium formate, sodium formate, cyclohexadiene, hydrazine and the like can be used, and 2 equivalents to a large excess amount of them are preferably used.

As the solvent, for example, methanol, ethanol, toluene, ethyl acetate, acetonitrile, diethyl ether, THF, 1,2-dimethoxyethane (DME), dioxane, DMF, DMA, NMP, water and the like can be used, or a mixed solvent thereof or the like can also be used.

Production Method 3

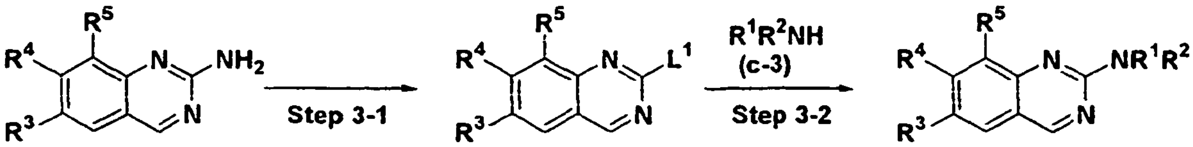


(c-1) (c-2) (I)

(wherein $R^1$, $R^2$, $R^3$, $R^4$ and $R^5$ have the same meanings as defined above, respectively, and $L^1$ represents a chlorine atom, a bromine atom or an iodine atom)

Step 3-1

Compound (c-2) can be synthesized by subjecting Compound (c-1) to Sandmeyer reaction.

In other words, Compound (c-2) can be obtained by reacting, for example, Compound (c-1) obtained according to Production Method 1 or the like, with 1 to 100 equivalents of nitrrite in the presence of, if necessary, 1 to 1000 equivalents of acid and 1 to 1000 equivalents of halogen sources, in a solvent or without solvent at the temperature between -30°C and boiling point of solvent used for 5 minutes to 100 hours.

As the nitrite, for example, nitrous acid, nitrite such as sodium nitrite, halogenated nitrosyl such as nitrosyl chloride, alkyl nitrite such as *tert*-butyl nitrite, isoamyl nitrite, and the like can be used.

As the acid, for example, hydroiodic acid, hydrobromic acid, hydrochloric acid and the like can be used.

As the halogen source, for example, copper (I) chloride, copper (I) bromide, copper (I) iodide, copper (II) chloride, copper (II) bromide, copper (II) iodide, potassium iodide, diiodomethane and the like can be used.

As the solvent, for example, alcohols such as methanol, and ethanol, ethers such as THF, and dioxane, acetone, DMSO, DMF, water and the like can be used, or a mixed solvent thereof or the like can be also used.

Step 3-2

Compound (I) can be obtained by reacting, Compound (c-2) and 1 to 1000 equivalents of amine (c-3) in a solvent or without a solvent in the presence of, if necessary, 1 to 100 equivalents of base, at the temperature between 0°C and the boiling point of the solvent used, preferably from 0 to 100°C for 5 minutes to 48 hours.

Furthermore, if necessary, reaction can also be carried out in the presence of 0.0001 to 2 equivalents, preferably 0.01 to 0.1 equivalents of palladium complex.

As the palladium complex, for example, tetrakis(triphenylphosphine)palladium, dichlorobis(triphenylphosphine)palladium, [bis(1,2-diphenylphosphino)ethane]dichloropalladium, [1,1'-bis(diphenylphosphino)ferrocene]dichloropalladium and the like can be used. Also the reaction of forming a palladium complex in a reaction mixture by using a combination of palladium precursor and phosphine compound can be carried out.

As the palladium precursor, for example, palladium acetate, palladium chloride, tris(dibenzylideneacetone)dipalladium, palladium-carbon and the like can be used.

As the phosphine compound, for example, triphenylphosphine, 1,1'-bis(diphenylphosphino)ferrocene, bis(1,2-diphenylphosphino)ethane, bis(1,3-diphenylphosphino)propane, bis(1,4-diphenylphosphino)butane, 2,2'-bis(diphenylphosphino)-1,1'-binaphthyl, bis(1,4-dicyclohexylphosphino)butane and the like can be used.

As the base, for example, inorganic bases such as potassium carbonate, sodium carbonate, cesium carbonate, potassium phosphate, potassium hydroxide, and potassium acetate, or organic bases such as pyridine, and triethylamine can be used.

As the solvent, for example, aliphatic hydrocarbons such as pentane, hexane, and cyclohexane, aromatic hydrocarbons such as benzene, toluene, and xylene, alcohols such as methanol, ethanol, propanol, and butanol, tetralin, diphenyl ether, ethyl acetate, dichloromethane, chloroform, dichloroethane, carbon tetrachloride, pyridine, acetonitrile, DMF, DMA, NMP, 1,3-dimethyl-2-imidazolidinone, DMSO, sulfolane, dimethylsulfone, THF, dioxane, dimethoxyethane and the like can be used, or a mixed solvent thereof can be also used.

Compound (c-3) can be obtained as a commercially available product, or can be obtained by known methods [for example, a process described in Jikken Kagaku Koza (Courses in Experimental Chemistry), vol. 20, p. 279, Maruzen (1992), and the like] or the methods described in the Reference Example.

Production Method 4

Compound (I) can be produced, for example, according to the following step.

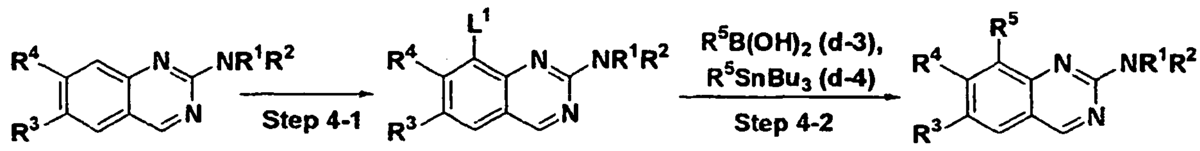


(d-1) (d-2) (I)

(wherein Bu, $R^1$, $R^2$, $R^3$, $R^4$, $R^5$ and $L^1$ have the same meanings as defined above, respectively)

Step 4-1

Compound (d-2) can be synthesized, for example, using Compound (d-1) obtained according to Production Method 1, when $L^1$ is a bromine atom, Compound (d-2) can be synthesized according to Step 1-1 of Production Method 1. When $L^1$ is an iodine atom, Compound (d-2) can be synthesized by reacting Compound (d-1) with a base such as sodium hydroxide and potassium hydroxide, and sodium iodide, in a solvent such as methanol and ethanol, in the presence of sodium chlorite. When $L^1$ is a chlorine atom, Compound (d-2) can be obtained by reacting Compound (d-1) with chlorine or N-chlorosuccinimide in a solvent such as chloroform and carbon tetrachloride.

Step 4-2

Compound (I) can be synthesized according to Step 1-3 of Production Methodd 1.
Compounds (d-3) and (d-4) can be obtained as a commercially available product, or Compounds (d-3) and (d-4) can be obtained by known methods [for example, see Jikken Kagaku Koza (Courses in Experimental Chemistry), vol. 24, Japan Society for Analytical Chemistry (1992), and the like] or the methods according to those.

Production Method 5

Compound (I-2) in which $R^1$ is optionally substituted lower alkanoyl, optionally substituted cycloalkylcarbonyl, optionally substituted lower alkoxycarbonyl, or $-C(=O)NR^6R^7$ (wherein $R^6$ and $R^7$ have the same meanings as defined above, respectively), can be produced according to, for example, the following step.
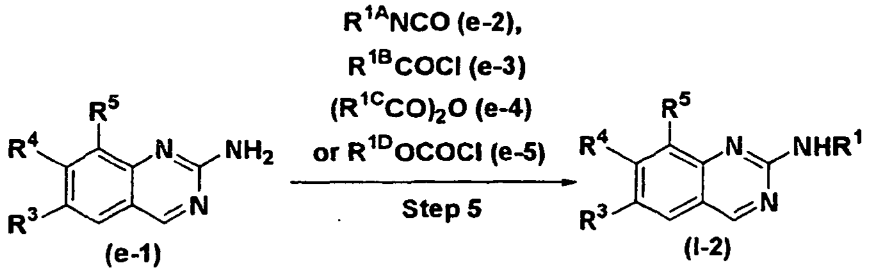


(e-1) (I-2)

(wherein $R^3$, $R^4$ and $R^5$ have the same meanings as defined above, respectively; $R^{1A}$ represents optionally substituted lower alkyl in the definition of $R^6$; $R^{1B}$ represents lower alkyl moiety, cycloalkyl moiety or $-NR^6R^7$ (wherein $R^6$ and $R^7$ have the same meanings as defined above, respectively) moiety of optionally substituted lower alkanoyl, optionally substituted cycloalkylcarbonyl, and $-C(=O)NR^6R^7$ (wherein $R^6$ and $R^7$ have the same meanings as defined above, respectively) in the definition of $R^1$; $R^{1C}$ represents lower alkyl moiety or cycloalkyl moiety of optionally substituted lower alkanoyl, optionally substituted lower alkoxycarbonyl and optionally substituted cycloalkylcarbonyl in the definition $R^1$; and $R^{1D}$ represents lower alkyl moiety of optionally substituted lower alkoxycarbonyl in the definition of $R^1$).

Step 5

Compound (1-2) can be obtained by reacting, for example, Compound (e-1) obtained according to Production Method 1 and 1 to 1000 equivalents of isocyanate (e-2), acid chloride (e-3), acid anhydride (e-4) or alkoxycarbonyl chloride (e-5) in a solvent or without a solvent and in the presence of, if necessary 1 to 100 equivalents of a base, at a temperature between 0°C and the boiling point of the solvent used, preferably from 0 to 100°C for 5 minutes to 48 hours.
If necessary, reaction can be also carried out in the presence of 0.0001 to 2 equivalents, preferably 0.01 to 0.1 equivalents of a base.
As the base, for example, inorganic bases such as potassium carbonate, sodium carbonate, cesium carbonate, potassium phosphate, potassium hydroxide, and potassium acetate, organic bases such as pyridine, triethylamine, and DBU and the like can be used.
As the solvents, for example, aliphatic hydrocarbons such as pentane, hexane, and cyclohexane, aromatic hydrocarbons such as benzene, toluene, and xylene, tetralin, diphenyl ether, ethyl acetate, dichloromethane, chloroform, dichloroethane, carbon tetrachloride, pyridine, acetonitrile, DMF, DMA, NMP, 1,3-dimethyl-2-imidazolidinone, DMSO, sulfolane, dimethylsulfone, THF, dioxane, dimethoxyethane, water and the like can be used, or a mixed solvent thereof or the like can be used.
By appropriately combining the methods described above, Compound (I) with a desirable functional group at a desirable position can be obtained.
The intermediates and the desired compounds in the above-mentioned respective Production Methods can be isolated and purified by performing methods of a separation and purification that are usually used in organic synthetic chemistry, for example, filtration, extraction, washing, drying, concentration, recrystallization, various types of chromatography, and the like. Further, the intermediate can be subjected to the subsequent reaction without being purified.
In Compounds (I), stereoisomers such as geometric isomers, and optical isomers, and isomers such as tautomers may be present. The present invention includes all possible isomers including these isomers and mixtures thereof. To obtain a salt of Compound (I), when Compound (I) is obtained in a salt form directly, the salt may be purified as it is. When Compound (I) is obtained in a free form, Compound (I) is dissolved or suspended in a suitable solvent, and the

acid or the base is added to the mixture, then the resulting salt can be isolated and purified.

Furthermore, Compound (I) and pharmaceutically acceptable salts thereof may exist in the form of adducts with water or various solvents, and these adducts are also included in the present invention.

Specific examples of Compound (I) obtained by the present invention are shown in Tables 1 to 24. However, the compound of the present invention is not limited thereto.

In the Tables, Me represents methyl, Et represents ethyl, i-Pr represents isopropyl, t-Bu represents *tert*-butyl, and Ac represents acetyl.

[Table 1]

Table 1

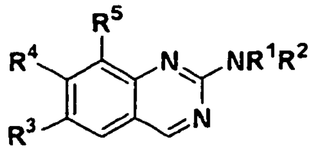

| Example No. | Compound No. | $NR^1R^2$ | $R^3$ | $R^4$ | $R^5$ |
|---|---|---|---|---|---|
| 1 | 1 | $NH_2$ | 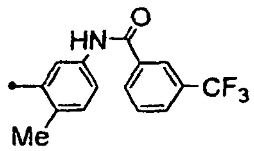 | OMe | 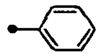 |
| 2 | 2 | $NH_2$ | 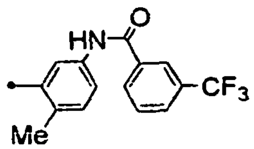 | OMe | 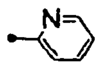 |
| 3 | 3 | $NH_2$ | 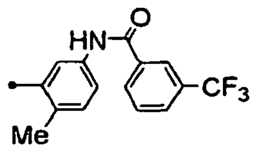 | OMe | 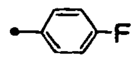 |
| 4 | 4 | $NH_2$ | 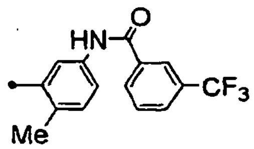 | OH | 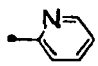 |
| 5 | 5 | $NH_2$ | 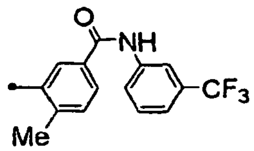 | OMe | 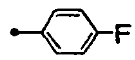 |
| 6 | 6 | $NH_2$ | 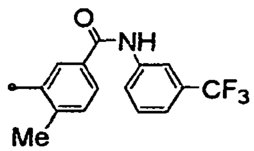 | OMe | 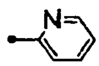 |
| 7 | 7 | $NH_2$ | 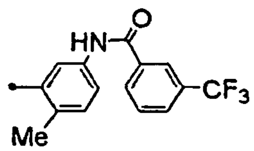 | OMe | 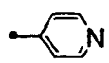 |
| 8 | 8 | $NH_2$ | 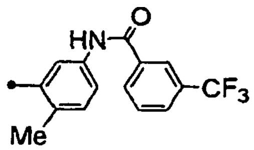 | OMe | 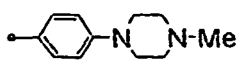 |

(continued)

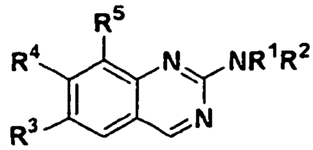

| Example No. | Compound No. | $NR^1R^2$ | $R^3$ | $R^4$ | $R^5$ |
|---|---|---|---|---|---|
| 9 | 9 | $NH_2$ | 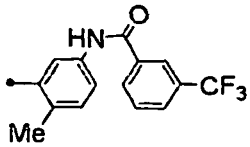 | OMe | 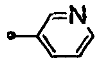 |
| 10 | 10 | $NH_2$ | 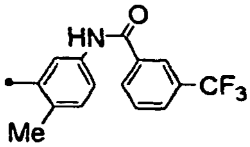 | OH | 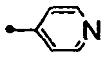 |

[Table 2]

Table 2

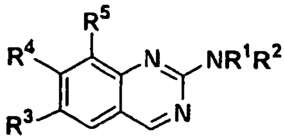

| Example No. | Compound No. | $NR^1R^2$ | $R^3$ | $R^4$ | $R^5$ |
|---|---|---|---|---|---|
| 11 | 11 | $NH_2$ | 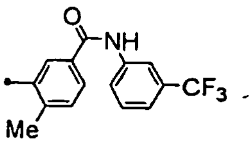 | OMe | 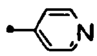 |
| 12 | 12 | $NH_2$ | 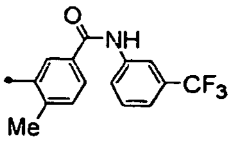 | OH | 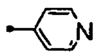 |
| 13 | 13 | $NH_2$ | 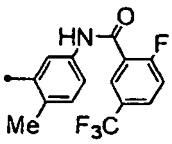 | OMe | 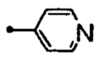 |
| 14 | 14 | $NH_2$ | 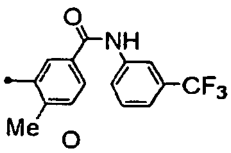 | OMe | 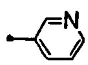 |
| 15 | 15 | $NH_2$ | 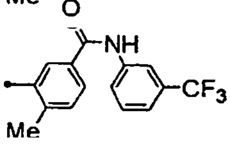 | OH | 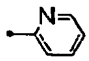 |
| 16 | 16 | $NH_2$ | 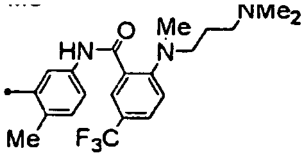 | OMe | 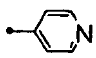 |

(continued)

Table 2

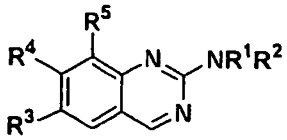

| Example No. | Compound No. | $NR^1R^2$ | $R^3$ | $R^4$ | $R^5$ |
|---|---|---|---|---|---|
| 17 | 17 | $NH_2$ | 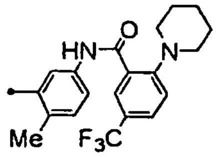 | OMe | 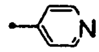 |
| 18 | 18 | $NH_2$ | 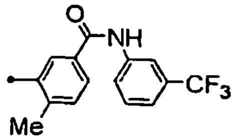 | OMe | 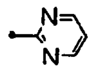 |
| 19 | 19 | $NH_2$ | 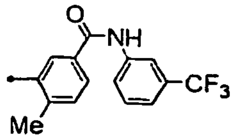 | OMe | 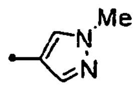 |
| 20 | 20 | $NH_2$ | 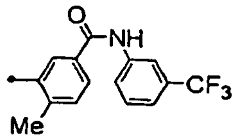 | OMe | 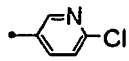 |

[Table 3]

Table 3

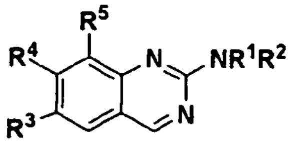

| Example No. | Compound No. | $NR^1R^2$ | $R^3$ | $R^4$ | $R^5$ |
|---|---|---|---|---|---|
| 21 | 21 | $NH_2$ | 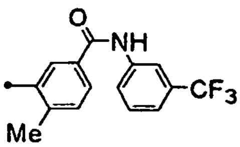 | OMe | 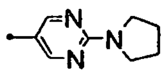 |
| 22 | 22 | $NH_2$ | 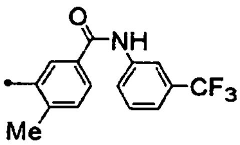 | OMe | 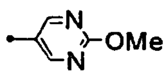 |
| 23 | 23 | $NH_2$ | 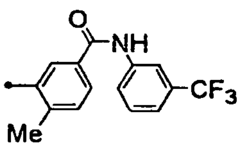 | OMe | 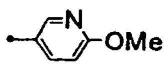 |
| 24 | 24 | $NH_2$ | 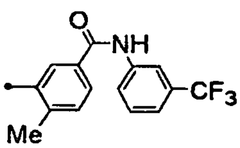 | OMe | 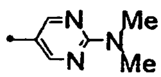 |

(continued)

Table 3

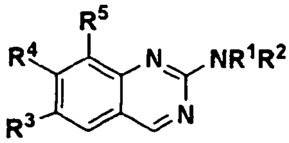

| Example No. | Compound No. | $NR^1R^2$ | $R^3$ | $R^4$ | $R^5$ |
|---|---|---|---|---|---|
| 25 | 25 | NHMe | 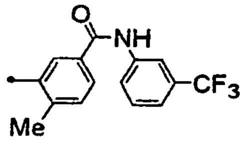 | OMe | 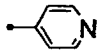 |
| 26 | 26 | NHMe | 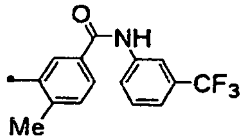 | OMe | 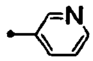 |
| 27 | 27 | NHMe | 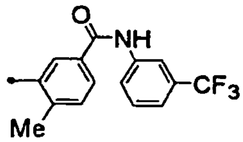 | OMe | 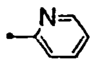 |
| 28 | 28 | $NH_2$ | 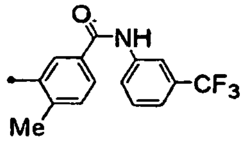 | OMe | 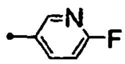 |
| 29 | 29 | $NH_2$ | 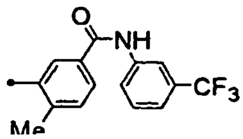 | OMe | 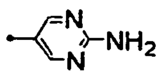 |
| 30 | 30 | $NH_2$ | 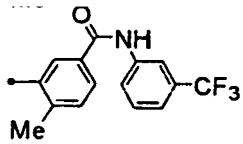 | OMe | 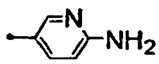 |

[Table 4]

Table 4

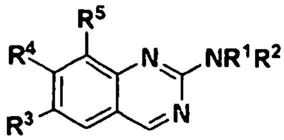

| Example No. | Compound No. | $NR^1R^2$ | $R^3$ | $R^4$ | $R^5$ |
|---|---|---|---|---|---|
| 31 | 31 | $NH_2$ | 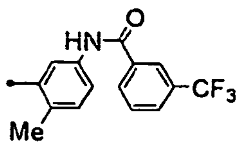 | OMe | 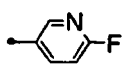 |
| 32 | 32 | $NH_2$ | 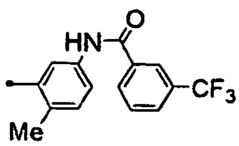 | OMe | 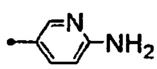 |

(continued)

Table 4

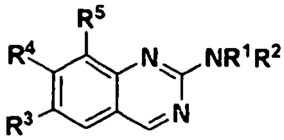

| Example No. | Compound No. | $NR^1R^2$ | $R^3$ | $R^4$ | $R^5$ |
|---|---|---|---|---|---|
| 33 | 33 | $NH_2$ | 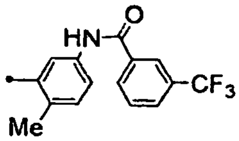 | OMe | 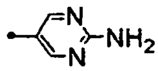 |
| 34 | 34 | $NH_2$ | 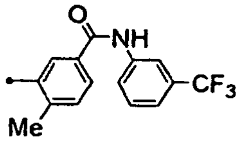 | OMe | 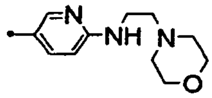 |
| 35 | 35 | $NH_2$ | 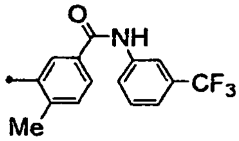 | OMe | 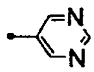 |
| 36 | 36 | $NH_2$ | 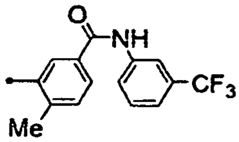 | OMe | 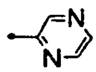 |
| 37 | 37 | $NH_2$ | 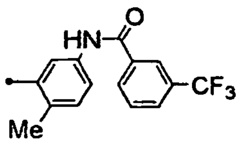 | OMe | 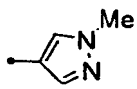 |
| 38 | 38 | $NH_2$ | 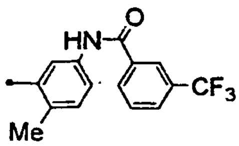 | OMe | 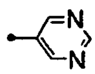 |
| 39 | 39 | $NH_2$ | 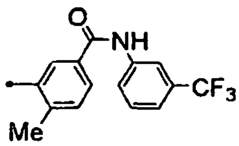 | OMe | 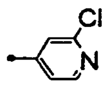 |
| 40 | 40 | NHi-Pr | 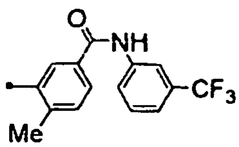 | OMe | 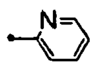 |

[Table 5]

Table 5

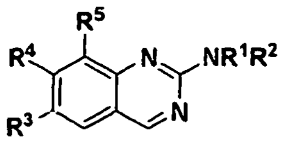

| Example No. | Compound No. | $NR^1R^2$ | $R^3$ | $R^4$ | $R^5$ |
|---|---|---|---|---|---|
| 41 | 41 | NHMe | 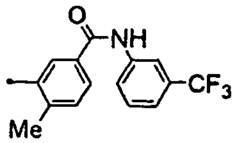 | OMe | 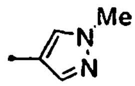 |
| 42 | 42 | NHMe | 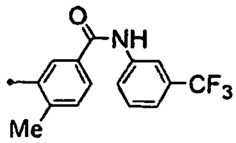 | OMe | 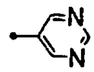 |
| 43 | 43 | $NH_2$ | 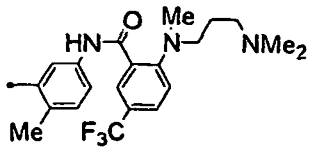 | OMe | 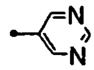 |
| 44 | 44 | $NH_2$ | 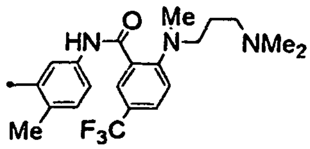 | OMe | 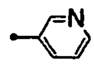 |
| 45 | 45 | $NH_2$ | 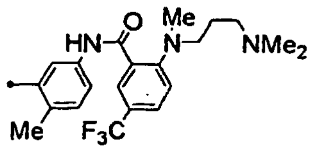 | OMe | 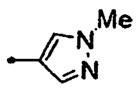 |
| 46 | 46 | $NH_2$ | 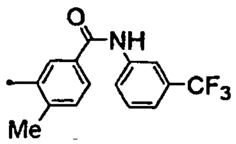 | OMe | 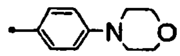 |
| 47 | 47 | $NH_2$ | 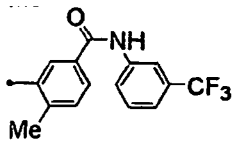 | OMe | 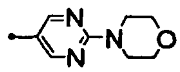 |
| 48 | 48 | $NH_2$ | 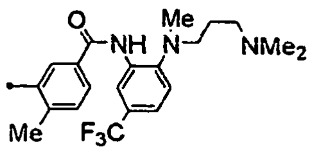 | OMe | 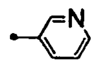 |
| 49 | 49 | $NH_2$ | 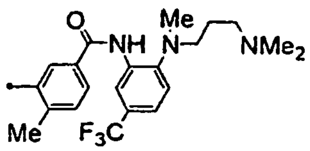 | OMe | 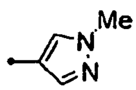 |
| 50 | 50 | NHMe | 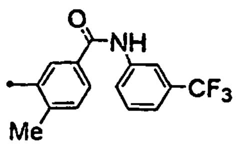 | OMe | 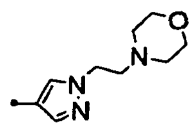 |

[Table 6]

Table 6

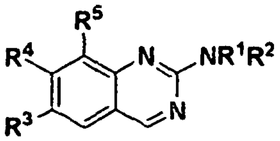

| Example No. | Compound No. | $NR^1R^2$ | $R^3$ | $R^4$ | $R^5$ |
|---|---|---|---|---|---|
| 51 | 51 | $NH_2$ | 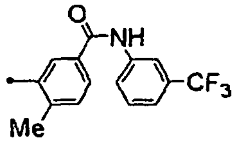 | OMe | 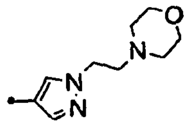 |
| 52 | 52 | $NH_2$ | 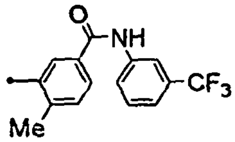 | OMe | 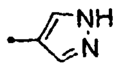 |
| 53 | 53 | $NH_2$ | 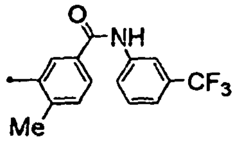 | OMe | 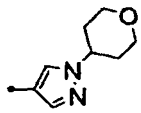 |
| 54 | 54 | $NH_2$ | 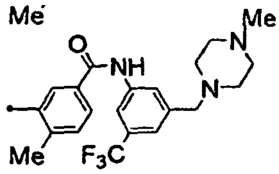 | OH | 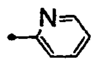 |
| 55 | 55 | $NH_2$ | 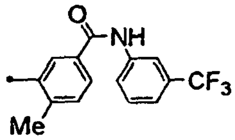 | OMe | 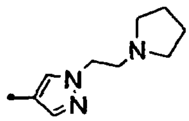 |
| 56 | 56 | $NH_2$ | 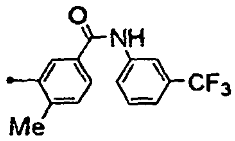 | OMe | 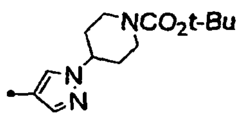 |
| 57 | 57 | $NH_2$ | 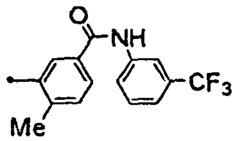 | OMe | 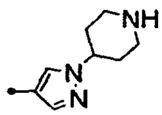 |
| 58 | 58 | $NH_2$ | 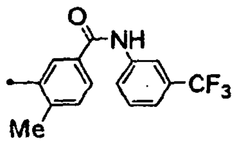 | OMe | 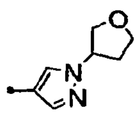 |
| 59 | 59 | $NH_2$ | 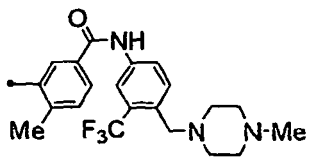 | OH | 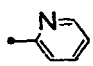 |
| 60 | 60 | $NH_2$ | 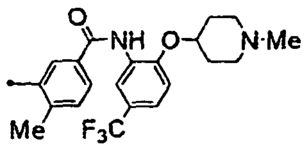 | OH | 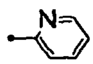 |

[Table 7]

Table 7

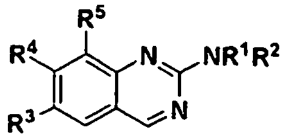

| Example No. | Compound No. | $NR^1R^2$ | $R^3$ | $R^4$ | $R^5$ |
|---|---|---|---|---|---|
| 61 | 61 | NHi-Pr | 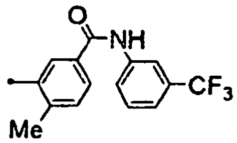 | OMe | 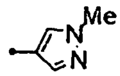 |
| 62 | 62 | NHi-Pr | 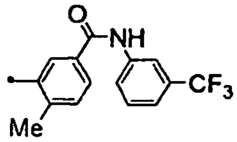 | OH | 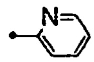 |
| 63 | 63 | $NH_2$ | 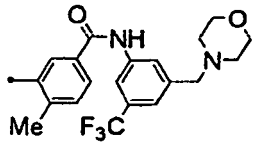 | OH | 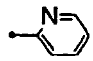 |
| 64 | 64 | $NH_2$ | 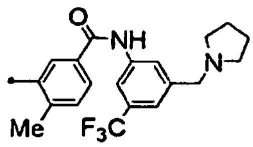 | OH | 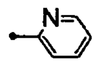 |
| 65 | 65 | $NH_2$ | 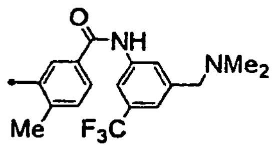 | OH | 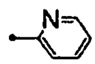 |
| 66 | 66 | $NH_2$ | 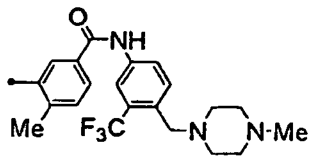 | OMe | 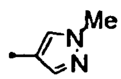 |
| 67 | 67 | 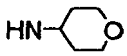 | 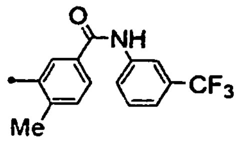 | OMe | 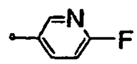 |
| 68 | 68 | 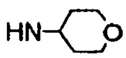 | 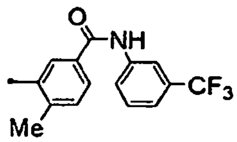 | OMe | 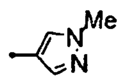 |
| 69 | 69 | 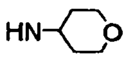 | 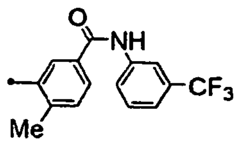 | OMe | 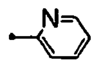 |
| 70 | 70 | 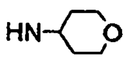 | 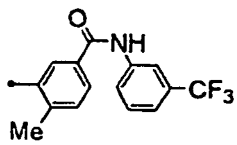 | OH | 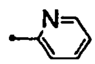 |

[Table 8]

Table 8

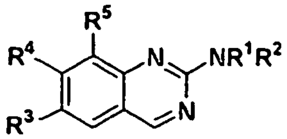

| Example No. | Compound No. | $NR^1R^2$ | $R^3$ | $R^4$ | $R^5$ |
|---|---|---|---|---|---|
| 71 | 71 | 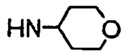 | 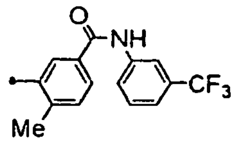 | OH | 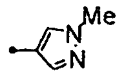 |
| 72 | 72 | $NH_2$ | 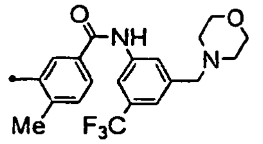 | OMe | 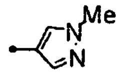 |
| 73 | 73 | $NH_2$ | 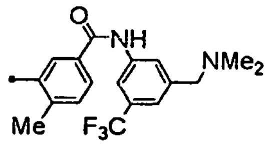 | OMe | 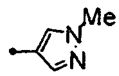 |
| 74 | 74 | $NH_2$ | 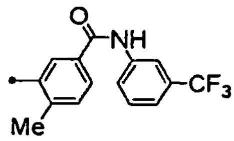 | OH | 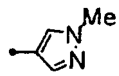 |
| 75 | 75 | 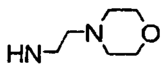 | 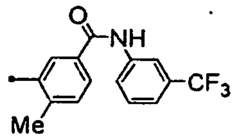 | OMe | 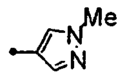 |
| 76 | 76 | $NH_2$ | 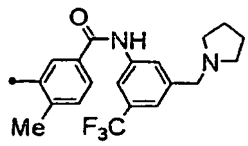 | OMe | 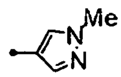 |
| 77 | 77 | $NH_2$ | 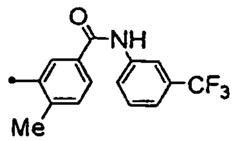 | OH | 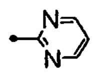 |
| 78 | 78 | $NH_2$ | 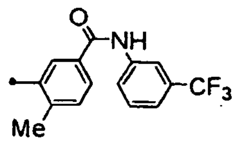 | OH | 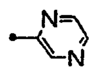 |
| 79 | 79 | $NH_2$ | 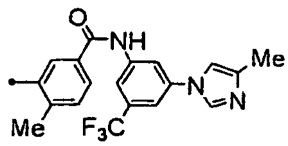 | OMe | 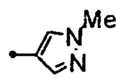 |
| 80 | 80 | $NH_2$ | 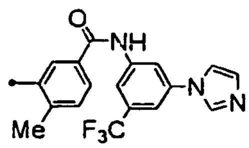 | OH | 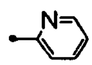 |

[Table 9]

Table 9

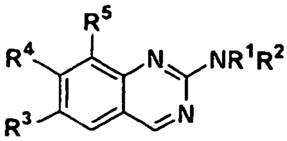

| Example No. | Compound No. | $NR^1R^2$ | $R^3$ | $R^4$ | $R^5$ |
|---|---|---|---|---|---|
| 81 | 81 | $NH_2$ | 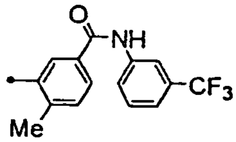 | OMe | 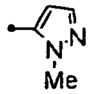 |
| 82 | 82 | $NH_2$ | 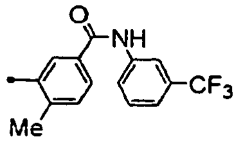 | OMe | 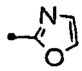 |
| 83 | 83 | $NH_2$ | 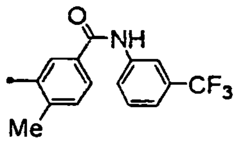 | OH | 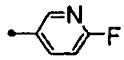 |
| 84 | 84 | $NH_2$ | 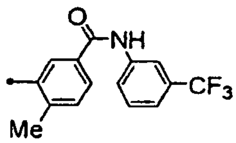 | OH | 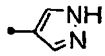 |
| 85 | 85 | $NH_2$ | 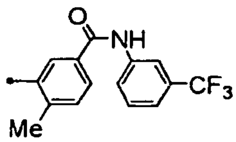 | OMe | 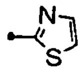 |
| 86 | 86 | $NH_2$ | 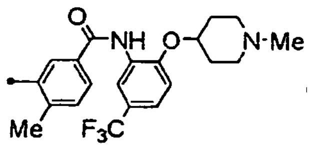 | Me | 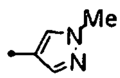 |
| 87 | 87 | $NH_2$ | 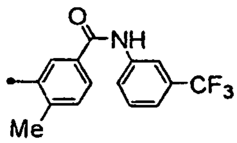 | OH | 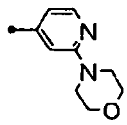 |
| 88 | 88 | $NH_2$ | 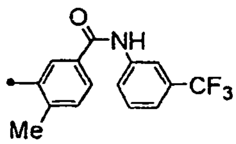 | OH | 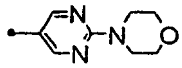 |
| 89 | 89 | $NH_2$ | 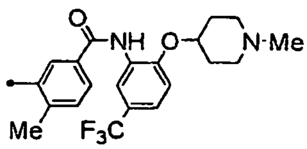 | OH | 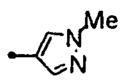 |
| 90 | 90 | $NH_2$ | 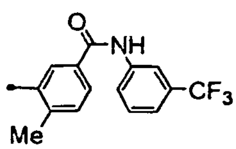 | OH | 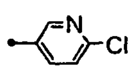 |

[Table 10]

Table 10

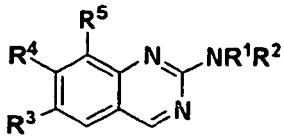

| Example No. | Compound No. | $NR^1R^2$ | $R^3$ | $R^4$ | $R^5$ |
|---|---|---|---|---|---|
| 91 | 91 | $NH_2$ | 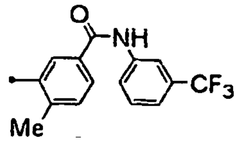 | OH | 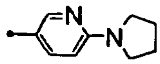 |
| 92 | 92 | $NH_2$ | 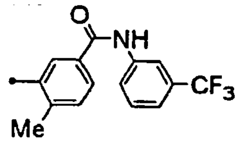 | OMe | 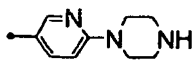 |
| 93 | 93 | $NH_2$ | 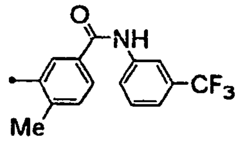 | OH | 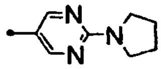 |
| 94 | 94 | $NH_2$ | 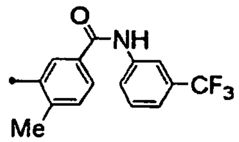 | OMe | 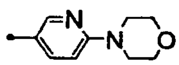 |
| 95 | 95 | $NH_2$ | 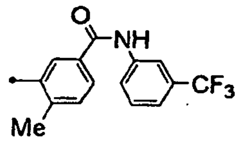 | OH | 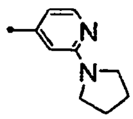 |
| 96 | 96 | $NH_2$ | 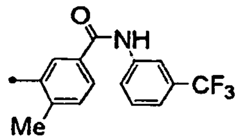 | OH | 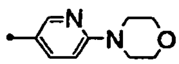 |
| 97 | 97 | NHMe | 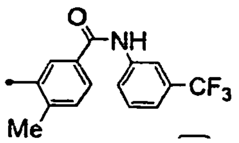 | OH | 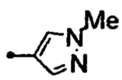 |
| 98 | 98 | $NH_2$ | 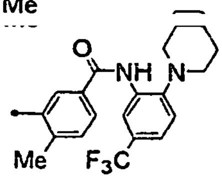 | OMe | 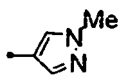 |
| 99 | 99 | $NH_2$ | 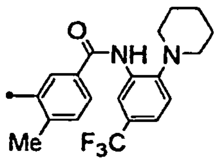 | OH | 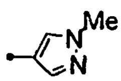 |
| 100 | 100 | $NH_2$ | 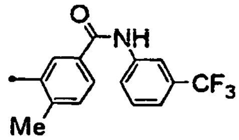 | OH | 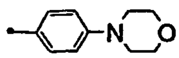 |

[Table 11]

Table 11

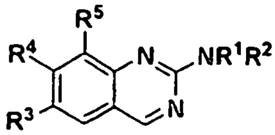

| Example No. | Compound No. | NR$^1$R$^2$ | R$^3$ | R$^4$ | R$^5$ |
|---|---|---|---|---|---|
| 101 | 101 | $NH_2$ | 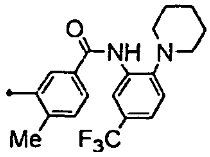 | OMe | 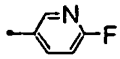 |
| 102 | 102 | $NH_2$ | 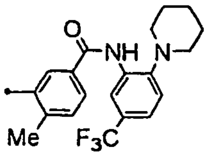 | OH | 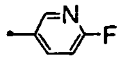 |
| 103 | 103 | $NH_2$ | 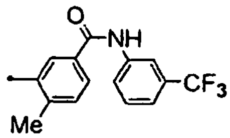 | OMe | 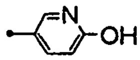 |
| 104 | 104 | $NH_2$ | 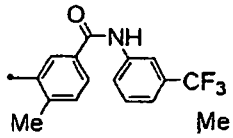 | OMe | 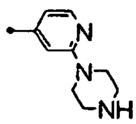 |
| 105 | 105 | $NH_2$ | 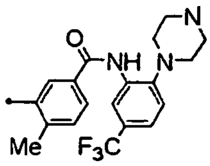 | OH | 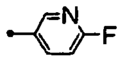 |
| 106 | 106 | $NH_2$ | 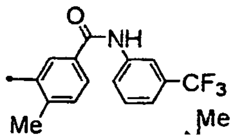 | OMe | 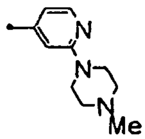 |
| 107 | 107 | $NH_2$ | 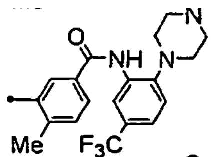 | OMe | 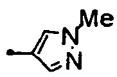 |
| 108 | 108 | $NH_2$ | 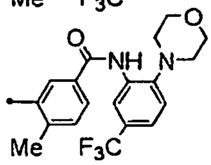 | OMe | 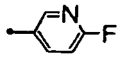 |
| 109 | 109 | $NH_2$ | 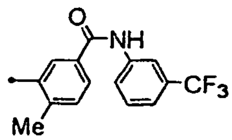 | OMe | 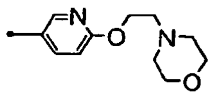 |
| 110 | 110 | $NH_2$ | 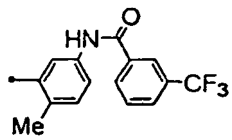 | OH | 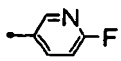 |

[Table 12]

Table 12

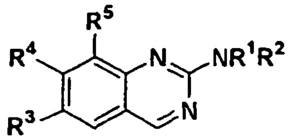

| Example No. | Compound No. | $NR^1R^2$ | $R^3$ | $R^4$ | $R^5$ |
|---|---|---|---|---|---|
| 111 | 111 | $NH_2$ | 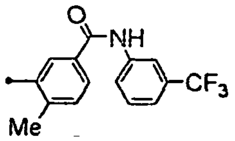 | OH | 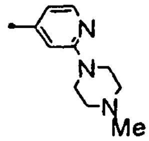 |
| 112 | 112 | $NH_2$ | 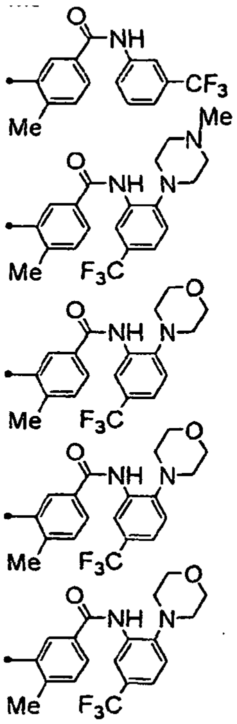 | OH | 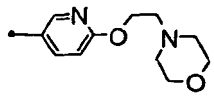 |
| 113 | 113 | $NH_2$ | | OH | 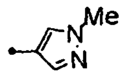 |
| 114 | 114 | $NH_2$ | | OH | 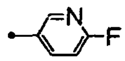 |
| 115 | 115 | $NH_2$ | | OMe | 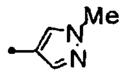 |
| 116 | 116 | $NH_2$ | | OH | 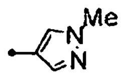 |
| 117 | 117 | $NH_2$ | 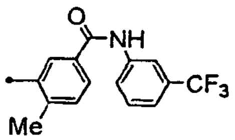 | OMe | 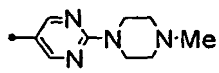 |
| 118 | 118 | $NH_2$ | 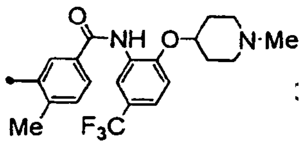 | OH | 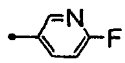 |
| 119 | 119 | $NH_2$ | 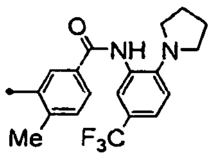 | OH | 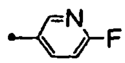 |
| 120 | 120 | $NH_2$ | 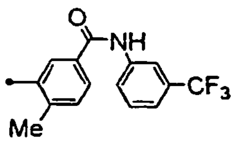 | OH | 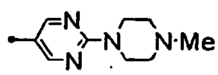 |

[Table 13]

Table 13

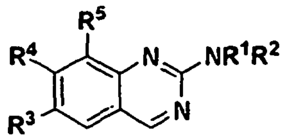

| Example No. | Compound No. | $NR^1R^2$ | $R^3$ | $R^4$ | $R^5$ |
|---|---|---|---|---|---|
| 121 | 121 | $NH_2$ | 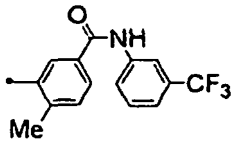 | OH | 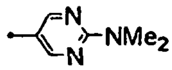 |
| 122 | 122 | $NH_2$ | 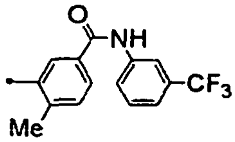 | OH | 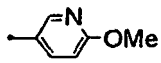 |
| 123 | 123 | $NH_2$ | 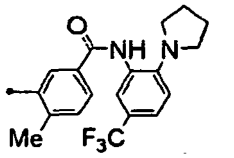 | OMe | 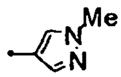 |
| 124 | 124 | $NH_2$ | 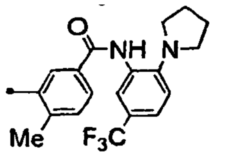 | OH | 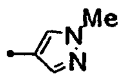 |
| 125 | 125 | $NH_2$ | 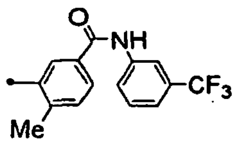 | OH | 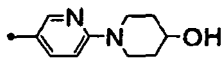 |
| 126 | 126 | $NH_2$ | 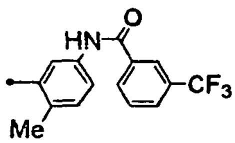 | OH | 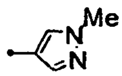 |
| 127 | 127 | $NH_2$ | 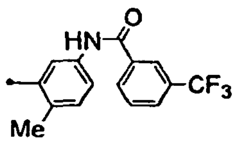 | OH | 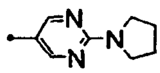 |
| 128 | 128 | $NH_2$ | 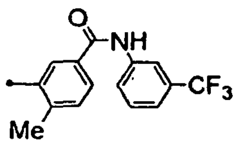 | OMe | 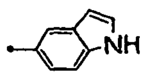 |
| 129 | 129 | $NH_2$ | 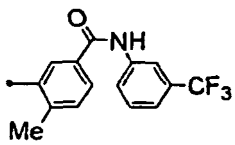 | OMe | 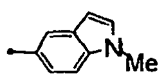 |
| 130 | 130 | $NH_2$ | 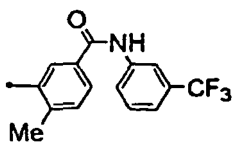 | OMe | 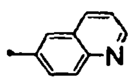 |

[Table 14]

Table 14

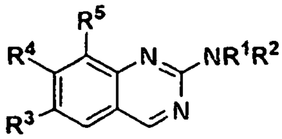

| Example No. | Compound No. | $NR^1R^2$ | $R^3$ | $R^4$ | $R^5$ |
|---|---|---|---|---|---|
| 131 | 131 | $NH_2$ | 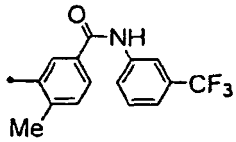 | OH | 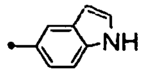 |
| 132 | 132 | $NH_2$ | 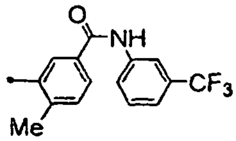 | OH | 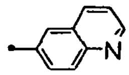 |
| 133 | 133 | $NH_2$ | 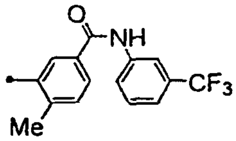 | OH | 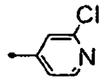 |
| 134 | 134 | $NH_2$ | 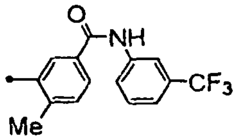 | OH | 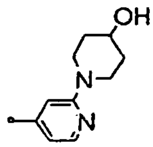 |
| 135 | 135 | NHMe | 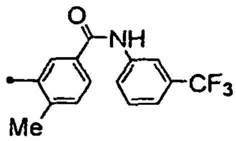 | OH | 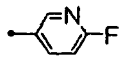 |
| 136 | 136 | $NH_2$ | 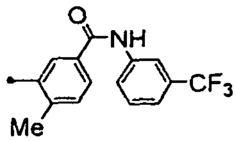 | OH | 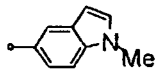 |
| 137 | 137 | $NH_2$ | 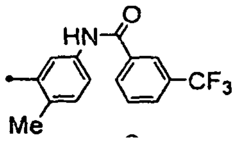 | OH | 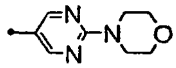 |
| 138 | 138 | $NH_2$ | 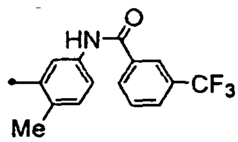 | OH | 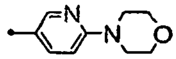 |
| 139 | 139 | $NH_2$ | 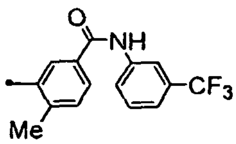 | OMe | 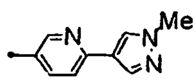 |
| 140 | 140 | $NH_2$ | 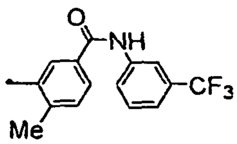 | OMe | 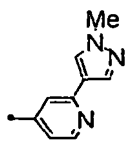 |

[Table 15]

Table 15

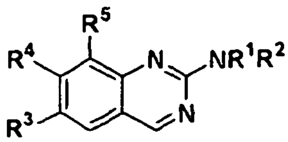

| Example No. | Compound No. | $NR^1R^2$ | $R^3$ | $R^4$ | $R^5$ |
|---|---|---|---|---|---|
| 141 | 141 | $NH_2$ | 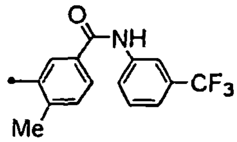 | OH | 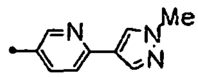 |
| 142 | 142 | $NH_2$ | 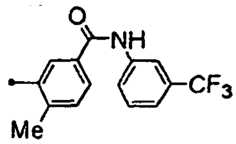 | OH | 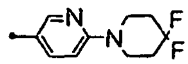 |
| 143 | 143 | NHAc | 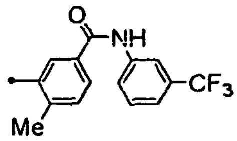 | OMe | 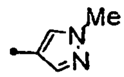 |
| 144 | 144 | 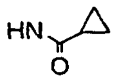 | 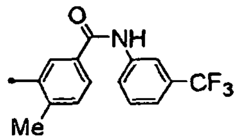 | OMe | 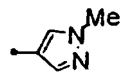 |
| 145 | 145 | $NH_2$ | 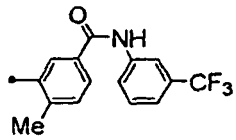 | OMe | 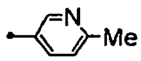 |
| 146 | 146 | $NH_2$ | 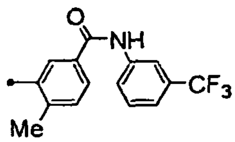 | OMe | 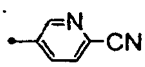 |
| 147 | 147 | $NH_2$ | 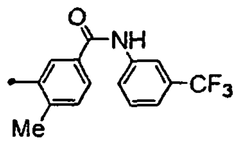 | OH | 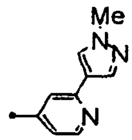 |
| 148 | 148 | $NH_2$ | 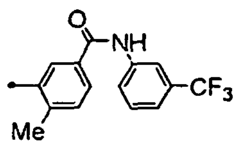 | OH | 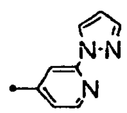 |
| 149 | 149 | $NH_2$ | 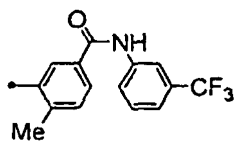 | OH | 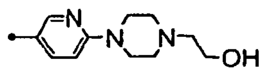 |
| 150 | 150 | NHAc | 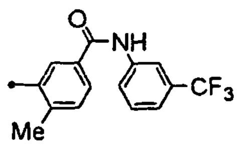 | OH | 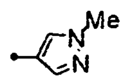 |

[Table 16]

Table 16

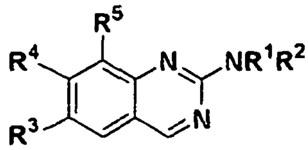

| Example No. | Compound No. | $NR^1R^2$ | $R^3$ | $R^4$ | $R^5$ |
|---|---|---|---|---|---|
| 151 | 151 | 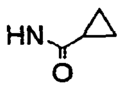 | 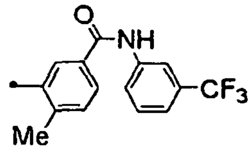 | OH | 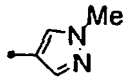 |
| 152 | 152 | $NH_2$ | 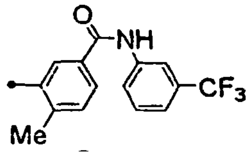 | OH | 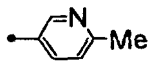 |
| 153 | 153 | $NH_2$ | 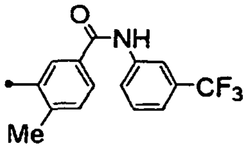 | OH | 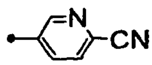 |
| 154 | 154 | $NH_2$ | 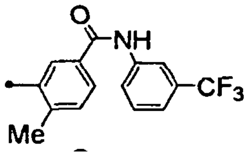 | OMe | 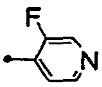 |
| 155 | 155 | $NH_2$ | 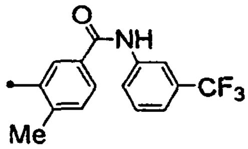 | OMe | 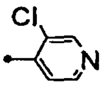 |
| 156 | 156 | $NH_2$ | 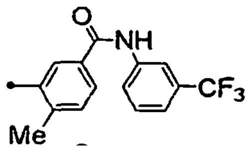 | OH | 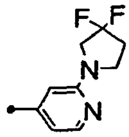 |
| 157 | 157 | $NH_2$ | 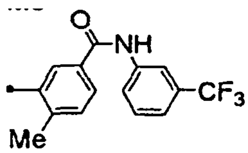 | OMe | 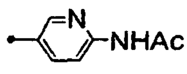 |
| 158 | 158 | $NH_2$ | 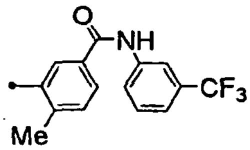 | OH | 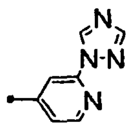 |

[Table 17]

Table 17

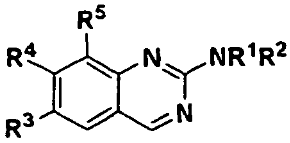

| Example No. | Compound No. | $NR^1R^2$ | $R^3$ | $R^4$ | $R^5$ |
|---|---|---|---|---|---|
| 159 | 159 | NHAc | 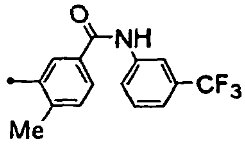 | OH | 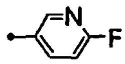 |
| 160 | 160 | 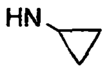 | 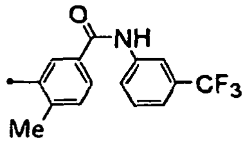 | OH | 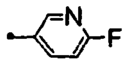 |
| 161 | 161 | $NH_2$ | 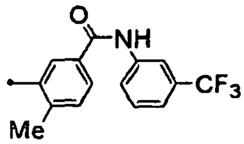 | OH | 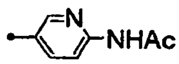 |
| 162 | 162 | $NH_2$ | 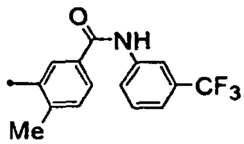 | OH | 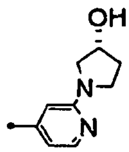 |
| 163 | 163 | $NH_2$ | 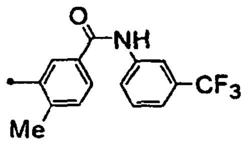 | OH | 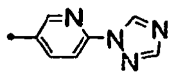 |
| 164 | 164 | $NH_2$ | 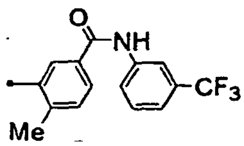 | OH | 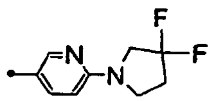 |
| 165 | 165 | $NH_2$ | 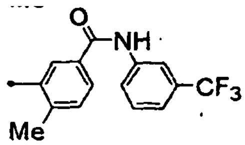 | OMe | 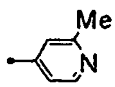 |
| 166 | 166 | $NH_2$ | 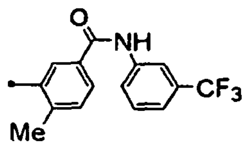 | OH | 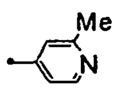 |
| 167 | 167 | $NH_2$ | 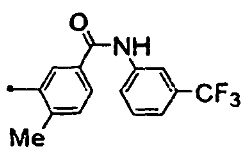 | OH | 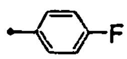 |
| 168 | 168 | $NH_2$ | 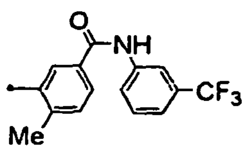 | OH | 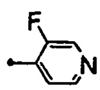 |

[Table 18]

Table 18

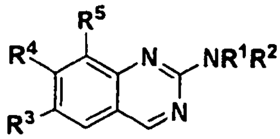

| Example No. | Compound No. | $NR^1R^2$ | $R^3$ | $R^4$ | $R^5$ |
|---|---|---|---|---|---|
| 169 | 169 | $NH_2$ | 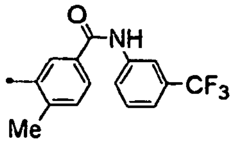 | OH | 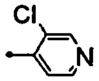 |
| 170 | 170 | $NH_2$ | 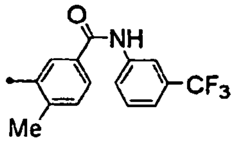 | OH | 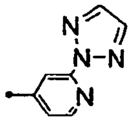 |
| 171 | 171 | $NH_2$ | 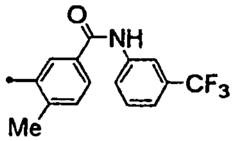 | OMe | 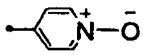 |
| 172 | 172 | $NH_2$ | 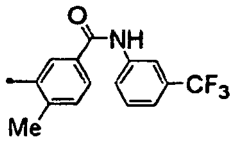 | OH | 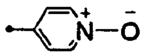 |
| 173 | 173 | $NH_2$ | 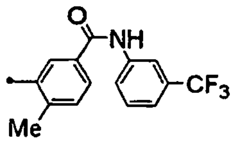 | OH | 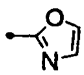 |
| 174 | 174 | $NH_2$ | 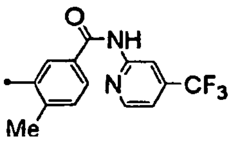 | OMe | 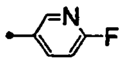 |
| 175 | 175 | 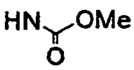 | 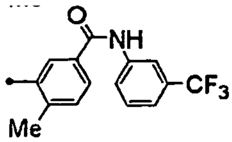 | OMe | 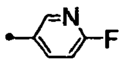 |
| 176 | 176 | NHAc | 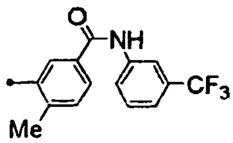 | OH | 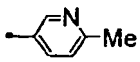 |
| 177 | 177 | NHAc | 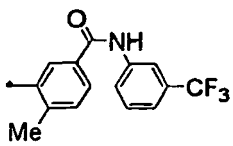 | OH | 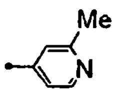 |
| 178 | 178 | 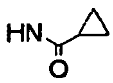 | 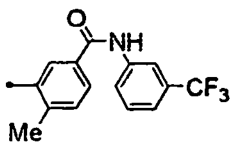 | OH | 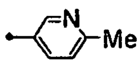 |

[Table 19]

Table 19

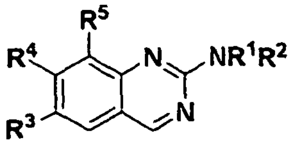

| Example No. | Compound No. | $NR^1R^2$ | $R^3$ | $R^4$ | $R^5$ |
|---|---|---|---|---|---|
| 179 | 179 | $NH_2$ | 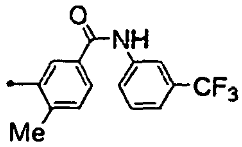 | OH | 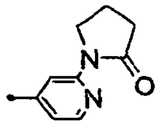 |
| 180 | 180 | $NH_2$ | 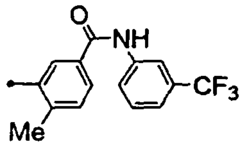 | OMe | 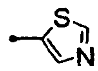 |
| 181 | 181 | $NH_2$ | 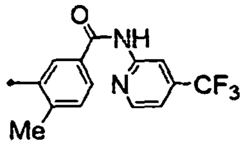 | OH | 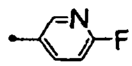 |
| 182 | 182 | $NH_2$ | 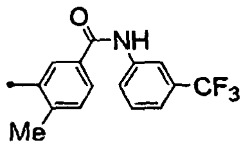 | OMe | 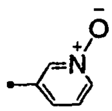 |
| 183 | 183 | $NH_2$ | 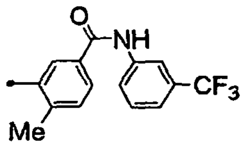 | OMe | 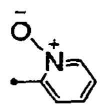 |
| 184 | 184 | $NH_2$ | 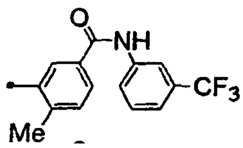 | OH | 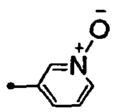 |
| 185 | 185 | $NH_2$ | 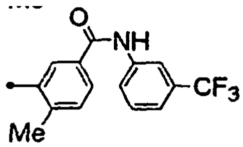 | OH | 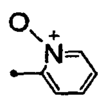 |
| 186 | 186 | 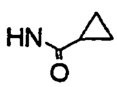 | 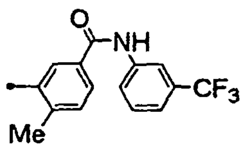 | OH | 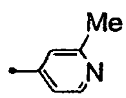 |
| 187 | 187 | $NH_2$ | 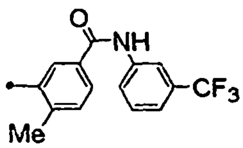 | OH | 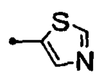 |
| 188 | 188 | $NH_2$ | 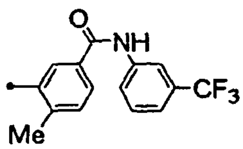 | OH | 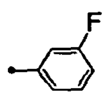 |

[Table 20]

Table 20

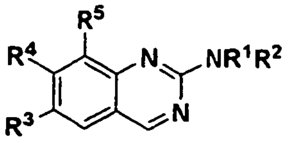

| Example No. | Compound No. | $NR^1R^2$ | $R^3$ | $R^4$ | $R^5$ |
|---|---|---|---|---|---|
| 189 | 189 | $NH_2$ | 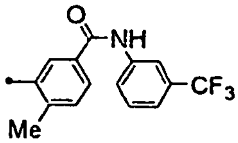 | OH | 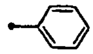 |
| 190 | 190 | $NH_2$ | 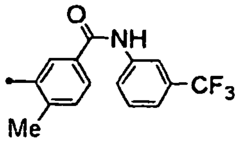 | OMe | 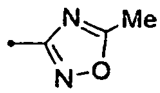 |
| 191 | 191 | $NH_2$ | 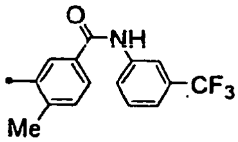 | OH | 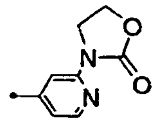 |
| 192 | 192 | $NH_2$ | 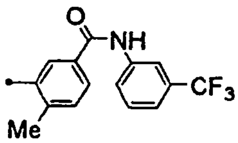 | OH | 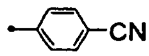 |
| 193 | 193 | NHMe | 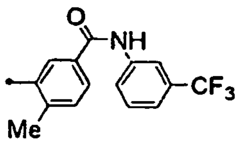 | OH | 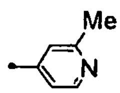 |
| 194 | 194 | $NH_2$ | 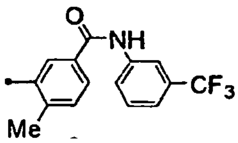 | OH | 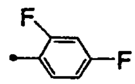 |
| 195 | 195 | $NH_2$ | 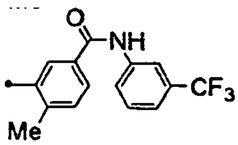 | OH | 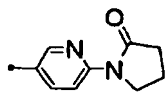 |
| 196 | 196 | $NH_2$ | 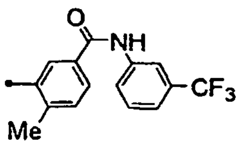 | OH | 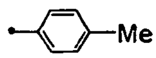 |
| 197 | 197 | $NH_2$ | 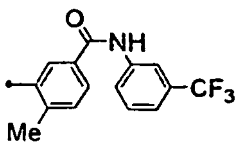 | OH | 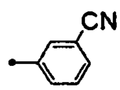 |
| 198 | 198 | 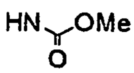 | 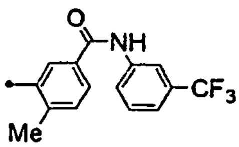 | OH | 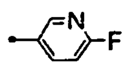 |

[Table 21]

Table 21

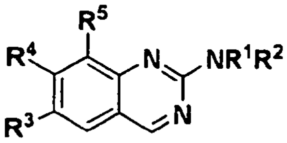

| Example No. | Compound No. | $NR^1R^2$ | $R^3$ | $R^4$ | $R^5$ |
|---|---|---|---|---|---|
| 199 | 199 | 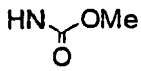 | 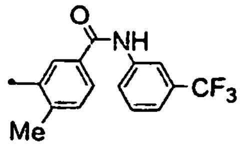 | OH | 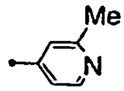 |
| 200 | 200 | $NH_2$ | 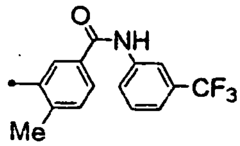 | OH | 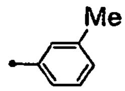 |
| 201 | 201 | $NH_2$ | 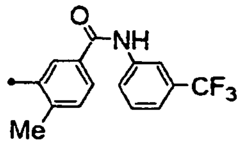 | OH | 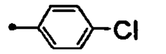 |
| 202 | 202 | $NH_2$ | 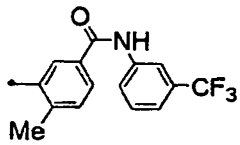 | OH | 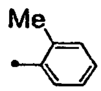 |
| 203 | 203 | $NH_2$ | 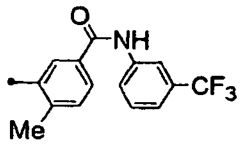 | OH | 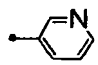 |
| 204 | 204 | $NH_2$ | 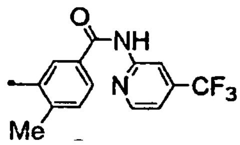 | OH | 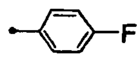 |
| 205 | 205 | $NH_2$ | 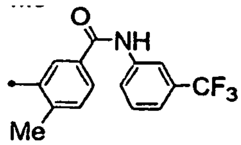 | OH | 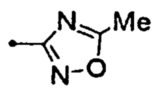 |
| 206 | 206 | NHAc | 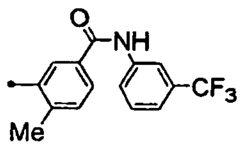 | OH | 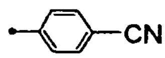 |
| 207 | 207 | $NH_2$ | 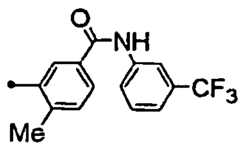 | OH | 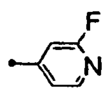 |
| 208 | 208 | $NH_2$ | 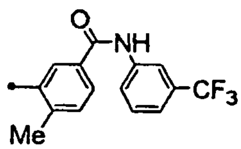 | OH | 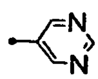 |
| Compound No. | | | | | |

[Table 22]

Table 22

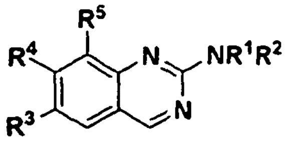

| Example No. | Compound No. | $NR^1R^2$ | $R^3$ | $R^4$ | $R^5$ |
|---|---|---|---|---|---|
| 209 | 209 | $NH_2$ | 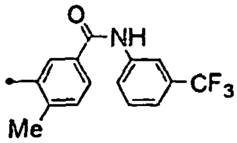 | OH | 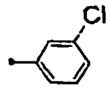 |
| 210 | 210 | $NH_2$ | 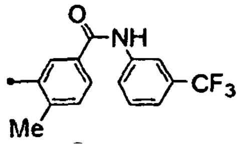 | OH | 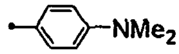 |
| 211 | 211 | $NH_2$ | 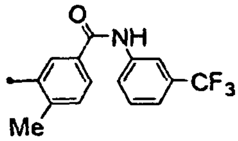 | OH | 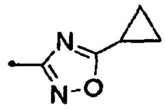 |
| 212 | 212 | 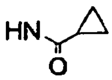 | 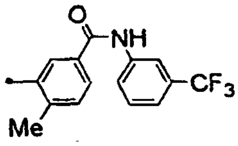 | OH | 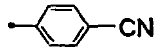 |
| 213 | 213 | $NH_2$ | 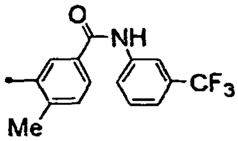 | OH | 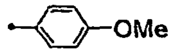 |
| 214 | 214 | $NH_2$ | 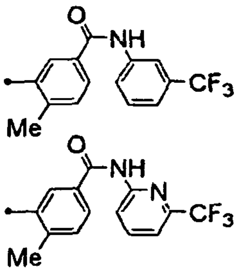 | OH | 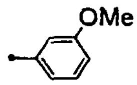 |
| 215 | 215 | $NH_2$ | | OMe | 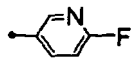 |
| 216 | 216 | $NH_2$ | 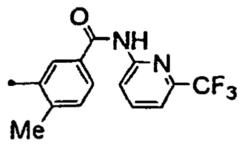 | OH | 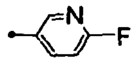 |
| 217 | 217 | $NH_2$ | 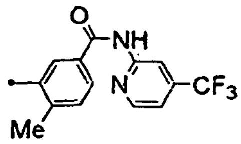 | OH | 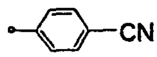 |
| 218 | 218 | 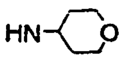 | 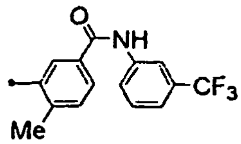 | OH | 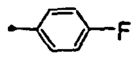 |

[Table 23]

Table 23

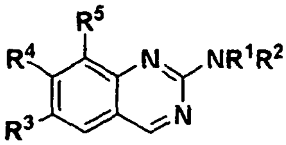

| Example No. | Compound No. | $NR^1R^2$ | $R^3$ | $R^4$ | $R^5$ |
|---|---|---|---|---|---|
| 219 | 219 | NHi-Pr | 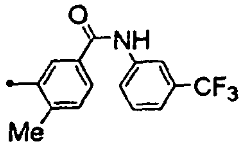 | OH | 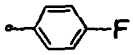 |
| 220 | 220 | NHi-Pr | 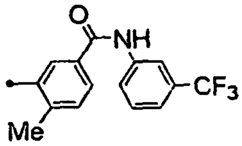 | OH | 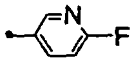 |
| 221 | 221 | NHMe | 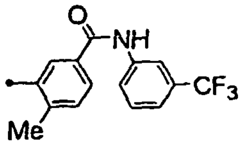 | OH | 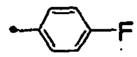 |
| 222 | 222 | NHi-Pr | 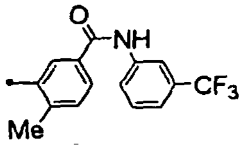 | OH | 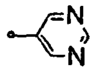 |
| 223 | 223 | $NH_2$ | 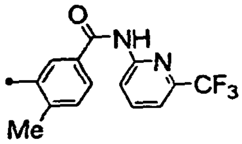 | OH | 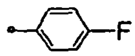 |
| 224 | 224 | $NH_2$ | 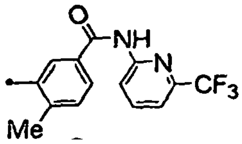 | OH | 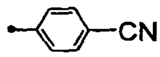 |
| 225 | 225 | NHi-Pr | 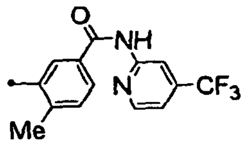 | OH | 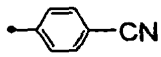 |
| 226 | 226 | NHi-Pr | 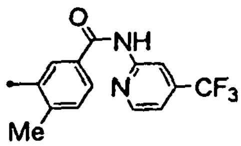 | OH | 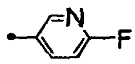 |
| 227 | 227 | 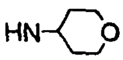 | 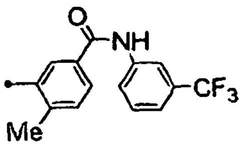 | OH | 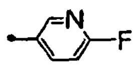 |
| 228 | 228 | 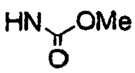 | 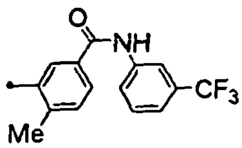 | OH | 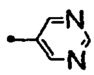 |

[Table 24]

Table 24

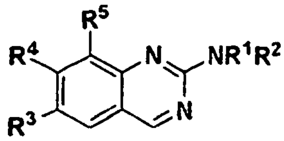

| Example No. | Compound No. | $NR^1R^2$ | $R^3$ | $R^4$ | $R^5$ |
|---|---|---|---|---|---|
| 229 | 229 | $NH_2$ | 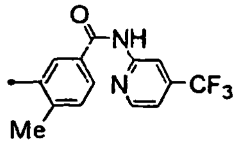 | OH | 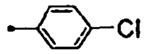 |
| 230 | 230 | NHi-Pr | 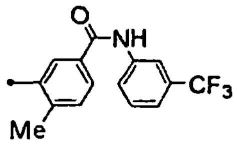 | OH | 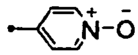 |
| 231 | 231 | NHi-Pr | 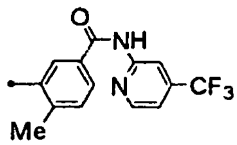 | OH | 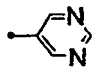 |
| 232 | 232 | NHi-Pr | 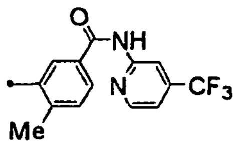 | OH | 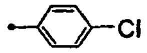 |
| 233 | 233 | 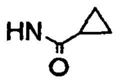 | 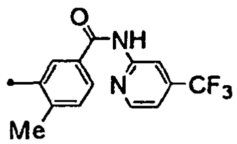 | OH | 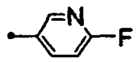 |
| 234 | 234 | $NH_2$ | 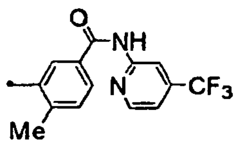 | OH | 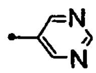 |
| 235 | 235 | 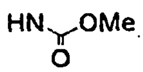 | 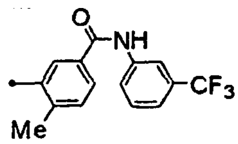 | OH | 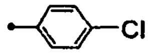 |
| 236 | 236 | 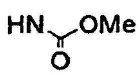 | 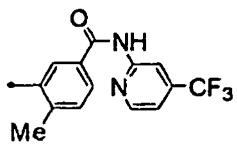 | OH | 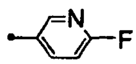 |
| 237 | 237 | $NH_2$ | 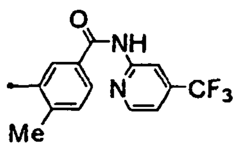 | OH | 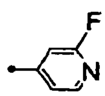 |
| 238 | 238 | 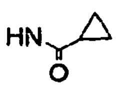 | 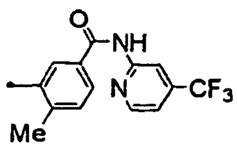 | OH | 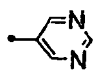 |

[Table 25]

Table 25

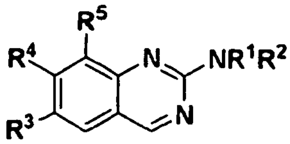

| Example No. | Compound No. | $NR^1R^2$ | $R^3$ | $R^4$ | $R^5$ |
|---|---|---|---|---|---|
| 239 | 239 | NHi-Pr | 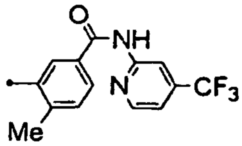 | OH | 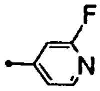 |
| 240 | 240 | 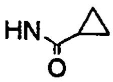 | 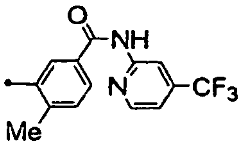 | OH | 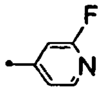 |
| 241 | 241 | NHi-Pr | 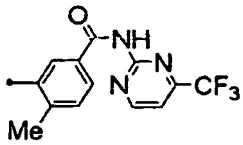 | OH | 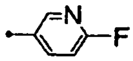 |
| 242 | 242 | $NH_2$ | 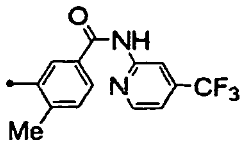 | OH | 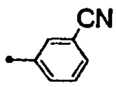 |
| 243 | 243 | $NH_2$ | 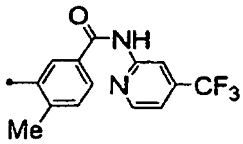 | OH | 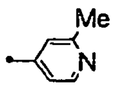 |
| 244 | 244 | $NH_2$ | 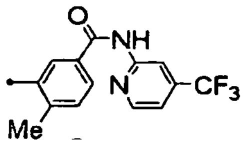 | OH | 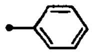 |
| 245 | 245 | NHi-Pr | 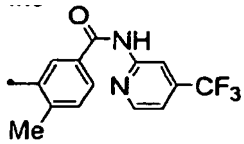 | OH | 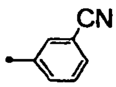 |
| 246 | 246 | $NH_2$ | 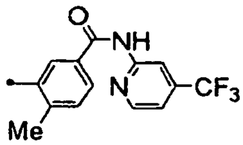 | OH | 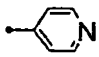 |
| 247 | 247 | NHi-Pr | 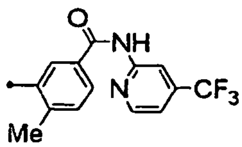 | OH | 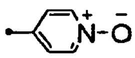 |
| 248 | 248 | 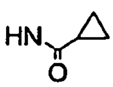 | 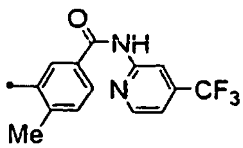 | OH | 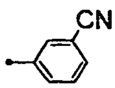 |

[Table 26]

Table 26

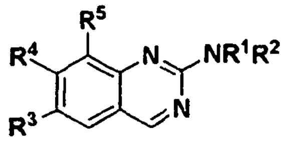

| Example No. | Compound No. | $NR^1R^2$ | $R^3$ | $R^4$ | $R^5$ |
|---|---|---|---|---|---|
| 249 | 249 | $NH_2$ | 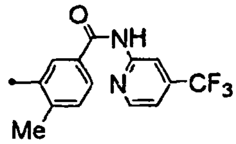 | OH | 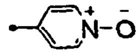 |
| 250 | 250 | $NH_2$ | 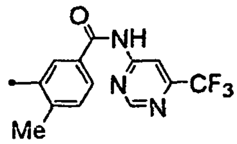 | OH | 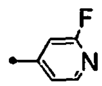 |
| 251 | 251 | $NH_2$ | 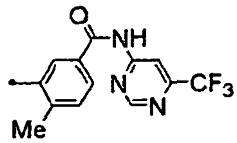 | OH | 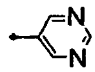 |
| 252 | 252 | $NH_2$ | 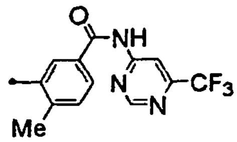 | OH | 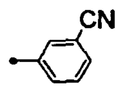 |
| 253 | 253 | $NH_2$ | 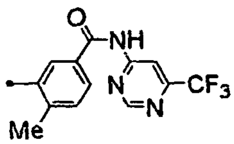 | OH | 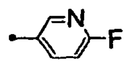 |
| 254 | 254 | $NH_2$ | 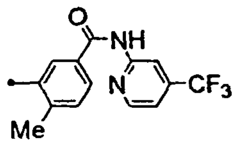 | OH | 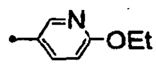 |
| 255 | 255 | $NH_2$ | 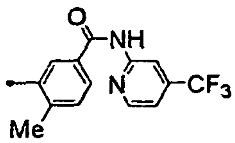 | OH | 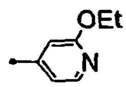 |
| 256 | 256 | $NH_2$ | 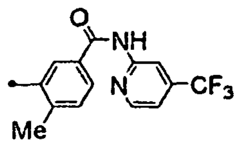 | OH | 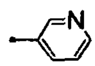 |
| 257 | 257 | $NH_2$ | 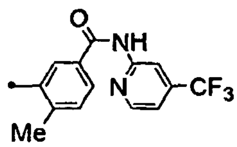 | OH | 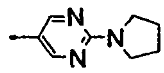 |
| 258 | 258 | $NH_2$ | 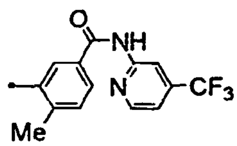 | OH | 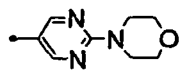 |

[Table 27]

Table 27

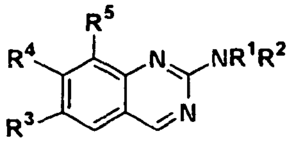

| Example No. | Compound No. | $NR^1R^2$ | $R^3$ | $R^4$ | $R^5$ |
|---|---|---|---|---|---|
| 259 | 259 | $NH_2$ | 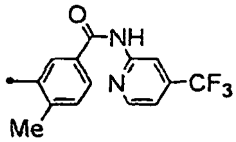 | OH | 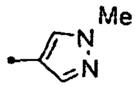 |
| 260 | 260 | $NH_2$ | 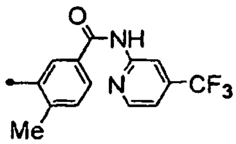 | OH | 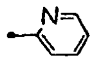 |
| 261 | 261 | $NH_2$ | 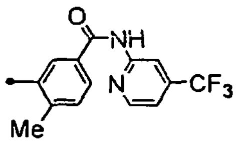 | OH | 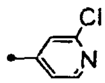 |
| 262 | 262 | $NH_2$ | 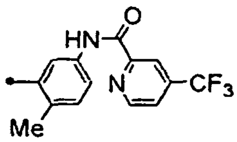 | OH | 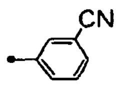 |
| 263 | 263 | 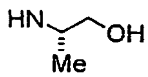 | 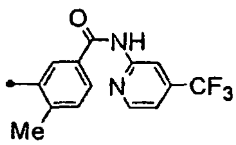 | OMe | 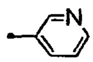 |
| 264 | 264 | 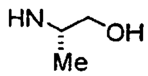 | 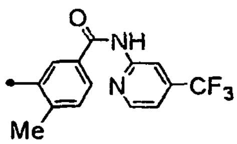 | OMe | 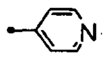 |
| 265 | 265 | 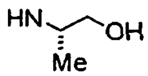 | 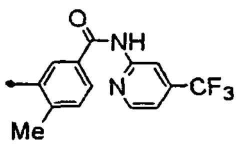 | OH | 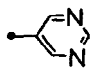 |
| 266 | 266 | 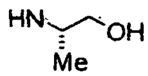 | 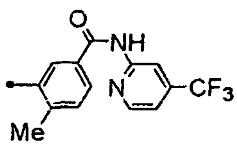 | OH | 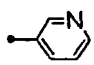 |
| 267 | 267 | 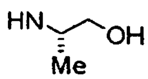 | 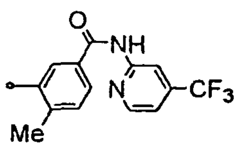 | OH | 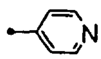 |
| 268 | 268 | $NH_2$ | 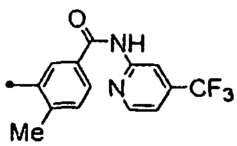 | OH | 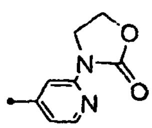 |

[Table 28] [0099]

Table 28

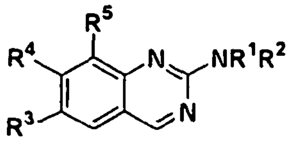

| Example No. | Compound No. | $NR^1R^2$ | $R^3$ | $R^4$ | $R^5$ |
|---|---|---|---|---|---|
| 269 | 269 | $NH_2$ | 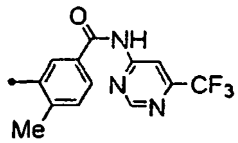 | OH | 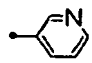 |
| 270 | 270 | $NH_2$ | 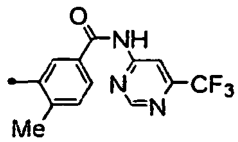 | OH | 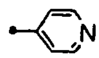 |
| 271 | 271 | $NH_2$ | 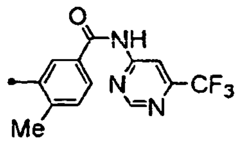 | OH | 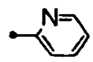 |
| 272 | 272 | $NH_2$ | 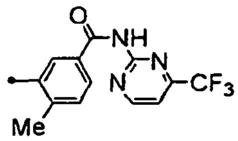 | OH | 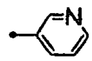 |
| 273 | 273 | $NH_2$ | 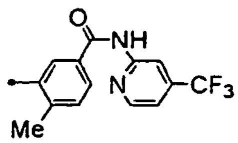 | OH | 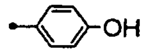 |
| 274 | 274 | $NH_2$ | 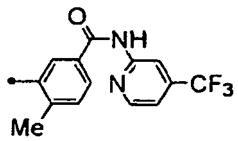 | OH | 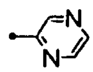 |
| 275 | 275 | $NH_2$ | 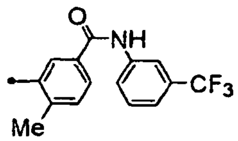 | OH | 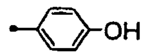 |

Hereinafter, pharmaceutical activities of typical compounds are described in detail in Test Examples.

Test Example 1: Tie-2 enzyme inhibitory activity

The following method was used in order to measure Tie-2 enzyme inhibitory activity. Functional domain of human Tie-2 (771-1124 end amino acid) (Carna Biosicence Inc., Catalogue No. 08-285) was used as Tie-2 enzyme. Biotinylated polyglutamic acid-tyrosine peptide (CisBio Bioassays, Catalogue No. 61GT0BLA) as a substrate was immobilized on a 96-well plate coated with NeutrAvidin (Pierce Corporation, Catalogue No.31000) and then was blocked with 0.25% gelatin (Bio-Rad Laboratories Inc., Catalogue No. 170-6537) to prepare a plate for measuring the kinase reaction. After adjusting the concentration of each test compound, test compounds were added to each well of the plate for measuring the kinase reaction, and the final concentratioins of respective reagents in the volume of 60 μL were as follows: 83 ng/mL of Tie-2, 20 mmol/L of Tris·Cl (pH 7.5), 5 mmol/L of β-glycerophosphate, 2 mmol/L of dithiothreitol, 0.1 mmol/L of $Na_3VO_4$, 10 mmol/L of $MnCl_2$, 50 μmol/L of ATP, 0.1% of Albumin bovine serum (BSA; Sigma Corporation, Catalogue No. A4503), and 0.1% of dimethylsulfoxide (DMSO). Then the enzyme reaction was carried out at 30°C for 40 minutes. The plate was washed with TBS-T [10 mmol/L Tris-Cl (pH 7.5), 150 mmol/L NaCl, and 0.05% Tween 20 (Bio-Rad Laboratories Inc., Catalogue No. 170-6531)] three times, and allowed to react with europium-labeled anti-phosphotyrosine antibody (PerkinElmer Inc. Catalogue No. AD0160) at room temperature for 60 minutes. The plate was further washed with TBS-T three times. Subsequently, DELFIA Enhancement Solution (PerkinElmer Inc. Catalogue No. 1244-105) was added to each well of the plate, and time-resolved fluoresence was measured (excitation wavelength: 340 nm, measuring wavelength: 615 nm). The relative activity (%) on the well to which the test compound was added was calculated with using the value on the well of the enzyme with addition of 0.1% DMSO being 100% and the value on the well with no addition of the enzyme being 0%. The Tie-2 inhibitory activity (%) of the test compound was determined by subtracting the relative activity (%) from 100. In other words, the value on the well of the enzyme with addition of 0.1% DMSO was defined as A, the value on the well with no addition of the enzyme was defined as B, and the value on the well with addition of a test compound was defined as C, whereby Tie-2 inhibition ratio (%) was calculated by equation 1.

Equation 1:

$$\text{Tie-2 inhibition ratio(\%)} = 100 - \frac{C - B}{A - B} \times 100$$

Then, $IC_{50}$ values of all compounds were calculated based on these.

In the present test, Compounds 4, 5, 6, 12, 14, 16, 18, 19, 22, 25, 32, 33, 34, 36, 46, 50, 51, 54, 59, 60, 62, 69, 75, 78, 79, 81, 82, 85, 91, 98, 100, 105, 109, 110, 111, 114, 117, 126, 128, 130, 134, 137, 144, 148, 150, 158, 163, 171, 173, 179, 186, 190, 199, 218, 226, 233, 235 and Compounds 239-275 showed $IC_{50}$ values of 20 nM or less.

From the results of the test, it was considered that Compound (I) and pharmaceutically acceptable salts thereof have inhibitory activity against Tie-2 enzyme, that is, Tie-2 kinase, and are effective in the treatment and/or the prevention of a disease associate with Tie-2 kinase (for example, ovarian cancer, breast cancer, renal cancer, prostate cancer, lung cancer, thyroid cancer, myeloid leukemia, hemangiomas, melanomas, astrocytomas, glioblastomas, psoriasis or pulmonary hypertension, or the like).

Test Example 2: KDR enzyme inhibitory activity

The following method was used in order to measure KDR enzyme inhibitory activity. The protein which was fused with His (histidine) tag at the N terminal of the functional domain of human KDR (amino acid 790-end) (Upstate Inc., Catalogue No. 14-630) was used as a KDR. Biotinylated polyglutamic acid-tyrosine substrate peptide (CisBio Biosassays, Catalogue No. 61GT0BLA) used as a substrate was immobilized on a 96-well plate coated with NeutrAvidin (Pierce Corporation, Catalogue No.31000), and then was blocked with 0.25% gelatin (Bio-Rad Laboratories Inc., Catalogue No. 170-6537) to prepare a plate for measuring the kinase reaction. To each well of the plate for measuring the kinase reaction were added reagents in the volume of 60 μL, and the final concentrations of the respective reagents were as follows: 33 ng/mL of His-Tag fused KDR protein, 20 mmol/L of Tris-Cl (pH 7.5), 5 mmol/L of β-glycerophosphate, 2 mmol/L of dithiothreitol, 0.1 mmol/L of $Na_3VO_4$:, 10 mmol/L of $MnCl_2$, 10 μmol/L of ATP, 0.1% of Albumin bovine serum (BSA; Sigma Corporation, Catalogue No. A4503), and 0.1% of dimethylsulfoxide (DMSO). Then the enzyme reaction was carried out at 25° C for 60 minutes. The plate was washed with TBS-T [10 mM Tris-Cl (pH 7.5), 150 mmol/L NaCl, and 0.05% Tween 20 (Bio-Rad Laboratories Inc., Catalogue No. 170-6531)] three times, and allowed to react with europium-labeled anti-phosphotyrosine antibody (PerkinElmer Inc. Catalogue No. AD0160) at room temperature for 60 minutes. The plate was further washed with TBS-T three times. Subsequently, DELFIA Enhancement Solution (PerkinElmer Inc. Catalogue No. 1244-105) was added to each well of the plate, and time-resolved fluoresence was measured (excitation wavelength: 340 nm, measuring wavelength: 615 nm). The relative activity (%) on the well to which the test compound was added was calculated using the value on the well of the enzyme with addition of 0.1% DMSO being 100% and the value on the well with no addition of the enzyme being 0%. The KDR inhibitory activity (%) of the test compound was determined by subtracting the relative activity (%) from 100. In other words, the value on the well of the enzyme with addition of 0.1% DMSO was defined as Al, the value on the well with no addition of the enzyme was defined as B1, and the value on the well with addition of a test compound was defined as C1, whereby KDR inhibition ratio (%) was calculated by equation 2. Then, $IC_{50}$ values of all compounds were calculated based on these.

Equation 2:

$$\text{KDR inhibition ratio(\%)} = 100 - \frac{C1 - B1}{A1 - B1} \times 100$$

As the results, the ratios of KDR $IC_{50}$/Tie-2 $IC_{50}$ for Compounds 4, 12, 14, 16, 18, 22, 25, 34, 46, 50, 54, 59, 60, 62, 69, 75, 78, 79, 98, 100, 105, 110, 111, 114, 126, 128, 134, 144, 148, 150, 158, 163, 173, 179, 186, 199, 218, 226, 233, 235 and Compounds 239-275 were 20 or more, and these compounds exhibited highly selective kinase inhibitory activity to Tie-2 kinase.

Compound (I) or a pharmaceutically acceptable salt thereof can be administered alone. However, usually it is preferably provided in various pharmaceutical preparations. Such pharmaceutical preparations are used in animals or humans.

The pharmaceutical preparations according to the present invention may contain Compound (I) or a pharmaceutically acceptable salt thereof alone as an active ingredient or as a mixture with any other active ingredient for other treatments. Furthermore, these pharmaceutical preparations are prepared by mixing the active ingredient with one or more pharmaceutical acceptable carriers (for example, diluents, solvents, excipients), then subjecting the mixture to any method well-known in the technical field of pharmaceutics.

As for the administration route, it is preferred to select the most effective route for the treatment. Examples of the administration route include oral and parenteral administration such as an intravenous route.

Examples of the dosage form include tablet, injection and the like.

For example, tablets suitable for the oral administration, or the like, can be produced using excipients such as lactose, disintegrants such as starch, lubricants such as magnesium stearate, binders such as hydroxypropyl cellulose, and the like.

For example, injections suitable for parenteral administration, or the like can be produced by using diluents or solvents such as a salt solution, a glucose solution, or a mixture of brine and a glucose solution, and the like.

The dosage and the dosage frequency of administration of Compound (I) or a pharmaceutically acceptable salt thereof may vary depending on the dosage form, age and body weight of a patient, nature or seriousness of the symptoms to be treated, and the like. In oral administration, in general, a dose of 0.01 to 1000 mg, preferably 0.05 to 100 mg, is administered to an adult patient once or several times a day. In parenteral administration, such as intravenous administration, a dose of 0.001 to 1000 mg, preferably 0.01 to 100 mg, is administered to an adult patient once or several times a day. However, these dosage and dosage frequencies vary depending on the various conditions described above.

Reference Example 1

5-Bromo-2-fluoro-4-methoxybenzaldehyde (Compound A1)

Potassium bromide (193 g, 1.62 mol) and bromine (33.0 mL, 649 mmol) were dissolved in water (1000 mL), and 2-fluoro-4-methoxybenzaldehyde (50.0 g, 324 mmol) was added thereto under ice-cooling, followed by stirring at room temperature for 3 hours. Water was added to the reaction mixture, and crystals were collected by filtration to obtain Compound A1 (72.2 g, 95%).

$^1$H NMR (300 MHz, $CDCl_3$) 8 (ppm) 3.98 (s, 3H), 6.78 (d, J = 11.7 Hz, 1H), 8.06 (d, J = 7.7 Hz, 1H), 10.17 (s 1H).

Reference Example 2

2-Amino-6-bromo-7-methoxyquinazoline (Compound A2)

Compound A1 (20.0 g, 85.8 mmol) was dissolved in DMA(300 mL), and guanidine carbonate (30.9 g,172 mmol) was added thereto, followed by stirring at 140°C for 2 hours. The reaction mixture was added to ice-water, and the resulting crystals were collected by filtration to obtain Compound A2 (16.4 g, 75%).

$^1$H NMR (300 MHz, DMSO-$d_6$) $\delta$ (ppm) 4.00 (s, 3H), 5.24 (br s, 2H), 6.92 (s, 1H), 7.88 (s, 1H), 8.82 (s, 1H).

Reference Example 3

6-Bromo-2-isopropylamino-7-methoxyquinazoline (Compound A3)

Compound A2 (6.0 g, 23.6 mmol) was dissolved in DMF (230 mL), followed by cooling to 0°C, and sodium hydride (60% in oil, 2.80 g, 64.9 mmol) was added thereto, followed by stirring for 30 minutes under argon atmosphere. To the reaction mixture was added isopropyl iodide (4.7 mL, 47.2 mmol), followed by stirring at 60°C for 2.5 hours. The reaction mixture was cooled to 0°C, sodium hydride (60% in oil, 2.80 g, 64.9 mmol) was added thereto, followed by stirring at room temperature for 30 minutes, and isopropyl iodide (4.7 mL, 47.2 mmol) was added thereto, followed by stirring at 60°C for 1 hour. The reaction mixture was poured into ice-water, followed by stirring at room temperature, and the precipitated solid was collected by filtration to obtain Compound A3 (7.35 g, 100%).

$^1$H NMR (300 MHz, DMSO-$d_6$) $\delta$ (ppm) 1.19 (d, J = 6.6 Hz, 6H), 3.96 (s, 3H), 4.11-4.23 (m, 1H), 6.93 (s, 1H), 7.27 (br

s, 1H), 8.05 (s, 1H), 8.90 (s, 1H).

Reference Example 4

4-Methyl-3-(4,4,5,5-tetramethyl-1,3,2-dioxaboran-2-yl)aniline (Compound A4)

3-Iodo-4-methylaniline (1.97 g, 8.45 mmol) and bis(pinacolate)diboron (2.32 g, 9.13 mmol) were dissolved in DMSO(23 mL), and potassium acetate (2.87 g, 29.2 mmol) and [1,1'-bis(diphenylphosphino)ferrocene]dichloropalladium (345 mg, 0.423 mmol) were added thereto, followed by stirring at 80°C for 4 hours under argon atmosphere. To the reaction mixture were added ethyl acetate and water, and insoluble materials were filtrated off through celite, followed by extraction with ethyl acetate. The organic layer was washed with water and brine, followed by drying over anhydrous magnesium sulfate. The solvent was evaporated under reduced pressure, and the residue was purified by si lica gel column chromatography (ethyl acetate/hexane = 6/1) to obtain Compound A4(1.36 g, 69%).
$^1$H NMR (300 MHz, $CDCl_3$) $\delta$ (ppm) 1.33 (s, 12H), 2.42 (s, 3H), 3.51 (br s, 2H), 6.68 (dd, J = 8.1, 2.7 Hz, 1H), 6.97 (d, J = 8.1 Hz, 1H), 7.11 (d, J = 2.7 Hz, 1H).

Reference Example 5

N-[4-Methyl-3-(4,4,5,5-tetramethyl-1,3,2-dioxaboran-2-yl)]-3-(trifluoromethyl)benzamide (Compound A5)

Compound A4 (0.50 g, 2.04 mmol) was dissolved in dichloromethane (21 mL), followed by cooling to 0°C, then triethylamine (0.387 mL, 2.78 mmol), and 3-trifluoromethylbenzoyl chloride (0.339 mL, 2.25 mmol) were added thereto, followed by stirring for 10 minutes. Water was added to the reaction mixture, followed by extraction with dichloromethane. The organic layer was washed with brine, followed by drying over anhydrous magnesium sulfate. Under reduced pressure, the solvent was evaporated to obtain Compound A5 (0.80 g, 91%).
$^1$H NMR (300 MHz, $CDCl_3$) $\delta$ (ppm) 1.35 (s, 12H), 2.53 (s, 3H), 7.22 (d, J = 6.6 Hz, 1H), 7.58-7.68 (m, 2H) 7.74-7.84 (m, 2H), 7.93-8.00 (m, 1H), 8.05 (d, J = 7.5 Hz, 1H) 8.12 (s, 1H).

Reference Example 6

2-Amino-7-methoxy-6-[2-methyl-5-(3-trifluoromethylbenzamide)phenyl]quinazoline (Compound A6)

Compound A2 (1.82 g, 7.16 mmol) was dissolved in dioxane (36 mL) and water (36 mL), and Compound A5 (3.48 g, 8.59 mmol), sodium carbonate (1.52 g, 14.3 mmol) and [1,1'-bis(diphenylphosphino)ferrocene]dichloropalladium (292 mg, 0.358 mmol) were added thereto, followed by stirring under heating and reflux for I hour. To the reaction mixture were added ethyl acetate and water, and the organic layer was separated. The organic layer was washed with water, followed by drying over anhydrous magnesium sulfate. The solvent was evaporated under reduced pressure, and the residue was purified by silica gel column chromatography (ethyl acetate) to obtain Compound A6 (2.4 g, 74%).
$^1$H NMR (300 MHz, $CDCl_3$) $\delta$ (ppm) 2.12 (s, 3H), 3.88 (s, 3H), 5.15 (s, 2H), 6.98 (s, 1H), 7.29 (d, J = 8.1 Hz, 1H), 7.49 (s, 1H), 7.50 (d, J = 2.1 Hz, 1H), 7.58-7.63 (m, 2H), 7.78-7.86 (m, 2H), 8.05 (d, J = 7.8 Hz, 1H), 8.11 (s, 1H), 8.87 (s, 1H).

Reference Example 7

2-Amino-8-bromo-7-methoxy-6-[2-methyl-5-(3-trifluoromethylbenzamide)phenyl]quinazoline (Compoun d A7)

Compound A6 (2.40 g, 5.30 mmol) was dissolved in chloroform (27 mL) and acetic acid (27 mL), then N-bromosuccinicimide (1.03 g, 5.83 mmol) was added thereto, followed by stirring at room temperature for 3 hours. The reaction mixture was poured into 2 mol/L of an aqueous sodium hydroxide solution, followed by extraction with ethyl acetate. The organic layer was washed with brine, followed by drying over anhydrous magnesium sulfate. The solvent was evaporated under reduced pressure, and the residue was purified by silica gel column chromatography (ethyl acetate/hexane = 1/1) to obtain Compound A7 (2.18 g, 78%).
$^1$H NMR (300 MHz, $CDCl_3$) $\delta$ (ppm) 2.19 (s, 3H), 3.57 (s, 3H), 5.46 (s, 2H), 7.33 (d, J = 9.0 Hz, 1H), 7.55 (s, 1H), 7.58-7.68 (m, 3H), 7.78-7.90 (m, 2H), 8.07 (d, J = 7.5 Hz, 1H), 8.13 (s, 1H), 8.94 (s, 1H).

Reference Example 8

4-Methyl-3-(4,4,5,5-tetramethyl-1,3,2-dioxaboran-2-yl) benzoic acid (Compound A8)

3-Iodo-4-methyl benzoic acid (26.2 g, 100 mmol) was dissolved in DMF (300 mL), and then bis(pinacolate)diboron (38.1 g, 150 mmol), [1,1'-bis(diphenylphosphino)ferrocene]dichloropalladium (8.17 g, 10.0 mmol) and potassium acetate (49.0 g, 500 mmol) were added thereto, followed by stirring under argon atmosphere at 100°C for 6 hours. To the reaction mixture was added 1 mol/L of hydrochloric acid, followed by extraction with ethyl acetate, an organic layer was washed with brine, followed by drying over anhydrous magnesium sulfate, and activated carbon (1 g) was added thereto, followed by stirring at room temperature for 1 hour. The reaction mixture was filtered through celite, followed by washing with ethyl acetate: The solvent was evaporated, and the resulting crystals were recrystallized from isopropyl ether, followed by filtration to obtain Compound A8 (18.5 g, 71%).
$^1$H NMR (300 MHz, $CDCl_3$) δ (ppm) 1.36 (s, 12H), 2.61 (s, 3H), 7.26 (d, J = 8.1 Hz, 1H), 8.02 (dd, J = 8.1, 1.8 Hz, 1H), 8.49 (d, J = 1.8 Hz, 1H).

Reference Example 9

4-Methyl-3-(4,4,5,5-tetramethyl-1,3,2-dioxaboran-2-yl)-N-(3-trifluoromethylphenyl)benzamide (Compoun d A9)

Compound A8 (7.13 g, 27.2 mmol) was dissolved in thionyl chloride (57 mL), followed by stirring at 60°C for 2 hours, then the thionyl chloride was evaporated under reduced pressure. The reaction mixture was dissolved in dichloromethane (136 mL), and 3-trifluoromethylaniline (4.0 mL, 32.6 mmol) and triethylamine (5.31 mL, 38.1 mmol) were added thereto, followed by stirring at room temperature for 3.5 hours. To the reaction mixture was added saturated aqueous sodium bicarbonate, followed by extraction with chloroform, and the organic layer was washed with brine, followed by drying over anhydrous magnesium sulfate. The solvent was evaporated under reduced pressure, and the residue was reslurried with cyclohexane to obtain Compound A9(9.32 g, 85%).
$^1$H NMR (300 MHz, $CDCl_3$) δ (ppm) 1.38 (s, 12H), 2.61 (s, 3H), 7.31 (d, J = 8.0 Hz, 1H), 7.39 (d, J = 7.9 Hz, 1H), 7.49 (t, J = 7.9 Hz, 1H), 7.85-8.03 (m, 4H), 8.17 (d, J = 2.0 Hz, 1H).
ESI m/z $(M+H)^+$ 406.

Reference Example 10

2-Amino-7-methoxy-6-[2-methyl-5-N-(3-trifluoromethylphenyl)carbamoylphenyl]quinazoline (Compound A10)

Compound A10 was obtained in the same manner as in Reference Example 6, using the compounds A2 and A9.
$^1$H NMR (270 MHz, $CDCl_3$) δ (ppm) 2.21 (s, 3H), 3.88 (s, 3H), 5.16 (s, 2H), 7.00 (s, 1H), 7.35-7.53 (m, 4H), 7.72 (d, J = 1.9 Hz, 1H), 7.85-8.05 (m, 4H), 8.87 (s, 1H).

Reference Example 11

2-Amino-8-bromo-7-methoxy-6-[2-methyl-5-N-(3-trifluoromethylphenyl)carbamoylphenyl]quinazoline (Compound A11)

Compound A11 was obtained in the same manner as in Reference Example 7, using Compound A10.
$^1$H NMR (270 MHz, $CDCl_3$) δ (ppm) 2.27 (s, 3H), 3.53 (s, 3H), 5.51 (s, 2H), 7.37-7.55 (m, 3H), 7.51 (s, 1H), 7.84-7.96 (m, 3 H), 7.99 (br s, 1H), 8.15 (br s, 1H), 8.92 (s, 1H).

Reference Example 12

2-Fluoro-N-[4-methyl-3-(4,4,5,5-tetramethyl-1,3,2-dioxaboran-2-yl)phenyl]-5-(trifluoromethyl)benzamide (Compound A12)

Compound A12 was obtained in the same manner as in Reference Example 5, using Compound A4 and 2-fluoro-5-trifluoromethylbenzoyl chloride.
$^1$H NMR (300 MHz, $CDCl_3$) δ (ppm) 1.36 (s, 12H), 2.53 (s, 3H), 7.21 (d, J = 8.4 Hz, 1H), 7.28-7.36 (m, 1H), 7.69 (d, J = 2.4 Hz, 1H), 7.74-7.82 (m, 1H), 7.92 (dd, J = 8.4, 2.4 Hz, 1H), 8.28-8.42 (m, 1H), 8.48-8.54 (m, 1H).

Reference Example 13

2-Amino-8-bromo-7-methoxy-6-[2-methyl-5-(2-fluoro-5-trifluoromethylbenzamide)phenyl]quinazoline (Compound A13)

Compound A13 was obtained in the same manner as in Reference Example 6 and Reference Example 7, using Compound A2 and Compound A12.
$^1$H NMR (300 MHz, $CDCl_3$) δ (ppm) 2.19 (s, 3H), 3.58 (s, 3H), 5.71 (s, 2H), 7.28-7.38 (m, 2H), 7.55 (s, 1H), 7.58-7.67 (m, 2H), 7.74-7.84 (m, 1H), 8.40-8.52 (m, 2H), 8.94 (s, 1H).

Reference Example 14

2-Acetylamino-6-bromo-7-methoxyquinazoline (Compound A14)

Compound A2 (2.00 g, 7.87 mmol) was suspended in pyridine (10 mL), and acetic anhydride (3.7 mL, 39.4 mmol) was added thereto, followed by stirring at 80°C for 2 hours. The reaction mixture was added to water, and the resulting crystals were collected by filtration to obtain Compound A14 (1.80 g, 77%).
$^1$H NMR (270 MHz, DMSO-$d_6$) δ (ppm) 2.27 (s, 3H), 4.04 (s, 3H), 7.24 (s, 1H), 8.36 (s, 1H), 9.26 (s, 1H), 10.62 (s, 1H).

Reference Example 15

6-Bromo-7-methoxy-2-(methylamino)quinazoline (Compound A15)

Compound A14 (1.80 g, 6.08 mmol) was dissolved in DMF (15 mL), and potassium carbonate (2.52 g, 18.2 mmol) and methyl iodide (0.95 mL, 17.0 mmol) were added thereto, followed by stirring at 60°C for 6 hours. The reaction mixture was added to water, the resulting crystals were collected by filtration, followed by dissolving in methanol (16 mL), and potassium carbonate (1.59 g, 11.5 mmol) was added thereto, followed by stirring at 60°C for 2 hours. The reaction mixture was added to water, and the resulting crystals were collected by filtration to obtain Compound A15 (1.51 g, 93%).
$^1$H NMR (270 MHz, $CDCl_3$) δ (ppm) 3.10 (d, J = 5.1 Hz, 3H), 4.01 (s, 3H), 5.26 (br s, 1H), 6.99 (s, 1H), 7.84 (s, 1H), 8.75 (s, 1H).

Reference Example 16

8-Bromo-7-methoxy-2-methylamino-6-[2-methyl-5-N-(3-trifluoromethylphenyl)carbamoylphenyl]quinazol ine (Compound A16)

Compound A16 was obtained in the same manner as in Reference Example 6 and Reference Example 7, using the compounds A15 and A9.
$^1$H NMR (270 MHz, $CDCl_3$) δ (ppm) 2.29 (s, 3H), 3.22 (d, J = 4.9 Hz, 3H), 3.55 (s, 3H), 5.42-5.55 (m, 1H), 7.38-7.56 (m, 4H), 7.80-7.98 (m, 5H), 8.89 (s, 1H).

Reference Example 17

3-(2-Amino-7-methoxyquinazolin-6-yl)-4-methyl benzoic acid (Compound A17)

Compound A2 (35.0 g, 138 mmol) was dissolved in dioxane (415 mL) and water (210 mL), and Compound A8 (43.4 g, 166 mmol), sodium carbonate (29.3 g, 276 mmol) and [1,1'-bis(diphenylphosphino)ferrocene]dichloropalladium (5.6 g, 6.9 mmol) were added thereto, followed by stirring under heating and reflux for 1 hour. To the reaction mixture were added water and ethyl acetate, and the organic layer was separated. To an aqueous layer was added 2 mol/L of hydrochloric acid, followed by adjusting the pH to about 5, and the resulting crystals were collected by filtration to obtain Compound A17 (42 g, 99%).
$^1$H NMR (270 MHz, DMSO-$d_6$) δ (ppm) 2.21 (s, 3H), 3.81 (s, 3H), 6.73 (s, 2H), 6.89 (s, 1H), 7.37 (d, J = 8.1 Hz, 1H), 7.54 (s, 1H), 7.68 (d, J = 1.6 Hz, 1H), 7.82 (dd, J = 8.1, 1.6 Hz, 1H), 8.92 (s, 1H).

Reference Example 18

3-(2-Amino-8-bromo-7-methoxyquinazolin-6-yl)-4-methyl benzoic acid (Compound A18)

Compound A17 (37.1 g, 120 mmol) was dissolved in acetic acid (600 mL), and N-bromosuccinicimide (25.6 g, 144 mmol) was added thereto, followed by stirring at room temperature for 18 hours. The reaction mixture was cooled to 0°C, 10 mol/L of an aqueous sodium hydroxide solution was added thereto for neutralization, and 6 mol/L of hydrochloric acid was added thereto to adjust the pH to about 4. The resulting crystals were collected by filtration, followed by reslurrying with methanol to obtain Compound A18 (35.6 g, 76%).
$^1$H NMR (300 MHz, DMSO-$d_6$) $\delta$ (ppm) 2.19 (s, 3H), 3.40 (s, 3H), 7.24 (s, 2H), 7.47 (d, J = 8.1 Hz, 1H), 7.73 (s, 1H), 7.83 (d, J = 2.1 Hz, 1H), 7.91 (dd, J = 8.1, 2.1 Hz, 1H), 9.08 (s, 1H), 11.06 (br s, 1H).

Reference Example 19

8-Bromo-2-isopropylamino-7-methoxy-6-[2-methyl-5-N-(3-trifluoromethylphenyl)carbamoylphenyl]quina zoline (Compound A19)

Compound A19 was obtained in the same manner as in Reference Example 6 and Reference Example 7, using Compound A3 and Compound A9.
$^1$H NMR (300 MHz, $CDCl_3$) $\delta$ (ppm) 1.36 (d, J = 6.4 Hz, 6H), 2.28 (s, 3H), 3.53 (s, 3H), 4.34-4.45 (m, 1H), 5.36 (s, 1H), 7.39-7.53 (m, 4H), 7.81-7.90 (m, 3H), 7.95-7.96 (m, 2H), 8.87 (s, 1H).

Reference Example 20

2-Amino-8-bromo-6-(5-N-{2-[N-(3-dimethylaminopropyl)-N-methylamino]-5-trifluoromethylphenyl}carb amoyl-2-methylphenyl)-7-methoxyquinazoline (Compound A20)

Compound A18 (0.50 g, 1.29 mmol) was dissolved in thionyl chloride (4.1 mL), followed by stirring at 60°C for 2 hours, then thionyl chloride was evaporated under reduced pressure. The reaction mixture was dissolved in dichloromethane (9 mL), and 2-[N-(3-dimethylaminopropyl)-N-methylamino]-5-trifluoromethylaniline (WO 06/039718) (0.43 g, 1.55 mmol) and triethylamine (0.25 mL, 1.80 mmol) were added thereto, followed by stirring at room temperature for 21 hours. To the reaction mixture was added saturated aqueous sodium bicarbonate, followed by extraction with chloroform, and the organic layer was washed with brine, followed by drying over anhydrous magnesium sulfate. The solvent was evaporated under reduced pressure, and the residue was purified by silica gel column chromatography (chloroform/methanol = 95/5) to obtain Compound A20 (0.25 g, 30%).
$^1$H NMR (270 MHz, $CDCl_3$) $\delta$ (ppm) 1.60-1.74 (m, 2H), 2.13 (s, 6H), 2.27 (s, 3H), 2.28-2.34 (m, 2H), 2.70 (s, 3H), 2.99 (t, J = 7.3 Hz, 2H), 3.54 (s, 3H), 5.62 (s, 2H), 7.28-7.38 (m, 2H), 7.44 (d, J = 7.8 Hz, 1H), 7.56 (s, 1H), 7.80-7.86 (m, 1H), 7.90-7.94 (m, 1H), 8.84-8.90 (m, 1H), 8.94 (s, 1H), 9.49 (s, 1H).
ESI m/z $(M+H)^+$ 645.

Reference Example 21

3-(4-Methylpiperazin-1-yl)methyl-5-trifluoromethylaniline (Compound A21)

Compound A21 was obtained according to the method described in WO 06/039718, using 3-nitro-5-trifluoromethyl benzoic acid and N-methylpiperazine.
$^1$H NMR (270 MHz, $CDCl_3$) $\delta$ (ppm) 2.29 (s, 3H), 2.34-2.60 (br m, 8H), 3.44 (s, 2H), 3.81 (br s, 2H), 6.78 (s, 1H), 6.83 (s, 1H), 6.95 (s, 1H).
ESI m/z $(M+H)^+$ 274.

Reference Example 22

2-Amino-8-bromo-7-methoxy-6-{2-methyl-5-N-[3-(4-methylpiperazin-1-yl)methyl-5-trifluoromethylpheny 1]carbamoylphenyl}quinazoline (Compound A22)

Compound A22 was obtained in the same manner as in Reference Example 20, using Compound A18 and Compound A21.
$^1$H NMR (270 MHz, $CDCl_3$) $\delta$ (ppm) 2.28 (s, 3H), 2.30 (s, 3H), 2.35-2.60 (br m, 8H), 3.45 (s, 2H), 3.52 (s, 3H), 5.53 (br

s, 2H), 7.37 (s, 1H), 7.45 (d, J = 8.4 Hz, 1H), 7.50 (s, 1H), 7.82 (s, 1H), 7.85-7.93 (m, 2H), 8.00 (s, 1H), 8.24 (s, 1H), 8.92 (s, 1H).
ESI m/z $(M+H)^+$ 643.

Reference Example 23

4-[(4-Methylpiperazin-1-yl)methyl]-3-trifluoromethylaniline (Compound A23)

Compound A23 was obtained in the same manner as in Reference Example 21, using 4-nitro-2-trifluoromethyl benzoic acid.
$^1$H NMR (300 MHz, $CDCl_3$) $\delta$ (ppm) 2.28 (s, 3H), 2.35-2.55 (br m, 8H), 3.53 (s, 2H), 3.76 (br s, 2H), 6.79 (dd, J = 8.2, 2.4 Hz, 1H), 6.92 (d, J = 2.6 Hz, 1H), 7.47 (d, J = 8.4 Hz, 1H).
ESI m/z $(M+H)^+$ 274.

Reference Example 24

2-Amino-8-bromo-7-methoxy-6-[2-methyl-5-N-{4-(4-methylpiperazin-1-yl)methyl-3-trifluoromethylpheny l}carbamoylphenyl]quinazoline (Compound A24)

Compound A24 was obtained in the same manner as in Reference Example 20, using Compound A18 and Compound A23.
$^1$H NMR (270 MHz, $CD_3OD$) $\delta$ (ppm) 2.95 (s, 3H), 3.01 (s, 3H), 3.17 (s, 5H), 4.24 (s, 3H), 4.35 (s, 2H), 8.06 (br s, 2H), 8.32 (d, J = 8.3 Hz, 1H), 8.49 (d, J = 8.6 Hz, 1H), 8.57 (s, 1H), 8.75-8.90 (m, 3H), 8.98 (d, J = 2.0 Hz, 1H), 9.91 (s, 1H), 11.23 (s, 1H).
ESI m/z $(M+H)^+$ 643.

Reference Example 25

2-Amino-8-bromo-7-methoxy-6-{2-methyl-5-N-[2-(1-methylpiperidin-4-yl)oxy-5-trifluoromethylphenyl]c arbamoylphenyl}quinazoline (Compound A25)

Compound A25 was obtained in the same manner as in Reference Example 20, using Compound A18 and 2-(1-methylpiperidin-4-yl)oxy-5-trifluoromethylaniline (WO 06/039718). $^1$H NMR (270 MHz, $CDCl_3$) $\delta$ (ppm) 1.82-1.98 (m, 2H), 2.01-2.14 (m, 2H), 2.19 (s, 3H), 2.25-2.42 (m, 5H), 2.52-2.66 (m, 2H), 3.55 (s, 3H), 4.50-4.61 (m, 1H), 5.47 (br s, 2H), 6.98 (d, J = 8.6 Hz, 1H), 7.29-7.37 (m, 1H), 7.46 (d, J = 8.6 Hz, 1H), 7.56 (s, 1H), 7.82-7.91 (m, 2H), 8.75 (s, 1H), 8.90-8.98 (m, 2H).
ESI m/z $(M+H)^+$ 644.

Reference Example 26

3-Morpholinomethyl-5-trifluoromethylaniline (Compound A26)

Compound A26 was obtained in the same manner as in Reference Example 21, using morpholine.
$^1$H NMR (270 MHz, DMSO-$d_6$) $\delta$ (ppm) 2.30-2.38 (m, 4H), 3.37 (s, 2H), 3.53-3.62 (m, 4H), 5.55 (br s, 2H), 6.71 (s, 1H), 6.73 (s, 1H), 6.78 (s, 1H).
ESI m/z $(M+H)^+$ 261.

Reference Example 27

2-Amino-8-bromo-7-methoxy-6-{2-methyl-5-N-[3-(morpholinomethyl)-5-trifluoromethylphenyl]carbamoy lphenyl}quinazoline (Compound A27)

Compound A27 was obtained in the same manner as in Reference Example 20, using Compound A18 and Compound A26.
$^1$H NMR (300 MHz, $CDCl_3$) $\delta$ (ppm) 2.28 (s, 3H), 2.40-2.50 (m, 4H), 3.54 (s, 3H), 3.67-3.75 (m, 4H), 5.47 (br s, 2H), 7.38 (s, 1H), 7.46 (d, J = 8.1 Hz, 1H), 7.54 (s, 1H), 7.80-7.95 (m, 4H), 8.02 (s, 1H), 8.95 (s, 1H). ESI m/z $(M+H)^+$ 630.

Reference Example 28

3-(Pyrrolidin-1-yl)methyl-5-trifluoromethylaniline (Compound A28)

Compound A28 was obtained in the same manner as in Reference Example 21, using pyrrolidine.
$^1$H NMR (300 MHz, $CDCl_3$) δ (ppm) 1.76-1.83 (m, 4H), 2.47-2.54 (m, 4H), 3.55 (s, 2H), 3.80 (br s, 2H), 6.78 (s, 1H), 6.85 (s, 1H), 6.95 (s, 1H).
ESI m/z $(M+H)^+$ 245.

Reference Example 29

2-Amino-8-bromo-7-methoxy-6-{2-methyl-5-N-[3-(pyrrolidin-1-yl)methyl-5-trifluoromethylphenyl]carba moylphenyl} quinazoline (Compound A29)

Compound A29 was obtained in the same manner as in Reference Example 20, using Compound A18 and Compound A28.
$^1$H NMR (300 MHz, $CDCl_3$) δ (ppm) 1.75-1.83 (m, 4H), 2.28 (s, 3H), 2.49-2.57 (m, 4H), 3.53 (s, 3H), 3.67 (s, 2H), 5.50 (br s, 2H), 7.37 (s, 1H), 7.45 (d, J = 8.1 Hz, 1H), 7.53 (s, 1H), 7.76 (s, 1H), 7.82-7.90 (m, 2H), 8.02 (s, 1H), 8.08 (s, 1H), 8.94 (s, 1H).
ESI m/z $(M+H)^+$ 614.

Reference Example 30

3-Dimethylaminomethyl-5-trifluoromethylaniline (Compound A30)

Compound A30 was obtained in the same manner as in Reference Example 21, using dimethylamine (51% aqueous solution).
$^1$H NMR (270 MHz, $CDCl_3$) δ (ppm) 2.24 (s, 6H), 3.36 (s, 2H), 3.81 (br s, 2H), 6.79 (s, 1H), 6.82 (s, 1H), 6.93 (s, 1H).
ESI m/z $(M+H)^+$ 219.

Reference Example 31

2-Amino-8-bromo-6-{5-N-[3-(dimethylaminomethyl)-5-trifluoromethylphenyl]carbamoyl-2-methylphenyl }-7-methoxyquinazoline (Compound A31)

Compound A31 was obtained in the same manner as in Reference Example 20, using Compound A18 and Compound A30.
$^1$H NMR (270 MHZ, $CDCl_3$) δ (ppm) 2.26 (s, 6H), 2.28 (s, 3H), 3.48 (s, 2H), 3.53 (s, 3H), 5.50 (br s, 2H), 7.36 (s, 1H), 7.45 (d, J = 7.9 Hz, 1H), 7.54 (s, 1H), 7.74 (s, 1H), 7.81-7.90 (m, 2H), 8.02 (s, 1H), 8.07 (s, 1H), 8.95 (s, 1H).
ESI m/z $(M+H)^+$ 588.

Reference Example 32

6-Bromo-2-iodo-7-methoxyquinazoline (Compound A32)

Compound A2 (16.0 g, 65.3 mmol) was dissolved in THF (330 mL), and diiodomethane (53.0 mL, 658 mmol), isoamyl nitrite (26.3 mL, 196 mmol) and copper iodide (3.73 g, 19.6 mmol) were added thereto, followed by stirring at 60°C for 8 hours. The insoluble material of the reaction mixture was filttrated off, and the solvent was evaporated under reduced pressure. To the residue was added hexane, and the resulting crystals were collected by filtration to obtain Compound A32 (13.7 g, 57%).
$^1$H NMR (300 MHz, DMSO-$d_6$) δ (ppm) 4.06 (s, 3H), 7.48 (s, 1H), 8.50 (s, 1H), 9.13 (s, 1H).

Reference Example 33

6-Bromo-7-methoxy-2-(4-tetrahydropyranylamino)quinazoline (Compound A33)

Compound A32 (1.42 g, 3.90 mmol) was dissolved in DMF (20 mL), and triethylamine (0.81 1 mL, 5.85 mmol) and 4-tetrahydropyranylamine (473.6 mg, 4.68 mmol) were added thereto, followed by stirring at 80°C overnight. To the

reaction mixture was added water, and the resulting crystals were collected by filtration to obtain Compound A33 (1.10 g, 84%).
$^1$H NMR (270 MHz, $CDCl_3$) δ (ppm) 1.52-1.68 (m, 2H), 2.06-2.18 (m, 2H), 3.52-3.64 (m, 2H), 4.01 (s, 3H), 3.98-4.06 (m, 2H), 4.10-4.30 (m, 1H), 5.12-5.24 (m, 1H), 6.93 (s, 1H), 7.84 (s, 1H), 8.76 (s, 1H).

Reference Example 34

8-Bromo-7-methoxy-6-[2-methyl-5-N-(3-trifluoromethylphenyl)carbamoylphenyl]-2-(4-tetrahydropyranyla mino)quinazoline (Compound A34)

Compound A34 was obtained in the same manner as in Reference Example 6 and Reference Example 7, using Compound A33 and Compound A9.
$^1$H NMR (270 MHz, $CDCl_3$) δ (ppm) 1.58-1.74 (m, 2H), 2.12-2.30 (m, 2H), 2.28 (s, 3H), 3.53 (s, 3H), 3.56-3.70 (m, 2H), 4.00-4.11 (m, 2H), 4.20-4.38 (m, 1H), 5.38-5.54 (m, 1H), 7.38-7.52 (m, 3H), 7.50 (s, 1H), 7.80-8.03 (m, 5H), 8.89 (s, 1H).

Reference Example 35

6-Bromo-7-methoxy-2-[2-(morpholino)ethylamino]quinazoline (Compound A35)

Compound A35 was obtained in the same manner as in Reference Example 33, using 2-(morpholino)ethylamine.
$^1$H NMR (270 MHz, $CDCl_3$) δ (ppm) 2.47-2.58 (m, 4H), 2.62-2.70 (m, 2H), 3.56-3.66 (m, 2H), 3.70-3.80 (m, 4H), 4.00 (s, 3H), 5.82-5.92 (m, 1H), 6.95 (s, 1H), 7.84 (s, 1H), 8.76 (s, 1H).

Reference Example 36

8-Bromo-7-methoxy-6-[2-methyl-5-N-(3-trifluoromethylphenyl)carbamoylphenyl]-2-[2-(morpholino)ethyl amino]quinazoline (Compound A36)

Compound A36 was obtained in the same manner as in Reference Example 6 and Reference Example 7, using Compound A35 and Compound A9.
$^1$H NMR (300 MHz, $CDCl_3$) δ (ppm) 2.28 (s, 3H), 2.54-2.66 (m, 4H), 2.70-2.80 (m, 2H), 3.46-3.56 (m, 2H), 3.53 (s, 3H), 3.70-3.84 (m, 4H), 6.14-6.22 (m, 1H), 7.37-7.54 (m, 4H), 7.80-7.98 (m, 4H), 8.13 (s, 1H), 8.88 (s, 1H).

Reference Example 37

2-Amino-7-hydroxy-6-[2-methyl-5-N-(3-trifluoromethylphenyl)carbamoylphenyl]quinazoline (Compound A3 7)

Compound A10 (5.56 g, 12.3 mmol) was dissolved in DMF (120 mL), and potassium *tert*-butoxide (6.89 mg, 61.5 mmol) and 1-dodecanethiol (14.6 mL, 61.5 mmol) were added thereto, followed by stirring at 100°C for 5 hours under argon atmosphere. To the reaction mixture was added water, the pH was adjusted to 7 using 6 mol/L hydrochloric acid, and then the precipitated crystals were separated by filtration. The crystals were reslurried with ethyl acetate to obtain Compound A37 (3.41 g, 63%).
$^1$H NMR (270 MHz, DMSO-$d_6$) δ (ppm) 2.22 (s, 3H), 6.70 (br s, 2H), 6.87 (s, 1H), 7.44-7.46 (m, 2H), 7.56-7.62 (m, 2H), 7.88-7.95 (m, 3H), 8.09 (d, J = 8.9 Hz, 1H), 8.24 (s, 1H), 8.90 (s, 1H), 10.47 (s, 1H).

Reference Example 38

2-Amino-7-hydroxy-8-iodo-6-[2-methyl-5-N-(3-trifluoromethylphenyl)carbamoylphenyl]quinazoline (Co mpound A38)

Compound A37 (3.41 g, 7.79 mmol) was dissolved in dichloromethane (160 mL), and bis(2,4,6-trimethylpyridine)iodonium (I) hexafluorophosphate (4.40 g, 8.57 mmol) was added thereto, followed by stirring at room temperature for 2 hours. The solvent was evaporated under reduced pressure, and the residue was reslurried with methanol to obtain Compound A38 (3.38 g, 77%).
$^1$H NMR (300 MHz, DMSO-$d_6$) δ (ppm) 2.19 (s, 3H), 7.01 (br s, 2H), 7.43-7.50 (m, 2H), 7.57-7.62 (m, 2H), 7.90-7.97 (m, 2H), 8.09 (d, J = 8.4 Hz, 1H), 8.24 (s, 1H), 8.86 (br s, 1H), 9.61 (br s, 1H), 10.46 (s, 1H). ESI m/z $(M+H)^+$ 565.

Reference Example 39

2-Amino-8-iodo-7-methoxy-6-[2-methyl-5-N-(3-trifluoromethylphenyl)carbamoylphenyl]quinazoline (Co mpound A39)

Compound A38 (3.05 g, 5.40 mmol) was dissolved in THF (110 mL), triphenylphosphine (2.83 g, 10.8 mmol) and methanol (1.09 mL, 27.0 mmol) were added thereto, then diethyl azodicarbonate (2.2 mol/L toluene solution: 4.91 mL, 10.8 mmol) was added dropwise thereinto under ice-cooling" followed by stirring at room temperature for 3 hours. The solvent was evaporated under reduced pressure, and the residue was purified by silica gel column chromatography (ethyl acetate/hexane = 6/4) to obtain Compound A39 (1.30 g, 42%).
$^1$H NMR (300 MHz, $CDCl_3$) $\delta$ (ppm) 2.28 (s, 3H), 3.47 (s, 3H), 5.46 (s, 2H), 7.36-7.52 (m, 3H), 7.51 (s, 1H), 7.80-8.00 (m, 4H), 8.11 (s, 1H), 8.94 (s, 1H).

Reference Example 40

7-Acetyloxy-2-amino-8-iodo-6-[2-methyl-5-N-(3-trifluoromethylphenyl)carbamoylphenyl]quinazoline (C ompound A40)

Compound A38 (564 mg, 1.00 mmol) was dissolved in dichloromethane (10 mL), and triethylamine (0.277 mL, 2.00 mmol) and acetate anhydride (0.283 mL, 3.00 mmol) were added thereto, followed by stirring at room temperature for 4 hours. The solvent was evaporated under reduced pressure, and to the residue were added water and ethyl acetate. The organic layer was separated, and washed with saturated aqueous sodium bicarbonate and brine, followed by drying over anhydrous magnesium sulfate. The solvent was evaporated under reduced pressure, and the residue was purified by silica gel column chromatography (ethyl acetate) to obtain Compound A40 (470 mg, 78%).
$^1$H NMR (270 MHz, $CDCl_3$) $\delta$ (ppm) 2.01 (s, 3H), 2.25 (s, 3H), 5.57 (s, 2H), 7.35-7.52 (m, 3H), 7.59 (s, 1H), 7.65-7.70 (m, 1H), 7.83-8.00 (m, 3H), 8.07 (s, 1H), 8.92 (s, 1H).

Reference Example 41:

2-Amino-8-bromo-7-methoxy-6-[2-methyl-5-N-(2-piperidino-5-trifluoromethylphenyl)carbamoylphenyl]q uinazoline (Compound A41)

Compound A18 (1.94 g, 5.00 mmol) was dissolved in DMF (10 mL), and 1-ethyl-3-(3-dimethylaminopropyl)carbodiimide hydrochloride (2.88 g, 15.0 mmol), 4-dimethylaminopyridine (0.915 g, 7.50 mmol) and 2-piperidino-5-trifluoromethylaniline (WO 06/039718) (1.46 g, 6.00 mmol) were added thereto, followed by stirring at 60°C for 3 hours. To the reaction mixture was added saturated aqueous sodium bicarbonate, followed by extraction with ethyl acetate/isopropanol (5/1), and the organic layer was washed with saturated brine, followed by drying over anhydrous sodium sulfate. The solvent was evaporated under reduced pressure, and the residue was purified by silica gel column chromatography (ethyl acetate) to obtain Compound A41(0.840 g, 27%).
$^1$H NMR (300 MHz, DMSO-$d_6$) $\delta$ (ppm) 1.44-1.56 (m, 2H), 1.57-1.69 (m, 4H), 2.25 (s, 3H), 2.82-2.91 (m, 4H), 3.44 (s, 3H), 7.28 (br s, 2H), 7.39 (d, J = 8.4 Hz, 1H), 7.49 (d, J = 8.4 Hz, 1H), 7.58 (d, J = 8.1 Hz, 1H), 7.78 (s, 1H), 7.90 (s, 1H), 7.94 (d, J = 8.1 Hz, 1H), 8.42 (s, 1H), 9.09 (s, 1H), 9.68 (s, 1H).
ESI m/z $(M+H)^+$ 614.

Reference Example 42

2-Amino-8-bromo-7-methoxy-6-{2-methyl-5-N-[2-(4-methylpiperazin-1-yl)-5-trifluoromethylphenyl]carba moylphenyl} quinazoline (Compound A42)

Compound A42 was obtained in the same manner as in Reference Example 41, using 2-(4-methylpiperazin-1-yl)-5-trifluoromethylaniline (WO 06/039718).
$^1$H NMR (270 MHz, $CDCl_3$) $\delta$ (ppm) 2.24 (s, 3H), 2.31 (s, 3H), 2.40-2.67 (br m, 4H), 2.88-3.02 (br m, 4H), 3.55 (s, 3H), 5.53 (br s, 2H), 7.27-7.40 (m, 2H), 7.50 (d, J = 7.9 Hz, 1H), 7.58 (s, 1H), 7.82-7.97 (m, 2H), 8.90 (s, 1H), 8.96 (s, 1H), 9.47 (s, 1H).
ESI m/z $(M+H)^+$ 629.

Reference Example 43

2-Amino-8-bromo-7-methoxy-6-[2-methyl-5-N-(2-morpholino-5-trifluoromethylphenyl)carbamoylphenyl] quinazoline (Compound A43)

Compound A43 was obtained in the same manner as in Reference Example 41, using 2-morpholino-5 -trifluoromethylaniline (WO 06/039718).
$^1$H NMR (300 MHz, DMSO-$d_6$) $\delta$ (ppm) 2.22 (s, 3H), 2.87-2.96 (m, 4H), 3.43 (s, 3H), 3.66-3.76 (m, 4H), 7.24 (br s, 2H), 7.40 (d, J = 8.4 Hz, 1H), 7.46-7.58 (m, 2H), 7.77 (s, 1H), 7.85-7.96 (m, 2H), 8.34 (s, 1H), 9.08 (s, 1H), 9.76 (s, 1H).
ESI m/z $(M+H)^+$ 616.

Reference Example 44

2-Amino-8-bromo-7-methoxy-6-{2-methyl-5-N-[2-(pyrrolidin-1-yl)-5-trifluoromethylphenyl]carbamoylph enyl}quinazoline (Compound A44)

Compound A44 was obtained in the same manner as in Reference Example 41, using 2-(pyrrolidin-1-yl)-5-trifluoromethylaniline (WO 06/039718).
$^1$H NMR (270 MHz, $CDCl_3$) $\delta$ (ppm) 1.92-2.01 (m, 4H), 2.29 (s, 3H), 3.10-3.20 (m, 4H), 3.55 (s, 3H), 5.53 (br s, 2H), 7.15 (d, J = 8.6 Hz, 1H), 7.35 (d, J = 8.3 Hz, 1H), 7.46 (d, J = 7.6 Hz, 1H), 7.56 (s, 1H), 7.80-7.89 (m, 2H), 8.51 (s, 1H), 8.77 (s, 1H), 8.95 (s, 1H).
ESI m/z $(M+H)^+$ 600.

Reference Example 45

2-Amino-8-bromo-7-methoxy-6-{2-methyl-5-N-[3-(4-methyl-1H-imidazol-1-yl)-5-trifluoromethylphenyl]c arbamoylphenyl}quinazoline (Compound A45)

Compound A45 was obtained in the same manner as in Reference Example 41, using Compound A18 and 3-(4-methyl-1H-imidazol-1-yl)-5-trifluoromethylaniline.
$^1$H NMR (270 MHz, $CDCl_3$) $\delta$ (ppm) 1.25 (s, 3H), 2.27 (s, 3H), 3.48 (s, 3H), 5.58 (br s, 2H), 7.04 (s, 1H), 7.32 (s, 1H), 7.41 (s, 1H), 7.46 (d, J = 8.6 Hz, 1H), 7.77 (s, 1H), 7.98-8.06 (m, 3H), 8.22 (s, 1H), 8.86 (s, 1H), 9.37 (s, 1H).
ESI m/z $(M+H)^+$ 611.

Reference Example 46

2-Amino-8-bromo-6-{5-N-[3-(1H-imidazol-1-yl)-5-trifluoromethylphenyl]carbamoyl-2-methylphenyl}-7-methoxyquinazoline (Compound A46)

Compound A46 was obtained in the same manner as in Reference Example 41, using Compound A18 and 3-(1H-imidazol-1-yl)-5-trifluoromethylaniline.
$^1$H NMR (270 MHz, $CDCl_3$) $\delta$ (ppm) 1.69 (s, 3H), 2.28 (s, 3H), 3.50 (s, 3H), 5.48 (br s, 2H), 7.21 (s, 1H), 7.33 (s, 1H), 7.39 (s, 1H), 7.44-7.51 (m, 2H), 7.88 (s, 1H), 7.94-8.02 (m, 3H), 8.24-8.28 (m, 1H), 8.91 (s, 1H), 8.91 (s, 1H).
ESI m/z $(M+H)^+$ 597.

Reference Example 47

7-Acetyloxy-8-bromo-2-methylamino-6-[2-methyl-5-N-(3-trifluoromethylphenyl)carbamoylphenyl]quinaz oline (Compound A47)

Compound A47 was obtained in the same manner as in Reference Example 37 and Reference Example 40, using Compound A16.
**[0136]** [0109] ESI m/z $(M+H)^+$ 573.

Reference Example 48

6-Bromo-2-cyclopropylamino-7-hydroxyquinazoline (Compound A48)

Compound A48 was obtained in the same manner as in Reference Example 33 and Reference Example 37, using Compound A32 and cyclopropylamine.
$^1$H NMR (270 MHz, DMSO-$d_6$) δ (ppm) 0.42-0.53 (m, 2H), 0.63-0.75 (m, 2H), 2.70-2.88 (m, 1H), 6.93 (s, 1H), 7.49 (br s, 1H), 8.00 (s, 1H), 8.85 (s, 1H), 11.26 (s, 1H).

Reference Example 49

7-Benzyloxy-6-bromo-2-cyclopropylaminoquinazoline (Compound A49)

Compound A48 (0.367 g, 1.32 mmol) was dissolved in DMF (2.6 mL), and potassium carbonate (0.365 g, 2.64 mmol) and benzyl bromide (0.24 mL, 2.0 mmol) were added thereto, followed by stirring at room temperature for 1 hour. To the reaction mixture was added water, followed by extraction with ethyl acetate, and the organic layer was washed with water and brine, followed by drying over anhydrous magnesium sulfate. The solvent was evaporated under reduced pressure, and the residue was reslurried with hexane to obtain compound 49(0.436 g, 89%).
$^1$H NMR (270 MHz, $CDCl_3$) δ (ppm) 0.55-0.67 (m, 2H), 0.84-0.95 (m, 2H), 2.81-2.98 (m, 1H), 5.27 (s, 2H), 5.44 (br s, 1H), 7.09 (s, 1H), 7.34-7.56 (m, 5H), 7.89 (s, 1H), 8.79 (s, 1H).

Reference Example 50

7-Benzyloxy-2-cyclopropylamino-6-[2-methyl-5-N-(3-trifluoromethylphenyl)carbamoylphenyl]quinazolin e (Compound A50)

Compound A50 was obtained in the same manner as in Reference Example 6, using Compound A49 and Compound A9.
$^1$H NMR (270 MHz, $CDCl_3$) δ (ppm) 0.52-0.62 (m, 2H), 0.82-0.92 (m, 2H), 2.23 (s, 3H), 2.84-2.94 (m, 1H), 5.17 (s, 2H), 5.42 (d, J = 8.9 Hz, 1H), 7.12-7.52 (m, 10H), 7.68-7.72 (m, 1H), 7.80-7.94 (m, 3H), 8.18 (s, 1H), 8.80 (s, 1H).

Reference Example 51

2-Cyclopropylamino-7-hydroxy-6-[2-methyl-5-N-(3-trifluoromethylphenyl)carbamoylphenyl]quinazoline (Compound A51)

Compound A50 (100 mg, 0.18 mmol) was suspended in ethanol (1.4 mL) and DMF (1.4 mL). Under argon atmosphere, ammonium formate (164 mg, 2.6 mmol) and palladium-carbon (50% water-containing, 20 mg) were added thereto in this order, followed by stirring at 60°C for 1 hour. The insoluble materials were filtered through celite, and the solvent was evaporated under reduced pressure to obtain Compound A51 (90.5 mg, 100%).
ESI m/z $(M+H)^+$ 479.

Reference Example 52

7-Acetyloxy-2-cyclopropylamino-8-iodo-6-[2-methyl-5-N-(3-trifluoromethylphenyl)carbamoylphenyl]quin azoline (Compound A52)

Compound A52 was obtained in the same manner as in Reference Example 38 and Reference Example 40, using Compound A51.
ESI m/z $(M+H)^+$ 647.

Reference Example 53

2-Amino-8-bromo-7-methoxy-6-[2-methyl-5-N-(4-trifluoromethylpyridin-2-yl)carbamoylphenyl]quinazoli ne (Compound A53)

Compound A18 (0.72 g, 1.85 mmol) was dissolved in thionyl chloride (9.3 mL), followed by stirring at 60°C for 2 hours, and thionyl chloride was evaporated under reduced pressure. The reaction mixture was dissolved in pyridine

(9.3 mL), and 2-amino-4-trifluoromethyl pyridine (0.3 g, 1.85 mmol) was added thereto, followed by stirring at 80°C for 2 hours. To the reaction mixture was added saturated aqueous sodium bicarbonate, followed by extraction with ethyl acetate, and the organic layer was washed with brine, followed by drying over anhydrous magnesium sulfate. The solvent was evaporated under reduced pressure, and the residue was purified by silica gel column chromatography (hexane/ethyl acetate = 1/1) to obtain Compound A53 (0.35 g, 36%).
$^1$H NMR (270 MHz, DMSO-$d_6$) δ (ppm) 2.21 (s, 3H), 3.44 (s, 3H), 7.25 (br s, 2H), 7.46-7.57 (m, 2H), 7.76 (s, 1H), 7.98-8.08 (m, 2H), 8.55 (s, 1H), 8.66 (d, J = 5.0 Hz, 1H), 9.10 (s, 1H), 11.33 (s, 1H).
ESI m/z $(M+H)^+$ 532.

Reference Example 54

2-Amino-8-cyano-7-methoxy-6-[2-methyl-5-N-(4-trifluoromethylpyridin-2-yl)carbamoylphenyl]quinazolin e (Compound A54)

Compound A11 (3.0 g, 5.65 mmol) was dissolved in DMF (28 mL), and copper cyanide (0.76 g, 8.48 mmol) was added thereto, followed by stirring at 150°C for 5 hours under argon air flow. The insoluble materials of the reaction mixture were filtrated off through celite, and saturated aqueous sodium bicarbonate was added thereto, followed by extraction with ethyl acetate. The organic layer was washed with brine, followed by drying over anhydous sodium sulfate. The solvent was evaporated under reduced pressure, and the residue was purified by silica gel column chromatography (chloroform/methanol = 5/1) to obtain Compound A54 (1.3 g, 48%).
$^1$H NMR (270 MHz, DMSO-$d_6$) δ (ppm) 2.21 (s, 3H), 3.80 (s, 3H), 7.40-7.67 (m, 5H), 7.90-8.12 (m, 4H), 8.22 (s, 1H), 9.14 (s, 1H), 10.48 (s, 1H).

Reference Example 55

(Z)-2-Amino-7-methoxy-6-[2-methyl-5-N-(3-trifluoromethylphenyl)carbamoylphenyl]quinazoline-8-(N'-hydroxycarboximidamide) (Compound A55)

Compound A54 (1.26 g, 2.64 mmol) was suspended in ethanol (36 mL), and hydroxyamine·hydrochloride (0.92 g, 8.48 mmol) was added thereto, followed by stirring under heating and reflux for 2 hours. To the reaction mixture was added saturated aqueous sodium bicarbonate, followed by extraction with ethyl acetate, and the organic layer was washed with brine, followed by drying over anhydous sodium sulfate. The solvent was evaporated under reduced pressure, and the residue was purified by silica gel column chromatography (chloroform/methanol = 5/1) to obtain Compound A55 (0.61 g, 45%).
H NMR (270 MHz, DMSO-$d_6$) δ (ppm) 2.22 (s, 3H), 3.58 (s, 3H), 5.78 (br s, 2H), 6.98 (br s, 2H), 7.38-7.54 (m, 2H), 7.54-7.65 (m, 1H), 7.71 (s, 1H), 7.87-8.02 (m, 2H), 8.08 (d, J = 8.3 Hz, 1H), 8.23 (s, 1H), 9.04 (s, 1H), 9.27 (s, 1H), 10.50 (s, 1H).
ESI m/z $(M+H)^+$ 511.

Reference Example 56

(Z)-2-Acetylamino-7-methoxy-6-[2-methyl-5-N-(3-trifluoromethylphenyl)carbamoylphenyl]quinazoline-8-(N'-hydroxycarboximidamide) (Compound A56)

Compound A55 (0.20 g, 0.392 mmol) was dissolved in pyridine (2.0 mL), and acetyl chloride (0.17 mL, 2.35 mmol) was added thereto under ice-cooling, followed by stirring at room temperature for 2 hours and then stirring at 90°C for 2 hours. To the reaction mixture was added saturated aqueous sodium bicarbonate, followed by extraction with ethyl acetate, and the organic layer was washed with brine, followed by drying over anhydous sodium sulfate. The solvent was evaporated under reduced pressure, and the residue was purified by silica gel column chromatography (chloroform/methanol = 20/1) to obtain Compound A56 (0.23 g, 94%).
$^1$H NMR (270 MHz, $CDCl_3$, 50°C) δ (ppm) 2.15 (s, 3H), 2.18 (s, 3H), 2.24 (s, 3H), 2.45 (s, 3H), 3.53 (s, 3H), 7.27-7.40 (m, 3H), 7.67 (s, 1H), 7.78-7.96 (m, 3H), 8.03 (s, 1H), 8.88-9.04 (m, 2H), 9.08 (s, 1H), 9.16 (br s, 1H).
ESI m/z $(M+H)^+$ 637.

Reference Example 57

2-Amino-8-bromo-7-hydroxy-6-[2-methyl-5-N-(3-trifluoromethylphenyl)carbamoylphenyl]quinazoline (Compound A57)

Compound A57 was obtained in the same manner as in Reference Example 37, using Compound A11.
$^1$H NMR (300 MHz, DMSO-$d_6$) δ (ppm) 2.20 (s, 3H), 6.74 (br s, 2H), 7.38-7.48 (m 3H), 7.53-7.63 (m, 1H), 7.82-7.98 (m, 2H), 8.09 (d, J = 8.9 Hz, 1H), 8.24 (s, 1H), 8.66 (br s, 1H), 10.44 (s, 1H).

Reference Example 58

2-Amino-7-benzyloxy-8-bromo-6-[2-methyl-5-N-(3-trifluoromethylphenyl)carbamoylphenyl]quinazoline ( Compound A58)

Compound A58 was obtained in the same manner as in Reference Example 49, using Compound A57.
$^1$H NMR (270 MHz, $CDCl_3$) δ (ppm) 2.28 (s, 3H), 4.30-4.80 (m, 2H), 5.46 (s, 2H), 6.87-6.99 (m, 2H), 7.18-7.26 (m, 3H), 7.38-7.50 (m, 3H), 7.58 (s, 1H), 7.70-7.99 (m, 5H), 8.97 (s, 1H).

Reference Example 59

2-Amino-8-bromo-7-methoxy-6-[2-methyl-5-N-(2-trifluoromethylpyridin-6-yl)carbamoylphenyl]quinazoli ne (Compound A59)

Compound A59 was obtained in the same manner as in Reference Example 53, using Compound A18 and 2-amino-6-trifluoromethylpyridine.
$^1$H NMR (270 MHz, DMSO-$d_6$) δ (ppm) 2.21 (s, 3H), 3.45 (s, 3H), 7.25 (br s, 2H), 7.49 (d, J = 7.9 Hz, 1H), 7.65 (d, J = 7.3 Hz, 1H), 7.77 (s, 1H), 7.98-8.19 (m, 3H), 8.47 (d, J = 7.9 Hz, 1H), 9.10 (s, 1H), 11.21 (s, 1H).
ESI m/z $(M+H)^+$ 532.

Reference Example 60

3-(8-Bromo-2-isopropylamino-7-methoxyquinazolin-6-yl)-4-methyl benzoic acid (Compound A60)

Compound A60 was obtained in the same manner as in Reference Example 17 and Reference Example 18, using Compound A3 and Compound A8.
$^1$H NMR (300 MHz, DMSO-$d_6$) δ (ppm) 1.25 (d, J = 9.0 Hz, 6H), 2.18 (s, 3H), 3.40 (s, 3H), 4.15-4.32 (m, 1H), 7.46 (d, J = 6.0 Hz, 1H), 7.58-7.68 (m, 1H), 7.70 (s, 1H), 7.81 (s, 1H), 7.89 (d, J = 6.0 Hz, 1H), 9.04 (s, 1H).

Reference Example 61

8-Bromo-2-isopropylamino-7-methoxy-6-[2-methyl-5-N-(4-trifluoromethylpyridin-2-yl)carbamoylphenyl] quinazoline (Compound A61)

Compound A61 was obtained in the same manner as in Reference Example 53, using Compound A60.
$^1$H NMR (270 MHz, DMSO-$d_6$) δ (ppm) 1.27 (d, J = 6.3 Hz, 6H), 2.21 (s, 3H), 3.45 (s, 3H), 4.16-4.38 (m, 1H), 7.46-7.57 (m, 2H), 7.58-7.70 (m, 1H), 7.74 (s, 1H), 7.98-8.08 (m, 2H), 8.55 (s, 1H), 8.67 (d, J = 5.0 Hz, 1H), 9.07 (s, 1H), 11.33 (s, 1H).
ESI m/z $(M+H)^+$ 574.

Reference Example 62

2-Amino-8-bromo-7-hydroxy-6-[2-methyl-5-N-(4-trifluoromethylpyridin-2-yl)carbamoylphenyl]quinazolin e (Compound A62)

Compound A62 was obtained in the same manner as in Reference Example 37, using Compound A53.
$^1$H NMR (300 MHz, DMSO-$d_6$) δ (ppm) 2.20 (s, 3H), 7.06 (br s, 2H), 7.40-7.62 (m, 3H), 7.90-8.05 (m, 2H), 8.54 (s, 1H), 8.66 (d, J = 5.1 Hz, 1H), 8.84 (s, 1H), 11.25 (s, 1H).
ESI m/z $(M+H)^+$ 518.

Reference Example 63

2-Amino-7-benzyloxy-8-bromo-6-[2-methyl-5-N-(4-trifluoromethylpyridin-2-yl)carbamoylphenyl]quinazo line (Compound A63)

Compound A63 was obtained in the same manner as in Reference Example 49, using Compound A62.
$^1H$ NMR (270 MHz, $CDCl_3$) δ (ppm) 1.61 (s, 3H), 2.25 (s, 2H), 5.52 (br s, 2H), 6.90-7.00 (m, 2H), 7.14-7.25 (m, 3H), 7.28-7.33 (m, 1H), 7.43 (d, J = 8.3 Hz, 1H), 7.57 (s, 1H), 7.73 (d, J = 2.0 Hz, 1H), 7.87-7.94 (m, 1H), 8.48 (d, J = 5.3 Hz, 1H), 8.64-8.72 (m, 2H), 8.97 (s, 1H).
ESI m/z $(M+H)^+$ 608.

Reference Example 64

4-Methyl-3-(4,4,5,5-tetramethyl-1,3,2-dioxaboran-2-yl)-N-(4-trifluoromethylpyrimidin-6-yl)benzamide (Compound A64)

Compound A64 was obtained in the same manner as in Reference Example 9, using the 4-amino-6-trifluoromethylpyrimidine and Compound A8.
H NMR (300 MHz, $CDCl_3$) δ (ppm) 1.38 (s, 12H), 2.63 (s, 3H), 7.35 (d, J = 8.4 Hz, 1H), 7.95 (dd, J = 8.4, 2.2 Hz, 1H), 8.24 (d, J = 2.2 Hz, 1H), 8.75 (s, 1H), 8.87 (s, 1H), 9.03 (s, 1H).

Reference Example 65

2-Amino-8-bromo-7-methoxy-6-[2-methyl-5-N-(4-trifluoromethylpyrimidin-6-yl)carbamoylphenyl]quinaz oline (Compound A65)

Compound A65 was obtained in the same manner as in Reference Example 6 and Reference Example 7, using Compound A64 and Compound A2.
$^1H$ NMR (300 MHz, $CDCl_3$) δ (ppm) 2.30 (s, 3H), 3.54 (s, 3H), 5.48 (s, 2H), 7.50 (d, J = 7.7 Hz, 1H), 7.55 (s, 1H), 7.87-7.92 (m, 2H), 8.72 (d, J = 1.5 Hz, 1H), 8.82 (s, 1H), 8.96 (s, 1H), 9.02 (s, 1H).

Reference Example 66

N-[4-Methyl-3-(4,4,5,5-tetramethyl-1,3,2-dioxaboran-2-yl)phenyl]-4-(trifluoromethyl)picolinamide (Comp ound A66)

Compound A66 was obtained in the same manner as in Reference Example 5 using the 4-trifluoromethyl-2-picolinic acid and Compound A4.
$^1H$ NMR (300 MHz, $CDCl_3$) δ (ppm) 1.36 (s, 13H), 2.54 (s, 3H), 7.70 (d, J = 5.0 Hz, 1H), 7.80 (d, J = 2.6 Hz, 1H), 8.09 (dd, J = 8.4, 2.6 Hz, 1H), 8.54 (s, 1H), 8.80 (d, J = 5.0 Hz, 1H), 9.89 (s, 1H).

Reference Example 67

2-Amino-8-bromo-7-methoxy-6-[2-methyl-5-(4-trifluoromethylpicolinamide)phenyl]quinazoline (Compou nd A67)

Compound A67 was obtained in the same manner as in Reference Example 6 and Reference Example 7, using Compound A66 and Compound A2.
$^1H$ NMR (300 MHz, $CDCl_3$) δ (ppm) 2.20 (s, 3H), 3.58 (s, 3H), 5.42 (s, 2H), 7.35 (d, J = 8.1 Hz, 1H), 7.57 (s, 1H), 7.71-7.78 (m, 3H), 8.53 (s, 1H), 8,81 (d, J = 5.1 Hz, 1H), 8.94 (s, 1H), 9.94 (s, 1H).

Reference Example 68

(S)-6-Bromo-2-(1-hydroxypropan-2-yl amino)-7-methoxyquinazoline (Compound A68)

Compound A68 was obtained in the same manner as in Reference Example 33, using (S)-2-aminopropan-1-ol and Compound A32.
$^1H$ NMR (270 MHz, $CDCl_3$) δ (ppm) 1.32 (d, J = 6.9 Hz, 3H), 3.70 (dd, J = 10.9, 7.2 Hz, 1H), 3.83 (dd, J = 10.9, 2.6 Hz, 1H), 4.00 (s, 3H), 4.19-4.28 (m, 1H), 5.37 (br s, 1H), 6.90 (s, 1H), 7.85 (s, 1H), 8.76 (s, 1H).

Reference Example 69

4-Methyl-3-(4,4,5,5-tetramethyl-1,3,2-dioxaboran-2-yl)-N-(4-trifluoromethylpyridin-2-yl)benzamide (Compound A69)

Compound A69 was obtained in the same manner as in Reference Example 9, using the 2-amino-4-trifluoromethylpyridine and Compound A8.
$^1$H NMR (270 MHz, $CDCl_3$) δ (ppm) 1.37 (s, 12H), 2.62 (s, 3H), 7.29 (d, J = 5.4 Hz, 2H), 7.33 (d, J = 8.0 Hz, 2H), 7.96 (dd, J = 8.0, 2.2 Hz, 1H), 8.26 (d, J = 2.2 Hz, 1H), 8.49 (d, J = 5.4 Hz, 1H), 8.74 (s, 1H), 8.79 (s, 1H).

Reference Example 70

(S)-8-Bromo-2-(1-hydroxypropan-2-ylamino)-7-methoxy-6-[2-methyl-5-N-(4-trifluoromethylpyridin-2-yl)carbamoylphenyl] quinazoline (Compound A70)

Compound A70 was obtained in the same manner as in Reference Example 6 and Reference Example 7, using Compound A68 and Compound A69.
$^1$H NMR (300 MHz, $CDCl_3$) δ (ppm) 1.38 (d, J = 6.9 Hz, 3H), 2.27 (s, 3H), 3.53 (s, 3H), 3.78-3.83 (m, 1H), 3.89-3.96 (m, 1H), 4.33-4.41 (m, 1H), 5.63 (d, J = 6.3 Hz, 1H), 7.30 (d, J = 4.6 Hz, 1H), 7.47 (d, J = 8.3 Hz, 1H), 7.53 (s, 1H), 7.86-7.92 (m, 2H), 8.48 (d, J = 5.3 Hz, 1H), 8.70 (s, 1H), 8.75 (s, 1H), 8.90 (s, 1H).

Reference Example 71

8-Bromo-2-isopropylamino-7-methoxy-6-[2-methyl-5-N-(4-trifluoromethylpyrimidin-2-yl)carbamoylphen yl]quinazoline (Compound A71)

Compound A71 was obtained in the same manner as in Reference Example 53, using Compound A60 and 2-amino-4-trifluoromethylpyrimidine.
$^1$H NMR (300 MHz, $CDCl_3$) δ (ppm) 1.36 (d, J = 6.2 Hz, 6H), 2.27 (s, 3H), 3.53 (s, 3H), 4.30-4.50 (m, 1H), 5.40 (br s, 1H), 7.39 (d, J = 5.1 Hz, 1H), 7.41-7.52 (m, 2H), 7.85-7.97 (m, 2H), 8.83-8.92 (m, 2H), 8.98 (d, J=4.8Hz, 1H).

Reference Example 72

4-Methyl-3-(4,4,5,5-tetramethyl-1,3,2-dioxaboran-2-yl)-N-(4-trifluoromethylpyrimidin-2-yl)benzamide (Compound A72)

Compound A72 was obtained in the same manner as in Reference Example 9, using Compound A8 and 2-amino-4-trifluoromethylpyrimidine.
$^1$H NMR (270 MHz, $CDCl_3$) δ (ppm) 1.38 (s, 12H), 2.62 (s, 3H), 7.32 (d, J = 7.9 Hz, 1H), 7.38 (d, J = 5.0 Hz, 1H), 7.97-8.03 (m, 1H), 8.22-8.26 (m, 1H), 8.89 (br s, 1H), 8.98 (d, J = 5.0 Hz, 1H).

Reference Example 73

2-Amino-8-bromo-7-methoxy-6-[2-methyl-5-N-(4-trifluoromethylpyrimidin-2-yl)carbamoylphenyl]quinaz oline (Compound A73)

Compound A73 was obtained in the same manner as in Reference Example 6 and Reference Example 7, using Compound A72 and Compound A2.
$^1$H NMR (300 MHz, $CDCl_3$) δ (ppm) 2.27 (s, 3H), 3.53 (s, 3H), 5.66 (br s, 2H), 7.35-7.59 (m, 3H), 7.87-8.01 (m, 2H), 8.90-9.05 (m, 3H).

Example 1

2-Amino-7-methoxy-6-[2-methyl-5-(3-trifluoromethylbenzamide)phenyl]-8-phenylquinazoline (Compoun d 1)

Compound A7 (49 mg, 0.093 mmol) was dissolved in dioxane (0.6 mL) and water (0.6 mL), and phenyl boronic acid (13.5 mg, 0.111 mmol), sodium carbonate (19.7 mg, 0.186 mmol) and [1,1'-bis(diphenylphosphino)ferrocene]dichloropalladium (3.8 mg, 0.0047 mmol) were added thereto, followed by stirring for 5.5 hours under heating and reflux. To the reaction mixture was added water and ethyl acetate, and the organic layer was separated, followed by washing with water. The organic layer was dried over anhydrous magnesium sulfate, and the solvent was evaporated under reduced

pressure. The residue was purified by preparative thin layer chromatography (ethyl acetate/hexane = 1/1) to obtain Compound 1 (32.8 mg, 67%).
$^1$H NMR (300 MHz, $CDCl_3$) δ (ppm) 2.26 (s, 3H), 3.17 (s, 3H), 5.14 (s, 2H), 7.26-7.70 (m, 10H), 7.79-7.86 (m, 2H), 8.07 (d, J = 7.8 Hz, 1H), 8.13 (s, 1H), 8.97 (s, 1H).
ESI m/z $(M+H)^+$ 529.

Example 2

2-Amino-7-methoxy-6-[2-methyl-5-(3-trifluoromethylbenzamide)phenyl]-8-(2-pyridyl)quinazoline (Comp ound 2)

Compound A7 (186 mg, 0.350 mmol) was dissolved in DMF (1.9 mL) under argon atmosphere, and 2-(tributylstannyl)pyridine (258 mg, 0.700 mmol) and tetrakis(triphenylphosphine)palladium (20.2 mg, 0.0175 mmol) were added thereto, followed by stirring at 140°C for 4 hours. To the reaction mixture were added water and ethyl acetate, and the organic layer was separated, followed by washing with water. The organic layer was dried over anhydrous magnesium sulfate, and the solvent was evaporated under reduced pressure. The residue was purified by silica gel column chromatography (ethyl acetate/methanol = 4/1) to obtain Compound 2 (82.4 mg, 44%).
$^1$H NMR (300 MHz, $CDCl_3$) δ (ppm) 2.23 (s, 3H), 3.17 (s, 3H), 5.11 (s, 2H), 7.28-7.37 (m, 2H), 7.45-7.51 (m, 2H), 7.57 (s, 1H), 7.60 (d, J = 7.8 Hz, 1H), 7.70-7.84 (m, 3H), 8.07 (d, J = 8.1 Hz, 1H), 8.16 (s, 1H), 8.22 (br s, 1H), 8.77-8.82 (m, 1H), 8.93 (s, 1H).
ESI m/z $(M+H)^+$ 530.
Melting point 228-230°C.

Example 3

2-Amino-8-(4-fluorophenyl)-7-methoxy-6-[2-methyl-5-(3-trifluoromethylbenzamide)phenyl]quinazoline ( Compound 3)

Compound 3 was obtained in the same manner as in Example 1, using Compound A7 and 4-fluorophenyl boronic acid.
$^1$H NMR (300 MHz, $CDCl_3$) δ (ppm) 2.25 (s, 3H), 3.16 (s, 3H), 5.14 (s, 2H), 7.13-7.22 (m, 2H), 7.33 (d, J = 8.1 Hz, 1H), 7.48-7.70 (m, 6H), 7.78-7.85 (m, 2H), 8.07 (d, J = 8.2 Hz, 1H), 8.12 (s, 1H), 8.97 (s, 1H).
ESI m/z $(M+H)^+$ 547.
Melting point 134-138°C.

Example 4

2-Amino-7-hydroxy-6-[2-methyl-5-(3-trifluoromethylbenzamide)phenyl]-8-(2-pyridyl)quinazoline (Comp ound 4)

Compound 2 (100 mg, 0,189 mmol) was dissolved in DMF (3.3 mL), potassium *tert*-butoxide (424 mg, 3.78 mmol) and 1-dodecanethiol (0.91 mL, 3.78 mmol) were added thereto, then the mixture was stirred at 80°C for 2 hours under argon atmosphere. To the reaction mixture was added 1 mol/L hydrochloric acid to adjust the pH to 7, ethyl acetate was added thereto, and the organic layer was separated. The organic layer was washed with water and brine, followed by drying over anhydrous magnesium sulfate, and then the solvent was evaporated under reduced pressure. The residue was reslurried with hexane to obtain Compound 4(81.2 mg, 83%).
$^1$H NMR (300 MHz, $CDCl_3$) δ (ppm) 2.23 (s, 3H), 5.19 (s, 2H), 7.26-7.36 (m, 2H), 7.54 (s, 1H), 7.53-7.57 (m, 1H), 7.58-7.68 (m, 2H), 7.76-7.85 (m, 2H), 7.92-8.00 (m, 1H), 8.05 (d, J = 7.8 Hz, 1H), 8.12 (s, 1H), 8.38-8.43 (m, 1H), 8.82 (s, 1H), 9.70-9.76 (m, 1H).
ESI m/z $(M+H)^+$ 516.
Melting point 274-278°C.

Example 5

2-Amino-8-(4-fluorophenyl)-7-methoxy-6-[2-methyl-5-N-(3-trifluoromethylphenyl)carbamoylphenyl]quin azoline (Compound 5)

Compound 5 was obtained in the same manner as in Example 1, using Compound A11 and 4-fluorophenyl boronic acid.
$^1$H NMR (270 MHz, $CDCl_3$) δ (ppm) 2.34 (s, 3H), 3.12 (s, 3H), 5.14 (s, 2H), 7.10-7.24 (m, 2H), 7.38-7.60 (m, 6H), 7.80-8.01 (m, 5H), 8.98 (s, 1H).

ESI m/z $(M+H)^+$ 547.
Melting point 217-218°C.

Example 6

2-Amino-7-methoxy-6- [2-methyl-5-N-(3-trifluoromethylphenyl)carbamoylphenyl]-8-(2-pyridyl)quinazolin e (Compound 6)

Compound A11 (2.0 g, 3.76 mmol) was dissolved in DMF (19 mL), and 2-(tributylstannyl)pyridine (1.4 mL, 4.51 mmol), lithium chloride (478 mg, 11.3 mmol) and tetrakis(triphenylphosphine)palladium (217 mg, 0.19 mmol) were added thereto, and followed by stirring at 120°C for 5 hours under argon atmosphere. To the reaction mixture was added water and chloroform/isopropanol (5/1), and the organic layer was separated. The organic layer was washed with water and brine, followed by drying over anhydrous magnesium sulfate. The solvent was evaporated under reduced pressure, followed by purification with silica gel column chromatography (ethyl acetate/methanol = 5/1) to obtain Compound 6 (639 mg, 32%).
$^1$H NMR (300 MHz, $CDCl_3$) $\delta$ (ppm) 2.28 (s, 3H), 3.15 (s, 3H), 5.04 (s, 2H), 7.28-7.44 (m, 4H), 7.51 (s, 1H), 7.50-7.56 (m, 1H), 7.78-7.90 (m, 4H), 7.96 (s, 1H) 8.69 (s, 1H), 8.72-8.78 (m, 1H), 8.90 (s, 1H).
ESI m/z $(M+H)^+$ 530.
Melting point 251-253°C.

Example 7

2-Amino-7-methoxy-6-[2-methyl-5-(3-trifluoromethylbenzamide)phenyl]-8-(4-pyridyl)quinazoline (Comp ound 7)

Compound 7 was obtained in the same manner as in Example 1, using Compound A7 and 4-pyridine boronic acid.
$^1$H NMR (300 MHz, $CDCl_3$) $\delta$ (ppm) 2.25 (s, 3H), 3.19 (s, 3H), 5.14 (s, 2H), 7.33 (d, J = 8.4 Hz, 1H), 7.46-7.72 (m, 6H), 7.82 (d, J = 7.8 Hz, 1H), 7.92 (s, 1H), 8.08 (d, J = 7.8 Hz, 1H), 8.13 (s, 1H) 8.69-8.74 (m, 2H), 8.99 (s, 1H).
ESI m/z $(M+H)^+$ 530.
Melting point 214-216°C.

Example 8

2-Amino-7-methoxy-6-[2-methyl-5-(3-trifluoromethylbenzamide)phenyl]-8-[4-(4-methylpiperazin-1-yl)phenyl]quinazoline (Compound 8)

Compound 8 was obtained in the same manner as in Example 1, using Compound A7 and 1-methyl-4-[4-(4,4,5,5-tetramethyl-1,3,2-dioxaboran-2-yl)phenyl]piperazine.
$^1$H NMR (300 MHz, $CDCl_3$) $\delta$ (ppm) 2.25 (s, 3H), 2.38 (s, 3H), 2.57-2.65 (m, 4H), 3.17 (s, 3H), 3.29-3.35 (m, 4H), 5.09 (s, 2H), 7.00-7.07 (m, 2H), 7.32 (d, J = 8.1 Hz, 1H), 7.45-7.50 (m, 2H), 7.53 (s, 1H), 7.58-7.68 (m, 3H), 7.78-7.85 (m, 2H), 8.07 (d, J = 7.5 Hz, 1H), 8.13 (br s, 1H), 8.95 (s, 1H).
ESI m/z $(M+H)^+$ 627.
Melting point 178°C.

Example 9

2-Amino-7-methoxy-6-[2-methyl-5-(3-trifluoromethylbenzamide)phenyl]-8-(3-pyridyl)quinazoline (Compound 9)

Compound 9 was obtained in the same manner as in Example 1, using Compound A7 and 3-pyridine boronic acid.
$^1$H NMR (300 MHz, $CDCl_3$) $\delta$ (ppm) 2.26 (s, 3H), 3.18 (s, 3H), 5.12 (s, 2H), 7.34 (d, J = 8.4 Hz, 1H), 7.39-7.44 (m, 1H), 7.55-7.70 (m, 4H), 7.79-7.86 (m, 2H), 7.87-7.94 (m, 1H), 8.08 (d, J = 7.8 Hz, 1H), 8.13 (br s, 1H), 8.63 (dd, J = 5.1, 1.8 Hz, 1H), 8.80-8.83 (m, 1H), 8.99 (s, 1H).
ESI m/z $(M+H)^+$ 530.
Melting point 163-164°C

Example 10

2-Amino-7-hydroxy-6-[2-methyl-5-(3-trifluoromethylbenzamide)phenyl]-8-(4pyridyl)quinazoline (Compound 10)

Compound 10 was obtained in the same manner as in Example 4, using Compound 7. $^1$H NMR (270 MHz, DMSO-$d_6$) δ (ppm) 2.14 (s, 3H), 6.61 (br s, 2H), 7.28 (d, J = 8.6 Hz, 1H), 7.41 (d, J = 5.9 Hz, 2H), 7.57 (s, 1H), 7.68-7.81 (m, 3H), 7.96 (d, J = 7.8 Hz, 1H), 8.21-8.32 (m, 2H), 8.60 (d, J = 5.9 Hz, 2H), 8.94 (s, 1H), 9.00 (br s, 1H), 10.44 (s, 1H). ESI m/z $(M+H)^+$ 516.

Example 11

2-Amino-7-methoxy-6-[2-methyl-5-N-(3-trifluoromethylphenyl)carbamoylphenyl]-8-(4-pyridyl)quinazolin e (Compound 11)

Compound 11 was obtained in the same manner as in Example 1, using Compound A11 and 4-pyridine boronic acid.
$^1$H NMR (300 MHz, $CDCl_3$) δ (ppm) 2.34 (s, 3H), 3.14 (s, 3H), 5.17 (s, 2H), 7.39-7.52 (m, 5H), 7.64 (s, 1H), 7.80-8.10 (m, 5H), 8.72 (d, J = 6.0 Hz, 2H), 9.00 (s, 1H).
ESI m/z $(M+H)^+$ 530.
Melting point 187-188°C.

Example 12

2-Amino-7-hydroxy-6-[2-methyl-5-N-(3-trifluoromethylphenyl)carbamoylphenyl]-8-(4-pyridyl)quinazolin e (Compound 12)

Compound 12 was obtained in the same manner as in Example 4, using Compound 11. $^1$H NMR (270 MHz, DMSO-$d_6$) δ (ppm) 2.24 (s, 3H), 6.65 (s, 2H), 7.38-7.68 (in, 6H), 7.90-8.00 (m, 2H), 8.04-8.12 (m, 1H), 8.23 (s, 1H), 8.61 (d, J = 5.2 Hz, 2H), 8.94 (s, 1H), 9.09 (br s, 1H), 10.44 (s, 1H).
ESI m/z $(M+H)^+$ 516.
Melting point 281-286°C.

Example 13

2-Amino-6-[5-(2-fluoro-5-trifluoromethylbenzamide)-2-methylphenyl]-7-methoxy-8-(4-pyridyl)quinazolin e (Compound 13)

Compound 13 was obtained in the same manner as in Example 1, using Compound A13 and 4-pyridine boronic acid.
$^1$H NMR (270 MHz, $CDCl_3$) δ (ppm) 2.25 (s, 3H), 3.19 (s, 3H), 5.15 (s, 2H), 7.29-7.38 (m, 2H), 7.46-7.52 (m, 2H), 7.54-7.60 (m, 1H), 7.64 (s, 1H), 7.70 (d, J = 2.4 Hz, 1H), 7.74-7.84 (m, 1H), 8.40-8.52 (m, 2H), 8.68-8.74 (m, 2H), 8.99 (s, 1H).
ESI m/z $(M+H)^+$ 548.
Melting point 196°C.

Example 14

2-Amino-7-methoxy-6-[2-methyl-5-N-(3-trifluoromethylphenyl)carbamoylphenyl]-8-(3-pyridyl)quinazolin e (Compound 14)

Compound 14 was obtained in the same manner as in Example 1, using Compound A11 and 3-pyridine boronic acid.
$^1$H NMR (270 MHz, $CDCl_3$) δ (ppm) 2.35 (s, 3H), 3.13 (s, 3H), 5.16 (s, 2H), 7.38-7.54 (m, 4H), 7.62 (s, 1H), 7.80-7.95 (m, 5H), 8.01 (s, 1H), 8.60-8.72 (m, 1H), 8.82 (d, J = 1.6 Hz, 1H), 9.00 (s, 1H).
ESI m/z $(M+H)^+$ 530.
Melting point 211°C.

Example 15

2-Amino-7-hydroxy-6-[2-methyl-5-N-(3-trifluoromethylphenyl)carbamoylphenyl]-8-(2-pyridyl)quinazolin e (Compound 15)

Compound 15 was obtained in the same manner as in Example 4, using Compound 6. $^1H$ NMR (270 MHz, $CDCl_3$) δ (ppm) 2.31 (s, 3H), 5.20 (s, 2H), 7.29-7.54 (m, 6H), 7.77 (d, J =1.9 Hz, 1H), 7.82-8.00 (m, 5H), 8.38-8.44 (m, 1H), 8.80 (s, 1H), 9.76 (d, J = 8.6 Hz, 1H).
ESI m/z $(M+H)^+$ 516.

Example 16

2-Amino-6-(5-{2-[N-(3-dimethylaminopropyl)-N-methylamino]-5-trifluoromethylbenzamide}-2-methylph enyl)-7-methoxy-8-(4-pyridyl)quinazoline (Compound 16)

Compound 13 (80 mg, 0.15 mmol) was dissolved in dimethylsulfoxide (5.6 mL), and N-(dimethylaminopropyl)methylamine (0.024 mL, 0.16 mmol) was added thereto, followed by stirring at 80°C for 72 hours. To the reaction mixture was added water, followed by extraction with ethyl acetate, then the organic layer was washed with brine, followed by drying over anhydrous magnesium sulfate. The solvent was evaporated under reduced pressure, and the residue was purified by preparative thin layer chromatography (chloroform/methanol = 4/1) to obtain Compound 16 (71 mg, 75%).
$^1H$ NMR (270 MHz, $CDCl_3$) δ (ppm) 1.66-1.80 (m, 2H), 2.12 (s, 6H), 2.24 (s, 3H), 2.22-2.30 (m, 2H), 2.86 (s, 3H), 3.19 (s, 3H), 3.14-3.23 (m, 2H), 5.15 (s, 2H), 7.30 (d, J = 8.4 Hz, 1H), 7.39 (d, J = 8.1 Hz, 1H), 7.45-7.53 (m, 3H), 7.65 (s, 1H), 7.66-7.75 (m, 1H), 7.85 (d, J = 2.2 Hz, 1H), 8.53 (d, J = 1.6 Hz, 1H), 8.69-8.76 (m, 2H), 8.99 (s, 1H), 11.93 (s, 1H).
ESI m/z $(M+H)^+$ 644.
Melting point 187-189°C.

Example 17

2-Amino-7-methoxy-6-[2-methyl-5-(2-piperidino-5-triffuoromethylbenzamide)phenyl]-8-(4-pyridyl)quinaz oline (Compound 17)

Compound 17 was obtained in the same manner as in Example 16, using piperidine. $^1H$ NMR (270 MHz, $CDCl_3$) δ (ppm) 1.61-1.71 (m, 2H), 1.80-1.92 (m, 4H), 2.25 (s, 3H), 3.01-3.10 (m, 4H), 3.19 (s, 3H), 5.13 (s, 2H), 7.28-7.40 (m, 2H), 7.47-7.58 (m, 3H), 7.66 (s, 1H), 7.68-7.75 (m, 1H), 7.94 (d, J = 2.2 Hz, 1H), 8.51 (d, J = 2.2 Hz, 1H), 8.69-8.74 (m, 2H), 8.99 (s, 1H), 11.97 (s, 1H).
ESI m/z $(M+H)^+$ 613.
Melting point 154°C.

Example 18

2-Amino-7-methoxy-6-[2-methyl-5-N-(3-trifluoromethylphenyl)carbamoylphenyl]-8-(pyrimidin-2-yl)quina zoline (Compound 18)

Compound 18 was obtained in the same manner as in Example 6, using 2-(tributylstannyl)pyrimidine.
$^1H$ NMR (300 MHz, $CDCl_3$) δ (ppm) 2.33 (s, 3H), 3.27 (s, 3H), 5.07 (s, 2H), 7.34-7.49 (m, 4H), 7.62 (s, 1H), 7.80-7.90 (m, 3H), 7.98 (br s, 1H), 8.17 (br s, 1H), 8.92-8.98 (m, 3H).
ESI m/z $(M+H)^+$ 531.

Example 19

2-Amino-7-methoxy-8-(1-methyl-1H-pyrazol-4-yl)-6-[2-methyl-5-N-(3-trifluoromethylphenyl)carbamoylp henyl]quinazoline (Compound 19)

Compound 19 was obtained in the same manner as in Example 1, using Compound A11 and 1-methyl-4-(4,4,5,5-tetramethyl-1,3,2-dioxaboran-2-yl)-1H-pyrazole.
$^1H$ NMR (300 MHz, $CDCl_3$) δ (ppm) 2.34 (s, 3H), 3.27 (s, 3H), 4.01 (s, 3H), 5.22 (s, 2H), 7.38-7.54 (m 4H), 7.84-8.00 (m, 5H), 8.27-8.29 (m, 2H), 8.98 (s, 1H).

ESI m/z $(M+H)^+$ 533.
Melting point 195°C.

Example 20

2-Amino-8-(2-chloropyridin-5-yl)-7-methoxy-6-[2-methyl-5-N-(3-trifluoromethylphenyl)carbamoylphenyl ]quinazoline (Compound 20)

Compound 20 was obtained in the same manner as in Example 1, using Compound A11 and 2-chloro-5-pyridine boronic acid.
$^1H$ NMR (270 MHz, $CDCl_3$) $\delta$ (ppm) 2.34 (s, 3H), 3.16 (s, 3H), 5.17 (s, 2H), 7.38-7.54 (m, 4H), 7.64 (s, 1H), 7.80-7.98 (m, 6H), 8.62 (d, J = 2.2 Hz, 1H), 9.01 (s, 1H).
ESI m/z $(M+H)^+$ 564.
Melting point 165-167°C.

Example 21

2-Amino-7-methoxy-6-[2-methyl-5-N-(3-trifluoromethylphenyl)carbamoylphenyl]-8-[2-(pyrrolidin-1-yl)py rimidin-5-yl] quinazoline (Compound 21)

Compound 21 was obtained in the same manner as in Example 1, using Compound A11 and 2-(pyrrolidin-1-yl)-5-(4,4,5,5-tetramethyl-1,3,2-dioxaboran-2-yl)pyrimidine.
$^1H$ NMR (270 MHz, $CDCl_3$) $\delta$ (ppm) 1.98-2.23 (m, 4H), 2.33 (s, 3H), 3.22 (s, 3H), 3.60-3.76 (m, 4H), 5.18 (s, 2H), 7.36-7.53 (m, 3H), 7.55 (s, 1H), 7.80-8.08 (m, 5H), 8.66 (s, 2H), 8.97 (s, 1H).
ESI m/z $(M+H)^+$ 600.

Example 22

2-Amino-7-methoxy-8-(2-methoxypyrimidin-5-yl)-6-[2-methyl-5-N-(3-trifluoromethylphenyl)carbamoylp henyl]quinazoline (Compound 22)

Compound 22 was obtained in the same manner as in Example 1, using Compound A11 and 2-methoxy-5-pyrimidine boronic acid.
$^1H$ NMR (300 MHz, $CDCl_3$) $\delta$ (ppm) 2.34 (s, 3H), 3.19 (s, 3H), 4.10 (s, 3H), 5.18 (s, 2H), 7.39-7.52 (m, 3H), 7.63 (s, 1H), 7.81-7.99 (m, 5H), 8.81 (s, 2H), 9.01 (s, 1H).
ESI m/z $(M+H)^+$ 561.
Melting point 165-167°C.

Example 23

2-Amino-7-methoxy-8-(2-methoxypyridin-5-yl)-6-[2-methyl-5-N-(3-trifluoromethylphenyl)carbamoylphen yl]quinazoline (Compound 23)

Compound 23 was obtained in the same manner as in Example 1, using Compound A11 and 2-methoxy-5-pyridine boronic acid.
$^1H$ NMR (270 MHz, $CDCl_3$) $\delta$ (ppm) 2.34 (s, 3H), 3.16 (s, 3H), 4.02 (s, 3H), 5.16 (s, 2H), 6.87 (d, J = 8.4 Hz, 1H), 7.37-7.57 (m, 3H), 7.59 (s, 1H), 7.78-8.04 (m, 6H), 8.41 (d, J = 2.2 Hz, 1H), 8.99 (s, 1H).
ESI m/z $(M+H)^+$ 560.
Melting point 151-156°C.

Example 24

2-Amino-8-(2-dimethylaminopyrimidin-5-yl)-7-methoxy-6-[2-methyl-5-N-(3-trifluoromethylphenyl)carba moylphenyl] quinazoline (Compound 24)

Compound 24 was obtained in the same manner as in Example 1, using Compound A11 and 2-dimethylamino-5-(4,4,5,5-tetramethyl-1,3,2-dioxaboran-2-yl)pyrimidine.
$^1H$ NMR (270 MHz, $CDCl_3$) $\delta$ (ppm) 2.34 (s, 3H), 3.21 (s, 3H), 3.28 (s, 6H), 5.16 (s, 2H), 7.38-7.54 (m, 3H), 7.56 (s, 1H),

7.80-8.00 (m, 5H), 8.65 (s, 2H), 8.98 (s, 1H).
ESI m/z $(M+H)^+$ 574.
Melting point 159-161°C.

Example 25

7-Methoxy-2-methylamino-6-[2-methyl-5-N-(3-trifluoromethylphenyl)carbamoylphenyl]-8-(4-pyridyl)quin azoline (Compound 25)

Compound 25 was obtained in the same manner as in Example 1, using Compound A16 and 4-pyridine boronic acid. $^1$H NMR (270 MHz, $CDCl_3$) δ (ppm) 2.27 (s, 3H), 2.88 (d, J = 4.9 Hz, 3H), 3.09 (s, 3H), 5.10-5.23 (m, 1H), 7.30-7.60 (m, 6H), 7.70-7.95 (m, 5H),8.61-8.65 (m, 2H), 8.85 (s, 1H).
ESI m/z $(M+H)^+$ 544.
Melting point 215-216°C.

Example 26

7-Methoxy-2-methylamino-6-[2-methyl-5-N-(3-trifluoromethylphenyl)carbamoylphenyl]-8-(3-pyridyl)quin azoline (Compound 26)

Compound 26 was obtained in the same manner as in Example 1, using Compound A16 and 3-pyridine boronic acid.
$^1$H NMR (270 MHz, $CDCl_3$) δ (ppm) 2.27 (s, 3H), 2.88 (d, J = 5.1 Hz, 3H), 3.08 (s, 3H), 5.10-5.27 (m, 1H), 7.30-7.46 (m, 4H), 7.51 (s, 1H), 7.74-7.96 (m, 6H), 8.50-8.60 (m, 1H), 8.80-8.84 (m, 1H), 8.86 (s, 1H). ESI m/z $(M+H)^+$ 544.
Melting point 135-138°C.

Example 27

7-Methoxy-2-methylamino-6-[2-methyl-5-N-(3-trifluoromethylphenyl)carbamoylphenyl]-8-(2-pyridyl)quin azoline (Compound 27)

Compound 27 was obtained in the same manner as in Example 6, using Compound A16 and 2-(tributylstannyl)pyridine.
$^1$H NMR (270 MHz, $CDCl_3$) δ (ppm) 2.29 (s, 3H), 2.87 (d, J = 4.3 Hz, 3H), 3.21 (s, 3H), 5.10-5.23 (m, 1H), 7.30-7.46 (m, 4H), 7.48 (s, 1H), 7.61 (d, J = 5.9 Hz, 1H), 7.77-7.93 (m, 4H), 8.00 (s, 1H), 8.59 (br s, 1H), 8.70-8.81 (m, 1H), 8.85 (s, 1H), ESI m/z $(M+H)^+$ 544.
Melting point 144-146°C.

Example 28

2-Amino-8-(2-fluoropyridin-5-yl)-7-methoxy-6-[2-methyl-5-N-(3-trifluoromethylphenyl)carbamoylphenyl] quinazoline (Compound 28)

Compound 28 was obtained in the same manner as in Example 1, using Compound A11 and 2-fluoro-5-pyridine boronic acid.
$^1$H NMR (270 MHz, $CDCl_3$) δ (ppm) 2.34 (s, 3H), 3.15 (s, 3H), 5.18 (s, 2H), 7.06 (dd, J = 8.1, 2.7 Hz, 1H), 7.38-7.56 (m, 3H), 7.64 (s, 1H), 7.80-8.07 (m, 6H), 8.42-8.47 (m, 1H), 9.01 (s, 1H).
ESI m/z $(M+H)^+$ 548.
Melting point 144-146°C.

Example 29

2-Amino-8-(2-aminopyrimidin-5-yl)-7-methoxy-6-[2-methyl-5-N-(3-trifluoromethylphenyl)carbamoylphen yl]quinazoline (Compound 29)

Compound 29 was obtained in the same manner as in Example 1, using Compound A11 and 2-amino-5-(4,4,5,5-tetramethyl-1,3 ,2-dioxaboran-2-yl)pyrimidine.
$^1$H NMR (270 MHz, $CDCl_3$) δ (ppm) 2.34 (s, 3H), 3.21 (s, 3H), 5.13 (s, 2H), 5.21 (s, 2H), 7.38-7.54 (m, 3H), 7.59 (s, 1H),

7.81-8.03 (m, 5H), 8.61 (s, 2H), 8.99 (s, 1H).
ESI m/z $(M+H)^+$ 546.
Melting point 175-178°C.

Example 30

2-Amino-8-(2-aminopyridin-5-yl)-7-methoxy-6-[2-methyl-5-N-(3-trifluoromethylphenyl)carbamoylphenyl ]quinazoline (Compound 30)

Compound 30 was obtained in the same manner as in Example 1 using Compound A11 and 2-amino-5-(4,4,5,5-tetramethyl-1,3,2-dioxaboran-2-yl)pyridine.
$^1H$ NMR (270 MHz, $CDCl_3$) δ (ppm) 2.34 (s, 3H), 3.17 (s, 3H), 4.53 (s, 2H), 5.18 (s, 2H), 6.64 (d, J = 8.4 Hz, 1H), 7.36-7.54 (m, 3H), 7.55 (s, 1H), 7.71 (dd, J = 8.4, 2.2 Hz, 1H), 7.81-8.04 (m, 5H), 8.33 (d, J = 2.4 Hz, 1H), 8.97 (s, 1H).
ESI m/z $(M+H)^+$ 545.
Melting point 175-178°C.

Example 31

2-Amino-8-(2-fluoropyridin-5-yl)-7-methoxy-6-[2-methyl-5-(3-trifluoromethylbenzamide)phenyl]quinazol ine (Compound 31)

Compound 31 was obtained in the same manner as in Example 1, using Compound A7 and 2-fluoro-5-(4,4,5,5-tetramethyl-1,3,2-dioxaboran-2-yl)pyridine.
$^1H$ NMR (300 MHz, $CDCl_3$) δ (ppm) 2.26 (s, 3H), 3.20 (s, 3H), 5.14 (s, 2H), 7.05 (dd, J = 8.4, 3.0 Hz, 1H), 7.34 (dd, J = 8.1 Hz, 1H), 7.54-7.75 (m, 3H), 7.64 (s, 1H), 7.78-7.87 (m, 2H), 7.98-8.15 (m, 3H), 8.40-8.47 (m, 1H), 9.00 (s, 1H).
ESI m/z $(M+H)^+$ 548.
Melting point 130-135°C.

Example 32

2-Amino-8-(2-aminopyridin-5-yl)-7-methoxy-6-[2-methyl-5-(3-trifluoromethylbenzamide)phenyl]quinazol ine (Compound 32)

Compound 32 was obtained in the same manner as in Example 1, using Compound A7 and 2-amino-5-(4,4,5,5-tetramethyl-1,3,2-dioxaboran-2-yl)pyridine.
$^1H$ NMR (300 MHz, $CDCl_3$) δ (ppm) 2.27 (s, 3H), 3.21 (s, 3H), 4.53 (s, 2H), 5.17 (s, 2H), 6.63 (d, J = 8.4 Hz, 1H), 7.32 (d, J = 8.1 Hz, 1H), 7.55 (s, 1H), 7.57-7.76 (m, 4H), 7.81 (d, J = 7.5 Hz, 1H), 7.90 (s, 1H), 8.07 (d, J = 7.5 Hz, 1H), 8.13 (s, 1H), 8.32-8.35 (m, 1H), 8.96 (s, 1H).
ESI m/z $(M+H)^+$ 545.
Melting point 168-172°C.

Example 33

2-Amino-8-(2-aminopyrimidin-5-yl)-7-methoxy-6-[2-methyl-5-(3-trifluoromethylbenzamide)phenyl]quina zoline (Compound 33)

Compound 33 was obtained in the same manner as in Example 1, using Compound A7 and 2-amino-5-(4,4,5,5-tetramethyl-1,3,2-dioxaboran-2-yl)pyrimidine.
$^1H$ NMR (300 MHz, $CDCl_3$) δ (ppm) 2.25 (s, 3H), 3.26 (s, 3H), 5.11 (s, 2H), 5.17 (s, 2H), 7.34 (d, J = 8.1 Hz, 1H), 7.59 (s, 1H), 7.56-7.70 (m, 3H), 7.79-7.90 (m, 2H), 8.07 (d, J = 7.5 Hz, 1H), 8.13 (s, 1H), 8.61 (s, 2H), 8.98 (s, 1H).
ESI m/z $(M+H)^+$ 546.
Melting point 168-172°C.

Example 34

2-Amino-7-methoxy-6-[2-methyl-5-N-(3-trifluoromethylphenyl)carbamoylphenyl]-8-{2-[2-(morpholino)et hylamino]pyridin-5-yl}quinazoline (Compound 34)

Compound 28 (40 mg, 0.075 mmol) was dissolved in 2-(morpholino)ethylamine (0.74 mL), followed by stirring at 130°C for 22 hours. To the reaction mixture was added water, followed by extraction with ethyl acetate, and the organic layer was washed with brine, followed by drying over anhydrous magnesium sulfate. The solvent was evaporated under reduced pressure, and the residue was purified by preparative thin layer chromatography (chloroform/methanol = 10/1) to obtain Compound 34(8.7 mg, 12%).
$^1$H NMR (270 MHz, $CDCl_3$) $\delta$ (ppm) 2.33 (s, 3H), 2.40-2.55 (m, 4H), 2.60-2.70 (m, 2H), 3.18 (s, 3H), 3.36-3.50 (m, 2H), 3.65-3.80 (m, 4H), 5.19 (s, 2H), 5.15-5.20 (m, 1H), 6.53 (dd, J = 8.6, 0.5 Hz, 1H), 7.36-7.96 (m, 7H), 7.52 (s, 1H), 7.70 (dd, J = 8.6,2.1 Hz, 1H), 8.15 (br s, 1H), 8.32-8.38 (m, 1H), 8.95 (s, 1H).
ESI m/z $(M+H)^+$ 658.

Example 35

2-Amino-7-methoxy-6-[2-methyl-5-N-(3-trifluoromethylphenyl)carbamoylphenyl]-8-(pyrimidin-5-yl)quina zoline (Compound 35)

Compound 35 was obtained in the same manner as in Example 1, using Compound A11 and 5-pyrimidine boronic acid.
$^1$H NMR (270 MHz, $CDCl_3$) $\delta$ (ppm) 2.36 (s, 3H), 3.18 (s, 3H), 5.20 (s, 2H), 7.38-7.54 (m, 3H), 7.68 (s, 1H), 7.80-7.98 (m, 5H), 8.99-9.02 (m, 3H), 9.22 (s, 1H).
ESI m/z $(M+H)^+$ 531.

Example 3 6

2-Amino-7-methoxy-6-[2-methyl-5-N-(3-trifluoromethylphenyl)carbamoylphenyl]-8-(pyrazin-2-yl)quinazo line (Compound 36)

Compound 36 was obtained in the same manner as in Example 6, using Compound A11 and 2-(tributylstannyl)pyrazine.
$^1$H NMR (300 MHz, $CDCl_3$) $\delta$ (ppm) 2.32 (s, 3H), 3.23 (s, 3H), 5.14 (s, 2H), 7.38-7.50 (m, 3H), 7.65 (s, 1H), 7.84-7.92 (m, 3H), 7.96 (br s, 1H), 8.20 (s, 1H), 8.58 (d, J = 2.7 Hz, 1H), 8.73-8.78 (m, 1H), 8.82-8.85 (m, 1H), 8.98 (s, 1H).
ESI m/z $(M+H)^+$ 531.

Example 37

2-Amino-7-methoxy-8-(1-methyl-1H-pyrazol-4-yl)-6-[2-methyl-5-(3-trifluoromethylbenzamide)phenyl]qui nazoline (Compound 37)

Compound 37 was obtained in the same manner as in Example 1, using Compound A7 and 1-methyl-4-(4,4,5,5-tetramethyl-1,3,2-dioxaboran-2-yl)-1H-pyrazole.
$^1$H NMR (270 MHz, $CDCl_3$) $\delta$ (ppm) 2.24 (s, 3H), 3.32 (s, 3H), 4.01 (s, 3H), 5.18 (s, 2H), 7.33 (d, J = 9.2 Hz, 1H), 7.46 (s, 1H), 7.58-7.67 (m, 3H), 7.78-7.84 (m, 2H), 8.07 (d, J = 7.3 Hz, 1H), 8.13 (br s, 1H), 8.23-8.30 (m, 2H), 8.96 (s, 1H).
ESI m/z $(M+H)^+$ 533.

Example 38

2-Amino-7-methoxy-6-[2-methyl-5-(3-trifluoromethylbenzamide)phenyl]-8-(pyrimidin-5-yl)quinazoline ( Compound 38)

Compound 38 was obtained in the same manner as in Example 1, using Compound A7 and 5-pyrimidine boronic acid.
$^1$H NMR (270 MHz, $CDCl_3$) $\delta$ (ppm) 2.26 (s, 3H), 3.23 (s, 3H), 5.16 (s, 2H), 7.34 (d, J = 8.1 Hz, 1H), 7.55-7.92 (m, 6H), 8.08 (d, J = 7.6 Hz, 1H), 8.13 (s, 1H), 9.10-9.15 (m, 3H), 9.21 (s, 1H).
ESI m/z $(M+H)^+$ 531.

Example 39

2-Amino-8-(2-chloropyridin-4-yl)-7-methoxy-6-[2-methyl-5-N-(3-trifluoromethylphenyl)carbamoylphenyl ]quinazoline (Compound 39)

Compound 39 was obtained in the same manner as in Example 1, using Compound A11 and 2-chloro-4-pyridine boronic acid.
$^1$H NMR (270 MHz, $CDCl_3$) δ (ppm) 2.34 (s, 3H), 3.17 (s, 3H), 5.18 (s, 2H), 7.38-7.56 (m, 5H), 7.66 (s, 1H), 7.80-7.96 (m, 5H), 8.46-8.50 (m, 1H), 9.01 (s, 1H).
ESI m/z $(M+H)^+$ 564.

Example 40

2-Isopropylamino-7-methoxy-6-[2-methyl-5-N-(3-trifluoromethylphenyl)carbamoylphenyl]-8-(2-pyridyl)q uinazoline (Compound 40)

Compound 40 was obtained in the same manner as in Example 6, using Compound A19 and 2-(tributylstannyl)pyridine.
$^1$H NMR (300 MHz, $CDCl_3$) δ (ppm) 1.13 (d, J = 6.4 Hz, 6H), 2.31 (s, 3H), 3.22 (s, 3H), 3.78-3.92 (m, 1H), 5.06 (br d, 1H), 7.29-7.61 (m, 5H), 7.63-7.70 (m, 2H), 7.79-7.90 (m, 3H), 7.99 (s, 1H), 8.3-7 (s, 1H), 8.77-8.78 (m, 1H), 8.86 (s, 1H).
ESI m/z $(M+H)^+$ 572.

Example 41

7-Methoxy-2-methylamino-8-(1-methyl-1H-pyrazol-4-yl)-6-[2-methyl-5-N-(3-trifluoromethylphenyl)carba moylphenyl] quinazoline (Compound 41)

Compound 41 was obtained in the same manner as in Example 1, using Compound A16 and 1-methyl-4-(4,4,5,5-tetramethyl-1,3,2-dioxaboran-2-yl)-1H-pyrazole.
$^1$H NMR (270 MHz, $CDCl_3$) δ (ppm) 2.33 (s, 3H), 3.17 (d, J = 4.9 Hz, 3H), 3.30 (s, 3H), 4.01 (s, 3H), 5.28-5.36 (m, 1H), 7.38-7.54 (m, 4H), 7.82-8.00 (m, 5H), 8.39 (s, 1H), 8.47 (s, 1H), 8.90 (s, 1H).
ESI m/z $(M+H)^+$ 547.
Melting point 218°C.

Example 42

7-Methoxy-2-methylamino-6-[2-methyl-5-N-(3-trifluoromethylphenyl)carbamoylphenyl]-8-(pyrimidin-5-yl)quinazoline (Compound 42)

Compound 42 was obtained in the same manner as in Example 1, using Compound A16 and 5-pyrimidine boronic acid.
$^1$H NMR (270 MHz, $CDCl_3$) δ (ppm) 2.36 (s, 3H), 2.99 (d, J = 4.9 Hz, 3H), 3.19 (s, 3H), 5.28-5.36 (m, 1H), 7.38-7.54 (m, 3H), 7.63 (s, 1H), 7.81-8.00 (m, 5H), 8.95 (s, 1H), 9.08 (s, 2H), 9.20 (s, 1H).
ESI m/z $(M+H)^+$ 545.
Melting point 192°C.

Example 43

2-Amino-6-(5-{2-[N-(3-dimethylaminopropyl)-N-methylamino]-5-trifluoromethylbenzamide}-2-methylphenyl)-7-methoxy-8-(pyrimidin-5-yl)quinazoline (Compound 43)

Compound 43 was obtained in the same manner as in Example 1 and Example 16, using Compound A13 and 5-pyrimidine boronic acid.
$^1$H NMR (300 MHz, $CDCl_3$) δ (ppm) 1.68-1.83 (m, 2H), 2.12 (s, 6H), 2.25 (s, 3H), 2.24-2.30 (m, 2H), 2.87 (s, 3H), 3.14-3.21 (m, 2H), 3.24 (s, 3H), 5.21 (s, 2H), 7.31 (d, J = 8.1 Hz, 1H), 7.40 (d, J = 8.4 Hz, 1H), 7.46-7.52 (m, 1H), 7.69 (s, 1H), 7.68-7.75 (m, 1H), 7.88 (d, J = 2.4 Hz, 1H), 8.53 (d, J = 2.1 Hz, 1H), 9.00 (s, 2H), 9.01 (s, 1H), 9.21 (s, 1H), 11.96 (s, 1H).
ESI m/z $(M+H)^+$ 645.

Example 44

2-Amino-6-(5-{2-[N-(3-dimethylaminopropyl)-N-methylamino]-5-trifluoromethylbenzamide}-2-methylphenyl)-7-methoxy-8-(3-pyridyl)quinazoline (Compound 44)

Compound 44 was obtained in the same manner as in Example 1 and Example 16, using Compound A13 and 3-pyridine boronic acid.
$^1$H NMR (270 MHz, $CDCl_3$) $\delta$ (ppm) 1.68-1.81 (m, 2H), 2.11 (s, 6H), 2.24 (s, 3H), 2.22-2.26 (m, 2H), 2.87 (s, 3H), 3.19 (s, 3H), 3.16-3.21 (m, 2H), 5.17 (s, 2H), 7.30 (d, J = 8.4 Hz, 1H), 7.35-7.45 (m, 2H), 7.52 (dd, J = 8.4, 2.4 Hz, 1H), 7.64 (s, 1H), 7.66-7.73 (m, 1H), 7.82 (d, J = 2.4 Hz, 1H), 7.87-7.95 (m, 1H), 8.51-8.55 (m, 1H), 8.62 (dd, J = 4.9, 1.9 Hz, 1H), 8.78-8.83 (m, 1H), 8.99 (s, 1H), 11.90 (s, 1H).
ESI m/z $(M+H)^+$ 644.

Example 45

2-Amino-6-(5-{2-[N-(3-dimethylaminopropyl)-N-methylamino]-5-trifluoromethylbenzamide}-2-methylphenyl)-7-methoxy-8-(1-methyl-1H-pyrazol-4-yl)quinazoline (Compound 45)

Compound 45 was obtained in the same manner as in Example 1 and Example 16, using Compound A13 and 1-methyl-4-(4,4,5,5-tetramethyl-1,3,2-dioxaboran-2-yl)-1H-pyrazole.
$^1$H NMR (270 MHz, $CDCl_3$) $\delta$ (ppm) 1.65-1.80 (m, 2H), 2.11 (s, 6H), 2.22 (s, 3H), 2.20-2.29 (m, 2H), 2.85 (s, 3H), 3.13-3.21 (m, 2H), 3.32 (s, 3H), 4.01 (s, 3H), 5.19 (s, 2H), 7.30 (d, J = 8.4 Hz, 1H)7.37 (d, J = 8.6 Hz, 1H), 7.48 (s, 1H), 7.51-7.58 (m, 1H), 7.66-7.74 (m, 1H), 7.75 (d, J = 2.2 Hz, 1H), 8.24-8.28 (m, 2H), 8.51-8.55 (m, 1H), 8.96 (s, 1H), 11.87 (s, 1H).
ESI m/z $(M+H)^+$ 647.

Example 46

2-Amino-7-methoxy-6-[2-methyl-5-N-(3-trifluoromethylphenyl)carbamoylphenyl]-8-(4-morpholinophenyl )quinazoline (Compound 46)

Compound 46 was obtained in the same manner as in Example 1, using Compound A11 and 4-[4-(4,4,5,5-tetramethyl-1,3,2-dioxaboran-2-yl)phenyl]morpholine.
$^1$H NMR (270 MHz, $CDCl_3$) $\delta$ (ppm) 2.34 (s, 3H), 3.14 (s, 3H), 3.22-3.30 (m, 4H), 3.86-3.93 (m, 4H), 5.13 (s, 2H), 7.01 (d, J = 10.8 Hz, 2H), 7.37-7.53 (m, 6H), 7.83-8.03 (m, 5H), 8.96 (s, 1H).
ESI m/z $(M+H)^+$ 614.

Example 47

2-Amino-7-methoxy-6-[2-methyl-5-N-(3-trifluoromethylphenyl)carbamoylphenyl]-8-(2-morpholinopyrimi din-5-yl)quinazoline (Compound 47)

Compound 47 was obtained in the same manner as in Example 1, using Compound A11 and 2-morpholino-5-(4,4,5,5-tetramethyl-1,3,2-dioxaboran-2-yl)pyrimidine.
$^1$H NMR (270 MHz, $CDCl_3$) $\delta$ (ppm) 2.34 (s, 3H), 3.21 (s, 3H), 3.80-3.96 (m, 8H), 5.18 (s, 2H), 7.38-7.55 (m, 3H), 7.58 (s, 1H), 7.80-7.96 (m, 5H), 8.66 (s, 2H), 8.99 (s, 1H).
ESI m/z $(M+H)^+$ 616.

Example 48

2-Amino-6-(5-N-{2-[N-(3-dimethylaminopropyl)-N-methylamino]-5-trifluoromethylphenyl}carbamoyl-2-methylphenyl)-7-methoxy-8-(3-pyridyl)quinazoline (Compound 48)

Compound 48 was obtained in the same manner as in Example 1, using Compound A20 and 3-pyridine boronic acid.
$^1$H NMR (270 MHz, $CDCl_3$) $\delta$ (ppm) 1.57-1.70 (m, 2H), 2.08 (s, 6H), 2.18-2.26 (m, 2H), 2.35 (s, 3H), 2.70 (s, 3H), 2.94-3.02 (m, 2H), 3.13 (s, 3H), 5.20 (s 2H), 7.26-7.35 (m, 2H), 7.40-7.48 (m, 2H), 7.64 (s, 1H), 7.80 (dd, J = 6.2, 1.9 Hz, 1H), 7.90-7.96 (m, 1H), 7.97 (d, J = 1.6 Hz, 1H), 8.63 (dd, J = 5.1, 1.9 Hz, 1H), 8.81-8.84 (m, 1H), 8.87-8.92 (m,

1H), 9.00 (s, 1H), 9.51 (s, 1H).
ESI m/z $(M+H)^+$ 644.

Example 49

2-Amino-6-(5-N-{2-[N-(3-dimethylaminopropyl)-N-methylamino]-5-trifluoromethylphenyl}carbamoyl-2-methylphenyl)-7-methoxy-8-(1-methyl-1H-pyrazol-4-yl)quinazoline (Compound 49)

Compound 49 was obtained in the same manner as in Example 1, using Compound A20 and 1-methyl-4-(4,4,5,5-tetramethyl-1,3,2-dioxaboran-2-yl)-1H-pyrazole.
$^1$H NMR (270 MHz, $CDCl_3$) $\delta$ (ppm) 1.54-1.72 (m, 2H), 2.07 (s, 6H), 2.16-2.28 (m, 2H), 2.33 (s, 3H), 2.70 (s, 3H), 2.90-3.02 (m, 2H), 3.28 (s, 3H), 4.02 (s, 3H), 5.25 (s, 2H), 7.22-7.50 (m, 4H), 7.76-7.84 (m, 1H), 7.90-7.96 (m, 1H), 8.27 (s, 2H), 8.89 (br s, 1H), 8.97 (s, 1H), 9.51 (s, 1H).
ESI m/z $(M+H)^+$ 647.

Example 50

7-Methoxy-2-methylamino-6-[2-methyl-5-N-(3-trifluoromethylphenyl)carbamoylphenyl]-8-[1-(2-morpholinoethyl)-1H-pyrazol-4-yl]quinazoline (Compound 50)

Compound 50 was obtained in the same manner as in Example 1, using Compound A16 and 1-(2-morpholinoethyl)-4-(4,4,5,5-tetramethyl-1,3,2-dioxaboran-2-yl)-1H-pyrazole.
$^1$H NMR (270 MHz, $CDCl_3$) $\delta$ (ppm) 2.33 (s, 3H), 2.48-2.56 (m, 4H), 2.93 (t, J = 5.4 Hz, 2H), 3.17 (d, J = 5.4 Hz, 3H), 3.30 (s, 3H), 3.60-3.75 (m, 4H), 4.35 (t, J = 5.4 Hz, 2H), 5.31-5.37 (m, 1H), 7.38-7.54 (m, 4H), 7.82-7.98 (m, 5H), 8.47 (s, 1H), 8.51 (s, 1H), 8.91 (s, 1H).
ESI m/z $(M+H)^+$ 646.

Example 51

2-Amino-7-methoxy-6-[2-methyl-5-N-(3-trifluoromethylphenyl)carbamoylphenyl]-8-[1-(2-morpholinoethy 1)-1H-pyrazol-4-yl]quinazoline (Compound 51)

Compound 51 was obtained in the same manner as in Example 1, using Compound A11 and 1-(2-morpholinoethyl)-4-(4,4,5,5-tetramethyl-1,3,2-dioxaboran-2-yl)-1H-pyrazole.
$^1$H NMR (270 MHz, $CDCl_3$) $\delta$ (ppm) 2.33 (s, 3H), 2.50-2.58 (m, 4H), 2.94 (t, J = 5.4 Hz, 2H), 3.27 (s, 3H), 3.68-3.74 (m, 4H), 4.35 (t, J = 5.4 Hz, 2H), 5.26 (s, 2H), 7.38-7.54 (m, 4H) 7.82-8.02 (m 5H), 8.29 (s, 1H), 8.33 (s, 1H), 8.97 (s, 1H).
ESI m/z $(M+H)^+$ 632.

Example 52

2-Amino-7-methoxy-6-[2-methyl-5-N-(3-trifluoromethylphenyl)carbamoylphenyl]-8-(1H-pyrazol-4-yl)qui nazoline (Compound 52)

Compound 52 was obtained in the same manner as in Example 1, using Compound A11 and 4-(4,4,5,5-tetramethyl-1,3,2-dioxaboran-2-yl)-1H-pyrazole.
$^1$H NMR (300 MHz, $CDCl_3$) $\delta$ (ppm) 2.34 (s, 3H), 3.27 (s, 3H), 5.39 (s, 2H), 7.39-7.55 (m, 5H), 7.84-8.01 (m, 5H), 8.41 (s, 2H), 8.99 (s, 1H).
ESI m/z $(M+H)^+$ 519.

Example 53

2-Amino-7-methoxy-6-[2-methyl-5-N-(3-trifluoromethylphenyl)carbamoylphenyl]-8-[1-(tetrahydropyran-4-yl)-1H-pyrazol-4-yl]quinazoline (Compound 53)

Compound 53 was obtained in the same manner as in Example 1, using Compound A11 and 1-(tetrahydropyran-4-yl)-4-(4,4,5,5-tetramethyl-1,3,2-dioxaboran-2-yl)-1H-pyrazole (WO 06/021881).
$^1$H NMR (270 MHz, $CDCl_3$) $\delta$(ppm) 2.14-2.26 (m, 4H), 2.33 (s, 3H), 3.27 (s, 3H), 3.52-3.64 (m, 2H), 4.10-4.20 (m, 2H), 4.40-4.52 (m, 1H), 5.21 (s, 2H), 7.38-7.52 (m, 4H), 7.82-8.00 (m, 5H), 8.35 (s, 1H), 8.36 (s, 1H), 8.98 (s, 1H).

ESI m/z $(M+H)^+$ 603.

Example 54

2-Amino-7-hydroxy-6-{2-methyl-5-N-[3-(4-methylpiperazin-1-yl)methyl-5-trifluoromethylphenyl]carbam oylphenyl}-8-(2-pyridyl)quinazoline (Compound 54)

Compound 54 was obtained in the same manner as in Example 6 and Example 4, using Compound A22 and 2-(tributylstannyl)pyridine.
$^1$H NMR (270 MHz, DMSO-$d_6$) δ (ppm) 2.14 (s, 3H), 2.24 (s, 3H), 2.27-2.44 (m, 8H), 3.51 (s, 2H), 7.01 (br s, 2H), 7.32 (s, 1H), 7.43-7.51 (m, 2H), 7.67 (s, 1H), 7.90-7.97 (m, 2H), 8.00 (s, 1H), 8.02-8.12 (m, 1H), 8.17 (s, 1H), 8.59 (d, J = 4.6 Hz, 1H), 8.91 (s, 1H), 9.97 (d, J = 8.6 Hz, 1H), 10.44 (s, 1H).
ESI m/z $(M+H)^+$ 628.

Example 55

2-Amino-7-methoxy-6-[2-methyl-5-N-(3-tritluoromethylphenyl)carbamoylphenyl]-8-{1-[2-(pyrrolidin-1-yl)ethyl]-1H-pyrazol-4-yl}quinazoline (Compound 55)

Compound 55 was obtained in the same manner as in Example 1, using Compound A11 and 1-[2-(pyrrolidin-1-yl)ethyl]-4-(4,4,5,5-tetramethyl-1,3,2-dioxaboran-2-yl)-1H-pyrazole (WO 06/021884).
$^1$H NMR (270 MHz, $CDCl_3$) δ (ppm) 1.71-1.81 (m, 4H), 2.33 (s, 3H), 2.50-2.65 (m, 4H), 3.05 (t, J = 5.4 Hz, 2H), 3.27 (s, 3H), 4.37 (t, J = 5.4 Hz, 2H), 5.24 (s, 2H), 7.38-7.55 (m, 4H), 7.82-8.00 (m, 5H), 8.30 (s, 1H), 8.35 (s, 1H), 8.97 (s, 1H).
ESI m/z $(M+H)^+$ 616.

Example 56

2-Amino-8-{1-[1-(tert-butoxycarbonyl)piperidin-4-yl]-1H-pyrazol-4-yl} -7-methoxy-6-[2-methyl-5 -N-(3-trifluoromethylphenyl)carbamoylphenyl]quinazoline (Compound 56)

Compound 56 was obtained in the same manner as in Example 1, using Compound A1 and 1-[1-(*tert*-butoxycarbonyl)piperidin-4-yl]-4-(4,4,5,5-tetramethyl-1,3,2-dioxaboran-2-yl)-1H-pyrazole (WO 06/021881).
$^1$H NMR (270 MHz, $CDCl_3$) δ (ppm) 1.48 (s, 9H), 1.91-2.08 (m, 2H), 2.15-2.27 (m, 2H), 2.35 (s, 3H), 2.85-3.02 (m, 2H), 3.26 (s, 3H), 4.20-4.42 (m, 3H), 5.23 (s, 2H), 7.40-7.54 (m, 4H), 7.82-8.03 (m, 5H), 8.34 (s, 1H), 8.34 (s, 1H), 8.98 (s, 1H).
ESI m/z $(M+H)^+$ 702.

Example 57

2-Amino-7-methoxy-6-[2-methyl-5-N-(3-trifluoromethylphenyl)carbamoylphenyl]-8-[1-(piperidin-4-yl)-1H-pyrazol-4-yl] quinazoline (Compound 57)

Compound 56 (62 mg, 0.089 mmol) was dissolved in 1,4-dioxane (0.5 mL), and 4 mol/L hydrochloric acid/1,4-dioxane (0.7 mL) was added thereto, followed by stirring at room temperature for 20 minutes. To the reaction mixture was added saturated aqueous sodium bicarbonate, followed by extraction with ethyl acetate, and the organic layer was washed with brine, followed by drying over anhydrous magnesium sulfate. The solvent was evaporated under reduced pressure, and the residue was reslurried with diisopropyl ether to obtain Compound 57 (27 mg, 51%).
$^1$H NMR (270 MHz, $CDCl_3$) δ (ppm) 2.00-2.16 (m, 2H), 2.21-2.37 (m, 2H), 2.33 (s, 3H), 2.79-2.92 (m, 2H), 3.27 (s, 3H), 3.26-3.40 (m, 2H), 4.28-4.40 (m, 1H), 5.23 (s, 2H), 7.38-7.52 (m, 4H), 7.81-8.00 (m, 5H), 8.33 (s, 1H), 8.40 (s, 1H), 8.96 (s, 1H).
ESI m/z $(M+H)^+$ 602.

Example 58

2-Amino-7-methoxy-6-[2-methyl-5-N-(3-trifluoromethylphenyl)carbamoylphenyl]-8-[1-(tetrahydrofuran-3-yl)-1H-pyrazol-4-yl]quinazoline (Compound 58)

Compound 58 was obtained in the same manner as in Example 1, using Compound A11 and 1-(tetrahydrofuran-3-yl)-4-(4,4,5,5-tetramethyl-1,3,2-dioxaboran-2-yl)-1H-pyrazole (WO 06/021881).

$^1$H NMR (270 MHz, $CDCl_3$) δ (ppm) 2.33 (s, 3H), 2.42-2.60 (m, 2H), 3.27 (s, 3H), 3.95-4.05 (m, 1H), 4.10-4.26 (m, 3H), 5.00-5.20 (m, 1H), 5.22 (s, 2H), 7.38-7.54 (m, 4H), 7.82-8.00 (m, 5H), 8.34 (s, 1H), 8.38 (s, 1H), 8.98 (s, 1H).
ESI m/z $(M+H)^+$ 589.

Example 59

2-Amino-7-hydroxy-6-{2-methyl-5-N-[4-(4-methylpiperazin-1-yl)methyl-3-trifluoromethylphenyl]carbam oylphenyl}-8-(2-pyridyl)quinazoline (Compound 59)

Compound 59 was obtained in the same manner as in Example 6 and Example 4, using Compound A24 and 2-(tributylstannyl)pyridine.
$^1$H NMR (270 MHz, DMSO-$d_6$) δ (ppm) 2.14 (s, 3H), 2.23 (s, 3H), 2.25-2.45 (m, 8H), 3.54 (s, 2H), 7.01 (br s, 2H), 7.42-7.52 (m, 2H), 7.63-7.72 (m, 2H), 7.88-7.97 (m, 2H), 8.01-8.12 (m, 2H), 8.18 (s, 1H), 8.57-8.63 (m, 1H), 8.91 (s, 1H), 9.97 (d, J = 8.3 Hz, 1H), 10.41 (s, 1H).
ESI m/z $(M+H)^+$ 628.

Example 60

2-Amino-7-hydroxy-6-{2-methyl-5-N-[2-(1-methylpiperidin-4-yl)oxy-5-trifluoromethylphenyl]carbamoylp henyl}-8-(2-pyridyl)quinazoline (Compound 60)

Compound 60 was obtained in the same manner as in Example 6 and Example 4, using Compound A25 and 2-(tributylstannyl)pyridine.
$^1$H NMR (270 MHz, DMSO-$d_6$) δ (ppm) 1.65-1.87 (m, 4H), 1.91 (s, 3H), 2.10-2.22 (m, 2H), 2.25 (s, 3H), 2.30-2.42 (m, 2H), 4.58-4.68 (m, 1H), 7.01 (br s, 2H), 7.30 (d, J = 8.6 Hz, 2H), 7.43-7.52 (m, 3H), 7.68 (s, 1H), 7.80-7.91 (m, 3H), 8.08 (t, J = 8.6 Hz, 1H), 8.30 (s, 1H), 8.59 (d, J = 4.0 Hz, 1H), 8.90 (s, 1H), 9.43 (s, 1H), 9.97 (d, J = 8.6 Hz, 1H).
ESI m/z $(M+H)^+$ 629.

Example 61

2-Isopropylamino-7-methoxy-8-(1-methyl-1H-pyrazol-4-yl)-6-[2-methyl-5-N-(3-trifluoromethylphenyl)car bamoylphenyl] quinazoline (Compound 61)

Compound 61 was obtained in the same manner as in Example 1, using Compound A19 and 1-methyl-4-(4,4,5,5-tetramethyl-1,3,2-dioxaboran-2-yl)-1H-pyrazole.
$^1$H NMR (300 MHz, DMSO-$d_6$, 50°C) δ (ppm) 1.27 (d, J = 6.6 Hz 6H), 2.27 (s, 3H), 3.25 (s, 3H), 3.93 (s, 3H), 4.12-4.28 (m, 1H), 7.21 (d, J = 7.7 Hz, 1H), 7.43 (d, J = 8.4 Hz, 1H), 7.51 (d, J = 8.1 Hz, 1H), 7.55-7.63 (m, 2H), 7.95-8.03 (m, 2H), 8.08 (d, J = 8.4 Hz, 1H), 8.22 (s, 1H), 8.27 (s, 1H), 8.47 (s, 1H), 9.06 (s, 1H), 10.40 (s, 1H).
ESI m/z $(M+H)^+$ 575.

Example 62

7-Hydroxy-2-isopropylamino-6-[2-methyl-5-N-(3-trifluoromethylphenyl)carbamoylphenyl]-8-(2-pyridyl)q uinazoline (Compound 62)

Compound 62 was obtained in the same manner as in Example 4, using Compound 40.
$^1$H NMR (300 MHz, DMSO-$d_6$, 50°C) δ (ppm) 1.30 (d, J = 6.6 Hz, 6H), 2.26 (s, 3H), 4.15-4.32 (m, 1H), 7.27 (d, J = 7.7 Hz, 1H), 7.38-7.51 (m, 3H), 7.53-7.61 (m, 1H), 7.67 (s, 1H), 7.90-7.98 (m, 2H), 8.05-8.15 (m, 2H), 8.22 (s, 1H), 8.61 (d, J = 3.7 Hz, 1H), 8.93 (s, 1H), 9.86 (d, J = 8.4 Hz, 1H), 10.40 (s, 1H).
ESI m/z $(M+H)^+$ 558.

Example 63

2-Amino-7-hydroxy-6-{2-methyl-5-N-[3-morpholinomethyl-5-trfluoromethylphenyl]carbamoylphenyl}-8-(2-pyridyl)quinazoline (Compound 63)

Compound 63 was obtained in the same manner as in Example 6 and Example 4, using compound A27 and 2-(tributylstannyl)pyridine.

$^1H$ NMR (300 MHz, DMSO-$d_6$) δ (ppm) 2.24 (s, 3H), 2.32-2.40 (m, 4H), 3.52 (s, 2H), 3.53-3.60 (m, 4H), 7.02 (br s, 2H), 7.34 (s, 1H), 7.42-7.51 (m, 3H), 7.67 (s, 1H), 7.90-7.98 (m, 3H), 8.00-8.12 (m, 3H), 8.19 (s, 1H), 8.60 (d, J = 5.1 Hz, 1H), 8.91 (s, 1H), 9.97 (d, J = 8.4 Hz, 1H), 10.45 (s, 1H).
ESI m/z $(M+H)^+$ 615.

Example 64

2-Amino-7-hydroxy-6-{2-methyl-5-N-[3-(pyrrolidin-1-yl)methyl-5-trifluoromethylphenyl]carbamoylpheny 1}-8-(2-pyridyl) quinazoline (Compound 64)

Compound 64 was obtained in the same manner as in Example 6 and Example 4, using Compound A29 and 2-(tributylstannyl)pyridine.
$^1H$ NMR (270 MHz, $CDCl_3$) δ (ppm) 1.73-1.85 (m, 4H), 2.32 (s, 3H), 2.45-2.59 (m, 4H), 3.65 (s, 2H), 5.20 (br s, 2H), 7.27-7.37 (m, 2H), 7.45 (d, J = 7.9 Hz, 1H), 7.53 (s, 1H), 7.69-7.79 (m, 2H), 7.83-7.89 (m, 1H), 7.92-8.02 (m, 3H), 8.42 (d, J = 4.0 Hz, 1H), 8.84 (s, 1H), 9.77 (d, J = 8.6 Hz, 1H).
ESI m/z $(M+H)^+$ 599.

Example 65

2-Amino-6-{5-N-[3-(dimethylaminomethyl)-5-trifluoromethylphenyl]carbamoyl-2-methylphenyl}-7-hydroxy-8-(2-pyridyl) quinazoline (Compound 65)

Compound 65 was obtained in the same manner as in Example 6 and Example 4, using Compound A31 and 2-(tributylstannyl)pyridine.
$^1H$ NMR (300 MHz, $CDCl_3$) δ (ppm) 2.25 (s, 6H), 2.33 (s, 3H), 3.46 (s, 2H), 5.20 (br s, 2H), 7.25-7.40 (m, 2H), 7.45 (d, J = 7.9 Hz, 1H), 7.54 (s, 1H), 7.71 (s, 1H), 7.77 (d, J = 2.0 Hz, 1H), 7.86 (dd, J = 7.9, 2.0 Hz, 1H), 7.93-8.03 (m, 3H), 8.42 (d, J = 5.0 Hz, 1H), 8.84 (s, 1H), 9.77 (d, J = 8.6 Hz, 1H).
ESI m/z $(M+H)^+$ 573.

Example 66

2-Amino-7-methoxy-6-{2-methyl-5-N-[4-(4-methylpiperazin-1-yl)methyl-3-trifluoromethylphenyl]carbam oylphenyl}-8-(1-methyl-1H-pyrazol-4-yl)quinazoline (Compound 66)

Compound 66 was obtained in the same manner as in Example 1, using Compound A24 and 1-methyl-4-(4,4,5,5-tetramethyl-1,3,2-dioxaboran-2-yl)-1H-pyrazole.
$^1H$ NMR (270 MHz, $CDCl_3$, 40°C) δ (ppm) 2.28 (s, 3H), 2.32 (s, 3H), 2.39-2.56 (m, 8H), 3.27 (s, 3H), 3.63 (s, 2H), 3.99 (s, 3H), 5.23 (br s, 2H), 7.40-7.48 (m, 2H), 7.76 (d, J = 8.3 Hz, 1H), 7.81-7.92 (m, 4H), 8.04 (s, 1H), 8.24 (d, J = 2.6 Hz, 2H), 8.95 (s, 1H).
ESI m/z $(M+H)^+$ 645.

Example 67

8-(2-Fluoropyridin-5-yl)-7-methoxy-6-[2-methyl-5-N-(3-trifluoromethylphenyl)carbamoylphenyl]-2-(4-tetrahydropyranylamino)quinazoline (Compound 67)

Compound 67 was obtained in the same manner as in Example 1, using Compound A34 and 2-fluoro-5-pyridine boronic acid.
$^1H$ NMR (270 MHz, $CDCl_3$) δ (ppm) 1.45-1.67 (m, 2H), 1.90-2.03 (m, 2H), 2.34 (s, 3H), 3.18 (s, 3H), 3.40-3.54 (m, 2H), 3.80-4.10 (m, 3H), 5.24 (d, J = 5.4 Hz, 1H), 7.04 (dd, J = 8.4, 3.0 Hz, 1H), 7.37-7.55 (m, 3H), 7.60 (s, 1H), 7.80-7.98 (m, 5H), 8.01-8.10 (m, 1H), 8.45-8.50 (m, 1H), 8.95 (s, 1H).
ESI m/z $(M+H)^+$ 632.

Example 68

7-Methoxy-8-(1-methyl-1H-pyrazol-4-yl)-6-[2-methyl-5-N-(3-trifluoromethylphenyl)carbamoylphenyl]-2-( 4-tetrahydropyranylamino)quinazoline (Compound 68)

Compound 68 was obtained in the same manner as in Example 1, using Compound A34 and 1-methyl-4-(4,4,5,5-tetramethyl-1,3,2-dioxaboran-2-yl)-1H-pyrazole.
$^1$H NMR (270 MHz, $CDCl_3$) δ (ppm) 1.60-1.75 (m, 2H), 2.10-2.21 (m, 2H), 2.33 (s, 3H), 3.29 (s, 3H), 3.58-3.69 (m, 2H), 4.02 (s, 3H), 4.00-4.13 (m, 2H), 4.14-4.29 (m, 1H), 5.30 (d, J = 7.8 Hz, 1H), 7.39-7.52 (m, 4H), 7.82-8.00 (m, 5H), 8.29 (s, 1H), 8.36 (s, 1H), 8.92 (s, 1H).
ESI m/z $(M+H)^+$ 617.

Example 69

7-Methoxy-6-[2-methyl-5-N-(3-trifluoromethylphenyl)carbamoylphenyl]-8-(2-pyridyl)-2-(4-tetrahydropyra nylamino) quinazoline (Compound 69)

Compound 69 was obtained in the same manner as in Example 6, using Compound A34 and 2-(tributylstannyl)pyridine.
$^1$H NMR (270 MHz, $CDCl_3$) δ (ppm) 1.40-1.60 (m, 2H), 1.83-2.00 (m, 2H), 2.30 (s, 3H), 3.22 (s, 3H), 3.22-3.40 (m, 2H), 3.60-3.80 (m, 1H), 3.88-4.00 (m, 2H), 5.20 (d, J = 7.3 Hz, 1H), 7.30-7.57 (m, 6H), 7.77-7.91 (m, 4H), 7.98 (br s, 1H), 8.41 (s, 1H), 8.76-8.80 (m, 1H), 8.87 (s, 1H).
ESI m/z $(M+H)^+$ 614.

Example 70

7-Hydroxy-6-[2-methyl-5-N-(3-trifluoromethylphenyl)carbamoylphenyl]-8-(2-pyridyl)-2-(4-tetrahydropyra nylamino) quinazoline (Compound 70)

Compound 70 was obtained in the same manner as in Example 4, using Compound 69.
$^1$H NMR (300 MHz, $CDCl_3$) δ (ppm) 1.60-1.80 (m, 2H), 2.15-2.25 (m, 2H), 2.32 (s, 3H), 3.56-3.68 (m, 2H), 4.05-4.15 (m, 2H), 4.15-4.31 (m, 1H), 5.31 (d, J = 8.1 Hz, 1H), 7.29-7.52 (m, 6H), 7.75-8.02 (m, 6H), 8.41-8.46 (m, 1H), 8.79 (s, 1H), 9.84 (d, J = 8.4 Hz, 1H).
ESI m/z $(M+H)^+$ 600.

Example 71

7-Hydroxy-8-(1-methyl-1H-pyrazol-4-yl)-6-[2-methyl-5-N-(3-trifluoromethylphenyl)carbamoylphenyl]-2-( 4-tetrahydropyranylamino)quinazoline (Compound 71)

Compound 71 was obtained in the same manner as in Example 4, using Compound 68.
$^1$H NMR (270 MHz, $CDCl_3$) δ (ppm) 1.60-1.80 (m, 2H), 2.10-2.20 (m, 2H), 2.34 (s, 3H), 3.58-3.66 (m, 2H), 4.06 (s, 3H), 4.05-4.30 (m, 3H), 5.22-5.37 (m, 1H), 7.41-7.57 (m, 4H), 7.85-8.00 (m, 4H), 8.08 (s, 1H), 8.11 (s, 1H), 8.20 (s, 1H), 8.87 (s, 1H).
ESI m/z $(M+H)^+$ 603.

Example 72

2-Amino-7-methoxy-6-[2-methyl-5-N-(3-morpholinomethyl-5-trifluoromethylphenyl)carbamoylphenyl]-8-(1-methyl-1H-pyrazol-4-yl)quinazoline (Compound 72)

Compound 72 was obtained in the same manner as in Example 1, using Compound A27 and 1-methyl-4-(4,4,5,5-tetramethyl-1,3,2-dioxaboran-2-yl)-1H-pyrazole.
$^1$H NMR (270 MHz, $CDCl_3$) δ (ppm) 2.34 (s, 3H), 2.42-2.51 (m, 4H), 3.27 (s, 3H), 3.54 (s, 2H), 3.68-3.77 (m, 4H), 4.02 (s, 3H), 5.23 (br s, 2H), 7.38 (s, 1H), 7.43-7.51 (m, 2H), 7.81-7.89 (m, 3H), 7.91 (s, 1H), 8.01 (s, 1H), 8.26 (s, 2H), 8.98 (s, 1H).
ESI m/z $(M+H)^+$ 632.

Example 73

2-Amino-6-{5-N-[3-(dimethylaminomethyl)-5-trifluoromethylphenyl]carbamoyl-2-methylphenyl}-7-methoxy-8-(1-methyl-1H-pyrazol-4-yl)quinazoline (Compound 73)

Compound 73 was obtained in the same manner as in Example 1, using Compound A31 and 1-methyl-4-(4,4,5,5-tetramethyl-1,3,2-dioxaboran-2-yl)-1H-pyrazole.
$^1$H NMR (270 MHz, $CDCl_3$) $\delta$ (ppm) 2.26 (s, 6H), 2.34 (s, 3H), 3.27 (s, 3H), 3.48 (s, 2H), 4.02 (s, 3H), 5.23 (br s, 2H), 7.35 (s, 1H), 7.42-7.52 (m, 2H), 7.72 (s, 1H), 7.82-7.89 (m, 2H), 8.02 (s, 2H), 8.27 (d, J = 2.6 Hz, 2H), 8.98 (s, 1H).
ESI m/z $(M+H)^+$ 590.

Example 74

2-Amino-7-hydroxy-8-(1-methyl-1H-pyrazol-4-yl)-6-[2-methyl-5-N-(3-trifluoromethylphenyl)carbamoylp henyl]quinazoline (Compound 74)

Compound 74 was obtained in the same manner as in Example 4, using Compound 19.
$^1$H NMR (300 MHz, DMSO-$d_6$) $\delta$ (ppm) 2.21 (s, 3H), 3.90 (s, 3H), 6.70 (br s, 2H), 7.40-7.50 (m, 3H), 7.52-7.63 (m, 1H), 7.92-8.00 (m, 2H), 8.02-8.13 (m, 2H), 8.23 (s, 1H), 8.36 (s, 1H), 8.90 (s, 1H), 8.85-9.02 (m, 1H), 10.46 (s, 1H).
ESI m/z $(M+H)^+$ 519.

Example 75

7-Methoxy-8-(1-methyl-1H-pyrazol-4-yl)-6-[2-methyl-5-N-(3-trifluoromethylphenyl)carbamoylphenyl]-2-[ 2-(morpholino) ethylamino]quinazoline (Compound 77)

Compound 75 was obtained in the same manner as in Example 1 using Compound A36 and 1-methyl-4-(4,4,5,5-tetramethyl-1,3,2-dioxaboran-2-yl)-1H-pyrazole.
$^1$H NMR (270 MHz, $CDCl_3$) $\delta$ (ppm) 2.32 (s, 3H), 2.50-2.60 (m, 4H), 2.68 (t, J = 5.9 Hz, 2H), 3.28 (s, 3H), 3.60-3.71 (m, 2H), 3.71-3.80 (m, 4H), 4.16 (s, 3H), 5.93 (br s, 1H), 7.38-7.51 (m, 4H), 7.83-8.00 (m, 4H), 8.12 (s, 1H), 8.30 (s, 1H), 8.45 (s, 1H), 8.90 (s, 1H).
ESI m/z $(M+H)^+$ 646.

Example 76

2-Amino-7-methoxy-8-(1-methyl-1H-pyrazol-4-yl)-6-[2-methyl-5-N-(3-pyrrolidinylmethyl-5-trifluorometh ylphenyl)carbamoylphenyl]quinazoline (Compound 76)

Compound 76 was obtained in the same manner as in Example 1, using Compound A29 and 1-methyl-4-(4,4,5,5-tetramethyl-1,3,2-dioxaboran-2-yl)-1H-pyrazole.
$^1$H NMR (300 MHz, $CDCl_3$) $\delta$ (ppm) 1.76-1.84 (m, 4H), 2.33 (s, 3H), 2.48-2.58 (m, 4H), 3.27 (s, 3H), 3.67 (s, 2H), 4.02 (s, 3H), 5.23 (br s, 2H), 7.36 (s, 1H), 7.41-7.50 (m, 2H), 7.74 (s, 1H), 7.81-7.88 (m, 2H), 8.01 (s, 1H), 8.05 (s, 1H), 8.26 (d, J = 2.9 Hz, 2H), 8.98 (s, 1H).
ESI m/z $(M+H)^+$ 616.

Example 77

2-Amino-7-hydroxy-6-[2-methyl-5-N-(3-triftuoromethylphenyl)carbamoylphenyl]-8-(pyrimidin-2-yl)quinazoline (Compound 77)

Compound 77 was obtained in the same manner as in Example 4, using Compound 18.
$^1$H NMR (300 MHz, DMSO-$d_6$, 50°C) $\delta$ (ppm) 2.26 (s, 3H), 6.58 (br s, 2H), 7.38-7.50 (m, 3H), 7.54-7.61 (m, 1H), 7.69 (s, 1H), 7.92-7.98 (m, 2H), 8.08 (d, J = 8.4 Hz, 1H), 8.23 (s, 1H), 8.92-8.97 (m, 3H), 10.40 (s, 1H).
ESI m/z $(M+H)^+$ 517.

Example 78

2-Amino-7-hydroxy-6-[2-methyl-5-N-(3-trifluoromethylphenyl)carbamoylphenyl]-8-(pyrazin-2-yl)quinazo line (Compound 78)

Compound 78 was obtained in the same manner as in Example 4, using Compound 36.
$^1$H NMR (300 MHz, DMSO-$d_6$, 50°C) $\delta$ (ppm) 2.25 (s, 3H), 7.08 (br s, 2H), 7.38-7.51 (m, 2H), 7.53-7.62 (m, 1H), 7.74 (s, 1H), 7.91-7.98 (m, 2H), 8.08 (d, J = 8.8 Hz, 1H), 8.22 (s, 1H), 8.62-8.70 (m, 2H), 8.96 (s, 1H), 10.39 (s, 1H), 10.68 (brs, 1H).
ESI m/z $(M+H)^+$ 517.

Example 79

2-Amino-7-methoxy-6-{2-methyl-5-N-[3-(4-methyl-1H-imidazol-1-yl)-5-trifluoromethylphenyl]carbamoyl phenyl}-8-(1-methyl-1H-pyrazol-4-yl)quinazoline (Compound 79)

Compound 79 was obtained in the same manner as in Example 1, using Compound A45 and 1-methyl-4-(4,4,5,5-tetramethyl-1,3,2-dioxaboran-2-yl)-1H-pyrazole.
$^1$H NMR (270 MHz, $CDCl_3$) $\delta$ (ppm) 2.28 (s, 3H), 2.34 (s, 3H), 3.26 (s, 3H), 4.00 (s, 3H), 5.25 (br s, 2H) 7.07 (s, 1H), 7.35 (s, 1H), 7.43-7.52 (m, 2H), 7.75 (s, 1H), 7.82 (d, J = 1.0 Hz, 1H), 7.86-7.93 (m, 2H), 8.21-8.29 (m, 3H), 8.45 (s, 1H), 8.96 (s, 1H).
ESI m/z $(M+H)^+$ 613.

Example 80

2-Amino-7-hydroxy-6-{5-N-[3-(imidazol-1-yl)-5-trifluoromethylphenyl]carbamoyl-2-methylphenyl}-8-(2-pyridyl)quinazoline (Compound 80)

Compound 80 was obtained in the same manner as in Example 6 and Example 4, using Compound A46 and 2-(tributylstannyl)pyridine.
$^1$HNMR (300 MHz, DMSO-$d_6$) $\delta$ (ppm) 2.25 (s, 3H), 7.03 (br s, 2H), 7.14 (s, 1H), 7.43-7.53 (m, 2H), 7.68 (s, 1H), 7.73-7.80 (m, 2H), 7.91-8.00 (m, 2H), 8.02-8.12 (m, 1H), 8.22 (s, 1H), 8.28-8.34 (m, 2H), 8.60 (d, J = 4.0 Hz, 1H), 8.92 (s, 1H), 9.98 (d, J = 8.4 Hz, 1H), 10.63 (s, 1H).
ESI m/z $(M+H)^+$ 582.

Example 81

2-Amino-7-methoxy-8-(1-methyl-1H-pyrazol-5-yl)-6-[2-methyl-5-N-(3-trifluoromethylphenyl)carbamoylp henyl]quinazoline (Compound 81)

Compound 81 was obtained in the same manner as in Example 1, using Compound A11 and 2-methyl-3-(4,4,5,5-tetramethyl-1,3,2-dioxaboran-2-yl)-2H-pyrazole.
$^1$H NMR (300 MHz, $CDCl_3$, 40°C) $\delta$(ppm) 2.29 (s, 3H), 3.22 (s, 3H), 3.75 (s, 3H), 5.33 (br s, 2H), 6.40 (d, J = 2.2 Hz, 1H), 7.35-7.52 (m, 3H), 7.61-7.65 (m, 2H), 7.80-7.94 (m, 4H), 8.09 (s, 1H), 8.96 (s, 1H).
ESI m/z $(M+H)^+$ 533.

Example 82

2-Amino-7-methoxy-6-[2-methyl-5-N-(3-trifluoromethylphenyl)carbamoylphenyl]-8-(oxazol-2-yl)quinazol ine (Compound 82)

Compound A39 (173 mg, 0.30 mmol) was dissolved in DMF (3 mL), and 2-tributylstannyloxazole (0.31 mL, 1.5 mmol), tri-*tert*-butylphosphine-tetrafluoroborate (35 mg, 0.12 mmol), cesium fluoride (137 mg, 0.90 mmol) and tris(dibenzylideneacetone)dipalladium (62 mg, 0.060 mmol) were added thereto, followed by stirring at 120°C for 2 hours. To the reaction mixture were added water and ethyl acetate, and the organic layer was separated, followed by washing with aqueous ammonium fluoride and brine. The organic layer was dried over anhydrous magnesium sulfate, and the solvent was evaporated under reduced pressure. The residue was purified by preparative thin layer chromatography (chloroform/methanol = 97/3) to obtain Compound 82 (28 mg, 18%).

$^1H$ NMR (300 MHz, $CDCl_3$, 40°C) δ (ppm) 2.26 (s, 3H), 3.32 (s, 3H), 5.22 (br s, 2H), 7.33-7.47 (m, 4H), 7.59 (s, 1H), 7.80-7.84 (m, 1H), 7.85-7.92 (m, 3H), 7.98 (s, 1H), 8.49 (s, 1H), 8.90 (s, 1H).
ESI m/z $(M+H)^+$ 520.

Example 83

2-Amino-8-(2-fluoropyridin-5-yl)-7-hydroxy-6-[2-methyl-5-N-(3-trifluoromethylphenyl)carbamoylphenyl] quinazoline (Compound 83)

Compound A40 (467 mg, 0.77 mmol) was dissolved in dioxane (8 mL) and water (4 mL), and 2-fluoro-5-pyridine boronic acid (130 mg, 0.92 mmol), sodium carbonate (163 mg, 1.54 mmol) and [1,1'-bis(diphenylphosphino)ferrocene] dichloropalladium (32 mg, 0.039 mmol) were added thereto, followed by stirring for 2 hours and under heating and reflux. To the reaction mixture were added water and ethyl acetate, and the organic layer was separated, followed by washing with brine. The organic layer was dried over anhydrous magnesium sulfate, and the solvent was evaporated under reduced pressure. The residue was purified by silica gel column chromatography (ethyl acetate/hexane = 9/1) to obtain Compound 83 (300 mg, 73%).
$^1H$ NMR (270 MHz, DMSO-$d_6$) δ (ppm) 2.26 (s, 3H), 6.66 (s, 2H), 7.27 (dd, J = 8.2, 2.6 Hz, 1H), 7.41-7.51 (m, 2H), 7.55-7.67 (m, 2H), 7.93-8.03 (m, 3H), 8.10 (d, J = 7.9 Hz, 1H), 8.19-8.29 (m, 2H), 8.97 (s, 1H), 9.20 (br s, 1H), 10.47 (s, 1H).
ESI m/z $(M+H)^+$ 534.

Example 84

2-Aanino-7-hydroxy-6-[2-methyl-5-N-(3-trifluoromethylphenyl)carbamoylphenyl]-8-(1H-pyrazol-4-yl)quinazoline (Compound 84)

Compound 84 was obtained in the same manner as in Example 4, using Compound 52.
$^1H$ NMR (300 MHz, DMSO-$d_6$) δ (ppm) 2.22 (s, 3H), 6.70 (br s, 2H), 7.39-7.53 (m, 3H), 7.54-7.64 (m, 1H), 7.92-8.01 (m, 2H), 8.10 (d, J = 8.1 Hz, 1H), 8.15-8.45 (m, 2H), 8.92 (s, 2H), 10.47 (s, 1H), 12.82 (br s, 1H). ESI m/z $(M+H)^+$ 505.

Example 85

2-Amino-7-methoxy-6-[2-methyl-5-N-(3-trifluoromethylphenyl)carbamoylphenyl]-8-(thiazol-2-yl)quinazol ine (Compound 85)

Compound 85 was obtained in the same manner as in Example 82, using 2-tributylstannylthiazole.
$^1H$ NMR (300 MHz, $CDCl_3$, 40°C) δ (ppm) 2.28 (s, 3H), 3.28 (s, 3H), 5.24 (br s, 2H), 7.32-7.46 (m, 3H), 7.54-7.60 (m, 2H), 7.83-7.91 (m, 3H), 7.96-8.03 (m, 2H), 8.59 (s, 1H), 8.92 (s, 1H).
ESI m/z $(M+H)^+$ 536.

Example 86

2-Amino-7-methoxy-6-(2-methyl-5-N-{2-( 1-methylpiperidin-4-yl)oxy-5-trifluoromethylphenyl}carbamoyl phenyl)-8-(1-methyl-1H-pyrazol-4-yl)quinazoline (Compound 86)

Compound 86 was obtained in the same manner as in Example 1, using Compound A25 and 1-methyl-4-(4,4,5,5-tetramethyl-1,3,2-dioxaboran-2-yl)-1H-pyrazole.
$^1H$ NMR (270 MHz, $CDCl_3$) δ (ppm) 1.82-1.97 (m, 2H), 2.04-2.17 (m, 2H), 2.16 (s, 3H), 2.26-2.39 (in, 5H), 2.50-2.65 (m, 2H), 3.27 (s, 3H), 4.02 (s, 3H), 4.49-4.62 (m, 1H), 5.23 (br s, 2H), 6.97 (d, J = 8.6 Hz, 1H), 7.28-7.36 (m, 1H), 7.43-7.50 (m, 2H), 7.81-7.94 (m, 2H), 8.27 (s, 2H), 8.76 (s, 1H), 8.91-9.01 (m, 2H).
ESI m/z $(M+H)^+$ 646.

Example 87

2-Amino-7-hydroxy-6-[2-methyl-5-N-(3-trifluoromethylphenyl)carbamoylphenyl]-8-(2-morpholinopyridi n-4-yl)quinazoline (Compound 87)

Compound 39 (116 mg, 0.206 mmol) and morpholine (2 mL) were mixed, then the mixture was stirred at 130°C for 16 hours. To the reaction mixture was added water, followed by extraction with ethyl acetate, and the organic layer

was washed with brine, followed by drying over anhydrous magnesium sulfate. The solvent was evaporated under reduced pressure, and the residue was purified by silica gel column chromatography (chloroform/methanol = 85/15) to obtain Compound 87 (60 mg, 48%).
$^1$H NMR (300 MHz, DMSO-$d_6$, 80°C) δ (ppm) 2.24 (s, 3H), 3.42-3.51 (m, 4H), 3.67-3.75 (m, 4H), 6.37 (br s, 2H), 6.67-6.72 (m, 1H), 6.78 (s, 1H), 7.36-7.48 (m, 2H), 7.52-7.61 (m, 2H), 7.90-7.97 (m, 2H), 8.07 (d, J = 8.4 Hz, 1H), 8.15-8.22 (m, 2H), 8.90 (br s, 1H), 10.28 (s, 1H).
ESI m/z $(M+H)^+$ 601.

Example 88

2-Amino-7-hydroxy-6-[2-methyl-5-N-(3-trifluoromethylphenyl)carbamoylphenyl]-8-(2-morpholinopyrimi din-5-yl)quinazoline (Compound 88)

Compound 88 was obtained in the same manner as in Example 4, using Compound 47.
$^1$H NMR (270 MHz, $CDCl_3$) δ (ppm) 2.31 (s, 3H), 3.76-3.93 (m, 8H), 5.14 (s, 2H), 7.39-7.54 (m, 4H), 7.82-8.02 (m, 5H), 8.57 (s, 2H), 8.89 (s, 1H).
ESI m/z $(M+H)^+$ 602.

Example 89

2-Amino-7-hydroxy-6-(2-methyl-5-N-{2-(1-methylpiperidin-4-yl)oxy-5-trifluoromethylphenyl}carbamoyl phenyl)-8-(1-methyl-1H-pyrazol-4-yl)quinazoline (Compound 89)

Compound 89 was obtained in the same manner as in Example 4, using Compound 86.
$^1$H NMR (270 MHz, DMSO-$d_6$) δ (ppm) 1.65-1.79 (m, 2H), 1.80-1.95 (m, 5H), 2.10-2.25 (m, 5H), 2.30-2.45 (m, 2H), 3.89 (s, 3H), 4.57-4.70 (m, 1H), 6.67 (br s, 2H), 7.31 (d, J = 8.4 Hz, 1H), 7.40-7.52 (m, 3H), 7.75-7.92 (m, 2H), 8.11 (s, 1H), 8.27-8.41 (m, 2H), 8.86 (s, 1H), 9.43 (s, 1H).
ESI m/z $(M+H)^+$ 632.

Example 90

2-Amino-8-(2-chloropyridin-5-yl)-7-hydroxy-6-[2-methyl-5-N-(3-trifluoromethylphenyl)carbamoylphenyl] quinazoline (Compound 90)

Compound 90 was obtained in the same manner as in Example 4, using Compound 20.
$^1$H NMR (300 MHz, DMSO-$d_6$, 50°C) δ (ppm) 2.26 (s, 3H), 6.57 (br s, 2H), 7.38-7.50 (m, 2H), 7.54-7.67 (m, 3H), 7.84-7.90 (m, 1H), 7.92-7.99 (m, 2H), 8.09 (d, J = 8.1 Hz, 1H), 8.22 (s, 1H), 8.40-8.43 (m, 1H), 8.94 (s, 1H), 10.39 (s, 1H).
ESI m/z $(M+H)^+$ 550.

Example 91

2-Amino-7-hydroxy-6-[2-methyl-5-N-(3-trifluoromethylphenyl)carbamoylphenyl]-8-[2-(pyrrolidin-1-yl)py ridin-5-yl]quinazoline (Compound 91)

Compound 20 (220 mg, 0.39 mmol) was dissolved in NMP(2 mL), and pyrrolidine (0.16 mL, 1.95 mmol) was added thereto, followed by stirring at 130°C for 1.5 hours under microwave irradiation. To the reaction mixture was added water, followed by extraction with ethyl acetate, and the organic layer was washed with brine, followed by drying over anhydrous magnesium sulfate. The solvent was evaporated under reduced pressure, and the residue was purified by silica gel column chromatography (chloroform/methanol = 85/15) to obtain Compound 91 (57 mg, 25%).
$^1$H NMR (300 MHz, DMSO-$d_6$, 80°C) δ (ppm) 1.92-2.03 (m, 4H), 2.25 (s, 3H), 3.40-3.50 (m, 4H), 6.28 (br s, 2H), 6.52 (d, J = 8.8 Hz, 1H), 7.37-7.61 (m, 6H), 7.90-7.96 (m, 2H), 8.04-8.10 (m, 2H), 8.20 (s, 1H), 8.89 (s, 1H), 10.28 (s, 1H).
ESI m/z $(M+H)^+$ 585.

Example 92

2-Amino-7-methoxy-6-[2-methyl-5-N-(3-trifluoromethylphenyl)carbamoylphenyl]-8-[2-(piperazin-1-yl)py ridin-5-yl] quinazoline (Compound 92)

Compound 92 was obtained in the same manner as in Example 1, using Compound A11 and 1-[5-(4,4,5,5-tetramethyl-1,3,2-dioxaboran-2-yl)pyridin-2-yl]piperazine.
$^1$H NMR (300 MHz, DMSO-$d_6$, 50°C) $\delta$ (ppm) 2.28 (s, 3H), 2.78-2.86 (m, 4H), 3.10 (s, 3H), 3.42-3.51 (m, 4H), 6.66 (br s, 2H), 6.86 (d, J = 8.8 Hz, 1H), 7.43 (d, J = 7.7 Hz, 1H), 7.50 (d, J = 7.7 Hz, 1H), 7.54-7.65 (m, 2H), 7.70 (s, 1H), 7.94-8.03 (m, 2H), 8.08 (d, J = 8.8 Hz, 1H), 8.21 (d, J = 2.2 Hz, 2H), 9.08 (s, 1H), 10.38 (s, 1H).
ESI m/z $(M+H)^+$ 614.

Example 93

2-Amino-7-hydroxy-6-[2-methyl-5-N-(3-trifluoromethylphenyl)carbamoylphenyl]-8-[2-(pyrrolidin-1-yl)py rimidin-5-yl] quinazoline (Compound 93)

Compound 93 was obtained in the same manner as in Example 4, using Compound 21.
$^1$H NMR (300 MHz, DMSO-$d_6$, 80°C) $\delta$ (ppm) 1.92-2.01 (m, 4H), 2.25 (s, 3H), 3.52-3.61 (m, 4H), 7.45 (br s, 2H), 7.52-7.61 (m, 2H), 7.91-7.97 (m, 2H), 8.07 (d, J = 8.4 Hz, 1H), 8.20 (s, 1H), 8.33 (s, 2H), 8.91 (s, 1H), 10.28 (s, 1H).
ESI m/z $(M+H)^+$ 586.

Example 94

2-Amino-7-methoxy-6-[2-methyl-5-N-(3-trifluoromethylphenyl)carbamoylphenyl]-8-(2-morpholinopyridi n-5-yl)quinazoline (Compound 94)

Compound 94 was obtained in the same manner as in Example 1, using Compound A11 and 2-morpholino-5-(4,4,5,5-tetramethyl-1,3,2-dioxaboran-2-yl)pyridine.
$^1$H NMR (300 MHz, DMSO-$d_6$, 50°C) $\delta$ (ppm) 2.28 (s, 3H), 3.09 (s, 3H), 3.47-3.55 (m, 4H), 3.70-3.77 (m, 4H), 6.67 (br s, 2H), 6.91 (d, J = 8.8 Hz, 1H), 7.43 (d, J = 8.4 Hz, 1H), 7.50 (d, J = 8.1 Hz, 1H), 7.54-7.62 (m, 1H), 7.63-7.69 (m, 1H), 7.71 (s, 1H), 7.94-8.03 (m, 2H), 8.08 (d, J = 8.4 Hz, 1H), 8.19-8.26 (m, 2H), 9.09 (s, 1H), 10.38 (s, 1H).
ESI m/z $(M+H)^+$ 615.

Example 95

2-Amino-7-hydroxy-6-[2-methyl-5-N-(3-trifluoromethylphenyl)carbamoylphenyl]-8-[2-(pyrrolidin-1-yl)py ridin-4-yl]quinazoline (Compound 95)

Compound 95 was obtained in the same manner as in Example 91, using Compound 39.
$^1$H NMR (300 MHz, DMSO-$d_6$, 80°C) $\delta$ (ppm) 1.91-1.98 (m, 4H), 2.24 (s, 3H), 3.37-3.46 (m, 4H), 6.37 (br s, 2H), 6.43 (s, 1H), 6.50-6.57 (m, 1H), 7.36-7.48 (m, 2H), 7.52-7.61 (m, 2H), 7.90-7.97 (m, 2H), 8.03-8.13 (m, 2H), 8.20 (s, 1H), 8.90 (s, 1H), 10.28 (s, 1H).
ESI m/z $(M+H)^+$ 585.

Example 96

2-Amino-7-hydroxy-6-[2-methyl-5-N-(3-trifluoromethylphenyl)carbamoylphenyl]-8-(2-morpholinopyridi n-5-yl)quinazoline (Compound 96)

Compound 96 was obtained in the same manner as in Example 4, using Compound 94.
$^1$H NMR (300 MHz, DMSO-$d_6$, 80°C) $\delta$ (ppm) 2.25 (s, 3H), 3.47-3.55 (m, 4H), 3.70-3.77 (m, 4H), 6.30 (br s, 2H), 6.88 (d, J = 8.4 Hz, 1H), 7.37-7.48 (m, 2H), 7.52-7.61 (m, 3H), 7.90-7.98 (m, 2H), 8.07 (d, J = 8.4 Hz, 1H), 8.13-8.23 (m, 2H), 8.60 (br s, 1H), 8.91 (s, 1H), 10.28 (s, 1H).
ESI m/z $(M+H)^+$ 601.

Example 97

7-Hydroxy-2-methylamino-8-(1-methyl-1H-pyrazol-4-yl)-6-[2-methyl-5-N-(3-trifluoromethylphenyl)carba moylphenyl] quinazoline (Compound 97)

Compound 97 was obtained in the same manner as in Example 4, using Compound 41.
$^1$H NMR (300 MHz, DMSO-$d_6$, 80°C) $\delta$ (ppm) 2.23 (s, 3H), 2.96 (d, J = 4.8 Hz, 3H), 3.91 (s, 3H), 6.87-6.98 (br m, 1H), 7.37-7.50 (m, 3H), 7.52-7.60 (m, 1H), 7.92-8.00 (m, 2H), 8.08 (d, J = 7.7 Hz, 1H), 8.21 (s, 2H), 8.36 (s, 1H), 8.67 (br s, 1H), 8.90 (s, 1H), 10.30 (s, 1H).
ESI m/z $(M+H)^+$ 533.

Example 98

2-Amino-7-methoxy-6-[2-methyl-5-N-(2-piperidino-5-trifluoromethylphenyl)carbamoylphenyl]-8-(1-methyl-1H-pyrazol-4-yl)quinazoline (Compound 98)

Compound 98 was obtained in the same manner as in Example 1, using Compound A41 and 1-methyl-4-(4,4,5,5-tetramethyl-1,3,2-dioxaboran-2-yl)-1H-pyrazole.
$^1$H NMR (300 MHz, $CDCl_3$) $\delta$ (ppm) 1.50-1.70 (m, 2H), 1.68-1.78 (m, 4H), 2.35 (s, 3H), 2.81-2.91 (m, 4H), 3.28 (s, 3H), 4.02 (s, 3H), 5.23 (br s, 2H), 7.22-7.26 (m, 1H), 7.31-7.37 (m, 1H), 7.45-7.52 (m, 2H), 7.85-7.96 (m, 2H), 8.29 (s, 2H), 8.90 (d, J = 1.5 Hz, 1H), 8.97 (s, 1H), 9.52 (s, 1H).
ESI m/z $(M+H)^+$ 616.

Example 99

2-Amino-7-hydroxy-6-[2-methyl-5-N-(2-piperidino-5-trifluoromethylphenyl)carbamoylphenyl]-8-(1-methyl-1H-pyrazol-4-yl)quinazoline (Compound 99)

Compound 99 was obtained in the same manner as in Example 4, using Compound 98.
$^1$H NMR (270 MHz, DMSO-$d_6$) $\delta$ (ppm) 1.41-1.54 (m, 2H), 1.58-1.72 (m, 5H), 2.25 (s, 3H), 2.82-2.92 (m, 4H), 3.91 (s, 3H), 6.72 (br s, 2H), 7.35-7.57 (m, 4H), 7.82 (s, 1H), 7.90 (d, J = 8.3 Hz, 1H), 8.14 (s, 1H), 8.41 (s, 1H), 8.49 (d, J = 1.7 Hz, 1H), 8.89 (s, 1H), 9.67 (s, 1H).
ESI m/z $(M+H)^+$ 602.

Example 100

2-Amino-7-hydroxy-6-[2-methyl-5-N-(3-trifluoromethylphenyl)carbamoylphenyl]-8-(4-morpholinophenyl) quinazoline (Compound 100)

Compound 100 was obtained in the same manner as in Example 4, using Compound 46.
$^1$H NMR (300 MHz, DMSO-$d_6$-, 80°C) $\delta$ (ppm) 2.25 (s, 3H), 3.14-3.22 (m, 4H), 3.72-3.80 (m, 4H), 6.23 (br s, 2H), 7.00 (d, J = 8.7 Hz, 2H), 7.26 (d, J = 8.7 Hz, 2H), 7.36-7.46 (m, 2H), 7.50-7.60 (m, 2H), 7.90-7.98 (m, 2H), 8.04-8.10 (m, 1H), 8.20 (s, 1H), 8.90 (s, 1H), 10.27 (s, 1H).
ESI m/z $(M+H)^+$ 600.

Example 101

2-Amino-8-(2-fluoropyridin-5-yl)-7-methoxy-6-[2-methyl-5-N-(2-piperidino-5-trifluoromethylphenyl)carb amoylphenyl] quinazoline (Compound 101)

Compound 101 was obtained in the same manner as in Example 1, using Compound A41 and 2-fluoro-5-pyridine boronic acid.
$^1$H NMR (270 MHz, $CDCl_3$) $\delta$ (ppm) 1.55-1.66 (m, 2H), 1.69-1.81 (m, 4H), 2.36 (s, 3H), 2.81-2.93 (m, 4H), 3.16 (s, 3H), 5.18 (br s, 2H), 7.03-7.11 (m, 1H), 7.22-7.29 (m, 1H), 7.31-7.39 (m, 1H), 7.49 (d, J = 7.9 Hz, 1H), 7.65 (s, 1H), 7.84-7.92 (m, 1H), 7.95 (d, J = 2.0 Hz, 1H), 7.99-8.10 (m, 1H), 8.45 (d, J = 2.3 Hz, 1H), 8.90 (d, J = 1.7 Hz, 1H), 9.00 (s, 1H), 9.51 (s, 1H).
ESI m/z $(M+H)^+$ 631.

Example 102

2-Amino-8-(2-fluoropyridin-5-yl)-7-hydroxy-6-[2-methyl-5-N-(2-piperidino-5-trifluoromethylphenyl)carba moylphenyl] quinazoline (Compound 102)

Compound 102 was obtained in the same manner as in Example 4, using Compound 101.
$^1$H NMR (270 MHz, DMSO-$d_6$) δ (ppm) 1.42-1.56 (m, 2H), 1.60-1.73 (m, 4H), 2.28 (s 3H), 2.82-2.92 (m, 4H), 6.67 (br s, 2H), 7.23-7.31 (m, 1H), 7.37-7.58 (m, 3H), 7.66 (s, 1H), 7.81-8.04 (m, 3H), 8.23 (d, J = 1.7 Hz, 1H), 8.47 (d, J = 2.0 Hz, 1H), 8.95 (s, 1H), 9.24 (br s, 1H), 9.66 (s, 1H).
ESI m/z $(M+H)^+$ 617.

Example 103

2-Amino-8-(2-hydroxypyridin-5-yl)-7-methoxy-6-[2-methyl-5-N-(3-trifluoromethylphenyl)carbamoylphen yl]quinazoline (Compound 103)

Compound 103 was obtained in the same manner as in Example 1, using Compound A11 and 2-hydroxy-5-(4,4,5,5-tetramethyl-1,3,2-dioxaboran-2-yl)pyridine.
$^1$H NMR (300 MHz, DMSO-$d_6$, 80°C) δ (ppm) 2.27 (s, 3H), 3.20 (s, 3H), 6.40 (d, J = 9.5 Hz, 1H), 6.58 (br s, 2H), 7.37-7.51 (m, 3H), 7.52-7.61 (m, 2H), 7.68 (s, 1H), 7.94-8.00 (m, 2H), 8.03-8.10 (m, 1H), 8.19 (s, 1H), 9.07 (s, 1H), 10.28 (s, 1H), 11.42 (br s, 1H).
ESI m/z $(M+H)^+$ 546.

Example 104

2-Amino-7-methoxy-6-[2-methyl-5-N-(3-trifluoromethylphenyl)carbamoylphenyl]-8-[2-(piperazin-1-yl)py ridin-4-yl] quinazoline (Compound 104)

Compound 104 was obtained in the same manner as in Example 1, using Compound A11 and 1-[4-(4,4,5,5-tetramethyl-1,3,2-dioxaboran-2-yl)pyridin-2-yl]piperazine.
$^1$H NMR (300 MHz, DMSO-$d_6$ 50°C) δ (ppm) 2.28 (s, 3H), 2.75-2.83 (m, 4H), 3.14 (s, 3H), 3.37-3.45 (m, 4H), 6.65-6.69 (m, 1H), 6.73 (br s, 2H), 6.76 (s, 1H), 7.43 (d, J = 8.4 Hz, 1H), 7.50 (d, J = 7.7 Hz, 1H), 7.54-7.62 (m, 1H), 7.75 (s, 1H), 7.94-8.02 (m, 2H), 8.08 (d, J = 8.4 Hz, 1H), 8.16 (d, J = 5.1 Hz, 1H), 8.21 (s, 1H), 9.09 (s, 1H), 10.39 (s, 1H).
ESI m/z $(M+H)^+$ 614.

Example 105

2-Amino-8-(2-fluoropyridin-5-yl)-7-hydroxy-6-{2-methyl-5-N-[2-(4-methylpiperazin-1-yl)-5-trifluoromethylphenyl]carbamoylphenyl}quinazoline (Compound 105)

Compound 105 was obtained in the same manner as in Example 1 and Example 4, using Compound A42 and 2-fluoro-5-pyridine boronic acid.
$^1$H NMR (270 MHz, DMSO-$d_6$) δ (ppm) 2.03 (s, 3H), 2.28 (s, 3H), 2.38-2.50 (m, 4H), 2.87-2.98 (m, 4H), 6.63 (br s, 2H), 7.21-1.30 (m, 1H), 7.38-7.58 (m, 3H), 7.65 (s, 1H), 7.77-8.05 (m, 3H), 8.23 (s, 1H), 8.47 (s, 1H), 8.92 (br s, 1H), 9.67 (s,1H).
ESI m/z $(M+H)^+$ 632.

Example 106

2-Amino-7-methoxy-8-[2-(4-methylpiperazin-1-yl)pyridin-4-yl]-6-[2-methyl-5-N-(3-trifluoromethylphenyl )carbamoylphenyl]quinazoline (Compound 106)

Compound 106 was obtained in the same manner as in Example 1, using compound A11 and 1-methyl-4-[4-(4,4,5,5-tetramethyl-1,3,2-dioxaboran-2-yl)pyridin-2-yl]piperazine.
$^1$H NMR (300 MHz, $CDCl_3$, 40°C) δ (ppm) 2.33 (s, 3H), 2.36 (s, 3H), 2.51-2.59 (m, 4H), 3.17 (s, 3H), 3.60-3.68 (m, 4H), 5.15 (br s, 2H), 6.76 (d, J = 8.8 Hz, 1H), 7.36-7.54 (m, 4H), 7.71-7.78 (m, 1H), 7.81-7.95 (m, 4H), 8.03 (s, 1H), 8.44 (d, J = 2.2 Hz, 1H), 8.95 (s, 1H).
ESI m/z $(M+H)^+$ 628.

Example 107

2-Amino-7-methoxy-6-{2-methyl-5-N-[2-(4-methylpiperazin-1-yl)-5-trifluoromethylphenyl]carbamoylphe nyl}-8-(1-methyl-1H-pyrazol-4-yl)quinazoline (Compound 107)

Compound 107 was obtained in the same manner as in Example 1, using Compound A42 and 1-methyl-4-(4,4,5,5-tetramethyl-1,3,2-dioxaboran-2-yl)-1H-pyrazole.
$^1$H NMR (270 MHz, $CDCl_3$) δ (ppm) 2.19 (s, 3H), 2.36 (s, 3H), 2.44-2.62 (m, 4H), 2.91-3.01 (m, 4H), 3.29 (s, 3H), 4.02 (s, 3H), 5.23 (br s, 2H), 7.25-7.31 (m, 1H), 7.32-7.39 (m, 1H), 7.46-7.54 (m, 2H), 7.87-7.96 (m, 2H), 8.28 (s, 2H), 8.91 (d, J = 1.7 Hz, 1H), 8.98 (s, 1H), 9.47 (s, 1H).
ESI m/z $(M+H)^+$ 631.

Example 108

2-Amino-8-(2-fluoropyridin-5-yl)-7-methoxy-6-[2-methyl-5-N-(2-morpholino-5-trifluoromethylphenyl)car bamoylphenyl] quinazoline (Compound 108)

Compound 108 was obtained in the same manner as in Example 1, using Compound A43 and 2-fluoro-5-pyridine boronic acid.
$^1$H NMR (270 MHz, $CDCl_3$) δ (ppm) 2.37 (s, 3H), 2.92-3.00 (m, 4H), 3.15 (s, 3H), 3.84-3.93 (m, 4H), 5.19 (br s, 2H), 7.03-7.11 (m, 1H), 7.26-7.33 (m, 1H), 7.35-7.43 (m, 1H), 7.50 (d, J = 7.9 Hz, 1H), 7.65 (s, 1H), 7.81-7.88 (m, 1H), 7.95 (d, J=2.0 Hz, 1H), 7.99-8.10 (m, 1H), 8.43-8.48 (m, 1H), 8.91 (d, J = 1.7 Hz, 1H), 9.01 (s, 1H), 9.46 (s, 1H).
ESI m/z $(M+H)^+$ 633.

Example 109

2-Amino-7-methoxy-6-[2-methyl-5-N-(3-trifluoromethylphenyl)carbamoylphenyl]-8-{2-[2-(morpholino)et hyloxy]pyridin-5-yl}quinazoline (Compound 109)

Compound 103 (126 mg, 0.23 mmol) was dissolved in THF (5 mL), and triphenylphosphine (121 mg, 0.46 mmol) and 2-morpholinoethanol (0.084 mL, 0.69 mmol) were added, then diethyl azodicarbonate (2.2 mol/L toluene solution, 0.21 mL, 0.46 mmol) was added dropwise thereto under ice-cooling, followed by stirring at room temperature for 24 hours. The solvent was evaporated under reduced pressure, and the residue was purified by silica gel column chromatography (methanol/ethyl acetate = 3/7) to obtain Compound 109 (60 mg, 40%).
$^1$H NMR (300 MHz, $CDCl_3$, 50°C) δ (ppm) 2.31 (s, 3H), 2.48-2.56 (m, 4H), 2.79 (t, J = 6.4 Hz, 2H), 3.25 (s, 3H), 3.60-3.67 (m, 4H), 4.12 (t, J = 6.4 Hz, 2H), 5.31 (br s, 2H), 6.57-6.64 (m, 1H), 7.36-7.52 (m, 3H), 7.53-7.63 (m, 3H), 7.81-7.95 (m, 4H), 8.17 (s, 1H), 8.96 (s, 1H).
ESI m/z $(M+H)^+$ 659.

Example 110

2-Amino-8-(2-fluoropyridin-5-yl)-7-hydroxy-6-[2-methyl-5-(3-trifluoromethylbenzamide)phenyl]quinazoli ne (Compound 110)

Compound 31 (121 mg, 0.22 mmol) was dissolved in NMP (2 mL), and lithium chloride (92 mg, 2.2 mmol) was added thereto, followed by stirring at 150°C for 3 hours under microwave irradiation. To the reaction mixture was added water, followed by extraction with ethyl acetate, and the organic layer was washed with brine, followed by drying over anhydrous magnesium sulfate. The solvent was evaporated under reduced pressure, and the residue was purified by silica gel column chromatography (ethyl acetate/hexane = 8/2) to obtain Compound 110 (27 mg, 23%).
$^1$H NMR (300 MHz, $CDCl_3$, 50°C) δ (ppm) 2.21 (s, 3H), 5.08 (br s, 2H), 6.98-7.05 (m, 1H), 7.37 (d, J = 8.4 Hz, 1H), 7.54 (s, 1H), 7.57-7.70 (m 3H), 7.81 (d, J = 8.1 Hz, 1H), 7.90 (s, 1H), 7.95-8.08 (m, 2H) 8.11 (s, 1H), 8.40 (d, J = 2.6 Hz, 1H), 8.87 (s, 1H).
ESI m/z $(M+H)^+$ 534.

Example 111

2-Amino-7-hydroxy-8-[2-(4-methylpiperazin-1-yl)pyridin-4-yl]-6-[2-methyl-5-N-(3-trifluoromethylphenyl )carbamoylphenyl]quinazoline (Compound 111)

Compound 111 was obtained in the same manner as in Example 4, using Compound 106.
$^1$H NMR (300 MHz, DMSO-$d_6$, 80°C) δ (ppm) 2.25 (s, 6H), 2.40-2.47 (m, 2H), 3.51-3.58 (m, 4H), 6.30 (br s, 2H), 6.87 (d, J = 8.8 Hz, 1H), 7.37-7.48 (m, 2H), 7.51-7.60 (m, 3H), 7.90-7.97 (m, 2H), 8.07 (d, J = 8.4 Hz, 1H), 8.14 (d, J = 2.2 Hz, 1H), 8.20 (s, 1H), 8.90 (s, 1H), 10.28 (s, 1H).
ESI m/z $(M+H)^+$ 614.

Example 112

2-Amino-7-hydroxy-6-[2-methyl-5-N-(3-trifluoromethylphenyl)carbamoylphenyl]-8-{2-[2-(morpholino)et hyloxy]pyridin-5-yl}quinazoline (Compound 114)

Compound 114 was obtained in the same manner as in Example 4, using Compound 109.
$^1$H NMR (300 MHz, DMSO-$d_6$, 80°C) δ (ppm) 2.25 (s, 3H), 2.43-2.47 (m, 4H), 2.65 (t, J = 6.8 Hz, 2H), 3.49-3.56 (m, 4H), 4.02-4.06 (m, 2H), 4.04 (t, J = 6.8 Hz, 2H), 6.38 (br s, 2H), 6.43 (d, J = 9.2 Hz, 1H), 7.37-7.48 (m, 3H), 7.52-7.60 (m, 2H), 7.66 (d, J = 2.6 Hz, 1H), 7.89-7.98 (m, 2H), 8.07 (d, J = 8.4 Hz, 1H), 8.20 (s, 1H), 8.91 (s, 1H), 10.28 (s, 1H).
ESI m/z $(M+H)^+$ 645.

Example 113

2-Amino-7-hydroxy-6-{2-methyl-5-N-[2-(4-methylpiperazin-1-yl)-5-trifluoromethylphenyl]carbamoylphen yl}-8-(1-methyl-1H-pyrazol-4-yl)quinazoline (Compound 113)

Compound 113 was obtained in the same manner as in Example 4, using Compound 107.
$^1$H NMR (270 MHz, DMSO-$d_6$) δ (ppm) 1.98 (s, 3H), 2.23 (s, 3H), 2.34-2.45 (m, 4H), 2.85-2.94 (m, 4H), 3.89 (s, 3H), 6.69 (br s, 2H), 7.36-7.56 (m, 4H), 7.80 (s, 1H), 7.91 (d, J = 7.9 Hz, 1H), 8.11 (s, 1H), 8.39 (s, 1H), 8.47 (s, 1H), 8.89 (s, 1H), 9.65 (s, 1H).
ESI m/z $(M+H)^+$ 617.

Example 114

2-Amino-8-(2-fluoropyridin-5-yl)-7-hydroxy-6-[2-methyl-5-N-(2-morpholino-5-trifluoromethylphenyl)car bamoylphenyl] quinazoline (Compound 114)

Compound 114 was obtained in the same manner as in Example 4, using Compound 108.
$^1$H NMR (270 MHz, DMSO-$d_6$) δ (ppm) 2.27 (s, 3H), 2.88-2.98 (m, 4H), 3.70-3.79 (m, 4H), 6.64 (br s, 2H), 7.20-7.29 (m, 1H), 7.38-7.56 (m, 3H), 7.65 (s, 1H) 7.80-8.04 (m, 3H), 8.22 (d, J = 1.7 Hz, 1H), 8.39 (d, J = 1.7 Hz, 1H), 8.92 (br s, 1H), 9.74 (s, 1H).
ESI m/z $(M+H)^+$ 619.

Example 115

2-Amino-7-methoxy-6-[2-methyl-5-N-(2-morpholino-5-trifluoromethylphenyl)carbamoylphenyl]-8-(1-met hyl-1H-pyrazol-4-yl)quinazoline (Compound 115)

Compound 115 was obtained in the same manner as in Example 1, using Compound A43 and 1-methyl-4-(4,4,5,5-tetramethyl-1,3,2-dioxaboran-2-yl)-1H-pyrazole.
$^1$H NMR (270 MHz, DMSO-$d_6$) δ (ppm) 2.28 (s, 3H), 2.87-2.98 (m, 4H), 3.22 (s, 3H), 3.68-3.77 (m, 4H), 3.92 (s, 3H), 6.95 (br s, 2H), 7.36-7.63 (m, 4H), 7.88-7.96 (m, 2H), 8.26-8.31 (m, 1H), 8.38 (d, J = 1.7 Hz, 1H), 8.62 (s, 1H), 9.05 (s, 1H), 9.76 (s, 1H).
ESI m/z $(M+H)^+$ 618.

Example 116

2-Amino-7-hydroxy-6-[2-methyl-5-N-(2-morpholino-5-trifluoromethylphenyl)carbamoylphenyl]-8-(1-methyl-1H-pyrazol-4-yl)quinazoline (Compound 116)

Compound 116 was obtained in the same manner as in Example 4, using Compound 115.
$^1$H NMR (270 MHz DMSO-$d_6$) δ (ppm) 2.23 (s, 3H), 2.88-2.96 (m, 4H), 3.70-3.78 (m, 4H), 3.89 (s, 3H), 6.71 (br s, 2H), 7.38-7.56 (m, 4H), 7.83 (s, 1H), 7.86-7.96 (m, 1H), 8.10 (s, 1H), 8.34-8.44 (m, 2H), 8.88 (s, 1H), 9.74 (s, 1H).
ESI m/z $(M+H)^+$ 604.

Example 117

2-Amino-7-methoxy-8-[2-(4-methylpiperazin-1-yl)pyrimidin-5-yl]-6-[2-methyl-5-N-(3-trifluoromethylphe nyl)carbamoylphenyl]quinazoline (Compound 117)

Compound 117 was obtained in the same manner as in Example 1, using Compound A11 and 1-methyl-4-[5-(4,4,5,5-tetramethyl-1,3,2-dioxaboran-2-yl)pyrimidin-2-yl]piperazine.
$^1$H NMR (300 MHz, $CDCl_3$, 40°C) δ (ppm) 2.33 (s, 3H), 2.36 (s, 3H), 2.48-2.55 (m, 4H), 3.22 (s, 3H), 3.90-3.97 (m, 4H), 5.17 (br s, 2H), 7.36-7.52 (m, 3H), 7.55 (s, 1H), 7.80-7.95 (m, 4H), 7.98 (s, 1H), 8.63 (s, 2H), 8.97 (s, 1H).
ESI m/z $(M+H)^+$ 629.

Example 118

2-Amino-8-(2-fluoropyridin-5-yl)-7-hydroxy-6-{2-methyl-5-N-[2-(1-methylpiperidin-4-yl)oxy-5-trifluoro methylphenyl]carbamoylphenyl}quinazoline (Compound 118)

Compound 118 was obtained in the same manner as in Example 1 and Example 4, using Compound A25 and 2-fluoro-5-pyridine boronic acid.
$^1$H NMR (270 MHz, DMSO-$d_6$) δ (ppm) 1.67-1.82 (m, 2H), 1.83-1.97 (m, 2H), 2.00 (s, 3H), 2.15-2.32 (m, 5H), 2.40-2.50 (m, 18H), 4.61-4.72 (m, 1H), 6.65 (br s, 2H), 7.22-7.38 (m, 2H), 7.49 (d, J = 7.9 Hz, 2H), 7.64 (s, 1H), 7.82-7.93 (m, 2H), 7.93-8.04 (m, 1H), 8.22 (d, J = 2.0 Hz, 1H), 8.28-8.35 (m, 1H), 8.92 (s, 1H), 9.48 (s, 1H).
ESI m/z $(M+H)^+$ 647.

Example 119

2-Amino-8-(2-fluoropyridin-5-yl)-7-hydroxy-6-{2-methyl-5-N-[2-(pyrrolidin-1-yl)-5-trifluoromethylpheny 1]carbamoylphenyl}quinazoline (Compound 119)

Compound 119 was obtained in the same manner as in Example 1 and Example 4, using Compound A44 and 2-fluoro-5-pyridine boronic acid.
$^1$H NMR (270 MHz, DMSO-$d_6$) δ (ppm) 1.78-1.88 (m, 4H), 2.24 (s, 3H), 3.31-3.38 (m, 4H), 6.66 (br s, 2H), 6.87 (d, J = 8.6 Hz, 1H), 7.22-7.50 (m, 4H), 7.64 (s, 1H), 7.87-8.03 (m, 3H), 8.22 (d, J = 1.7 Hz, 1H), 8.32 (s, 0H), 8.97 (s, 1H), 9.18 (br s, 1H), 10.01 (s, 1H).
ESI m/z $(M+H)^+$ 603.

Example 120

2-Amino-7-hydroxy-8-[2-(4-methylpiperazin-1-yl)pyrimidin-5-yl]-6-[2-methyl-5-N-(3-trifluoromethylphen yl)carbamoylphenyl]quinazoline (Compound 120)

Compound 120 was obtained in the same manner as in Example 4, using Compound 117.
$^1$H NMR (300 MHz, DMSO-$d_6$, 120°C) δ (ppm) 2.26 (s, 3H), 2.34 (s, 3H), 2.50-2.58 (m, 4H), 3.81-3.90 (m, 4H), 6.22 (br s, 2H), 7.34-7.47 (m, 2H), 7.50-7.59 (m, 2H), 7.90-7.98 (m, 2H), 8.07 (d, J = 8.4 Hz, 1H), 8.19 (s, 1H), 8.41 (s, 2H), 8.90 (s, 1H) 10.17 (s, 1H).
ESI m/z $(M+H)^+$ 615.

Example 121

2-Amino-8-(2-dimethylaminopyrimidin-5-yl)-7-hydroxy-6-[2-methyl-5-N-(3-trifluoromethylphenyl)carba moylphenyl] quinazoline (Compound 121)

Compound 121 was obtained in the same manner as in Example 4, using Compound 24.
$^1$H NMR (300 MHz, DMSO-$d_6$, 80°C) $\delta$ (ppm) 2.25 (s, 3H), 3.19 (s, 6H), 6.39 (br s, 2H), 7.36-7.48 (m, 2H), 7.52-7.60 (m, 2H), 7.91-7.98 (m, 2H), 8.07 (d, J = 8.4 Hz, 1H), 8.21 (s, 1H), 8.35 (s, 2H), 8.91 (s, 1H), 10.28 (s, 1H).
ESI m/z $(M+H)^+$ 560.

Example 122

2-Amino-7-hydroxy-8-(2-methoxypyridin-5-yl)-6-[2-methyl-5-N-(3-trifluoromethylphenyl)carbamoylphen yl]quinazoline (Compound 122)

Compound 122 was obtained in the same manner as in Example 4, using Compound 23.
$^1$H NMR (270 MHz, DMSO-$d_6$) $\delta$ (ppm) 2.25 (s, 3H), 3.90 (s, 3H), 6.57 (br s, 2H), 6.89 (d, J = 8.1 Hz, 1H), 7.40-7.74 (m, 5H), 7.91-7.99 (m, 2H), 8.11-8.18 (m, 2H), 8.25 (s, 1H), 8.61 (s, 1H), 8.90 (s, 1H), 10.47 (s, 1H).
ESI m/z $(M+H)^+$ 546.

Example 123

2-Amino-7-methoxy-8-(1-methyl-1H-pyrazol-4-yl)-6-{2-methyl-5-N-[2-(pyrrolidin-1-yl)-5-trifluoromethyl phenyl]carbamoylphenyl}quinazoline (Compound 123)

Compound 123 was obtained in the same manner as in Example 1, using Compound A44 and 1-methyl-4-(4,4,5,5-tetramethyl-1,3,2-dioxaboran-2-yl)-1H-pyrazole.
$^1$H NMR (270 MHz, $CDCl_3$) $\delta$ (ppm) 1.91-2.01 (m, 4H), 2.34 (s, 3H), 3.10-3.21 (m, 4H), 3.28 (s, 3H), 4.02 (s, 3H), 5.23 (br s, 2H), 7.15 (d, J = 8.3 Hz, 1H), 7.30-7.38 (m, 1H), 7.43-7.51 (m, 2H), 7.80-7.86 (m, 1H), 7.90 (d, J = 1.7 Hz, 1H), 8.28 (s, 2H), 8.55 (s, 1H), 8.75 (s, 1H), 8.97 (s, 1H).
ESI m/z $(M+H)^+$ 602.

Example 124

2-Amino-7-hydroxy-8-(1-methyl-1H-pyrazol-4-yl)-6-{2-methyl-5-N-[2-(pyrrolidin-1-yl)-5-trifluoromethyl phenyl]carbamoylphenyl}quinazoline (Compound 124)

Compound 124 was obtained in the same manner as in Example 4, using Compound 123.
$^1$H NMR (300 MHz, DMSO-$d_6$) $\delta$ (ppm) 1.76-1.87 (m, 4H), 2.19 (s, 3H), 3.31-3.38 (m, 4H), 3.89 (s, 3H), 6.68 (br s, 2H), 6.85 (d, J = 8.8 Hz, 1H), 7.32 (s, 1H), 7.36-7.47 (m, 3H), 7.85-7.96 (m, 2H), 8.09 (s, 1H), 8.37 (s, 1H), 8.89 (s, 1H), 10.00 (s, 1H).
ESI m/z $(M+H)^+$ 588.

Example 125

2-Amino-7-hydroxy-8-[2-(4-hydroxypiperidin-1-yl)pyridin-5-yl]-6-[2-methyl-5-N-(3-trifluoromethylpheny l)carbamoylphenyl]quinazoline (Compound 125)

Compound 83 (102 mg, 0.19 mmol) was dissolved in NMP (1 mL), and 4-hydroxypiperidin (58 mg, 0.57 mmol) was added thereto, followed by stirring at 150°C for 3 hours under microwave irradiation. To the reaction mixture was added water, followed by extraction with ethyl acetate, and the organic layer was washed with brine, followed by drying over anhydrous magnesium sulfate. The solvent was evaporated under reduced pressure, and the residue was purified by thin-layer chromatography (ethyl acetate/methanol = 20/l) to obtain Compound 125 (56 mg, 49%).
$^1$H NMR (270 MHz, DMSO-$d_6$) $\delta$ (ppm) 1.28-1.46 (m, 2H), 1.74-1.88 (m, 2H), 2.23 (s, 3H), 3.00-3.15 (m, 2H), 3.62-3.78 (m, 1H), 4.00-4.13 (m, 2H), 4.76 (d, J = 4.0 Hz, 1H), 6.53 (br s, 2H), 6.89 (d, J = 8.9 Hz, 1H), 7.39-7.61 (m, 5H), 7.88-7.96 (m, 2H), 8.03-8.10 (m, 2H), 8.22 (s, 1H), 8.89 (s, 1H), 10.46 (s, 1H).
ESI m/z $(M+H)^+$ 615.

Example 126

2-Amino-7-hydroxy-8-(1-methyl-1H-pyrazol-4-yl)-6-[2-methyl-5-(3-trifluoromethylbenzamide)phenyl]qui nazoline (Compound 126)

Compound 126 was obtained in the same manner as in Example 4, using Compound 37.
$^1$H NMR (270 MHz, DMSO-$d_6$) δ (ppm) 2.12 (s, 3H), 3.88 (s, 3H), 6.39 (br s, 2H), 7.18-7.32 (m, 2H), 7.61 (s, 1H), 7.69-7.83 (m, 2H), 7.96 (d, J = 7.6 Hz, 1H), 8.22-8.42 (m, 3H), 8.50-8.76 (m, 2H), 10.42 (s, 1H).
ESI m/z $(M+H)^+$ 519.

Example 127

2-Amino-7-hydroxy-6-[2-methyl-5-(3-trifluoromethylbenzamide)phenyl]-8-(2-pyrrolidinylpyrimidin-5-yl) quinazoline (Compound 127)

Compound 127 was obtained in the same manner as in Example 1 and Example 4, using Compound A7 and 2-pyrrolidinyl-5-(4,4,5,5-tetramethyl-1,3,2-dioxaboran-2-yl)pyrimidine.
$^1$H NMR (270 MHz, DMSO-$d_6$) δ (ppm) 1.87-1.98 (in, 4H), 2.14 (s, 3H), 3.47-3.58 (m, 4H), 7.15-7.42 (m, 2H), 7.54-7.83 (m, 3H), 7.96 (d, J = 7.6 Hz, 1H), 8.21-8.45 (m, 4H), 10.41 (s, 1H).
ESI m/z $(M+H)^+$ 586.

Example 128

2-Amino-8-(indol-5-yl)-7-methoxy-6-[2-methyl-5-N-(3-trifluoromethylphenyl)carbamoylphenyl]quinazoli ne (Compound 128)

Compound 128 was obtained in the same manner as in Example 1, using Compound A11 and 5-(4,4,5,5-tetramethyl-1,3,2-dioxaboran-2-yl)indole.
$^1$H NMR (270 MHz, DMSO-$d_6$) δ (ppm) 2.30 (s, 3H), 2.99 (s, 3H), 6.45 (s, 1H), 6.68 (br s, 2H), 7.12 (d, J = 8.6 Hz, 1H), 7.35 (s, 1H), 7.40-7.65 (m, 5H), 7.71 (s, 1H), 7.92-8.14 (m, 3H), 8.23 (s, 1H), 9.09 (s, 1H), 10.47 (s, 1H), 11.09 (s, 1H).
ESI m/z $(M+H)^+$ 568.

Example 129

2-Amino-7-methoxy-8-(N-methylindol-5-yl)-6-[2-methyl-5-N-(3-trifluoromethylphenyl)carbamoylphenyl] quinazoline (Compound 129)

Compound 129 was obtained in the same manner as in Example 1, using Compound A11 and 1-methyl-5-(4,4,5,5-tetramethyl-1,3,2-dioxaboran-2-yl)indole.
$^1$H NMR (270 MHz, $CDCl_3$) δ (ppm) 2.36 (s, 3H), 3.09 (s, 3H), 3.81 (s, 3H), 5.08 (br s, 2H), 6.47 (d, J = 3.0 Hz, 1H), 7.06 (d, J = 3.0 Hz, 1H), 7.34-7.53 (m, 6H), 7.75 (s, 1H), 7.80-7.97 (m, 4H), 8.07 (s, 1H), 8.95 (s, 1H).
ESI m/z $(M+H)^+$ 5 82.

Example 130

2-Amino-7-methoxy-6-[2-methyl-5-N-(3-trifluoromethylphenyl)carbamoylphenyl]-8-(quinolin-6-yl)quinaz oline (Compound 130)

Compound 130 was obtained in the same manner as in Example 1, using Compound A11 and 6-(4,4,5,5-tetramethyl-1,3,2-dioxaboran-2-yl)quinoline.
$^1$H NMR (270 MHz, $CDCl_3$) δ (ppm) 2.38 (s, 3H), 3.10 (s, 3H), 5.14 (br s, 2H), 7.35-7.54 (m, 4H), 7.62 (s, 1H), 7.81-7.96 (m, 5H), 8.03 (s, 2H), 8.20 (d, J = 8.6 Hz, 2H), 8.95 (d, J = 2.6 Hz, 1H), 9.01 (s, 1H).
ESI m/z $(M+H)^+$ 580.

Example 131

2-Amino-7-hydroxy-8-(indol-5-yl)-6-[2-methyl-5-N-(3-trifluoromethylphenyl)carbamoylphenyl]quinazolin e (Compound 131)

Compound 131 was obtained in the same manner as in Example 4, using Compound 128.
$^1$H NMR (270 MHz, DMSO-$d_6$) δ (ppm) 2.28 (s, 3H), 6.10-6.48 (m, 3H), 7.04 (d, J = 8.6 Hz, 1H), 7.27-7.63 (m, 7H), 7.83-7.99 (m, 2H), 8.10 (d, J = 7.9 Hz, 1H), 8.25 (s, 1H), 8.83 (br s, 1H), 10.47 (s, 1H), 11.01 (s, 1H).
ESI m/z $(M+H)^+$ 554.

Example 132

2-Amino-7-hydroxy-6-[2-methyl-5-N-(3-trifluoromethylphenyl)carbamoylphenyl]-8-(quinolin-6-yl)quinaz oline (Compound 132)

Compound 132 was obtained in the same manner as in Example 4, using Compound 130.
$^1$H NMR (270 MHz, DMSO-$d_6$) δ (ppm) 2.29 (s, 3H), 6.55 (br s, 2H), 7.43-7.66 (m, 5H), 7.76 (d, J = 8.6 Hz, 1H), 7.90-8.03 (m, 3H), 8.04-8.15 (m, 2H), 8.25 (s, 1H), 8.41 (d, J = 7.9 Hz, 1H), 8.86-9.04 (m, 2H), 10.48 (s, 1H).
ESI m/z $(M+H)^+$ 566.

Example 133

2-Amino-8-(2-chloropyridin-4-yl)-7-hydroxy-6-[2-methyl-5-N-(3-trifluoromethylphenyl)carbamoylphenyl] quinazoline (Compound 133)

Compound 133 was obtained in the same manner as in Example 4, using Compound 39.
$^1$H NMR (300 MHz, DMSO-$d_6$, 80°C) δ (ppm) 2.25 (s, 3H), 6.47 (br s, 2H), 7.36-7.52 (m, 4H), 7.52-7.61 (m, 1H), 7.63 (s, 1H), 7.91-7.98 (m, 2H), 8.07 (d, J = 8.4 Hz, 1H), 8.20 (s, 1H), 8.43 (d, J = 4.8 Hz, 1H), 8.92 (s, 1H), 10.27 (s, 1H).
ESI m/z $(M+H)^+$ 550.

Example 134

2-Amino-7-hydroxy-8-[2-(4-hydroxypiperidin-1-yl)pyridin-4-yl]-6-[2-methyl-5-N-(3-trifluoromethylpheny l)carbamoylphenyl]quinazoline (Compound 134)

Compound 134 was obtained in the same manner as in Example 125, using Compound 39.
$^1$H NMR (300 MHz, DMSO-$d_6$, 80°C) δ (ppm) 1.34-1.52 (m, 2H), 1.72-1.88 (m, 2H), 2.24 (s, 3H), 3.08-3.18 (m, 2H), 3.65-3.78 (m, 1H), 3.92-4.07 (m, 2H), 4.39 (s, 1H), 6.35 (br s, 2H), 6.60 (d, J = 5.1 Hz, 1H), 6.76 (s, 1H), 7.36-7.49 (m, 2H), 7.52-7.62 (m, 2H), 7.88-7.98 (m, 2H), 8.04-8.23 (m, 3H), 8.58 (br s, 1H), 8.92 (s, 1H), 10.28 (s, 1H).
ESI m/z $(M+H)^+$ 615.

Example 135

8-(2-Fluoropyridin-5-yl)-7-hydroxy-2-methylamino-6-[2-methyl-5-N-(3-trifluoromethylphenyl)carbamoylp henyl]quinazoline (Compound 135)

Compound 135 was obtained in the same manner as in Example 83, using Compound A47.
$^1$H NMR (270 MHz, DMSO-$d_6$) δ (ppm) 2.27 (s 3H), 2.71 (br s, 3H), 7.12-7.28 (m, 1H), 7.25 (dd, J = 8.6, 3.0 Hz, 1H), 7.40-7.63 (m, 3H), 7.66 (s, 1H), 7.93-8.00 (m, 2H), 8.04-8.18 (m, 2H), 8.25 (br s, 1H), 8.32 (br s, 1H), 8.96 (s, 1H), 9.30 (br s, 1H), 10.48 (s, 1H).
ESI m/z $(M+H)^+$ 548.

Example 136

2-Amino-7-hydroxy-8-(N-methylindol-5-yl)-6-[2-methyl-5-N-(3-trifluoromethylphenyl)carbamoylphenyl] quinazoline (Compound 136)

Compound 136 was obtained in the same manner as in Example 4, using Compound 129.

$^{1}H$ NMR (270 MHz, $CDCl_3$) δ (ppm) 2.33 (s, 3H), 3.76 (s, 3H), 5.01 (br s, 2H), 5.99 (s, 1H), 6.32 (s, 1H), 7.05 (d, J = 3.0 Hz, 1H), 7.17-7.26 (m, 1H), 7.31-7.47 (m, 4H), 7.63 (s, 1H), 7.77-7.94 (m, 4H), 8.08 (s, 1H), 8.85 (s, 1H).
ESI m/z $(M+H)^+$ 568.

Example 137

2-Amino-7-hydroxy-6-[2-methyl-5-(3-trifluoromethylbenzamide)phenyl]-8-(2-morpholinopyrimidin-5-yl)q uinazoline (Compound 137)

Compound 137 was obtained in the same manner as in Example 1 and Example 4, using Compound A7 and 2-morpholino-5-(4,4,5,5-tetramethyl-1,3,2-dioxaboran-2-yl)pyrimidine.
$^{1}H$ NMR (270 MHz, DMSO-$d_6$) δ (ppm) 2.14 (s, 3H), 3.65-3.80 (m, 8H), 6.63 (br s, 2H), 7.28 (d, J = 8.3 Hz, 1H), 7.54 (s, 1H), 7.64-7.83 (m, 3H), 7.97 (d, J = 7.6 Hz, 1H), 8.22-8.33 (m, 2H), 8.40 (s, 2H), 8.93 (s, 1H), 9.09 (br s, 1H), 10.45 (s, 1H).
ESI m/z $(M+H)^+$ 602.

Example 138

2-Amino-7-hydroxy-6-[2-methyl-5-(3-trifluoromethylbenzamide)phenyl]-8-(2-morpholinopyridin-5-yl)qui nazoline (Compound 138)

Compound 138 was obtained in the same manner as in Example 1 and Example 4, using Compound A7 and 2-morpholino-5-(4,4,5,5-tetramethyl-1,3,2-dioxaboran-2-yl)pyridine.
$^{1}H$ NMR (270 MHz, DMSO-$d_6$) δ (ppm) 2.14 (s, 3H), 3.41-3.52 (m, 4H), 3.68-3.77 (m, 4H), 6.26 (br s, 2H), 6.87 (d, J = 8.9 Hz, 1H), 7.23 (d, J = 8.3 Hz, 1H), 7.38 (s, 1H), 7.56-7.83 (m, 4H), 7.96 (d, J = 7.9 Hz, 1H), 8.14-8.33 (m, 3H), 8.71 (br s, 1H), 10.42 (s, 1H).
ESI m/z $(M+H)^+$ 601.

Example 139

2-Amino-7-methoxy-8-[2-(1-methyl-1H-pyrazol-4-yl)pyridin-5-yl]-6-[2-methyl-5-N-(3-trifluoromethylphe nyl)carbamoylphenyl]quinazoline (Compound 139)

Compound 139 was obtained in the same manner as in Example 1, using Compound 20 and 1-methyl-4-(4,4,5,5-tetramethyl-1,3,2-dioxaboran-2-yl)-1H-pyrazole.
$^{1}H$ NMR (300 MHz, $CDCl_3$, 50°C) δ (ppm) 2.34 (s, 3H), 3.17 (s, 3H), 3.96 (s, 3H), 5.13 (br s, 2H), 7.35-7.52 (m, 3H), 7.53-7.60 (m, 2H), 7.80-7.95 (m, 5H), 7.95-8.02 (m, 3H), 8.77 (d, J = 1.5 Hz, 1H), 8.98 (s, 1H).
ESI m/z $(M+H)^+$ 610.

Example 140

2-Amino-7-methoxy-8-[2-(1-methyl-1H-pyrazol-4-yl)pyridin-4-yl]-6-[2-methyl-5-N-(3-trifluoromethylphe nyl)carbamoylphenyl]quinazoline (Compound 140)

Compound 140 was obtained in the same manner as in Example 1, using Compound 39 and 1-methyl-4-(4,4,5,5-tetramethyl-1,3,2-dioxaboran-2-yl)-1H-pyrazole.
$^{1}H$ NMR (270 MHz, $CDCl_3$, 50°C) δ (ppm) 2.34 (s, 3H), 3.19 (s, 3H), 3.94 (s, 3H), 5.15 (br s, 2H), 7.24-7.30 (m, 1H), 7.33-7.52 (m, 4H), 7.58-7.65 (m, 2H), 7.79-7.97 (m, 6H), 8.01 (s, 1H), 8.60-8.66 (m, 1H), 8.98 (s, 1H).
ESI m/z $(M+H)^+$ 610.

Example 141

2-Amino-7-hydroxy-8-[2-(1-methyl-1H-pyrazol-4-yl)pyridin-5-yl]-6-[2-methyl-5-N-(3-trifluoromethylphe nyl)carbamoylphenyl]quinazoline (Compound 141)

Compound 141 was obtained in the same manner as in Example 4, using Compound 139.
$^{1}H$ NMR (300 MHz, DMSO-$d_6$, 80°C) δ (ppm) 2.27 (s, 3H), 3.90 (s, 3H), 6.36 (br s, 2H), 7.35-7.50 (m, 2H), 7.51-7.79 (m, 4H), 7.90-8.02 (in, 3H), 8.08 (d, J = 8.4 Hz, 1H), 8.18-8.26 (m, 2H), 8.52 (s, 1H), 8.83 (br s, 1H), 8.94 (br s, 1H),

10.29 (s, 1H).
ESI m/z $(M+H)^+$ 596.

Example 142

2-Amino-8-[2-(4,4-difluoropiperidin-1-yl)pyridin-5-yl]-7-hydroxy-6-[2-methyl-5-N-(3-trifluoromethylphen yl)carbamoylphenyl]quinazoline (Compound 142)

Compound 28 (0.10 g, 0.183 mmol) was dissolved in NMP (0.92 mL), and 4,4-difluoropiperidin·hydrochloride (0.087 g, 0.183 mmol) and diisopropylethylamine (0.16 mL, 0.915 mmol) were added thereto, followed by stirring at 160°C for 5 hours under microwave irradiation. To the reaction mixture was added saturated aqueous sodium bicarbonate, followed by extraction with ethyl acetate, and the organic layer was washed with brine, followed by drying over anhydous sodium sulfate. The solvent was evaporated under reduced pressure, and the residue was purified by thin-layer silica gel column chromatography (chloroform/methanol = 10/1) to obtain compound 142 (0.05 g, 43%).
$^1$H NMR (270 MHz, DMSO-$d_6$) $\delta$ (ppm) 1.94-2.12 (m, 4H), 2.25 (s, 3H), 3.70-3.80 (m, 4H), 6.58 (br s, 2H), 7.04 (d, J = 8.6 Hz, 1H), 7.40-7.50 (m, 2H), 7.54-7.63 (m, 3H), 7.90-7.99 (m, 2H), 8.05-8.17 (m, 2H), 8.24 (s, 1H), 8.93 (s, 1H), 10.47 (s, 1H).
ESI m/z $(M+H)^+$ 635.

Example 143

2-Acetylamino-7-methoxy-8-(1-methyl-1H-pyrazol-4-yl)-6-[2-methyl-5-N-(3-trifluoromethylphenyl)carba moylphenyl] quinazoline (Compound 143)

Compound 19 (40 mg, 0.075 mmol) was suspended in pyridine (0.5 mL), and acetate anhydride (0.035 mL, 0.36 mmol) was added thereto, followed by stirring at 80°C for 2 hours. The reaction mixture was added to water, and the resulting crystals were collected by filtration to obtain Compound 143 (33.3 mg, 77%).
$^1$H NMR (270 MHz, $CDCl_3$) $\delta$ (ppm) 2.33 (s, 3H), 2.49 (s, 3H), 3.34 (s, 3H), 4.03 (s, 3H), 7.36-7.55 (m, 3H), 7.56 (s, 1H), 7.85-8.00 (m, 4H), 8.18 (br s, 1H), 8.29 (s, 1H), 8.64 (s, 1H), 8.93 (br s, 1H), 9.17 (s, 1H),
ESI m/z $(M+H)^+$ 575.

Example 144

2-Cyclopropylcarbonylamino-7-methoxy-8-(1-methyl-1H-pyrazol-4-yl)-6-[2-methyl-5-N-(3-trifluoromethy lphenyl)carbamoylphenyl]quinazoline (Compound 144)

Compound 144 was obtained in the same manner as in Example 143, using Compound 19 and cyclopropanecarbonyl chloride.
$^1$H NMR (270 MHz, $CDCl_3$) $\delta$ (ppm) 0.92-1.02 (m, 2H), 1.20-1.31 (m, 2H), 2.12-2.28 (m, 1H), 2.33 (s, 3H), 3.34 (s, 3H), 4.02 (s, 3H), 7.37-7.53 (m, 3H), 7.57 (s, 1H), 7.85-8.00 (m, 4H), 8.10 (s, 1H), 8.35 (s, 1H), 8.57 (br s, 1H), 9.05 (br s, 1H), 9.17 (s, 1H).
ESI m/z $(M+H)^+$ 601.

Example 145

2-Amino-7-methoxy-8-(2-methylpyridin-5-yl)-6-[2-methyl-5-N-(3-trifluoromethylphenyl)carbamoylphenyl ]quinazoline (Compound 145)

Compound 145 was obtained in the same manner as in Example 1, using Compound A11 and 2-methyl-5-(4,4,5,5-tetramethyl-1,3,2-dioxaboran-2-yl)pyridine.
$^1$H NMR (270 MHz, $CDCl_3$) $\delta$ (ppm) 2.34 (s, 3H), 2.65 (s, 3H), 3.15 (s, 3H), 5.15 (s, 2H), 7.24-7.31 (m, 1H), 7.36-7.53 (m, 3H), 7.60 (s, 1H), 7.79-8.03 (m, 6H), 8.69-8.73 (m, 1H), 8.99 (s, 1H).
ESI m/z $(M+H)^+$ 544.

Example 146

2-Amino-8-(2-cyanopyridin-5-yl)-7-methoxy-6-[2-methyl-5-N-(3-trifluoromethylphenyl)carbamoylphenyl] quinazoline (Compound 146)

Compound 146 was obtained in the same manner as in Example 1, using Compound A11 and 2-cyano-5-(4,4,5,5-tetramethyl-1,3,2-dioxaboran-2-yl)pyridine.
$^1$H NMR (270 MHz, $CDCl_3$) δ (ppm) 2.35 (s, 3H), 3.15 (s, 3H), 5.19 (s, 2H), 7.39-7.54 (m, 3H), 7.69 (s, 1H), 7.77-7.96 (m, 6H), 8.08 (dd, J = 7.9, 2.0 Hz, 1H), 8.93-8.95 (m, 1H), 9.02 (s, 1H).
ESI m/z $(M+H)^+$ 555.

Example 147

2-Amino-7-hydroxy-8-[2-(1-methyl-1H-pyrazol-4-yl)pyridin-4-yl]-6-[2-methyl-5-N-(3-trifluoromethylphe nyl)carbamoylphenyl]quinazoline (Compound 147)

Compound 147 was obtained in the same manner as in Example 4, using Compound 140.
$^1$H NMR (300 MHz, DMSO-$d_6$, 80°C) δ (ppm) 2.26 (s, 3H), 3.87 (s, 3H), 6.38 (br s, 2H), 7.13-7.20 (m, 1H), 7.37-7.50 (m, 2H), 7.52-7.65 (m, 3H), 7.90-7.99 (m, 3H), 8.08 (d, J = 7.7 Hz, 1H), 8.21 (s, 2H), 8.54 (d, J = 5.1 Hz, 1H), 8.93 (br s, 1H), 10.29 (s, 1H).
ESI m/z $(M+H)^+$ 596.

Example 148

2-Amino-7-hydroxy-6-[2-methyl-5-N-(3-trifluoromethylphenyl)carbamoylphenyl]-8-[2-(1H-pyrazol-1-yl)p yridin-4-yl] quinazoline (Compound 148)

Compound 133 (0.14 g, 0.25 mmol) was dissolved in NMP (1 mL), and pyrazole (0.085 g, 1.25 mmol), copper (1) iodide (0.010 g, 0.05 mmol) and potassium carbonate (0.069 g, 0.50 mmol) were added thereto, followed by stirring at 150°C for 14 hours under microwave irradiation. To the reaction mixture was added water, and the pH was adjusted to 7 with 1 mol/L hydrochloric acid, followed by extraction with ethyl acetate/isopropanol (5/1), then the organic layer was washed with brine, followed by drying over anhydous sodium sulfate. The solvent was evaporated under reduced pressure, and the residue was purified by silica gel column chromatography (ethyl acetate/methanol = 9/1) to obtain Compound 148 (0.043 g, 30%).
$^1$H NMR (300 MHz, DMSO-$d_6$, 80°C) δ (ppm) 2.27 (s, 3H), 6.42 (br s, 2H), 6.52-6.58 (m, 1H), 7.32-7.50 (m, 3H), 7.52-7.61 (m, 1H), 7.65 (s, 1H), 7.76 (s, 1H), 7.91-7.99 (m, 3H), 8.08 (d, J = 8.1 Hz, 1H), 8.21 (s, 1H), 8.49 (d, J = 5.1 Hz, 1H), 8.64 (d, J = 2.6 Hz, 1H), 8.94 (br s, 1H), 10.28 (s, 1H).
ESI m/z $(M+H)^+$ 582.

Example 149

2-Amino-7-hydroxy-8-{2-[4-(2-hydroxyethyl)piperazin-1-yl]pyridin-5-yl}-6-[2-methyl-5-N-(3-trifluorome thylphenyl)carbamoylphenyl]quinazoline (Compound 149)

Compound 149 was obtained in the same manner as in Example 142, using Compound 28 and 1-(2-hydroxyethyl)piperazine.
$^1$H NMR (300 MHz, DMSO-$d_6$) δ (ppm) 2.25 (s, 3H), 2.40-2.59 (m, 6H), 3.47-3.60 (m, 6H), 4.44 (t, J = 5.5 Hz, 1H), 6.54 (br s, 2H), 6.90 (d, J = 8.8 Hz, 1H), 7.40-7.50 (m, 2H), 7.51-7.64 (m, 3H), 7.91-7.98 (m, 2H), 8.06-8.14 (m, 2H), 8.24 (s, 1H), 8.90 (br s, 1H), 10.46 (s, 1H).
ESI m/z $(M+H)^+$ 644.

Example 150

2-Acetylamino-7-hydroxy-8-(1-methyl-1H-pyrazol-4-yl)-6-[2-methyl-5-N-(3-trifluoromethylphenyl)carba moylphenyl] quinazoline (Compound 150)

Compound 150 was obtained in the same manner as in Example 4, using Compound 143.
$^1$H NMR (270 MHz, DMSO-$d_6$) δ (ppm) 2.22 (s, 3H), 2.22 (s, 3H), 3.87 (s, 3H), 7.38-7.50 (m, 3H), 7.58 (t, J = 8.0 Hz,

1H), 7.88-8.00 (m, 2H), 8.10 (d, J = 8.9 Hz, 1H), 8.26 (s, 1H), 8.67-8.91 (m, 2H), 9.23 (s, 1H), 10.30 (br s, 1H), 10.46 (s, 1H). ESI m/z $(M+H)^+$ 561.

Example 151

2-Cyclopropylcarbonylamino-7-hydroxy-8-(1-methyl-1H-pyrazol-4-yl)-6-[2-methyl-5-N-(3-trifluoromethy lphenyl)carbamoylphenyl]quinazoline (Compound 151)

Compound 151 was obtained in the same manner as in Example 4, using Compound 144.
$^1H$ NMR (270 MHz, DMSO-$d_6$) $\delta$ (ppm) 0.75-0.98 (m, 4H), 2.22 (s, 3H), 2.15-2.28 (m, 1H), 3.85 (s, 3H), 7.33-7.47 (m, 3H), 7.58 (t, J = 7.9 Hz, 1H), 7.83-7.96 (m, 2H), 8.05-8.14 (m, 1H), 8.26 (s, 1H), 8.70-8.88 (m, 2H), 9.19 (s, 1H), 10.46 (s, 1H), 10.56 (br s, 1H).
ESI m/z $(M+H)^+$ 587.

Example 152

2-Amino-7-hydroxy-8-(2-methylpyridin-5-yl)-6-[2-methyl-5-N-(3-trifluoromethylphenyl)carbamoylphenyl ]quinazoline (Compound 152)

Compound 152 was obtained in the same manner as in Example 4, using Compound 145.
$^1H$ NMR (270 MHz, DMSO-$d_6$) $\delta$ (ppm) 2.25 (s, 3H), 2.49 (s, 3H), 6.21 (br s, 2H), 7.23 (d, J = 7.8 Hz, 1H), 7.37-7.46 (m, 3H), 7.58 (t, J = 7.9 Hz, 1H), 7.71 (dd, J = 7.9, 2.3 Hz, 1H), 7.85-7.95 (m, 2H), 8.10 (d, J = 7.9 Hz, 1H), 8.25 (s, 1H), 8.47-8.50 (m, 1H), 8.69 (br s, 1H), 10.46 (s, 1H).
ESI m/z $(M+H)^+$ 530.

Example 153

2-Amino-8-(2-cyanopyridin-5-yl)-7-hydroxy-6-[2-methyl-5-N-(3-trifluoromethylphenyl)carbamoylphenyl] quinazoline (Compound 153)

Compound 153 was obtained in the same manner as in Example 4, using Compound 146.
$^1H$ NMR (270 MHz, DMSO-$d_6$) $\delta$ (ppm) 2.25 (s, 3H), 5.79 (br s, 1H), 7.20 (br s, 1H), 7.29-7.36 (m, 1H), 7.39-7.45 (m, 1H), 7.52-7.61 (m, 1H), 7.76-7.88 (m, 3H), 8.05-8.13 (m, 1H), 8.26 (s, 1H), 8.30-8.42 (m, 2H), 9.13 (s, 1H), 10.42 (s, 1H).
ESI m/z $(M+H)^+$ 541.

Example 154

2-Amino-8-(3-fluoropyridin-4-yl)-7-methoxy-6-[2-methyl-5-N-(3-trifluoromethylphenyl)carbamoylphenyl] quinazoline (Compound 154)

Compound A39 (0.20 g, 0.346 mmol) was dissolved in DMF (1.7 mL), and 3-fluoro-4-(tributylstannyl)pyridine (0.27 g, 0.692 mmol), bis(triphenylphosphi)palladium (II) dichloride (0.024 g, 0.0346 mmol) and copper (I) iodide (0.0066 g, 0.0346 mmol) were added thereto, followed by stirring at 160°C for 10 minutes under argon air flow. To the reaction mixture were added ethyl acetate and water, and insoluble materials were filtrated off through celite, followed by extraction with ethyl acetate. An organic layer was washed with brine, followed by drying over anhydous sodium sulfate. The solvent was evaporated under reduced pressure, and the residue was purified by silica gel column chromatography (ethyl acetate) to obtain Compound 154 (0.14 g, 75%).
$^1H$ NMR (300 MHz, $CDCl_3$) $\delta$ (ppm) 2.33 (s, 3H), 3.20 (s, 3H), 5.19 (br s, 2H), 7.34-7.56 (m, 4H), 7.66 (s, 1H), 7.80-7.98 (m, 4H), 8.08 (s, 1H), 8.44-8.64 (m, 2H), 8.99 (s, 1H).
ESI m/z $(M+H)^+$ 548.

Example 155

2-Amino-8-(3-chloropyridin-4-yl)-7-methoxy-6-[2-methyl-5-N-(3-trifluoromethylphenyl)carbamoylphenyl ]quinazoline (Compound 155)

Compound 155 was obtained in the same manner as in Example 154, using Compound A39 and 3-chloro-4-(tributylstannyl)pyridine.

$^{1}H$ NMR (270 MHz, $CDCl_3$) δ (ppm) 2.33 (s, 3H), 3.19 (s, 3H), 5.13 (br s, 2H), 7.34 (d, J = 5.0 Hz, 1H), 7.36-7.54 (m, 3H), 7.68 (s, 1H), 7.79-8.00 (m, 5H), 8.59 (d, J = 5.0 Hz, 1H), 8.75 (s, 1H), 9.00 (s, 1H).
ESI m/z $(M+H)^+$ 564.

Example 156

2-Amino-8-[2-(3,3-difluoropyrrolidin-1-yl)pyridin-4-yl]-7-hydroxy-6-[2-methyl-5-N-(3-trifluoromethylphe nyl)carbamoylphenyl]quinazoline (Compound 156)

Compound 156 was obtained in the same manner as in Example 142, using Compound 133 and 2,2-difluoro-pyrrolidine·hydrochloride.
$^{1}H$ NMR (300 MHz, DMSO-$d_6$, 80°C) δ (ppm) 2.25 (s, 3H), 2.42-2.62 (m, 2H), 3.66 (t, J = 7.1 Hz, 2H), 3.86 (t, J = 13.4 Hz, 2H), 6.37 (br s, 2H), 6.55 (s, 1H), 6.65-6.72 (m, 1H), 7.35-7.49 (m, 2H), 7.51-7.63 (m, 2H), 7.89-7.98 (m, 2H), 8.07 (d, J = 8.1 Hz, 1H), 8.12-8.24 (m, 2H), 8.63 (br s, 1H), 8.92 (br s, 1H), 10.28 (s, 1H).
ESI m/z $(M+H)^+$ 621.

Example 157

8-[2-(Acetylamino)pyridin-5-yl]-2-amino-7-methoxy-6-[2-methyl-5-N-(3-trifluoromethylphenyl)carbamoy lphenyl]quinazoline (Compound 157)

Compound 157 was obtained in the same manner as in Example 1, using Compound A11 and 2-acetylamino-5-(4,4,5,5-tetramethyl-1,3,2-dioxaboran-2-yl)pyridine.
$^{1}H$ NMR (300 MHz, $CDCl_3$, 50°C) δ (ppm) 2.22 (br s, 3H), 2.33 (s, 3H), 3.15 (s, 3H), 5.18 (br s, 2H), 7.34-7.53 (m, 3H), 7.58 (s, 1H), 7.80-7.97 (m, 6H), 8.00 (s, 1H), 8.11 (s, 1H), 8.27 (d, J = 8.4 Hz, 1H), 8.45-8.49 (m, 1H), 8.97 (s, 1H).
ESI m/z $(M+H)^+$ 587.

Example 158

2-Amino-7-hydroxy-6-[2-methyl-5-N-(3-trifluoromethylphenyl)carbamoylphenyl]-8-[2-(1H-1,2,4-triazol-1-yl)pyridin-4-yl] quinazoline (Compound 158)

Compound 158 was obtained in the same manner as in Example 148, using Compound 133 and 1,2,4-triazol.
$^{1}H$ NMR (300 MHz, DMSO-$d_6$, 80°C) δ (ppm) 2.27 (s, 3H), 6.45 (br s, 2H), 7.36-7.49 (m, 2H), 7.50-7.61 (m, 2H), 7.66 (s, 1H), 7.89-7.99 (m, 3H), 8.08 (d, J = 8.4 Hz, 1H), 8.18-8.24 (m, 2H), 8.57 (d, J = 5.1 Hz, 1H), 8.95 (br s, 1H), 9.34 (s, 1H), 10.28 (s, 1H).
ESI m/z $(M+H)^+$ 583.

Example 159

2-Acetylamino-8-(2-fluoropyridin-5-yl)-7-hydroxy-6-[2-methyl-5-N-(3-trifluoromethylphenyl)carbamoylp henyl]quinazoline (Compound 159)

Compound 159 was obtained in the same manner as in Example 143 and Example 4, using Compound 28.
$^{1}H$ NMR (300 MHz, DMSO-$d_6$) δ (ppm) 2.11 (s, 3H), 2.27 (s, 3H), 7.20-7.30 (m, 1H), 7.40-7.63 (m, 4H), 7.80-7.90 (m, 1H), 7.92-8.03 (m, 2H), 8.05-8.15 (m, 2H), 8.24 (s, 1H), 8.34 (s, 1H), 10.48 (s, 1H).
ESI m/z $(M+H)^+$ 576.

Example 160

2-Cyclopropylamino-8-(2-fluoropyridin-5-yl)-7-hydroxy-6-[2-methyl-5-N-(3-trifluoromethylphenyl)carba moylphenyl] quinazoline (Compound 160)

Compound 160 was obtained in the same manner as in Example 83, using Compound A52. $^{1}H$ NMR (270 MHz, DMSO-$d_6$) δ (ppm) 0.40-0.65 (m, 4H), 2.27 (s, 3H), 2.55-2.62 (m, 1H), 7.20-7.26 (m, 1H), 7.33-7.70 (m, 5H), 7.90-8.20 (m, 4H), 8.25 (s, 1H), 8.36 (s, 1H), 8.96 (s, 1H), 9.25-9.37 (m, 1H), 10.48 (s, 1H).
ESI m/z $(M+H)^+$ 574.

Example 161

8-(2-Acetylaminopyridin-5-yl)-2-amino-7-hydroxy-6-[2-methyl-5-N-(3-trifluoromethylphenyl)carbamoylp henyl]quinazoline (Compound 161)

Compound 161 was obtained in the same manner as in Example 4, using Compound 157.
$^1$H NMR (300 MHz, DMSO-$d_6$, 80°C) δ (ppm) 2.13 (s, 3H), 2.26 (s, 3H), 6.35 (br s, 2H), 7.37-7.49 (m, 2H), 7.51-7.63 (m, 2H), 7.72-7.80 (m, 1H), 7.89-7.99 (m, 2H), 8.02-8.14 (m, 2H), 8.20 (s, 1H), 8.25-8.32 (m, 1H), 8.78 (br s, 1H), 8.94 (s, 1H), 10.20 (s, 1H), 10.28 (s, 1H).
ESI m/z $(M+H)^+$ 573.

Example 162

(R)-2-Amino-7-hydroxy-8-[2-(3-hydroxypyrrolidin-1-yl)pyridin-4-yl]-6-[2-methyl-5-N-(3-trifluoromethylp henyl)carbamoylphenyl]quinazoline (Compound 162)

Compound 162 was obtained in the same manner as in Example 142, using Compound 133 and (R)-2-hydroxypyrrolidine hydrochloride.
$^1$H NMR (300 MHz, DMSO-$d_6$, 80°C) δ (ppm) 1.83-1.95 (m, 1H), 1.97-2.10 (m, 1H), 2.25 (s, 3H), 3.29-3.37 (m, 1H), 3.44-3.57 (m, 3H), 4.35-4.45 (m, 1H), 4.69 (d, J = 3.7 Hz, 1H), 6.26-6.45 (m, 3H), 6.49-6.56 (m, 1H), 7.36-7.47 (m, 2H), 7.52-7.61 (m, 2H), 7.88-7.97 (m, 2H), 8.03-8.12 (m, 2H), 8.20 (s, 1H), 8.54 (br s, 1H), 8.90 (br s, 1H), 10.28 (s, 1H).
ESI m/z $(M+H)^+$ 601.

Example 163

2-Amino-7-hydroxy-6-[2-methyl-5-N-(3-trifluoromethylphenyl)carbamoylphenyl]-8-[2-(1H-1,2,4-triazol-1-yl)pyridin-5-yl] quinazoline (Compound 163)

Compound 163 was obtained in the same manner as in Example 148, using Compound 90 and 1,2,4-triazol.
$^1$H NMR (300 MHz, DMSO-$d_6$, 80°C) δ (ppm) 2.27 (s, 3H), 6.42 (br s, 2H), 7.35-7.49 (m, 2H), 7.53-7.60 (m, 1H), 7.64 (s, 1H), 7.90-7.99 (m, 3H), 8.04-8.13 (m, 2H), 8.21 (s, 1H), 8.27 (s, 1H), 8.56 (d, J = 1.8 Hz, 1H), 8.94 (s, 1H), 9.34 (s, 1H), 10.29 (s, 1H).
ESI m/z $(M+H)^+$ 583.

Example 164

2-Amino-8-[2-(3,3-difluoropyrrolidin-1-yl)pyridin-5-yl]-7-hydroxy-6-[2-methyl-5-N-(3-trifluoromethylphe nyl)carbamoylphenyl]quinazoline (Compound 164)

Compound 164 was obtained in the same manner as in Example 142, using Compound 90 and 3,3-difluoropyrrolidine hydrochloride.
$^1$H NMR (300 MHz, DMSO-$d_6$, 80°C) δ (ppm) 2.25 (s, 3H), 2.47-2.64 (m, 2H), 3.63-3.73 (m, 2H), 3.82-3.96 (m, 2H), 6.29 (br s, 2H), 6.64 (d, J = 8.8 Hz, 1H), 7.37-7.48 (m, 2H), 7.50-7.61 (m, 3H), 7.90-7.98 (m, 2H), 8.02-8.23 (m, 3H), 8.56 (br s, 1H), 8.91 (br s, 1H), 10.28 (s, 1H).
ESI m/z $(M+H)^+$ 621.

Example 165

2-Amino-7-methoxy-8-(2-methylpyridin-4-yl)-6-[2-methyl-5-N-(3-trifluoromethylphenyl)carbamoylphenyl ]quinazoline (Compound 165)

Compound 165 was obtained in the same manner as in Example 1, using Compound A11 and 2-methyl-4-(4,4,5,5-tetramethyl-1,3,2-dioxaboran-2-yl)pyridine.
$^1$H NMR (300 MHz, $CDCl_3$, 50°C) δ (ppm) 2.33 (s, 3H), 2.63 (s, 3H), 3.15 (s, 3H), 5.14 (br s, 2H), 7.22-7.29 (m, 1H), 7.32 (s, 1H), 7.36-7.53 (m, 3H), 7.59 (s, 1H), 7.79-8.00 (m, 5H), 8.59 (d, J = 5.1 Hz, 1H), 8.97 (s, 1H).
ESI m/z $(M+H)^+$ 544.

Example 166

2-Amino-7-hydroxy-8-(2-methylpyridin-4-yl)-6-[2-methyl-5-N-(3-trifluoromethylphenyl)carbamoylphenyl ]quinazoline (Compound 166)

Compound 166 was obtained in the same manner as in Example 4, using Compound 165.
$^1$H NMR (270 MHz, DMSO-$d_6$, 80°C) δ (ppm) 2.25 (s, 3H), 2.52 (s, 3H), 6.39 (br s, 2H), 7.20 (d, J = 4.9 Hz, 1H), 7.26 (s, 1H), 7.35-7.48 (m, 2H), 7.52-7.63 (m, 2H), 7.90-7.98 (m, 2H), 8.07 (d, J = 8.6 Hz, 1H), 8.20 (s, 1H), 8.48 (d, J = 5.3 Hz, 1H), 8.91 (s, 1H), 10.28 (s, 1H).
ESI m/z $(M+H)^+$ 530.

Example 167

2-Amino-8-(4-fluorophenyl)-7-hydroxy-6-[2-methyl-5-N-(3-trifluoromethylphenyl)carbamoylphenyl]quina zoline (Compound 167)

Compound 167 was obtained in the same manner as in Example 4, using Compound 5.
$^1$H NMR (270 MHz, DMSO-$d_6$, 80°C) δ (ppm) 1.11 (s, 3H), 2.25 (s, 3H), 6.28 (br s, 2H), 7.16-7.27 (m, 2H), 7.36-7.48 (m, 4H), 7.50-7.62 (m, 2H), 7.90-7.98 (m, 2H), 8.07 (d, J = 8.2 Hz, 1H), 8.20 (s, 1H), 8.92 (br s, 1H), 10.27 (s, 1H).
ESI m/z $(M+H)^+$ 533.

Example 168

2-Amino-8-(3-fluoropyridin-4-yl)-7-hydroxy-6-[2-methyl-5-N-(3-trifluoromethylphenyl)carbamoylphenyl] quinazoline (Compound 168)

Compound 168 was obtained in the same manner as in Example 4, using Compound 154.
$^1$H NMR (270 MHz, DMSO-$d_6$) δ (ppm) 2.25 (s, 3H), 6.71 (br s, 2H), 7.40-7.52 (m, 3H), 7.54-7.65 (m, 1H), 7.70 (s, 1H), 7.92-8.02 (m, 2H), 8.10 (d, J = 7.9 Hz, 1H), 8.25 (s, 1H), 8.45-8.51 (m, 1H), 8.62 (s, 1H), 8.96 (br s, 1H), 9.43 (br s, 1H), 10.48 (s, 1H).
ESI m/z $(M+H)^+$ 534.

Example 169

2-Amino-8-(3-chloropyridin-4-yl)-7-hydroxy-6-[2-methyl-5-N-(3-trifluoromethylphenyl)carbamoylphenyl] quinazoline (Compound 169)

Compound 169 was obtained in the same manner as in Example 4, using Compound 155.
$^1$H NMR (270 MHz, DMSO-$d_6$) δ (ppm) 2.25 (s, 3H), 6.67 (br s, 2H), 7.37-7.52 (m, 3H), 7.54-7.64 (m, 1H), 7.68 (s, 1H), 7.92-8.00 (m, 2H), 8.10 (d, J = 8.3 Hz, 1H), 8.25 (s, 1H), 8.56 (d, J = 5.0 Hz, 1H), 8.72 (s, 1H), 8.95 (br s, 1H), 9.31 (br s, 1H), 10.49 (s, 1H).
ESI m/z $(M+H)^+$ 550.

Example 170

2-Amino-7-hydroxy-6-[2-methyl-5-N-(3-trifluoromethylphenyl)carbamoylphenyl]-8-[2-(2H-1,2,3-triazol-2-yl)pyridin-4-yl] quinazoline (Compound 170)

Compound 170 was obtained in the same manner as in Example 148, using Compound 133 and 1,2,3-triazol.
$^1$H NMR (300 MHz, DMSO-$d_6$, 80°C) δ (ppm) 2.27 (s, 3H), 6.41 (br s, 2H), 7.36-7.49 (m, 2H), 7.52-7.60 (m, 2H), 7.64 (s, 1H), 7.91-7.98 (m, 2H), 8.02-8.12 (m, 4H), 8.21 (s, 1H), 8.60 (d, J = 5.1 Hz, 1H), 8.92 (br s, 1H), 10.28 (s, 1H).
ESI m/z $(M+H)^+$ 583.

Example 171

2-Amino-7-methoxy-6-[2-methyl-5-N-(3-trifluoromethylphenyl)carbamoylphenyl]-8-(pyridine-1-oxid-4-yl )quinazoline (Compound 171)

Compound 171 was obtained in the same manner as in Example 154, using Compound A11 and 4-(tributylstannyl)pyridine-1-oxide (WO 03/093273).
$^1$H NMR (270 MHz, $CDCl_3$) δ (ppm) 2.32 (s, 3H), 3.15 (s, 3H), 5.38 (br s, 2H), 7.35-7.53 (m, 3H), 7.56 (d, J = 7.3 Hz, 2H), 7.63 (s, 1H), 7.86-7.92 (m, 1H), 7.93-8.01 (m, 3H), 8.23 (d, J = 7.3 Hz, 2H), 8.54 (br s, 1H), 8.99 (s, 1H).
ESI m/z $(M+H)^+$ 546.

Example 172

2-Amino-7-hydroxy-6-[2-methyl-5-N-(3-trifluoromethylphenyl)carbamoylphenyl]-8-(pyridine-1-oxid-4-yl) quinazoline (Compound 172)

Compound 172 was obtained in the same manner as in Example 4, using Compound 171.
$^1$H NMR (270 MHz, DMSO-$d_6$) δ (ppm) 2.25 (s, 3H), 6.71 (br s, 2H), 7.40-7.52 (m, 4H), 7.55-7.66 (m, 2H), 7.92-7.99 (m, 2H), 8.10 (d, J = 8.1 Hz, 1H), 8.21-8.29 (m, 3H), 8.95 (br s, 1H), 9.35 (br s, 1H), 10.49 (s, 1H).
ESI m/z $(M+H)^+$ 532.

Example 173

2-Amino-7-hydroxy-6-[2-methyl-5-N-(3-trifluoromethylphenyl)carbamoylphenyl]-8-(oxazol-2-yl)quinazol ine (Compound 173)

Compound 173 was obtained in the same manner as in Example 4, using Compound 82.
$^1$H NMR (300 MHz, DMSO-$d_6$, 80°C) δ (ppm) 2.23 (s, 3H), 6.87 (br s, 2H), 7.37-7.61 (m, 4H), 7.72 (s, 1H), 7.88-7.98 (m, 2H), 8.06 (d, J = 8.8 Hz, 1H), 8.19 (s, 1H), 8.27 (s, 1H), 8.92 (s, 1H), 10.28 (br s, 1H).
ESI m/z $(M+H)^+$ 506.

Example 174

2-Amino-8-(2-fluoropyridin-5-yl)-7-methoxy-6-[2-methyl-5-N-(4-trifluoromethylpyridin-2-yl)carbamoylp henyl]quinazoline (Compound 174)

Compound 174 was obtained in the same manner as in Example 1, using Compound A53 and 2-fluoro-5-pyridine boronic acid.
$^1$H NMR (300 MHz, $CDCl_3$) δ (ppm) 2.36 (s, 3H), 3.14 (s, 3H), 5.27 (br s, 2H), 7.02-7.08 (m, 1H), 7.28-7.33 (m, 1H), 7.48 (d, J = 8.1 Hz, 1H), 7.62 (s, 1H), 7.88-7.95 (m, 2H), 7.99-8.08 (m, 1H), 8.43-8.50 (m, 2H), 8.72 (s, 1H), 8.84 (br s, 1H), 8.99 (s, 1H).
ESI m/z $(M+H)^+$ 549.

Example 175

8-(2-Fluoropyridin-5-yl)-7-methoxy-2-methoxycarbonylamino-6-[2-methyl-5-N-(3-trifluoromethylphenyl) carbamoylphenyl]quinazoline (Compound 175)

Compound 175 was obtained in the same manner as in Example 143, using Compound 28 and methyl chloroformate.
$^1$H NMR (270 MHz, $CDCl_3$) δ (ppm) 2.35 (s, 3H), 3.20 (s, 3H), 3.84 (s, 3H), 7.11 (dd, J = 8.3, 2.6 Hz, 1H), 7.38-7.54 (m, 3H), 7.72 (s, 1H), 7.80 (s, 1H), 7.85-8.03 (m, 5H), 8.14-8.24 (m, 1H), 8.52 (d, J = 2.6 Hz, 1H), 9.27 (s, 1H).
ESI m/z $(M+H)^+$ 606.

Example 176

2-Acetylamino-7-hydroxy-8-(2-methylpyridin-5-yl)-6-[2-methyl-5-N-(3-trifluoromethylphenyl)carbamoylp henyl]quinazoline (Compound 176)

Compound 176 was obtained in the same manner as in Example 143 and Example 4, using Compound 145 and acetate anhydride.
$^1$H NMR (270 MHz, DMSO-$d_6$) δ (ppm) 2.15 (s, 3H), 2.25 (s, 3H), 2.43 (s, 3H), 7.10 (d, J = 8.1 Hz, 1H), 7.25-7.45 (m, 3H), 7.52-7.61 (m, 1H), 7.96-7.90 (m, 3H), 8.04-8.12 (m, 1H), 8.26 (s, 1H), 8.49 (s, 1H), 8.63 (s, 1H), 9.51 (br s, 1H), 10.47 (s, 1H).
ESI m/z $(M+H)^+$ 572.

Example 177

2-Acetylamino-7-hydroxy-8-(2-methylpyridin-4-yl)-6-[2-methyl-5-N-(3-trifluoromethylphenyl)carbamoylp henyl]quinazoline (Compound 177)

Compound 177 was obtained in the same manner as in Example 143 and Example 4, using Compound 165 and acetate anhydride.
$^1$H NMR (270 MHz, DMSO-$d_6$) δ (ppm) 2.13 (s, 3H), 2.26 (s, 3H), 2.53 (s, 3H), 7.30-7.64 (m, 5H), 7.85 (s, 1H), 7.94-8.02 (m, 2H), 8.10 (d, J = 8.1 Hz, 1H), 8.24 (s, 1H), 8.49 (d, J = 5.4 Hz, 1H), 9.20 (br s, 1H), 10.34 (br s, 1H), 10.49 (s, 1H).
ESI m/z $(M+H)^+$ 572.

Example 178

2-Cyclopropylcarbonylamino-7-hydroxy-8-(2-methylpyridin-5-yl)-6-[2-methyl-5-N-(3-trifluoromethylphen yl)carbamoylphenyl]quinazoline (Compound 178)

Compound 178 was obtained in the same manner as in Example 143 and Example 4, using Compound 145 and cyclopropanecarbonyl chloride.
$^1$H NMR (270 MHz, DMSO-$d_6$) δ (ppm) 0.60-0.72 (m, 2H), 0.75-0.83 (m, 2H), 2.27 (s, 3H), 2.40-2.50 (m, 1H), 2.53 (s, 3H), 7.34 (d, J = 8.1 Hz, 1H), 7.42-7.63 (m, 3H), 7.73-7.80 (m, 1H), 7.89 (s, 1H), 7.95-8.03 (m, 2H), 8.10 (d, J = 8.1 Hz, 1H), 8.25 (s, 1H), 8.50 (s, 1H), 9.29 (br s, 1H), 9.65 (br s, 1H), 10.49 (s, 1H), 10.53 (br s, 1H).
ESI m/z $(M+H)^+$ 598.

Example 179

2-Amino-7-hydroxy-6-[2-methyl-5-N-(3-trifluoromethylphenyl)carbamoylphenyl]-8-[2-(2-oxopyrrolidin-1-yl)pyridin-4-yl] quinazoline (Compound 179)

Compound 133 (0.138 g, 0.25 mmol) was dissolved in dioxane (2.0 mL) and tert-butanol (1.0 mL), and 2-pyrrolidone (0.095 mL, 1.25 mmol), tris(dibenzylideneacetone)dipalladium(0)(0.026 g, 0.025 mmol), 2-dicyclohexylphosphino-(N,N'-dimethylamino)biphenyl (0.020 g, 0.05 mmol) and cesium carbonate (0.25 g, 0.75 mmol) were added thereto, followed by stirring at 110°C for 3 hours under argon air flow. To the reaction mixture was added saturated aqueous sodium bicarbonate, followed by extraction with ethyl acetate/isopropanol (5/1), then the organic layer was washed with brine, followed by drying over anhydous sodium sulfate. The solvent was evaporated under reduced pressure, and the residue was purified by silica gel column chromatography (ethyl acetate/methanol = 9/1) to obtain Compound 179 (0.070 g, 47%).
$^1$H NMR (300 MHz, DMSO-$d_6$, 80°C) δ (ppm) 2.02-2.15 (m, 2H), 2.25 (s, 3H), 2.52-2.62 (m, 2H), 4.03-4.11 (m, 2H), 6.36 (br s, 2H), 7.11-7.15 (m, 1H), 7.37-7.48 (m, 2H), 7.52-7.63 (m, 2H), 7.90-7.98 (m, 2H), 8.07 (d, J = 8.1 Hz, 1H), 8.21 (s, 1H), 8.32 (s, 1H), 8.38-8.42 (m, 1H), 8.82 (br s, 1H), 8.92 (br s, 1H), 10.28 (s, 1H).
ESI m/z $(M+H)^+$ 599.

Example 180

2-Amino-7-methoxy-6-[2-methyl-5-N-(3-trifluoromethylphenyl)carbamoylphenyl]-8-(thiazol-5-yl)quinazol ine (Compound 180)

Compound 180 was obtained in the same manner as in Example 82, using Compound A11 and 5-(tributylstannyl)thiazole.
$^1H$ NMR (300 MHz, $CDCl_3$, 50°C) δ (ppm) 2.32 (s, 3H), 3.30 (s, 3H), 5.28 (br s, 2H), 7.36-7.52 (m, 3H), 7.55 (s, 1H), 7.82-7.96 (m, 4H), 8.05 (br s, 1H), 8.82 (s, 1H), 8.92 (s, 1H), 8.98 (s, 1H).
ESI m/z $(M+H)^+$ 536.

Example 181

2-Amino-8-(2-fluoropyridin-5-yl)-7-hydroxy-6-[2-methyl-5-N-(4-trifluoromethylpyridin-2-yl)carbamoylph enyl]quinazoline (Compound 181)

Compound 181 was obtained in the same manner as in Example 4, using Compound 174.
$^1H$ NMR (300 MHz, DMSO-$d_6$) δ (ppm) 2.26 (s, 3H), 6.64 (br s, 2H), 7.22-7.30 (m, 1H), 7.42-7.56 (m, 2H), 7.66 (s, 1H), 7.94-8.05 (m, 3H), 8.23 (s, 1H), 8.55 (s, 1H), 8.67 (d, J = 5.1 Hz, 1H), 8.95 (br s, 1H), 8.95 (br s, 1H), 11.25 (s, 1H).
ESI m/z $(M+H)^+$ 535.

Example 182

2-Amino-7-methoxy-6-[2-methyl-5-N-(3-trifluoromethylphenyl)carbamoylphenyl]-8-(pyridine-1-oxid-3-yl)quinazoline (Compound 182)

Compound 182 was obtained in the same manner as in Example 154, using Compound A11 and 3-(tributylstannyl)pyridine-1-oxide (WO 03/093273).
$^1H$ NMR (300 MHz, DMSO-$d_6$) δ (ppm) 2.29 (s, 3H), 3.15 (s, 3H), 6.97 (br s, 2H), 7.39-7.62 (m, 5H), 7.85 (s, 1H), 7.95-8.12 (m, 3H), 8.20-8.28 (m, 3H), 9.14 (s, 1H), 10.48 (s, 1H).
ESI m/z $(M+H)^+$ 546.

Example 183

2-Amino-7-methoxy-6-[2-methyl-5-N-(3-trifluoromethylphenyl)carbamoylphenyl]-8-(pyridine-1-oxid-2-yl)quinazoline (Compound 183)

Compound 183 was obtained in the same manner as in Example 154, using Compound A39 and 2-(tributylstannyl)pyridine-1-oxide (WO 03/093273).
$^1H$ NMR (270 MHz, $CDCl_3$, 50°C) δ (ppm) 2.28 (s, 3H), 3.31 (s, 3H), 5.07 (br s, 2H), 7.27-7.44 (m, 7H), 7.57 (s, 1H), 7.80-7.92 (m, 3H), 8.00 (br s, 1H), 8.33-8.39 (m, 1H), 8.86 (s, 1H).
ESI m/z $(M+H)^+$ 546.

Example 184

2-Amino-7-hydroxy-6-[2-methyl-5-N-(3-trifluoromethylphenyl)carbamoylphenyl]-8-(pyridine-1-oxid-3-yl)quinazoline (Compound 184)

Compound 184 was obtained in the same manner as in Example 4, using Compound 182.
$^1H$ NMR (300 MHz, DMSO-$D_6$) δ (ppm) 2.26 (s, 3H), 6.75 (br s, 2H), 7.34 (d, J = 7.7 Hz, 1H), 7.40-7.53 (m, 3H), 7.55-7.70 (m, 2H), 7.92-8.00 (m, 2H), 8.05-8.27 (m, 4H), 8.95 (s, 1H), 8.95 (br s, 1H), 10.48 (s, 1H).
ESI m/z $(M+H)^+$ 532.

Example 185

2-Amino-7-hydroxy-6-[2-methyl-5-N-(3-trifluoromethylphenyl)carbamoylphenyl]-8-(pyridine-1-oxid-2-yl)quinazoline (Compound 185)

Compound 185 was obtained in the same manner as in Example 4, using Compound 183.
$^1$H NMR (300 MHz, DMSO-$d_6$, 80°C) δ (ppm) 2.23 (s, 3H), 6.54 (br s, 2H), 7.35-7.48 (m, 2H), 7.50-7.67 (m, 2H), 7.70-7.81 (m, 2H), 7.86-7.96 (m, 2H), 8.06 (d, J = 8.1 Hz, 1H), 8.13-8.24 (m, 2H), 8.63 (d, J = 6.2 Hz, 1H), 8.98 (s, 1H), 10.29 (s, 1H).
ESI m/z $(M+H)^+$ 532.

Example 186

2-Cyclopropylcarbonylamino-7-hydroxy-8-(2-methylpyridin-4-yl)-6-[2-methyl-5-N-(3-trifluoromethylphen yl)carbamoylphenyl]quinazoline (Compound 186)

Compound 186 was obtained in the same manner as in Example 143 and Example 4, using Compound 165 and cyclopropanecarbonyl chloride.
$^1$H NMR (270 MHz, DMSO-$d_6$) δ (ppm) 0.55-0.70 (m, 2H), 0.75-0.81 (m, 2H), 2.25 (s, 3H), 2.45 (s, 3H), 2.50-2.65 (m, 1H), 7.35-7.64 (m, 6H), 7.85-7.95 (m, 2H), 8.10 (d, J = 8.6 Hz, 1H), 8.25 (s, 1H), 8.33 (d, J = 5.3 Hz, 1H), 8.83 (br s, 1H), 10.16 (br s, 1H), 10.47 (s, 1H).
ESI m/z $(M+H)^+$ 598.

Example 187

2-Amino-7-hydroxy-6-[2-methyl-5-N-(3-trifluoromethylphenyl)carbamoylphenyl]-8-(thiazol-5-yl)quinazol ine (Compound 187)

Compound 187 was obtained in the same manner as in Example 4, using Compound 180.
$^1$H NMR (300 MHz, DMSO-$d_6$, 80°C) δ (ppm) 2.23 (s, 3H), 6.69 (br s, 2H), 7.36-7.50 (m, 2H), 7.51-7.62 (m, 2H), 7.91-7.99 (m, 2H), 8.07 (d, J = 8.1 Hz, 1H), 8.20 (s, 1H), 8.73 (s, 1H), 8.86 (s, 1H), 9.01 (s, 1H), 10.27 (s, 1H).
ESI m/z $(M+H)^+$ 522.

Example 188

2-Amino-8-(3-fluorophenyl)-7-hydroxy-6-[2-methyl-5-N-(3-trifluoromethylphenyl)carbamoylphenyl]quina zoline (Compound 188)

Compound 188 was obtained in the same manner as in Example 1 and Example 4, using Compound A11 and 3-fluorophenyl boronic acid.
$^1$H NMR (300 MHz, DMSO-$d_6$ 80°C) δ (ppm) 2.25 (s, 3H), 6.36 (br s, 2H), 7.07-7.24 (m, 3H), 7.37-7.49 (m, 3H), 7.52-7.61 (m, 2H), 7.91-7.97 (m, 2H), 8.07 (d, J = 8.8 Hz, 1H), 8.21 (s, 1H), 8.91 (s, 1H), 10.28 (s, 1H).
ESI m/z $(M+H)^+$ 533.

Example 189

2-Amino-7-hydroxy-6-[2-methyl-5-N-(3-trifluoromethylphenyl)carbamoylphenyl]-8-phenylquinazoline (C ompound 189)

Compound 189 was obtained in the same manner as in Example 1 and Example 4, using Compound A11 and phenyl boronic acid.
$^1$H NMR (300 MHz, DMSO-$d_6$, 80°C) δ (ppm) 2.26 (s, 3H), 6.30 (br s, 2H), 7.29-7.48 (m, 7H), 7.52-7.60 (m, 2H), 7.91-7.97 (m, 2H), 8.08 (d, J = 8.8 Hz, 1H), 8.21 (s, 1H), 8.91 (s, 1H), 10.28 (s, 1H).
ESI m/z $(M+H)^+$ 515.

Example 190

2-Amino-7-methoxy-8-(5-methyl-1,2,4-oxadiazol-3-yl)-6-[2-methyl-5-N-(3-trifluoromethylphenyl)carbam oylphenyl] quinazoline (Compound 190)

Compound A56 (0.22 g, 0.338 mmol) was dissolved in ethanol (6.8 mL), and camphor-10-sulfonic acid (0.24 g, 1.01 mmol) was added thereto, followed by stirring for 6 hours under heating and reflux. To the reaction mixture was added saturated aqueous sodium bicarbonate, followed by extraction with ethyl acetate, and the organic layer was washed with brine, followed by drying over anhydous sodium sulfate. The solvent was evaporated under reduced pressure, and the residue was purified by silica gel column chromatography (chloroform/methanol = 5/1) to obtain Compound 190 (0.10g, 57%).
$^1$H NMR (300 MHz, $CDCl_3$) $\delta$ (ppm) 2.29 (s, 3H), 2.70 (s, 3H), 3.38 (s, 3H), 5.22 (br s, 2H), 7.36-7.52 (m, 3H), 7.65 (s, 1H), 7.82-7.93 (m, 3H), 7.99 (s, 1H), 8.18 (br s, 1H), 8.96 (s, 1H).
ESI m/z $(M+H)^+$ 535.

Example 191

2-Amino-7-hydroxy-6-[2-methyl-5-N-(3-trifluoromethylphenyl)carbamoylphenyl]-8-[2-(1,3-oxazolidin-2-o n-3-yl)pyridin-4-yl]quinazoline (Compound 191)

Compound 191 was obtained in the same manner as in Example 179, using Compound 133 and 2-oxazolidone.
$^1$H NMR (300 MHz, DMSO-$d_6$, 80°C) $\delta$ (ppm) 2.25 (s, 3H), 4.20-4.29 (m, 2H), 4.43-4.52 (m, 2H), 6.37 (br s, 2H), 7.13-7.18 (m, 1H), 7.37-7.48 (m, 2H), 7.52-7.65 (m, 2H), 7.91-7.99 (m, 2H), 8.04-8.14 (m, 2H), 8.21 (s, 1H), 8.37-8.42 (m, 1H), 8.93 (s, 1H), 10.28 (s, 1H).
ESI m/z $(M+H)^+$ 601.

Example 192

2-Amino-8-(4-cyanophenyl)-7-hydroxy-6-[2-methyl-5-N-(3-trifluoromethylphenyl)carbamoylphenyl]quina zoline (Compound 192)

Compound 192 was obtained in the same manner as in Example 1 and Example 4, using Compound A11 and 4-cyanophenyl boronic acid.
$^1$H NMR (300 MHz, DMSO-$d_6$, 80°C) $\delta$ (ppm) 2.25 (s, 3H), 6.36 (br s, 2H), 7.37-7.49 (m, 2H), 7.52-7.64 (m, 4H), 7.80-7.87 (m, 2H), 7.91-7.98 (m, 2H), 8.07 (d, J = 8.4 Hz, 1H), 8.20 (s, 1H), 8.70-9.00 (m, 2H), 10.27 (s, 1H).
ESI m/z $(M+H)^+$ 540.

Example 193

7-Hydroxy-2-methylamino-8-(2-methylpyridin-4-yl)-6-[2-methyl-5-N-(3-trifluoromethylphenyl)carbamoyl phenyl]quinazoline (Compound 193)

Compound 193 was obtained in the same manner as in Example 83, using Compound A47 and 2-methyl-4-(4,4,5,5-tetramethyl-1,3,2-dioxaboran-2-yl)pyridine.
$^1$H NMR (270 MHz, DMSO-$d_6$) $\delta$ (ppm) 2.26 (s, 3H), 2.51 (s, 3H), 2.74 (br s, 3H), 7.11 (br s, 1H), 7.27-7.50 (m, 4H), 7.53-7.65 (m, 2H), 7.93-8.00 (m, 2H), 8.11 (d, J = 8.1 Hz, 1H), 8.25 (s, 1H), 8.47 (d, J = 5.4 Hz, 1H), 8.92 (s, 1H), 10.47 (s, 1H).
ESI m/z $(M+H)^+$ 544.

Example 194

2-Amino-8-(2,4-difluorophenyl)-7-hydroxy-6-[2-methyl-5-N-(3-trifluoromethylphenyl)carbamoylphenyl]q uinazoline (Compound 194)

Step 1:

Compound A58 (0.20 g, 0.33 mmol) was dissolved in dioxane (4.0 mL) and water (2.0 mL), and 2,4-difluorophenyl boronic acid (0.16 g, 0.99 mmol), tris(dibenzylideneacetone)dipalladium(0) (0.034 g, 0.033 mmol), 2-(dicyclohexylphosphino)biphenyl (0.023 g, 0.066 mmol) and potassium phosphate (0.21 g, 0.99 mmol) were added thereto, followed by stirring at 130°C for 6 hours under argon air flow. To the reaction mixture was added saturated aqueous sodium bicarbonate, followed by extraction with ethyl acetate, then the organic layer was washed with brine, followed by drying over anhydous sodium sulfate. The solvent was evaporated under reduced pressure to obtain 2-amino-7-benzyloxy-8-(2,4-difluorophenyl)-6-[2-methyl-5-N-(3-trifluoromethylphenyl)carbamoylphenyl]quinazoline (0.125 g, 59%).

Step 2:

2-Amino-7-benzyloxy-8-(2,4-difluorophenyl)-6-[2-methyl-5-N-(3-trifluoromethyl phenyl)carbamoylphenyl]quinazoline (0.122 g, 0.19 mmol) was suspended in ethanol (2.0 mL) and DMF (0.5 mL). Under argon atmosphere, ammonium formate (10 mol/L aqueous solution 0.19 mL, 1.9 mmol) and palladium-carbon (50% water-containing, 24 mg) were added to the mixture in this order, followed by stirring at 60°C for 20 minutes. The insoluble materials were filtered through celite, and the solvent was evaporated under reduced pressure. To the residue was added water, followed by extraction with ethyl acetate, and the organic layer was washed with brine, followed by drying over anhydrous magnesium sulfate. The solvent was evaporated under reduced pressure, and the residue was purified by silica gel column chromatography (ethyl acetate/hexane = 1/1) to obtain Compound 194 (0.057 g, 54%).
$^1$H NMR (300 MHz, DMSO-$d_6$, 80°C) $\delta$ (ppm) 2.24 (s, 3H), 6.33 (br s, 2H), 7.06-7.23 (m, 2H), 7.30-7.49 (m, 3H), 7.52-7.65 (m, 2H), 7.91-7.98 (m, 2H), 8.07 (d, J = 9.2 Hz, 1H), 8.20 (s, 1H), 8.70-8.99 (m, 2H), 10.28 (s, 1H).
ESI m/z (M+H)$^+$ 551.

Example 195

2-Amino-7-hydroxy-6-[2-methyl-5-N-(3-trifluoromethylphenyl)carbamoylphenyl]-8-[2-(2-oxopyrrolidin-1-yl)pyridin-5-yl] quinazoline (Compound 195)

Compound 195 was obtained in the same manner as in Example 179 and Example 4, using Compound 20 and 2-pyrrolidone.
$^1$H NMR (270 MHz, DMSO-$d_6$, 80°C) $\delta$ (ppm) 2.04-2.15 (m, 2H), 2.26 (s, 3H), 2.55-2.65 (m, 2H), 4.03-4.12 (m, 2H), 6.36 (br s, 2H), 7.37-7.48 (m, 2H), 7.55 (d, J = 7.9 Hz, 1H), 7.60 (s, 1H), 7.77-7.84 (m, 1H), 7.91-7.98 (m, 2H), 8.07 (d, J = 8.6 Hz, 1H), 8.20 (s, 1H), 8.30-8.41 (m, 2H), 8.70-8.98 (m, 2H), 10.28 (s, 1H).
ESI m/z (M+H)$^+$ 599.

Example 196

2-Amino-7-hydroxy-8-(4-methylphenyl)-6-[2-methyl-5-N-(3-trifluoromethylphenyl)carbamoylphenyl]quin azoline (Compound 196)

Compound 196 was obtained in the same manner as in Example 1 and Example 4, using Compound A11 and 4-methylphenyl boronic acid.
$^1$H NMR (300 MHz, DMSO-$d_6$, 80°C) $\delta$ (ppm) 2.25 (s, 3H), 2.37 (s, 3H), 6.28 (br s, 2H), 7.20-7.30 (m, 4H), 7.36-7.48 (m, 2H), 7.52-7.60 (m, 2H), 7.80-7.97 (m, 3H), 8.07 (d, J = 8.4 Hz, 1H), 8.20 (s, 1H), 8.89 (s, 1H), 10.28 (s, 1H).
ESI m/z (M+H)$^+$ 529.

Example 197

2-Amino-8-(3-cyanophenyl)-7-hydroxy-6-[2-methyl-5-N-(3-trifluoromethylphenyl)carbamoylphenyl]quina zoline (Compound 197)

Compound 197 was obtained in the same manner as in Example 1 and Example 4, using Compound A11 and 3-cyanophenyl boronic acid.
$^1$H NMR (300 MHz, DMSO-$d_6$, 80°C) $\delta$ (ppm) 2.26 (s, 3H), 6.38 (br s, 2H), 7.36-7.50 (m, 2H), 7.52-7.67 (m, 3H), 7.71-7.81 (m, 3H), 7.91-7.99 (m, 2H), 8.07 (d, J = 8.1 Hz, 1H), 8.20 (s, 1H), 8.70-8.99 (m, 2H), 10.28 (s, 1H).
ESI m/z (M+H)$^+$ 540.

Example 198

8-(2-Fluoropyridin-5-yl)-7-hydroxy-2-methoxycarbonylamino-6-[2-methyl-5-N-(3-trifluoromethylphenyl)c arbamoylphenyl]quinazoline (Compound 198)

Compound 198 was obtained in the same manner as in Example 1, Example 143 and Step 2 of Example 194, using Compound A58, 2-fluoro-5-pyridine boronic acid and methyl chloroformate.
$^1$H NMR (270 MHz, DMSO-$d_6$) δ (ppm) 2.25 (s, 3H), 3.63 (s, 3H), 6.94-7.02 (m, 1H), 7.30-7.45 (m, 3H), 7.53-7.62 (m, 1H), 7.81-7.90 (m, 2H), 8.10 (d, J = 8.1 Hz, 1H), 8.26 (br s, 1H), 8.34-8.50 (m, 1H), 8.53-8.65 (m, 3H), 9.60 (br s, 1H), 10.45 (s, 1H).
ESI m/z $(M+H)^+$ 592.

Example 199

7-Hydroxy-2-methoxycarbonylamino-8-(2-methylpyridin-4-yl)-6-[2-methyl-5-N-(3-trifluoromethylphenyl) carbamoylphenyl]quinazoline (Compound 199)

Compound 199 was obtained in the same manner as in Example 1, Example 143 and Step 2 of Example 194, using Compound A58, 2-methyl-4-(4,4,5,5-tetramethyl-1,3,2-dioxaboran-2-yl)pyridine and methyl chloroformate.
$^1$H NMR (270 MHz, DMSO-$d_6$) δ (ppm) 2.25 (s, 3H), 2.53 (s, 3H), 3.64 (s, 3H), 7.40-7.70 (m, 5H), 7.75 (s, 1H), 7.90-8.01 (m, 2H), 8.10 (d, J = 8.6 Hz, 1H), 8.25 (s, 1H), 8.43 (d, J = 5.3 Hz, 1H), 9.06 (br s, 1H), 10.15 (br s, 1H), 10.47 (s, 1H).
ESI m/z $(M+H)^+$ 588.

Example 200

2-Amino-7-hydroxy-8-(3-methylphenyl)-6-[2-methyl-5-N-(3-trifluoromethylphenyl)carbamoylphenyl]quin azoline (Compound 200)

Compound 200 was obtained in the same manner as in Example 1 and Example 4, using Compound A11 and 3-methylphenyl boronic acid.
$^1$H NMR (300 MHz, DMSO-$d_6$, 80°C) δ (ppm) 2.25 (s, 3H), 2.36 (s, 3H), 6.30 (br s, 2H), 7.11-7.20 (m, 3H), 7.28-7.36 (m, 1H), 7.37-7.48 (m, 2H), 7.52-7.61 (m, 2H), 7.90-7.97 (m, 2H), 8.07 (d, J = 8.1 Hz, 1H), 8.21 (s, 1H), 8.90 (s, 1H), 10.28 (s, 1H).
ESI m/z $(M+H)^+$ 529.

Example 201

2-Amino-8-(4-chlorophenyl)-7-hydroxy-6-[2-methyl-5-N-(3-trifluoromethylphenyl)carbamoylphenyl]quin azoline (Compound 201)

Compound 201 was obtained in the same manner as in Example 1 and Example 4, using Compound A11 and 4-chlorophenyl boronic acid.
$^1$H NMR (300 MHz, DMSO-$d_6$, 80°C) δ (ppm) 2.25 (s, 3H), 6.33 (br s, 2H), 7.37-7.49 (m, 6H), 7.52-7.61 (m, 2H), 7.91-7.97 (m, 2H), 8.07 (d, J = 7.7 Hz, 1H), 8.20 (s, 1H), 8.63 (br s, 1H), 8.91 (s, 1H), 10.27 (s, 1H).
ESI m/z $(M+H)^+$ 549.

Example 202

2-Amino-7-hydroxy-8-(2-methylphenyl)-6-[2-methyl-5-N-(3-trifluoromethylphenyl)carbamoylphenyl]quin azoline (Compound 202)

Compound 202 was obtained in the same manner as in Step 1 of Example 194 and Example 4, using Compound A11 and 2-methylphenyl boronic acid.
$^1$H NMR (300 MHz, DMSO-$d_6$, 80°C) δ (ppm) 2.05 (s, 3H), 2.24 (s, 3H), 6.29 (br s, 2H), 7.10-7.17 (m, 1H), 7.19-7.32 (m, 3H), 7.37-7.47 (m, 2H), 7.52-7.62 (m, 2H), 7.79 (br s, 1H), 7.91-7.98 (m, 2H), 8.08 (d, J = 8.8 Hz, 1H), 8.21 (s, 1H), 8.91 (s, 1H), 10.30 (s, 1H).
ESI m/z $(M+H)^+$ 529.

Example 203

2-Amino-7-hydroxy-8-(3-pyridyl)-6-[2-methyl-5-N-(3-trifluoromethylphenyl)carbamoylphenyl]quinazolin e (Compound 203)

Compound 203 was obtained in the same manner as in Example 4, using Compound 14.
$^1$H NMR (300 MHz, DMSO-$d_6$, 80°C) δ (ppm) 2.26 (s, 3H), 6.35 (br s, 2H), 7.37-7.48 (m, 3H), 7.52-7.63 (m, 2H), 7.75-7.83 (m, 1H), 7.91-7.98 (m, 2H), 8.07 (d, J = 8.8 Hz, 1H), 8.21 (s, 1H), 8.48-8.53 (m, 1H), 8.59 (d, J = 1.5 Hz, 1H), 8.92 (s, 1H), 10.28 (s, 1H).
ESI m/z $(M+H)^+$ 516.

Example 204

2-Amino-8-(4-fluorophenyl)-7-hydroxy-6-[2-methyl-5-N-(4-trifluoromethylpyridin-2-yl)carbamoylphenyl] quinazoline (Compound 204)

Compound 204 was obtained in the same manner as in Example 1 and Example 4, using Compound A53 and 4-fluorophenyl boronic acid.
$^1$H NMR (270 MHz, DMSO-$d_6$) δ (ppm) 2.25 (s, 3H), 6.57 (br s, 2H), 7.20-7.32 (m, 2H), 7.35-7.57 (m, 4H), 7.61 (s, 1H), 7.95-8.05 (m, 2H), 8.55 (s, 1H), 8.67 (d, J = 5.3 Hz, 1H), 8.77 (br s, 1H), 8.94 (s, 1H), 11.27 (s, 1H).
ESI m/z $(M+H)^+$ 534.

Example 205

2-Amino-7-hydroxy-8-(5-methyl-1,2,4-oxadiazol-3-yl)-6-[2-methyl-5-N-(3-trifluoromethylphenyl)carbamo ylphenyl] quinazoline (Compound 205)

Compound 205 was obtained in the same manner as in Example 4, using Compound 190.
$^1$H NMR (270 MHz, DMSO-$d_6$) δ (ppm) 2.20 (s, 3H), 2.31 (s, 3H), 7.18 (br s, 2H), 7.45 (d, J = 7.6 Hz, 1H), 7.54-7.65 (m, 2H), 7.73 (s, 1H), 7.98-8.11 (m, 3H), 8.23 (s, 1H), 9.22 (s, 1H), 10.50 (s, 1H), 11.62 (br s, 1H).
ESI m/z $(M+H)^+$ 521.

Example 206

2-Acetylamino-8-(4-cyanophenyl)-7-hydroxy-6-[2-methyl-5-N-(3-trifluoromethylphenyl)carbamoylphenyl ]quinazoline (Compound 206)

Compound 206 was obtained in the same manner as in Example 1, Example 143 and Example 4, using Compound A11, 4-cyanophenyl boronic acid and acetate anhydride.
$^1$H NMR (300 MHz, DMSO-$d_6$) δ (ppm) 2.09 (s, 3H), 2.26 (s, 3H), 7.45 (d, J = 7.8 Hz, 1H), 7.52 (d, J = 8.4 Hz, 1H), 7.60 (dd, J = 8.4, 7.8 Hz, 1H), 7.70 (d, J = 8.4 Hz, 2H), 7.83-7.95 (m, 3H), 7.97-8.04 (m, 2H), 8.10 (d, J = 7.5 Hz, 1H), 8.24 (s, 1H), 9.28 (br s, 1H), 9.70 (br s, 1H), 10.39 (br s, 1H), 10.49 (s, 1H).
ESI m/z $(M+H)^+$ 582.

Example 207

2-Amino-8-(2-fluoropyridin-4-yl)-7-hydroxy-6-[2-methyl-5-N-(3-trifluoromethylphenyl)carbamoylphenyl] quinazoline (Compound 207)

Compound 207 was obtained in the same manner as in Example 1 and Step 2 of Example 194, using Compound A58 and 2-fluoro-4-pyridine boronic acid.
$^1$H NMR (300 MHz, DMSO-$d_6$, 80°C) δ (ppm) 2.25 (s, 3H), 6.47 (br s, 2H), 7.16 (s, 1H), 7.34-7.49 (m, 3H), 7.52-7.60 (m, 1H), 7.64 (s, 1H), 7.90-7.98 (m, 2H), 8.07 (d, J = 8.4 Hz, 1H), 8.17-8.29 (m, 2H), 8.93 (s, 1H), 9.08 (br s, 1H), 10.28 (s, 1H).
ESI m/z $(M+H)^+$ 534.

Example 208

2-Amino-7-hydroxy-6-[2-methyl-5-N-(3-trifluoromethylphenyl)carbamoylphenyl]-8-(pyrimidin-5-yl)quina zoline (Compound 208)

Compound 208 was obtained in the same manner as in Example 4, using Compound 35.
$^1$H NMR (300 MHz, DMSO-$d_6$, 80°C) $\delta$: 2.26 (s, 3H), 6.47 (br s, 2H), 7.37-7.50 (m, 2H), 7.52-7.60 (m, 1H), 7.66 (s, 1H), 7.93-7.99 (m, 2H), 8.07 (d, J = 8.8 Hz, 1H), 8.20 (s, 1H), 8.83 (s, 2H), 8.95 (s, 1H), 9.11 (s, 1H), 9.25 (br s, 1H), 10.28 (s, 1H).
ESI m/z $(M+H)^+$ 517.

Example 209

2-Amino-8-(3-chlorophenyl)-7-hydroxy-6-[2-methyl-5-N-(3-trifluoromethylphenyl)carbamoylphenyl]quin azoline (Compound 209)

Compound 209 was obtained in the same manner as in Example 1 and Example 4, using Compound A11 and 3-chlorophenyl boronic acid.
$^1$H NMR (300 MHz, DMSO-$d_6$, 80°C) $\delta$ (ppm) 2.25 (s, 3H), 6.35 (br s, 2H), 7.28-7.50 (m, 6H), 7.52-7.63 (m, 2H), 7.90-7.98 (m, 2H), 8.07 (d, J = 8.4 Hz, 1H), 8.20 (s, 1H), 8.67 (br s, 1H), 8.92 (s, 1H), 10.28 (s, 1H).
ESI m/z $(M+H)^+$ 548.

Example 210

2-Amino-8-(4-dimethylaminophenyl)-7-hydroxy-6-[2-methyl-5-N-(3-trifluoromethylphenyl)carbamoylphe nyl]quinazoline (Compound 210)

Compound 210 was obtained in the same manner as in Example 1 and Example 4, using Compound A11 and 4-(dimethylamino)phenyl boronic acid.
$^1$H NMR (300 MHz, DMSO-$d_6$, 80°C) $\delta$ (ppm) 2.25 (s, 3H), 2.95 (s, 6H), 6.24 (br s, 2H), 6.82 (d, J = 8.8 Hz, 2H), 7.22 (d, J = 8.8 Hz, 2H), 7.37-7.61 (m, 4H), 7.72 (br s, 1H), 7.88-7.98 (m, 2H), 8.07 (d, J = 8.4 Hz, 1H), 8.20 (s, 1H), 8.89 (s, 1H), 10.28 (s, 1H).
ESI m/z $(M+H)^+$ 558.

Example 211

2-Amino-8-(5-cyclopropyl-1,2,4-oxadiazol-3-yl)-7-hydroxy-6-[2-methyl-5-N-(3-trifluoromethylphenyl)car bamoylphenyl] quinazoline (Compound 211)

Compound 211 was obtained in the same manner as in Reference Example 56, Example 190 and Example 4, using Compound A55 and cyclopropylcarbonyl chloride.
$^1$H NMR (270 MHz, DMSO-$d_6$) $\delta$ (ppm) 0.84-0.94 (m, 4H), 1.96-2.12 (m, 1H), 2.31 (s, 3H), 7.16 (br s, 2H), 7.45 (d, J = 7.6 Hz, 1H), 7.54-7.66 (m, 2H), 7.73 (s, 1H), 7.97-8.13 (m, 3H), 8.23 (s, 1H), 9.22 (s, 1H), 10.50 (s, 1H), 11.93 (br s, 1H).
ESI m/z $(M+H)^+$ 547.

Example 212

8-(4-Cyanophenyl)-2-cyclopropylcarbonylamino-7-hydroxy-6-[2-methyl-5-N-(3-trifluoromethylphenyl)car bamoylphenyl]quinazoline (Compound 212)

Compound 206 was obtained in the same manner as in Example 1, Example 143 and Example 4, using Compound A11, 4-cyanophenyl boronic acid and acetic anhydride.
$^1$H NMR (270 MHz, DMSO-$d_6$) $\delta$ (ppm) 0.60-0.70 (m, 2H), 0.76-0.84 (m, 2H), 2.26 (s, 3H), 2.50-2.53 (m, 1H), 7.35-7.47 (m, 2H), 7.52-7.63 (m, 2H), 7.66-7.76 (m, 2H), 7.80-7.95 (m, 4H), 8.10 (d, J = 8.2 Hz, 1H), 8.26 (s, 1H), 10.47 (s, 1H).
ESI m/z $(M+H)^+$ 608.

Example 213

2-Amino-7-hydroxy-8-(4-methoxyphenyl)-6-[2-methyl-5-N-(3-trifluoromethylphenyl)carbamoylphenyl]qu inazoline (Compound 213)

Compound 213 was obtained in the same manner as in Example 1 and Step 2 of Example 192, using Compound A58 and 4-methoxyphenyl boronic acid.
$^1$H NMR (300 MHz, $CDCl_3$, 50°C) δ (ppm) 2.30 (s, 3H), 3.82 (s, 3H), 5.05 (br s, 2H), 5.75 (br s, 1H), 6.97-7.05 (m, 2H), 7.34-7.48 (m, 6H), 7.81 (d, J = 1.8 Hz, 1H), 7.84-7.94 (m, 3H), 8.05 (s, 1H), 8.84 (s, 1H).
ESI m/z $(M+H)^+$ 545.

Example 214

2-Amino-7-hydroxy-8-(3-methoxyphenyl)-6-[2-methyl-5-N-(3-trifluoromethylphenyl)carbamoylphenyl]qu inazoline (Compound 214)

Compound 214 was obtained in the same manner as in Example 1 and Step 2 of Example 194, using Compound A58 and 3-methoxyphenyl boronic acid.
$^1$H NMR (300 MHz, $CDCl_3$, 50°C) δ (ppm) 2.30 (s, 3H), 3.77 (s, 3H), 5.07 (br s, 2H), 5.81 (br s, 1H), 6.93-7.04 (m, 3H), 7.33-7.50 (m, 5H), 7.80-7.96 (m, 4H), 8.02 (s, 1H), 8.86 (s, 1H).
ESI m/z $(M+H)^+$ 545.

Example 215

2-Amino-8-(2-fluoropyridin-5-yl)-7-methoxy-6-[2-methyl-5-N-(2-trifluoromethylpyridin-6-yl)carbamoylp henyl]quinazoline (Compound 215)

Compound 215 was obtained in the same manner as in Example 1, using Compound A59 and 2-fluoro-5-pyridine boronic acid.
$^1$H NMR (270 MHz, $CDCl_3$) δ (ppm) 2.34 (s, 3H), 3.14 (s, 3H), 5.21 (br s, 2H), 7.01-7.09 (m, 1H), 7.42-7.51 (m, 2H), 7.64 (s, 1H), 7.87-7.99 (m, 3H), 7.99-8.08 (m, 1H), 8.42-8.47 (m, 1H), 8.61 (d, J = 8.6 Hz, 1H), 8.71 (s, 1H), 9.01 (s, 1H).
ESI m/z $(M+H)^+$ 549.

Example 216

2-Amino-8-(2-fluoropyridin-5-yl)-7-hydroxy-6-[2-methyl-5-N-(2-trifluoromethylpyridin-6-yl)carbamoylph enyl]quinazoline (Compound 216)

Compound 216 was obtained in the same manner as in Example 4, using Compound 215.
$^1$H NMR (300 MHz, DMSO-$d_6$) δ (ppm) 2.26 (s, 3H), 6.65 (br s, 2H), 7.22-7.30 (m, 1H), 7.45 (d, J = 8.4 Hz, 1H), 7.62-7.69 (m, 2H), 7.95-8.05 (m, 3H), 8.08-8.18 (m, 1H), 8.21-8.25 (m, 1H), 8.46 (d, J = 8.4 Hz, 1H), 8.95 (s, 1H), 9.15 (br s, 1H), 11.17 (s, 1H).
ESI m/z $(M+H)^+$ 535.

Example 217

2-Amino-8-(4-cyanophenyl)-7-hydroxy-6-[2-methyl-5-N-(4-trifluoromethylpyridin-2-yl)carbamoylphenyl] quinazoline (Compound 217)

Compound 217 was obtained in the same manner as in Example 1 and Example 4, using Compound A53 and 4-cyanophenyl boronic acid.
$^1$H NMR (300 MHz, DMSO-$d_6$) δ (ppm) 2.25 (s, 3H), 6.62 (br s, 2H), 7.42-7.56 (m, 2H), 7.57-7.67 (m, 3H), 7.89 (d, J = 8.4 Hz, 2H), 7.96-8.04 (m, 2H), 8.55 (s, 1H), 8.67 (d, J = 5.1 Hz, 1H), 8.88-9.12 (m, 2H), 11.26 (s, 1H).
ESI m/z $(M+H)^+$ 541.

Example 218

8-(4-Fluorophenyl)-7-hydroxy-6-[2-methyl-5-N-(3-trifluoromethylphenyl)carbamoylphenyl]-2-(4-tetrahydr opyranylamino)quinazoline (Compound 218)

Compound 218 was obtained in the same manner as in Example 1 and Example 4, using Compound A34 and 4-fluorophenyl boronic acid.
$^1$H NMR (300 MHz, DMSO-$d_6$, 80°C) δ (ppm) 1.40-1.57 (m, 2H), 1.75-1.85 (m, 2H), 2.26 (s, 3H), 3.20-3.30 (m, 2H), 3.64-3.85 (m, 1H), 3.79-3.90 (m, 2H), 6.82 (d, J = 6.6 Hz, 1H), 7.14-7.26 (m, 2H), 7.38-7.60 (m, 6H), 7.91-8.00 (m, 2H), 8.03-8.10 (m, 1H), 8.21 (s, 1H), 8.66 (br s, 1H), 8.92 (s, 1H), 10.29 (s, 1H).
ESI m/z $(M+H)^+$ 617.

Example 219

8-(4-Fluorophenyl)-7-hydroxy-2-isopropylamino-6-[2-methyl-5-N-(3-trifluoromethylphenyl)carbamoylphe nyl]quinazoline (Compound 219)

Compound 219 was obtained in the same manner as in Example 1 and Example 4, using Compound A19 and 4-fluorophenyl boronic acid.
$^1$H NMR (270 MHz, DMSO-$d_6$, 80°C) δ (ppm) 1.10 (d, J = 6.8 Hz, 6H), 2.26 (s, 3H), 3.78-3.95 (m, 1H), 6.60 (d, J = 7.3 Hz, 1H), 7.14-7.27 (m, 2H), 7.38-7.60 (m, 6H), 7.90-8.00 (m, 2H), 8.03-8.10 (m, 1H), 8.20 (s, 1H), 8.58 (s, 1H), 8.91 (s, 1H), 10.28 (s, 1H).
ESI m/z $(M+H)^+$ 575.

Example 220

8-(2-Fluoropyridin-5-yl)-7-hydroxy-2-isopropylamino-6-[2-methyl-5-N-(3-trifluoromethylphenyl)carbamo ylphenyl]quinazoline (Compound 220)

Compound 220 was obtained in the same manner as in Example 1 and Example 4, using Compound A19 and 2-fluoro-5-pyridine boronic acid.
$^1$H NMR (300 MHz, DMSO-$d_6$, 80°C) δ (ppm) 1.11 (d, J = 6.6 Hz, 6H), 2.26 (s, 3H), 3.79-3.94 (m, 1H), 6.72 (d, J = 7.2 Hz, 1H), 7.16-7.22 (m, 1H), 7.38-7.49 (m, 2H), 7.57 (t, J = 8.1 Hz, 1H), 7.60 (s, 1H), 7.92-8.10 (m, 4H), 8.21 (br s, 1H), 8.27-8.29 (m, 1H), 8.93 (s, 1H), 9.00 (br s, 1H), 10.29 (s, 1H).
ESI m/z $(M+H)^+$ 576.

Example 221

8-(4-Fluorophenyl)-7-hydroxy-2-methylamino-6-[2-methyl-5-N-(3-trifluoromethylphenyl)carbamoylpheny l]quinazoline (Compound 221)

Compound 221 was obtained in the same manner as in Example 1 and Example 4, using Compound A16 and 4-fluorophenyl boronic acid.
$^1$H NMR (270 MHz, DMSO-$d_6$, 80°C) δ (ppm) 2.26 (s, 3H), 2.74 (d, J = 5.1 Hz, 3H), 6.71-6.82 (m, 1H), 7.14-7.26 (m, 2H), 7.36-7.62 (m, 6H), 7.92-8.00 (m, 2H), 8.04-8.10 (m, 1H), 8.20 (s, 1H), 8.58 (br s, 1H), 8.91 (s, 1H), 10.28 (s, 1H).
ESI m/z $(M+H)^+$ 547.

Example 222

7-Hydroxy-2-isopropylamino-6-[2-methyl-5-N-(3-trifluoromethylphenyl)carbamoylphenyl]-8-(pyrimidin-5-yl)quinazoline (Compound 222)

Compound 222 was obtained in the same manner as in Example 1 and Example 4, using Compound A19 and 5-pyrimidine boronic acid.
$^1$H NMR (300 MHz, DMSO-$d_6$, 80°C) δ (ppm) 1.11 (d, J = 6.6 Hz, 6H), 2.27 (s, 3H), 3.81-3.94 (m, 1H), 6.80 (d, J = 7.5 Hz, 1H), 7.38-7.50 (m, 2H), 7.57 (t, J = 7.8 Hz, 1H), 7.64 (s, 1H), 7.92-8.00 (m, 2H), 8.05-8.10 (m, 1H), 8.21 (br s, 1H), 8.88 (s, 2H), 8.93 (s, 1H), 9.09 (s, 1H), 9.25 (br s, 1H), 10.29 (s, 1H).
ESI m/z $(M+H)^+$ 559.

Example 223

2-Amino-8-(4-fluorophenyl)-7-hydroxy-6-[2-methyl-5-N-(2-trifluoromethylpyridin-6-yl)carbamoylphenyl] quinazoline (Compound 223)

Compound 223 was obtained in the same manner as in Example 1 and Example 4, using Compound A59 and 4-fluorophenyl boronic acid.
$^1$H NMR (300 MHz, DMSO-$d_6$, 80°C) δ (ppm) 2.25 (s, 3H), 6.30 (br s, 2H), 7.16-7.26 (m, 2H), 7.37-7.46 (m, 3H), 7.55-7.62 (m, 2H), 7.94-8.03 (m, 2H), 8.04-8.13 (m, 1H), 8.42 (d, J = 8.4 Hz, 1H), 8.90 (s, 1H), 10.83 (br s, 1H).
ESI m/z $(M+H)^+$ 534.

Example 224

2-Amino-8-(4-cyanophenyl)-7-hydroxy-6-[2-methyl-5-N-(2-trifluoromethylpyridin-6-yl)carbamoylphenyl] quinazoline (Compound 224)

Compound 224 was obtained in the same manner as in Example 1 and Example 4, using Compound A59 and 4-cyanophenyl boronic acid.
$^1$H NMR (300 MHz, DMSO-$d_6$, 80°C) δ (ppm) 2.25 (s, 3H), 6.36 (br s, 2H), 7.43 (d, J = 8.4 Hz, 1H), 7.55-7.66 (m, 4H), 7.80-7.88 (m, 2H), 7.95-8.13 (m, 3H), 8.42 (d, J = 8.4 Hz, 1H), 8.92 (s, 1H), 10.83 (s, 1H).
ESI m/z $(M+H)^+$ 541.

Example 225

8-(4-Cyanophenyl)-7-hydroxy-2-isopropylamino-6-[2-methyl-5-N-(4-trifluoromethylpyridin-2-yl)carbamo ylphenyl]quinazoline (Compound 225)

Compound 225 was obtained in the same manner as in Example 1 and Example 4, using Compound A61 and 4-cyanophenyl boronic acid.
$^1$H NMR (300 MHz, DMSO-$d_6$) δ (ppm) 1.03-1.14 (m, 6H), 2.26 (s, 3H), 3.65-3.85 (m, 1H), 7.10 (br s, 1H), 7.42-7.56 (m, 2H), 7.60-7.74 (m, 3H), 7.83-7.93 (m, 2H), 7.97-8.05 (m, 2H), 8.55 (s, 1H), 8.67 (d, J = 5.1 Hz, 1H), 8.95 (s, 1H), 9.16 (br s, 1H), 11.28 (s, 1H).
ESI m/z $(M+H)^+$ 583.

Example 226

8-(2-Fluoropyridin-5-yl)-7-hydroxy-2-isopropylamino-6-[2-methyl-5-N-(4-trifluoromethylpyridin-2-yl)car bamoylphenyl] quinazoline (Compound 226)

Compound 226 was obtained in the same manner as in Example 1 and Example 4, using Compound A61 and 2-fluoro-5-pyridine boronic acid.
$^1$H NMR (300 MHz, DMSO-$d_6$) δ (ppm) 1.10 (d, J = 6.2 Hz, 6H), 2.26 (s, 3H), 3.60-3.95 (m, 1H), 7.11 (br s, 1H), 7.21-7.29 (m, 1H), 7.42-7.56 (m, 2H), 7.64 (s, 1H), 7.96-8.12 (m, 3H), 8.29 (s, 1H), 8.55 (s, 1H), 8.67 (d, J = 5.1 Hz, 1H), 8.95 (s, 1H), 9.25 (br s, 1H), 11.28 (s, 1H).
ESI m/z $(M+H)^+$ 577.

Example 227

8-(2-Fluoropyridin-5-yl)-7-hydroxy-6-[2-methyl-5-N-(3-trifluoromethylphenyl)carbamoylphenyl]-2-(4-tetrahydropyranylamino)quinazoline (Compound 227)

Compound 227 was obtained in the same manner as in Example 1 and Example 4, using Compound A34 and 2-fluoro-5-pyridine boronic acid.
$^1$H NMR (300 MHz, DMSO-$d_6$, 80°C) δ (ppm) 1.40-1.58 (m, 2H), 1.72-1.86 (m, 2H), 2.26 (s, 3H), 3.20-3.32 (m, 2H), 3.65-3.82 (m, 1H), 3.80-3.88 (m, 2H), 6.93 (d, J = 6.9 Hz, 1H), 7.16-7.22 (m, 1H), 7.38-7.48 (m, 2H), 7.52-7.60 (m, 1H), 7.62 (s, 1H), 7.92-8.10 (m, 4H), 8.20 (s, 1H), 8.24-8.30 (m, 1H), 8.95 (s, 1H), 9.05 (br s, 1H), 10.29 (s, 1H).
ESI m/z $(M+H)^+$ 618.

Example 228

7-Hydroxy-2-methoxycarbonylamino-6-[2-methyl-5-N-(3-trifluoromethylphenyl)carbamoylphenyl]-8-(pyrimidin-5-yl) quinazoline (Compound 228)

Compound 228 was obtained in the same manner as in Example 1, Example 143 and Step 2 of Example 194, using Compound A58, 5-pyrimidine boronic acid and methyl chloroformate.
$^1$H NMR (300 MHz, DMSO-$d_6$, 80°C) $\delta$ (ppm) 2.27 (s, 3H), 3.67 (s, 3H), 7.38-7.44 (m, 1H), 7.46-7.52 (m, 1H), 7.57 (t, J = 7.9 Hz, 1H), 7.89 (s, 1H), 7.96-8.02 (m, 2H), 8.04-8.10 (m, 1H), 8.20 (s, 1H), 8.98 (s, 2H), 9.12 (s, 1H), 9.25 (br s, 1H), 10.03 (br s, 1H), 10.30 (s, 1H).
ESI m/z $(M+H)^+$ 575.

Example 229

2-Amino-8-(4-chlorophenyl)-7-hydroxy-6-[2-methyl-5-N-(4-trifluoromethylpyridin-2-yl)carbamoylphenyl] quinazoline (Compound 229)

Compound 229 was obtained in the same manner as in Example 1 and Example 4, using Compound A53 and 4-chlorophenyl boronic acid.
$^1$H NMR (300 MHz, DMSO-$d_6$, 80°C) $\delta$ (ppm) 2.25 (s, 3H), 6.32 (br s, 2H), 7.39-7.47 (m, 6H), 7.59 (s, 1H), 7.94-8.03 (m, 2H), 8.50 (s, 1H), 8.63 (d, J = 5.1 Hz, 1H), 8.91 (s, 1H), 10.92 (s, 1H).
ESI m/z $(M+H)^+$ 550.

Example 230

7-Hydroxy-2-isopropylamino-6-[2-methyl-5-N-(3-trifluoromethylphenyl)carbamoylphenyl]-8-(pyridine-1-oxid-4-yl)quinazoline (Compound 230)

Compound 230 was obtained in the same manner as in Example 154 and Example 4, using Compound A19 and 4-(tributylstannyl)pyridine-1-oxide (WO 03/093273).
$^1$H NMR (300 MHz, DMSO-$d_6$) $\delta$ (ppm) 1.13 (d, J = 6.6 Hz, 6H), 2.25 (s, 3H), 3.75-3.98 (m, 1H), 7.19 (br s, 1H), 7.40-7.68 (m, 6H), 7.92-8.02 (m, 2H), 8.10 (d, J = 9.2 Hz, 1H), 8.19-8.30 (m, 3H), 8.95 (br s, 1H), 9.41 (s, 1H), 10.49 (s, 1H).
ESI m/z $(M+H)^+$ 574.

Example 231

7-Hydroxy-2-isopropylamino-6-[2-methyl-5-N-(4-trifluoromethylpyridin-2-yl)carbamoylphenyl]-8-(pyrimi din-5-yl)quinazoline (Compound 231)

Compound 231 was obtained in the same manner as in Example 1 and Example 4, using Compound A61 and 5-pyrimidine boronic acid.
$^1$H NMR (300 MHz, DMSO-$d_6$) $\delta$ (ppm) 1.10 (d, J = 6.6 Hz, 6H), 2.27 (s, 3H), 3.68-3.88 (m, 1H), 7.20 (br s, 1H), 7.43-7.57 (m, 2H), 7.68 (s, 1H), 7.96-8.06 (m, 2H), 8.55 (s, 1H), 8.67 (d, J = 4.8 Hz, 1H), 8.85-9.02 (m, 3H), 9.12 (s, 1H), 9.51 (br s, 1H), 11.28 (s, 1H).
ESI m/z $(M+H)^+$ 560.

Example 232

8-(4-Chlorophenyl)-7-hydroxy-2-isopropylamino-6-[2-methyl-5-N-(4-trifluoromethylpyridin-2-yl)carbamo ylphenyl] quinazoline (Compound 232)

Compound 232 was obtained in the same manner as in Example 1 and Example 4, using Compound A61 and 4-chlorophenyl boronic acid.
$^1$H NMR (300 MHz, DMSO-$d_6$) $\delta$ (ppm) 1.10 (d, J = 7.0 Hz, 6H), 2.25 (s, 3H), 3.68-3.92 (m, 1H), 7.02 (br s, 1H), 7.40-7.56 (m, 6H), 7.60 (s, 1H), 7.96-8.03 (m, 2H), 8.55 (s, 1H), 8.67 (d, J = 4.8 Hz, 1H), 8.87-9.02 (br m, 2H), 11.28 (s, 1H).
ESI m/z $(M+H)^+$ 592.

Example 233

2-Cyclopropylcarbonylamino-8-(2-fluoropyridin-5-yl)-7-hydroxy-6-[2-methyl-5-N-(4-trifluoromethylpyrid in-2-yl)carbamoylphenyl]quinazoline (Compound 233)

Compound 233 was obtained in the same manner as in Example 143 and Example 4, using Compound 174 and cyclopropylcarbonyl chloride.
$^1$H NMR (270 MHz, DMSO-$d_6$) δ (ppm) 0.62-0.87 (m, 4H), 2.27 (s, 3H), 2.36-2.50 (m, 1H), 7.22-7.32 (m, 1H), 7.46-7.56 (m, 2H), 7.93 (s, 1H), 8.00-8.16 (m, 3H), 8.33 (d, J = 2.3 Hz, 1H), 8.55 (s, 1H), 8.67 (d, J = 5.0 Hz, 1H), 9.30 (s, 1H), 9.77 (br s, 1H), 10.62 (s, 1H), 11.30 (s, 1H).
ESI m/z $(M+H)^+$ 603.

Example 234

2-Amino-7-hydroxy-6-[2-methyl-5-N-(4-trifluoromethylpyridin-2-yl)carbamoylphenyl]-8-(pyrimidin-5-yl) quinazoline (Compound 234)

Compound 234 was obtained in the same manner as in Example 1 and Example 4, using Compound A53 and 5-pyrimidine boronic acid.
$^1$H NMR (300 MHz, DMSO-$d_6$, 80°C) δ (ppm) 2.26 (s, 3H), 6.46 (br s, 2H), 7.40-7.50 (m, 2H), 7.66 (s, 1H), 7.96-8.04 (m, 2H), 8.51 (s, 1H), 8.63 (d, J = 5.1 Hz, 1H), 8.84 (s, 2H), 8.94 (s, 1H), 9.10 (s, 1H), 10.93 (s, 1H).
ESI m/z $(M+H)^+$ 518.

Example 235

8-(4-Chlorophenyl)-7-hydroxy-2-methoxycarbonylamino-6-[2-methyl-5-N-(3-trifluoromethylphenyl)carba moylphenyl] quinazoline (Compound 235)

Compound 235 was obtained in the same manner as in Example 1, Example 143 and Step 2 of Example 194, using Compound A58, 4-chlorophenyl boronic acid and methyl chloroformate.
$^1$H NMR (300 MHz, DMSO-$d_6$, 80°C) δ (ppm) 2.26 (s, 3H), 3.65 (s, 3H), 7.38-7.62 (m, 7H), 7.83 (s, 1H), 7.94-8.02 (m, 2H), 8.05-8.10 (m, 1H), 8.20 (br s, 1H), 9.23 (s, 1H), 9.88 (br s, 1H), 10.29 (s, 1H).
ESI m/z $(M+H)^+$ 607.

Example 236

8-(2-Fluoropyridin-5-yl)-7-hydroxy-2-methoxycarbonylamino-6-[2-methyl-5-N-(4-trifluoromethylpyridin-2-yl)carbamoylphenyl]quinazoline (Compound 236)

Compound 236 was obtained in the same manner as in Example 1, Example 143 and Step 2 of Example 194, using Compound A63, 2-fluoro-5-pyridine boronic acid and methyl chloroformate.
$^1$H NMR (270 MHz, DMSO-$d_6$) δ (ppm) 2.27 (s, 3H), 3.65 (s, 3H), 7.22-7.32 (m, 1H), 7.46-7.57 (m, 2H), 7.91 (s, 1H), 8.00-8.08 (m, 2H), 8.11-8.23 (m, 1H), 8.39 (s, 1H), 8.55 (s, 1H), 8.67 (d, J = 5.3 Hz, 1H), 9.27 (s, 1H), 9.74 (br s, 1H), 10.35 (s, 1H), 11.30 (s, 1H).
ESI m/z $(M+H)^+$ 593.

Example 237

2-Amino-8-(2-fluoropyridin-4-yl)-7-hydroxy-6-[2-methyl-5-N-(4-trifluoromethylpyridin-2-yl)carbamoylph enyl]quinazoline (Compound 237)

Compound 237 was obtained in the same manner as in Example 1 and Example 4, using Compound A53 and 2-fluoro-4-pyridine boronic acid.
$^1$H NMR (300 NHz, DMSO-$d_6$, 80°C) δ (ppm) 2.25 (s, 3H), 6.46 (br s, 2H), 7.17 (s, 1H), 7.36-7.49 (m, 3H), 7.64 (s, 1H), 7.95-8.03 (m, 2H), 8.25 (d, J = 5.1 Hz, 1H), 8.51 (s, 1H), 8.63 (d, J = 5.1 Hz, 1H), 8.92 (s, 1H), 10.92 (s, 1H).
ESI m/z $(M+H)^+$ 535.

Example 238

2-Cyclopropylcarbonylamino-7-hydroxy-6-[2-methyl-5-N-(4-trifluoromethylpyridin-2-yl)carbamoylphenyl ]-8-(pyrimidin-5-yl)quinazoline (Compound 238)

Compound 238 was obtained in the same manner as in Example 1, Example 143 and Example 4, using Compound A53, 5-pyrimidine boronic acid and cyclopropylcarbonyl chloride.
$^1$H NMR (270 MHz, DMSO-$d_6$, 80°C) δ (ppm) 0.64-0.88 (m, 4H), 2.27 (s, 3H), 2.34-2.47 (m, 1H), 7.43-7.52 (m, 2H), 7.91 (s, 1H), 7.99-8.08 (m, 2H), 8.51 (s, 1H), 8.64 (d, J = 5.0 Hz, 1H), 8.93 (s, 2H), 9.11 (s, 1H), 9.23 (s, 1H), 10.35 (br s, 1H), 10.95 (s, 1H).
ESI m/z $(M+H)^+$ 586.

Example 239

8-(2-Fluoropyridin-4-yl)-7-hydroxy-2-isopropylamino-6-[2-methyl-5-N-(4-trifluoromethylpyridin-2-yl)car bamoylphenyl] quinazoline (Compound 239)

Compound 239 was obtained in the same manner as in Example 1 and Example 4, using Compound A61 and 2-fluoro-4-pyridine boronic acid.
$^1$H NMR (300 MHz, DMSO-$d_6$, 80°C) δ (ppm) 1.13 (d, J = 6.6 Hz, 6H), 2.26 (s, 3H), 3.80-3.96 (m, 1H), 6.79 (d, J = 7.3 Hz, 1H), 7.22 (s, 1H), 7.41-7.49 (m, 3H), 7.63 (s, 1H), 7.97-8.02 (m, 2H), 8.25 (d, J = 5.1 Hz, 1H), 8.49-8.53 (m, 1H), 8.63 (d, J = 5.1 Hz, 1H), 8.93 (s, 1H), 9.07 (br s, 1H), 10.93 (s, 1H).
ESI m/z $(M+H)^+$ 577.

Example 240

2-Cyclopropylcarbonylamino-8-(2-fluoropyridin-4-yl)-7-hydroxy-6-[2-methyl-5-N-(4-trifluoromethylpyrid in-2-yl)carbamoylphenyl]quinazoline (Compound 240)

Compound 240 was obtained in the same manner as in Example 1, Example 143 and Example 4, using Compound A53 and 2-fluoro-4-pyridine boronic acid and cyclopropylcarbonyl chloride.
$^1$H NMR (270 MHz, DMSO-$d_6$, 80°C) δ (ppm) 0.60-0.74 (m, 2H), 0.78-0.88 (m, 2H), 2.26 (s, 3H), 2.42-2.60 (m, 1H), 7.28 (s, 1H), 7.42-7.53 (m, 3H), 7.90 (s, 1H), 7.98-8.08 (m, 2H), 8.26 (d, J = 5.3 Hz, 1H), 8.49-8.53 (m, 1H), 8.64 (d, J = 5.3 Hz, 1H), 9.23 (br s, 1H), 10.30 (br s, 1H), 10.95 (s, 1H).
ESI m/z $(M+H)^+$ 603.

Example 241

8-(2-Fluoropyridin-5-yl)-7-hydroxy-2-isopropylamino-6-[2-methyl-5-N-(4-trifluoromethylpyrimidin-2-yl)carbamoylphenyl]quinazoline (Compound 241)

Compound 241 was obtained in the same manner as in Example 1 and Example 4, using Compound A72 and 2-fluoro-5-pyridine boronic acid.
$^1$H NMR (270 MHz, DMSO-$d_6$) δ (ppm) 1.09 (d, J = 6.3 Hz, 6H), 2.26 (s, 3H), 3.64-3.89 (m, 1H), 7.12 (br s, 1H), 7.21-7.28 (m, 1H), 7.46 (d, J = 8.9 Hz, 1H), 7.63 (s, 1H), 7.73 (d, J = 5.0 Hz, 1H), 7.91-7.99 (m, 2H), 8.00-8.12 (m, 1H), 8.29 (s, 1H), 8.94 (s, 1H), 9.10 (d, J = 4.6 Hz, 1H), 9.26 (br s, 1H), 11.46 (s, 1H).
ESI m/z $(M+H)^+$ 578.

Example 242

2-Amino-8-(3-cyanophenyl)-7-hydroxy-6-[2-methyl-5-N-(4-trifluoromethylpyridin-2-yl)carbamoylphenyl] quinazoline (Compound 242)

Compound 242 was obtained in the same manner as in Example 1 and Example 4, using Compound A53 and 3-cyanophenyl boronic acid.
$^1$H NMR (270 MHz, DMSO-$d_6$, 80°C) δ (ppm) 2.26 (s, 3H), 6.38 (br s, 2H), 7.41-7.49 (m, 2H), 7.57-7.67 (m, 2H), 7.71-7.82 (m, 3H), 7.95-8.02 (m, 2H), 8.48-8.53 (m, 1H), 8.63 (d, J = 5.3 Hz, 1H), 8.92 (s, 1H), 10.92 (br s, 1H).
ESI m/z $(M+H)^+$ 541.

Example 243

2-Amino-7-hydroxy-8-(2-methylpyridin-4-yl)-6-[2-methyl-5-N-(4-trifluoromethylpyridin-2-yl)carbamoylp henyl]quinazoline (Compound 243)

Compound 243 was obtained in the same manner as in Example 1 and Example 4, using Compound A53 and 2-methyl-4-(4,4,5,5-tetramethyl-1,3,2-dioxaboran-2-yl)pyridine.
$^1$H NMR (270 MHz, DMSO-$d_6$, 80°C) $\delta$ (ppm) 2.25 (s, 3H), 2.47 (s, 3H), 6.39 (br s, 2H), 7.17-7.22 (m, 1H), 7.26 (s, 1H), 7.40-7.49 (m, 2H), 7.61 (s, 1H), 7.95-8.02 (m, 2H), 8.44-8.53 (m, 2H), 8.63 (d, J = 5.3 Hz, 1H), 8.91 (s, 1H), 10.93 (s, 1H).
ESI m/z $(M+H)^+$ 531.

Example 244

2-Amino-7-hydroxy-6-[2-methyl-5-N-(4-trifluoromethylpyridin-2-yl)carbamoylphenyl]-8-phenylquinazoli ne (Compound 244)

Compound 244 was obtained in the same manner as in Example 1 and Example 4, using Compound A53 and phenyl boronic acid.
$^1$H NMR (270 MHz, DMSO-$d_6$, 80°C) $\delta$ (ppm) 2.26 (s, 3H), 6.27 (br s, 2H), 7.28-7.49 (m, 7H), 7.58 (s, 1H), 7.93-8.03 (m, 2H), 8.48-8.53 (m, 1H), 8.63 (d, J = 5.3 Hz, 1H), 8.91 (s, 1H), 10.92 (br s, 1H).
ESI m/z $(M+H)^+$ 516.

Example 245

8-(3-Cyanophenyl)-7-hydroxy-2-isopropylamino-6-[2-methyl-5-N-(4-trifluoromethylpyridin-2-yl)carbamo ylphenyl]quinazoline (Compound 245)

Compound 245 was obtained in the same manner as in Example 1 and Example 4, using Compound A61 and 3-cyanophenyl boronic acid.
$^1$H NMR (270 MHz, DMSO-$d_6$, 80°C $\delta$ (ppm) 1.12 (d, J = 5.9 Hz, 6H), 2.27 (s, 3H), 3.73-3.95 (m, 1H), 6.72 (d, J = 7.6 Hz, 1H), 7.41-7.49 (m, 2H), 7.57-7.66 (m, 2H), 7.71-7.77 (m, 1H), 7.78-7.84 (m, 1H), 7.90 (s, 1H), 7.96-8.03 (m, 2H), 8.49-8.54 (m, 1H), 8.63 (d, J = 5.3 Hz, 1H), 8.92 (s, 2H), 10.93 (s, 1H).
ESI m/z $(M+H)^+$ 583.

Example 246

2-Amino-7-hydroxy-6-[2-methyl-5-N-(4-trifluoromethylpyridin-2-yl)carbamoylphenyl]-8-(pyridin-4-yl)qui nazoline (Compound 246)

Compound 246 was obtained in the same manner as in Example 1 and Example 4, using Compound A53 and 4-pyridine boronic acid.
$^1$H NMR (270 MHz, DMSO-$d_6$, 80°C) $\delta$ (ppm) 2.25 (s, 3H), 6.39 (br s, 2H), 7.39-7.49 (m, 4H), 7.63 (s, 1H), 7.95-8.02 (m, 2H), 8.49-8.52 (m, 1H), 8.57-8.66 (m, 3H), 8.93 (s, 1H), 10.93 (s, 1H).
ESI m/z $(M+H)^+$ 517.

Example 247

7-Hydroxy-2-isopropylamino-6-[2-methyl-5-N-(4-trifluoromethylpyridin-2-yl)carbamoylphenyl]-8-(pyridi ne-1-oxid-4-yl) quinazoline (Compound 247)

Compound 247 was obtained in the same manner as in Example 154 and Example 4, using Compound A61 and 4-(tributylstannyl)pyridine-1-oxide (WO 03/093273).
$^1$H NMR (270 MHz, DMSO-$d_6$) $\delta$ (ppm) 1.13 (d, J = 6.6 Hz, 6H), 2.25 (s, 3H), 3.75-3.97 (m, 1H), 7.18 (br s, 1H), 7.42-7.67 (m, 5H), 7.97-8.04 (m, 2H), 8.25 (d, J = 7.3 Hz, 2H), 8.55 (s, 1H), 8.67 (d, J = 5.3 Hz, 1H), 8.94 (s, 1H), 9.38 (br s, 1H), 11.29 (s, 1H).
ESI m/z $(M+H)^+$ 575.

Example 248

8-(3-Cyanophenyl)-2-cyclopropylcarbonylamino-7-hydroxy-6-[2-methyl-5-N-(4-trifluoromethylpyridin-2-yl)carbamoylphenyl]quinazoline (Compound 248)

Compound 248 was obtained in the same manner as in Example 1, Example 143 and Example 4, using Compound A53 and 3-cyanophenyl boronic acid and cyclopropylcarbonyl chloride.
$^1$H NMR (270 MHz, DMSO-$d_6$) δ (ppm) 0.58-0.71 (m, 2H), 0.75-0.86 (m, 2H), 2.27 (s, 3H), 2.42-2.60 (m, 1H), 7.46-7.57 (m, 2H), 7.61-7.70 (m, 1H), 7.79-7.88 (m, 2H), 7.90-7.98 (m, 2H), 8.00-8.09 (m, 2H), 8.56 (s, 1H), 8.67 (d, J = 5.3 Hz, 1H), 9.31 (br s, 1H), 9.67 (br s, 1H), 10.58 (br s, 1H), 11.30 (s, 1H).
ESI m/z $(M+H)^+$ 609.

Example 249

2-Amino-7-hydroxy-6-[2-methyl-5-N-(4-trifluoromethylpyridin-2-yl)carbamoylphenyl]-8-(pyridine-1-oxi d-4-yl)quinazoline (Compound 249)

Compound 249 was obtained in the same manner as in Example 154 and Example 4, using Compound A53 and 4-(tributylstannyl)pyridine-1-oxide synthesized by the method described in WO 03/093273.
$^1$H NMR (270 MHz, DMSO-$d_6$) δ (ppm) 2.25 (s, 3H), 6.72 (br s, 2H), 7.42-7.57 (m, 4H), 7.65 (s, 1H), 7.96-8.04 (m, 2H), 8.26 (d, J = 6.9 Hz, 2H), 8.55 (s, 1H), 8.67 (d, J = 5.3 Hz, 1H), 8.96 (s, 1H), 9.31 (br s, 1H), 11.28 (s, 1H).
ESI m/z $(M+H)^+$ 533.

Example 250

2-Amino-8-(2-fluoropyridin-4-yl)-7-hydroxy-6-[2-methyl-5-N-(4-trifluoromethylpyrimidin-6-yl)carbamoyl phenyl]quinazoline (Compound 250)

Compound 250 was obtained in the same manner as in Example 1 and Example 4, using Compound A65 and 2-fluoropyridin-4-yl boronic acid.
$^1$H NMR (300 MHz, $CDCl_3$) δ (ppm) 2.34 (s, 3H), 5.18 (s, 2H), 7.39 (d, J = 4.0 Hz, 1H), 7.54-7.61 (m, 3H), 7.90-7.95 (m, 3H), 8.36 (d, J = 5.5 Hz, 1H), 8.72 (s, 1H), 8.83 (s, 1H), 8.95 (s, 1H), 9.01 (s, 1H).
ESI m/z $(M+H)^+$ 536.

Example 251

2-Amino-7-hydroxy-6-[2-methyl-5-N-(4-trifluoromethylpyrimidin-6-yl)carbamoylphenyl]-8-(pyrimidin-5-yl)quinazoline (Compound 251)

Compound 251 was obtained in the same manner as in Example 1 and Example 4, using Compound A65 and pyrimidin-5-boronic acid.
$^1$H NMR (300 MHz, $CDCl_3$) δ (ppm) 2.34 (s, 3H), 5.22 (s, 2H), 7.55-7.61 (m, 2H), 7.91-7.97 (m, 2H), 8.69 (d, J = 1.1 Hz, 1H), 8.89 (s, 1H), 8.93 (s, 1H), 8.97 (s, 2H), 9.00 (s, 1H), 9.13 (s, 1H).
ESI m/z $(M+H)^+$ 519.

Example 252

2-Amino-8-(3-cyanophenyl)-7-hydroxy-6-[2-methyl-5-N-(4-trifluoromethylpyrimidin-6-yl)carbamoylphen yl]quinazoline (Compound 252)

Compound 252 was obtained in the same manner as in Example 1 and Example 4, using Compound A65 and 3-cyanophenyl boronic acid.
$^1$H NMR (300 MHz, $CDCl_3$) δ (ppm) 2.34 (s, 3H), 5.17 (s, 2H), 5.50 (s, 1H), 7.52-7.66 (m, 3H), 7.72-7.80 (m, 2H), 7.87-7.95 (m, 3H), 8.72 (d, J = 1.1 Hz, 1H), 8.86 (s, 1H), 8.93 (s, 1H), 9.01 (s, 1H).
ESI m/z $(M+H)^+$ 542.

Example 253

2-Amino-8-(2-fluoropyridin-5-yl)-7-hydroxy-6-[2-methyl-5-N-(4-trifluoromethylpyrimidin-6-yl)carbamoyl phenyl]quinazoline (Compound 253)

Compound 253 was obtained in the same manner as in Example 1 and Example 4, using Compound A65 and 2-fluoropyridin-5-yl boronic acid.
$^1$H NMR (300 MHz, DMSO-$d_6$) $\delta$ (ppm) 2.27 (s, 3H), 5.80 (s, 2H), 7.01 (dd, J = 8.1, 3.6 Hz, 1H), 7.24 (s, 1H), 7.33 (d, J = 8.1 Hz, 1H), 7.85-7.90 (m, 3H), 8.09-8.16 (m, 1H), 8.36-8.39 (m, 2H), 8.60 (s, 1H), 9.14 (s, 1H).
ESI m/z $(M+H)^+$ 536.

Example 254

2-Amino-8-(2-ethoxypyridin-5-yl)-7-hydroxy-6-[2-methyl-5-N-(4-trifluoromethylpyridin-2-yl)carbamoylp henyl]quinazoline (Compound 254)

Compound 254 was obtained as a by-product in the production of Example 181.
$^1$H NMR (300 MHz, DMSO-$d_6$, 80°C) $\delta$ (ppm) 1.35 (t, J = 7.0 Hz, 3H), 2.25 (s, 3H), 4.37 (q, J = 7.0 Hz, 2H), 6.39 (br s, 2H), 6.75-6.79 (m, 1H), 6.94-6.99 (m, 1H), 7.40-7.48 (m, 2H), 7.60 (s, 1H), 7.95-8.01 (m, 2H), 8.13-8.19 (m, 1H), 8.50 (s, 1H), 8.63 (d, J = 5.1 Hz, 1H), 8.90 (s, 1H), 10.91 (br s, 1H).
ESI m/z $(M+H)^+$ 561.

Example 255

2-Amino-8-(2-ethoxypyridin-4-yl)-7-hydroxy-6-[2-methyl-5-N-(4-trifluoromethylpyridin-2-yl)carbamoylp henyl]quinazoline (Compound 255)

Compound 255 was obtained as a by-product in the production of Example 237.
$^1$H NMR (300 MHz, DMSO-$d_6$, 80°C) $\delta$ (ppm) 1.35 (t, J = 7.0 Hz, 3H), 2.25 (s, 3H), 4.37 (q, J = 7.0 Hz, 2H), 6.39 (br s, 2H), 6.74-6.79 (m, 1H), 6.94-6.99 (m, 1H), 7.40-7.48 (m, 2H), 7.60 (s, 1H), 7.95-8.01 (m, 2H), 8.14-8.18 (m, 1H), 8.50 (s, 1H), 8.63 (d, J = 5.1 Hz, 1H), 8.90 (s, 1H), 10.91 (br s, 1H).
ESI m/z $(M+H)^+$ 561.

Example 256

2-Amino-7-hydroxy-6-[2-methyl-5-N-(4-trifluoromethylpyridin-2-yl)carbamoylphenyl]-8-(pyridin-3-yl)qui nazoline (Compound 256)

Compound 256 was obtained in the same manner as in Example 1 and Example 4, using Compound A53 and 3-pyridine boronic acid.
$^1$H NMR (300 MHz, DMSO-$d_6$, 80°C) $\delta$ (ppm) 2.26 (s, 3H), 6.35 (br s, 2H), 7.40-7.48 (m, 3H), 7.62 (s, 1H), 7.76-7.83 (m, 1H), 7.95-8.03 (m, 2H), 8.47-8.53 (m, 2H), 8.57-8.67 (m, 2H), 8.92 (s, 1H), 10.92 (br s, 1H).
ESI m/z $(M+H)^+$ 517.

Example 257

2-Amino-7-hydroxy-6-[2-methyl-5-N-(4-trifluoromethylpyridin-2-yl)carbamoylphenyl]-8-[2-(pyrrolidin-1-yl)pyrimidin-5-yl] quinazoline (Compound 257)

Compound 257 was obtained in the same manner as in Example 1 and Example 4, using Compound A53 and 2-(pyrrolidin-1-yl)-5-(4,4,5,5-tetramethyl-1,3,2-dioxaboran-2-yl)pyrimidine.
H NMR (300 MHz, DMSO-$d_6$, 80°C) $\delta$ (ppm) 1.92-2.02 (m, 4H), 2.25 (s, 3H), 3.51-3.62 (m, 4H), 6.36 (br s, 2H), 7.40-7.48 (m, 2H), 7.57 (s, 1H), 7.95-8.02 (m, 2H), 8.34 (s, 2H), 8.49-8.52 (m, 1H), 8.63 (d, J = 5.1 Hz, 1H), 8.90 (s, 1H), 10.92 (br s, 1H).
ESI m/z $(M+H)^+$ 587.

Example 258

2-Amino-7-hydroxy-6-[2-methyl-5-N-(4-trifluoromethylpyridin-2-yl)carbamoylphenyl]-8-[2-(morpholino) pyrimidin-5-yl] quinazoline (Compound 258)

Compound 258 was obtained in the same manner as in Example 1 and Example 4, using Compound A53 and 2-morpholino-5-(4,4,5,5-tetramethyl-1,3,2-dioxaboran-2-yl)pyrimidine.
H NMR (300 MHz, DMSO-$d_6$, 80°C) $\delta$ (ppm) 2.25 (s, 3H), 3.67-3.74 (m, 4H), 3.75-3.82 (m, 4H), 6.38 (br s, 2H), 7.42-7.48 (m, 2H), 7.59 (s, 1H), 7.96-8.02 (m, 2H), 8.42 (s, 2H), 8.51 (s, 1H), 8.63 (d, J = 5.1 Hz, 1H), 8.92 (s, 1H), 10.92 (s, 1H).
ESI m/z $(M+H)^+$ 603.

Example 259

2-Amino-7-hydroxy-8-(1-methyl-1H-pyrazol-4-yl)-6-[2-methyl-5-N-(4-trifluoromethylpyridin-2-yl)carbam oylphenyl] quinazoline (Compound 259)

Compound 259 was obtained in the same manner as in Example 1 and Example 4, using Compound A53 and 1-methyl-4-(4,4,5,5-tetramethyl-1,3,2-dioxaboran-2-yl)-1H-pyrazole.
$^1H$ NMR (300 MHz, DMSO-$d_6$, 80°C) $\delta$ (ppm) 2.23 (s, 3H), 3.91 (s, 3H), 6.44 (br s, 2H), 7.42-7.48 (m, 3H), 7.96-8.03 (m, 2H), 8.10 (s, 1H), 8.35 (s, 1H), 8.48-8.67 (m, 3H), 8.90 (s, 1H), 10.92 (s, 1H).
ESI m/z $(M+H)^+$ 520.

Example 260

2-Amino-7-hydroxy-6-[2-methyl-5-N-(4-trifluoromethylpyridin-2-yl)carbamoylphenyl]-8-(pyridin-2-yl)qui nazoline (Compound 260)

Compound 260 was obtained in the same manner as in Example 6 and Example 4, using Compound A53 and 2-(tributylstannyl)pyridine.
$^1H$ NMR (270 MHz, DMSO-$d_6$, 80°C) $\delta$ (ppm) 2.26 (s, 3H), 6.72 (br s, 2H), 7.40-7.49 (m, 3H), 7.66 (s, 1H), 7.96-8.09 (m, 3H), 8.49-8.53 (m, 1H), 8.55-8.66 (m, 2H), 8.90 (s, 1H), 9.87-9.94 (m, 1H), 10.92 (s, 1H).
ESI m/z $(M+H)^+$ 517.

Example 261

2-Amino-8-(2-chloropyridin-4-yl)-7-hydroxy-6-[2-methyl-5-N-(4-trifluoromethylpyridin-2-yl)carbamoylph enyl]quinazoline (Compound 261)

Compound 261 was obtained in the same manner as in Example 1 and Example 4, using Compound A53 and 2-chloro-4-pyridine boronic acid.
$^1H$ NMR (300 MHz, DMSO-$d_6$, 80°C) $\delta$ (ppm) 2.25 (s, 3H), 6.46 (br s, 2H), 7.41-7.51 (m, 4H), 7.65 (s, 1H), 7.96-8.04 (m, 2H), 8.41-8.47 (m, 1H), 8.51 (d, J = 0.7 Hz, 1H), 8.64 (d, J = 5.1 Hz, 1H), 8.94 (s, 1H), 10.92 (s, 1H).
ESI m/z $(M+H)^+$ 551.

Example 262

2-Amino-8-(3-cyanophenyl)-7-hydroxy-6-[2-methyl-5-(4-trifluoromethylpicolinamide)phenyl]quinazoline (Compound 262)

Compound 262 was obtained in the same manner as in Example 1 and Example 4, using Compound A67 and 3-cyanophenyl boronic acid.
$^1H$ NMR (300 MHz, $CDCl_3$) $\delta$ (ppm) 2.23 (s, 3H), 5.10 (s, 2H), 5.47 (s, 1H), 7.42 (d, J = 7.7 Hz, 1H), 7.57-7.62 (m, 2H), 7.68-7.74 (m, 2H), 7.78-7.84 (m, 3H), 7.91 (s, 1H), 8.53 (s, 1H), 8.81 (d, J = 4.8 Hz, 1H), 8.93 (s, 1H), 9.98 (s, 1H).
ESI m/z $(M+H)^+$ 541.

Example 263

(S)-2-(1-Hydroxypropan-2-ylamino)-7-methoxy-6-[2-methyl-5-N-(4-trifluoromethylpyridin-2-yl)carbamoy lphenyl]-8-(pyridin-3-yl)quinazoline (Compound 263)

Compound 263 was obtained in the same manner as in Example 1, using Compound A70 and 3-pyridine boronic acid.
$^1$H NMR (300 MHz, $CDCl_3$) 8 (ppm) 1.24 (d, J = 6.0 Hz, 3H), 2.34 (s, 3H), 3.15 (s, 3H), 3.61 (br s, 2H), 3.98-4.07 (m, 1H), 5.36 (d, J = 6.2 Hz, 1H), 7.26-7.31 (m, 2H), 7.42-7.49 (m, 2H), 7.60 (s, 1H), 7.87-7.91 (m, 3H), 8.47 (d, J = 5.1 Hz, 1H), 8.65 (dd, J = 5.1, 1.5 Hz, 1H), 8.71 (br s, 2H), 8.83 (s, 1H), 8.95 (s, 1H).
ESI m/z $(M+H)^+$ 589.

Example 264

(S)-2-(1-Hydroxypropan-2-ylamino)-7-methoxy-6-[2-methyl-5-N-(4-trifluoromethylpyridin-2-yl)carbamoy lphenyl]-8-(pyridin-4-yl)quinazoline (Compound 264)

Compound 264 was obtained in the same manner as in Example 1, using Compound A70 and 4-pyridine boronic acid.
$^1$H NMR (300 MHz, $CDCl_3$) $\delta$ (ppm) 1.23 (d, J = 7.0 Hz, 3H), 2.34 (s, 3H), 3.16 (s, 3H), 3.55 (br s, 1H), 3.68 (br s, 1H), 3.97-4.06 (m, 1H), 5.38 (d, J = 6.2 Hz, 1H), 7.29-7.32 (m, 1H), 7.46-7.49 (m, 3H), 7.62 (s, 1H), 7.87-7.91 (m, 3H), 8.48 (d, J = 5.1 Hz, 1H), 8.71-8.76 (m, 4H), 8.96 (s, 1H).
ESI m/z $(M+H)^+$ 589.

Example 265

(S)-7-Hydroxy-2-(1-hydroxypropan-2-ylamino)-6-[2-methyl-5-N-(4-trifluoromethylpyridin-2-yl)carbamoyl phenyl]-8-(pyrimidin-5-yl)quinazoline (Compound 265)

Compound 265 was obtained in the same manner as in Example 1 and Example 4, using Compound A70 and pyrimidin-5-boronic acid.
$^1$H NMR (300 MHz, $CDCl_3$) $\delta$ (ppm) 1.28 (d, J = 7.0 Hz, 3H), 2.38 (s, 3H), 3.60-3.67 (m, 1H), 3.81 (br s, 1H), 4.22 (br s, 1H), 5.33 (br s, 1H), 7.21-7.26 (m, 2H), 7.41-7.50 (m, 2H), 7.72-7.76 (m, 1H), 7.83-7.88 (m, 1H), 7.94-7.98 (m, 1H), 8.01-8.04 (m, 1H), 8.31-8.33 (m, 1H), 8.40-8.45 (m, 1H), 8.66-8.74 (m, 1H), 8.96 (s, 2H), 9.05 (br s, 1H).
ESI m/z $(M+H)^+$ 576.

Example 266

(S)-7-Hydroxy-2-(1-hydroxypropan-2-ylamino)-6-[2-methyl-5-N-(4-trifluoromethylpyridin-2-yl)carbamoyl phenyl]-8-(pyridin-3-yl)quinazoline (Compound 266)

Compound 266 was obtained in the same manner as in Example 4, using Compound 263.
$^1$H NMR (300 MHz, $CDCl_3$) $\delta$ (ppm) 1.21 (d, J = 7.0 Hz, 3H), 2.33 (s, 3H), 3.49-3.55 (m, 1H), 3.64 (br s, 1H), 3.99 (br s, 1H), 5.40 (br s, 1H), 7.26-7.29 (m, 2H), 7.40-7.51 (m, 4H), 7.86-7.90 (m, 3H), 8.41 (d, J = 5.5 Hz, 1H), 8.50 (d, J = 4.4 Hz, 1H), 8.65 (s, 1H), 8.70 (s, 1H), 8.79 (s, 1H), 8.82 (s, 1H).
ESI m/z $(M+H)^+$ 575.

Example 267

(S)-7-Hydroxy-2-(1-hydroxypropan-2-ylamino)-6-[2-methyl-5-N-(4-trifluoromethylpyridin-2-yl)carbamoyl phenyl]-8-(pyridin-4-yl)quinazoline (Compound 267)

Compound 267 was obtained in the same manner as in Example 4, using Compound 264.
$^1$H NMR (300 MHz, DMSO-$d_6$) $\delta$ (ppm) 1.07 (d, J = 6.6 Hz, 3H), 2.25 (s, 3H), 3.27-3.43 (m, 2H), 3.74 (br s, 1H), 4.58 (s, 1H), 6.89 (br s, 1H), 7.45-7.54 (m, 4H), 7.64 (s, 1H), 7.99-8.02 (m, 2H), 8.55 (s, 1H), 8.61 (d, J = 5.9 Hz, 2H), 8.67 (d, J = 5.0 Hz, 1H), 8.95 (s, 1H), 9.23 (s, 1H), 11.28 (br s, 1H).
ESI m/z $(M+H)^+$ 575.

Example 268

2-Amino-7-hydroxy-6-[2-methyl-5-N-(4-trifluoromethylpyridin-2-yl)carbamoylphenyl]-8-[2-(1,3-oxazolidi n-2-on-3-yl)pyridin-4-yl]quinazoline (Compound 268)

Compound 268 was obtained in the same manner as in Example 179, using Compound 261 and 2-oxazolidone. $^1$H NMR (300 MHz, DMSO-$d_6$, 80°C) δ (ppm) 2.25 (s, 3H), 4.24 (t, J = 7.9 Hz, 2H), 4.48 (t, J = 7.9 Hz, 2H), 6.35 (br s, 2H), 7.12-7.19 (m, 1H), 7.40-7.48 (m, 2H), 7.63 (s, 1H), 7.94-8.05 (m, 2H), 8.12 (s, 1H), 8.39 (d, J = 5.1 Hz, 1H), 8.50 (s, 1H), 8.63 (d, J = 5.1 Hz, 1H), 8.94 (s, 1H), 10.92 (s, 1H).
ESI m/z $(M+H)^+$ 602.

Example 269

2-Amino-7-hydroxy-6-[2-methyl-5-N-(4-trifluoromethylpyrimidin-6-yl)carbamoylphenyl]-8-(pyridin-3-yl)quinazoline (Compound 269)

Compound 269 was obtained in the same manner as in Example 1 and Example 4, using Compound A65 and 3-pyridine boronic acid.
$^1$H NMR (300 MHz, $CDCl_3$) δ (ppm) 2.27 (s, 3H), 6.64 (s, 2H), 7.46-7.51 (m, 2H), 7.65 (s, 1H), 7.78-7.82 (m, 1H), 8.01 (d, J = 9.9 Hz, 2H), 8.53 (dd, J = 4.8, 1.5 Hz, 1H), 8.57 (d, J = 1.5 Hz, 1H), 8.62 (d, J = 1.1 Hz, 1H), 8.97 (s, 1H), 9.10 (s, 1H), 9.18 (s, 1H), 11.81 (s, 1H).
ESI m/z $(M+H)^+$ 518.

Example 270

2-Amino-7-hydroxy-6-[2-methyl-5-N-(4-trifluoromethylpyrimidin-6-yl)carbamoylphenyl]-8-(pyridin-4-yl)quinazoline (Compound 270)

Compound 270 was obtained in the same manner as in Example 1 and Example 4, using Compound A65 and 4-pyridine boronic acid.
$^1$H NMR (300 MHz, DMSO-$d_6$) δ (ppm) 2.26 (s, 3H), 6.67 (s, 2H), 7.43 (d, J = 5.1 Hz, 2H), 7.49 (d, J = 8.1 Hz, 1H), 7.65 (s, 1H), 8.01 (d, J = 8.1 Hz, 2H), 8.63 (d, J = 5.1 Hz, 3H), 8.96 (s, 1H), 9.18 (s, 1H), 11.81 (s, 1H).
ESI m/z $(M+H)^+$ 518.

Example 271

2-Amino-7-hydroxy-6-[2-methyl-5-N-(-4-trifluoromethylpyrimidin-6-yl)carbamoylphenyl]-8-(pyridin-2-yl)quinazoline (Compound 271)

Compound 271 was obtained in the same manner as in Example 1 and Example 4, using Compound A65 and 2-pyridine boronic acid.
$^1$H NMR (270 MHz, $CDCl_3$) δ (ppm) 2.26 (s, 3H), 7.02 (s, 2H), 7.46-7.51 (m, 2H), 7.69 (s, 1H), 7.98-8.11 (m, 4H), 8.59-8.62 (m, 2H), 8.92 (s, 1H), 9.17 (s, 1H).
ESI m/z $(M+H)^+$ 518.

Example 272

2-Amino-7-hydroxy-6-[2-methyl-5-N-(4-trifluoromethylpyrimidin-2-yl)carbamoylphenyl]-8-(pyridin-3-yl)quinazoline (Compound 272)

Compound 272 was obtained in the same manner as in Example 1 and Example 4, using Compound A73 and 3-pyridine boronic acid.
$^1$H NMR (270 MHz, DMSO-$d_6$) δ (ppm) 2.26 (s, 3H), 6.67 (br s, 2H), 7.23-7.32 (m, 1H), 7.40-7.52 (m, 2H), 7.55-7.70 (m, 2H), 7.92-8.04 (m, 2H), 8.10 (d, J = 8.6 Hz, 1H), 8.21-8.27 (m, 2H), 8.97 (s, 1H), 9.21 (br s, 1H), 10.48 (s, 1H).
ESI m/z $(M+H)^+$ 518.

Example 273

2-Amino-7-hydroxy-8-(4-hydroxyphenyl)-6-[2-methyl-5-N-(4-trifluoromethylpyridin-2-yl)carbamoylphen yl]quinazoline (Compound 273)

Compound 273 was obtained in the same manner as in Example 1 and Example 4, using Compound A53 and 4-hydroxyphenyl boronic acid.
$^1$H NMR (300 MHz, DMSO-$d_6$, 100°C) δ (ppm) 2.25 (s, 3H), 6.11 (br s, 2H), 6.81-6.88 (m, 2H), 7.19 (d, J = 8.4 Hz, 2H), 7.38-7.46 (m, 2H), 7.53 (s, 1H), 7.92-8.01 (m, 2H), 8.48 (s, 1H), 8.61 (d, J = 5.1 Hz, 1H), 8.89 (s, 1H), 8.96 (s, 1H), 10.79 (s, 1H).
ESI m/z $(M+H)^+$ 532.

Example 274

2-Amino-7-hydroxy-6-[2-methyl-5-N-(4-trifluoromethylpyridin-2-yl)carbamoylphenyl]-8-(pyrazin-2-yl)qu inazoline (Compound 274)

Compound 274 was obtained in the same manner as in Example 6 and Example 4, using Compound A53 and 2-(tributylstannyl)pyrazine.
$^1$H NMR (300 MHz, DMSO-$d_6$, 80°C) δ (ppm) 2.26 (s, 3H), 6.94 (br s, 2H), 7.40-7.49 (m, 2H), 7.72 (s, 1H), 7.96-8.03 (m, 2H), 8.50 (s, 1H), 8.60-8.66 (m, 3H), 8.94 (s, 1H), 10.65 (s, 1H), 10.92 (s, 1H).
ESI m/z $(M+H)^+$ 518.

Example 275

2-Amino-7-hydroxy-8-(4-hydroxyphenyl)-6-[2-methyl-5-N-(3-trifluoromethylphenyl)carbamoylphenyl]qui nazoline (Compound 275)

Compound 275 was obtained in the same manner as in Example 1 and Example 4, using Compound A11 and 4-hydroxyphenyl boronic acid.
$^1$H NMR (300 MHz, DMSO-$d_6$, 80°C) δ (ppm) 2.25 (s, 3H), 6.22 (br s, 2H), 6.81-6.87 (m, 2H), 7.13-7.22 (m, 2H), 7.37-7.47 (m, 2H), 7.50-7.60 (m, 2H), 7.89-7.97 (m, 2H), 8.07 (d, J = 8.4 Hz, 1H), 8.20 (s, 1H), 8.90 (s, 1H), 9.08 (s, 1H), 10.27 (s, 1H).
ESI m/z $(M+H)^+$ 531.

Example 276

A tablet having the following composition is prepared according to the ordinary processes. Compound 10 (40 g), 286.8 g of lactose, and 60 g of potato starch are mixed together, and 120 g of 10% aqueous hydroxypropylcellulose solution is added thereto. After the resulting mixture is kneaded, granulated and dried according to the ordinary processes, the size of the granules is prepared for tablet pressing. Then, 1.2 g of magnesium stearate is added thereto and mixed, followed by pressing to make tablets by a tablet making machine (RT-15 type, Kikushi) having a pestle of 8 mm diameter to obtain a tablet (each tablet containing 20 mg of the active ingredient).
Table 29

| | | |
|---|---|---|
| Formulation | Compound 10 | 20 mg |
| | Lactose | 143.4 mg |
| | potato starch | 30 mg |
| | hydroxypropylcellulose | 6 mg |
| | magnesium stearate | 0.6 mg |
| | | 200 mg |

Example 277

The injection having the following composition is prepared according to ordinary processes. Compound 10 (1 g) and D-mannitol (5 g) are added into distilled injection water and mixed, then hydrochloric acid and an aqueous sodium

hydroxide solution are added to adjust the pH to 6, and the total volume is made 1000 mL with distilled injection water. The obtained mixed liquid is dispensed into glass vials at a volume of 2 mL per vial (each vial contains 2 mg of the active ingredient) under the sterile condition to obtain the injection.

Table 30

| | | |
|---|---|---|
| Formulation | Compound 10 | 2 mg |
| | D-mannitol | 10 mg |
| | hydrochloric acid | proper amount |
| | aqueous sodium hydroxide solution | proper amount |
| | distilled injection water | proper amount |
| | | 2.00 mL |

Industrial Applicability

The present invention provides a 2-aminoquinazoline derivative having Tie-2 kinase inhibitory activity, and the like. Said 2-aminoquinazoline derivative and the like can be used as an agent for treating and/or preventing a disease associated with Tie-2 kinase (for example, ovarian cancer, breast cancer, renal cancer, prostate cancer, lung cancer, thyroid cancer, myeloid leukemia, hemangiomas, melanomas, astrocytomas, glioblastomas, psoriasis or pulmonary hypertension, or the like).

## Claims

**1.** A 2-aminoquinazoline derivative represented by formula (I)

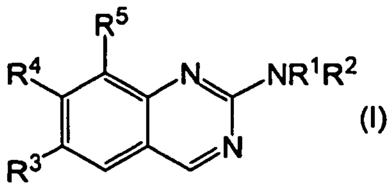

(I)

{wherein in formula (I), $R^1$ represents a hydrogen atom, optionally substituted $C_{1-10}$ alkyl, optionally substituted $C_{2-10}$ alkenyl, optionally substituted $C_{2-10}$ alkynyl, optionally substituted cycloalkyl, optionally substituted cycloalkenyl, optionally substituted $C_{2-11}$ alkanoyl, optionally substituted cycloalkylcarbonyl, optionally substituted $C_{1-10}$ alkoxy-carbonyl, optionally substituted aryl, an optionally substituted aliphatic heterocyclic group, an optionally substituted aromatic heterocyclic group, or
-C(=O)$NR^6R^7$ (wherein $R^6$ and $R^7$ may be the same or different and each represents a hydrogen atom or optionally substituted $C_{1-10}$ alkyl),
$R^2$ represents a hydrogen atom,
$R^3$ represents formula (II)

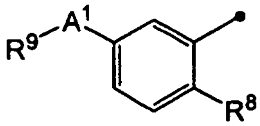

(II)

[wherein in formula (II), $A^1$ represents formula (III)

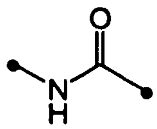 (III)

or formula (IV)

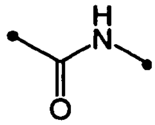 (IV)

,

$R^8$ represents a hydrogen atom or optionally substituted $C_{1-10}$ alkyl, and $R^9$ represents optionally substituted aryl or an optionally substituted aromatic heterocyclic group],
$R^4$ represents hydroxy, or optionally substituted $C_{1-10}$ alkoxy, and
$R^5$ represents optionally substituted aryl, or an optionally substituted aromatic heterocyclic group}, or
a pharmaceutically acceptable salt thereof.

**2.** The 2-aminoquinazoline derivative or a pharmaceutically acceptable salt thereof according to claim 1, wherein $R^1$ is a hydrogen atom, optionally substituted $C_{1-10}$ alkyl, optionally substituted $C_{2-10}$ alkenyl, optionally substituted $C_{2-10}$ alkynyl, optionally substituted cycloalkenyl, optionally substituted $C_{2-11}$ alkanoyl, optionally substituted cycloalkylcarbonyl, optionally substituted $C_{1-10}$ alkoxycarbonyl or -C(=O)$NR^6R^7$ (wherein $R^6$ and $R^7$ have the same meanings as defined above, respectively).

**3.** The 2-aminoquinazoline derivative or a pharmaceutically acceptable salt thereof according to claim 1, wherein $R^1$ is a hydrogen atom.

**4.** The 2-aminoquinazoline derivative or a pharmaceutically acceptable salt thereof according to claim 1, wherein $R^1$ is optionally substituted $C_{1-10}$ alkyl, optionally substituted $C_{2-11}$ alkanoyl, optionally substituted cycloalkylcarbonyl or optionally substituted $C_{1-10}$ alkoxycarbonyl.

**5.** The 2-aminoquinazoline derivative or a pharmaceutically acceptable salt thereof according to claim 1, wherein $R^1$ is optionally substituted cycloalkyl, optionally substituted aryl, an optionally substituted aliphatic heterocyclic group or an optionally substituted aromatic heterocyclic group.

**6.** The 2-aminoquinazoline derivative or a pharmaceutically acceptable salt thereof according to any one of claims 1 to 5, wherein $R^4$ is hydroxy or methoxy.

**7.** The 2-aminoquinazoline derivative or a pharmaceutically acceptable salt thereof according to any one of claims 1 to 6, wherein $R^8$ is methyl.

**8.** The 2-aminoquinazoline derivative or a pharmaceutically acceptable salt thereof according to any one of claims 1 to 7, wherein $R^9$ is a group represented by formula (V)

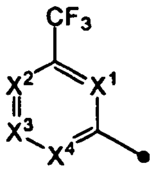 (V)

(wherein $X^1$, $X^2$, $X^3$, and $X^4$ may be the same or different, and each represents CH, C-$A^2$ {wherein $A^2$ represents halogen, optionally substituted $C_{1-10}$ alkyl, an optionally substituted aliphatic heterocyclic group, an optionally substituted aromatic heterocyclic group, optionally substituted $C_{1-10}$ alkoxy, optionally substituted aliphatic heterocycle-oxy, optionally substituted aromatic heterocycle-oxy, optionally substituted aliphatic heterocycle-methyl, optionally substituted aromatic heterocycle-methyl , $CH_2NR^{10}R^{11}$ (wherein $R^{10}$ and $R^{11}$ may be the same or different, and

each represents a hydrogen atom, or optionally substituted $C_{1-10}$ alkyl) or $NR^{12}R^{13}$ [wherein $R^{12}$ represents a hydrogen atom or optionally substituted $C_{1-10}$ alkyl, and $R^{13}$ represents a hydrogen atom, optionally substituted $C_{1-10}$ alkyl, an optionally substituted aliphatic heterocyclic group, an optionally substituted aromatic heterocyclic group or $-NR^{10A}R^{11A}$ (wherein $R^{10A}$ and $R^{11A}$ have the same meanings as $R^{10}$ and $R^{11}$ defined above, respectively)]}, or a nitrogen atom).

**9.** The 2-aminoquinazoline derivative or a pharmaceutically acceptable salt thereof according to claim 8, wherein $X^1$, $X^2$, $X^3$, and $X^4$ may be the same or different, and each is CH or a nitrogen atom.

**10.** The 2-aminoquinazoline derivative or a pharmaceutically acceptable salt thereof according to claim 8, wherein $A^2$ is an optionally substituted aliphatic heterocyclic group, $NR^{12}R^{13}$ (wherein $R^{12}$ and $R^{13}$ have the same meanings as defined above, respectively), optionally substituted aliphatic heterocycle-oxy, optionally substituted aliphatic heterocycle-methyl, or $CH_2NR_{10}R_{11}$ (wherein $R^{10}$ and $R^{11}$ have the same meanings as defined above, respectively).

**11.** The 2-aminoquinazoline derivative or a pharmaceutically acceptable salt thereof according to claim 8, wherein $A^2$ is $NR^{12}R^{13}$, wherein said $R^{12}$ is a hydrogen atom, and said $R^{13}$ is optionally substituted $C_{1-10}$ alkyl.

**12.** The 2-aminoquinazoline derivative or a pharmaceutically acceptable salt thereof according to claim 8, wherein one of $X^1$, $X^2$, $X^3$, and $X^4$ is a nitrogen atom, and the others may be the same or different, and each is CH or C-$A^2$ (wherein $A^2$ has the same meaning as defined above).

**13.** The 2-aminoquinazoline derivative or a pharmaceutically acceptable salt thereof according to claim 8, wherein among $X^1$, $X^2$ $X^3$ and $X^4$ one of $X^1$, $X^2$ and $X^4$ is a nitrogen atom, and the other three may be the same or different, and each is CH or C-$A^2$ (wherein $A^2$ has the same meaning as defined above).

**14.** The 2-aminoquinazoline derivative or a pharmaceutically acceptable salt thereof according to claim 8, wherein $X^1$, $X^2$, and $X^3$ may be the same or different, and each is CH or C-$A^2$ (wherein $A^2$ has the same meaning as defined above), and $X^4$ is a nitrogen atom.

**15.** The 2-aminoquinazoline derivative or a pharmaceutically acceptable salt thereof according to claim 8, wherein among $X^1$, $X^2$, $X^3$, and $X^4$, any two of them are nitrogen atoms, and the other two may be the same or different, and each is CH or C-$A^2$ (wherein $A^2$ has the same meaning as defined above).

**16.** The 2-aminoquinazoline derivative or a pharmaceutically acceptable salt thereof according to claim 8, wherein among $X^1$, $X^2$, $X^3$, and $X^4$,$X^1$ and $X^4$, or $X^2$ and $X^4$, are nitrogen atoms, and the other two may be the same or different, and each is CH or C-$A^2$ (wherein $A^2$ has the same meaning as defined above).

**17.** The 2-aminoquinazoline derivative or a pharmaceutically acceptable salt thereof according to any one of claims 1 to 16, wherein $A^1$ represents formula (III).

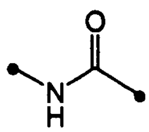 (III)

**18.** Use of the 2-aminoquinazoline derivative or a pharmaceutically acceptable salt thereof according to any one of claims 1 to 17 for the manufacture of a Tie-2 kinase inhibitor.

**19.** The 2-aminoquinazoline derivative or a pharmaceutically acceptable salt thereof according to any one of claims 1 to 17 for use in the inhibition of Tie-2 kinase.

**Patentansprüche**

**1.** 2-Aminochinazolinderivat der Formel (I)

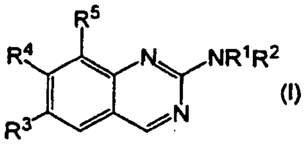

(I)

{wobei in Formel (I) $R^1$ ein Wasserstoffatom, einen gegebenenfalls substituierten $C_{1-10}$-Alkylrest, einen gegebenenfalls substituierten $C_{2-10}$-Alkenylrest, einen gegebenenfalls substituierten $C_{2-10}$-Alkinylrest, einen gegebenenfalls substituierten Cycloalkylrest, einen gegebenenfalls substituierten Cycloalkenylrest, einen gegebenenfalls substituierten $C_{2-11}$-Alkanoylrest, einen gegebenenfalls substituierten Cycloalkylcarbonylrest, einen gegebenenfalls substituierten $C_{1-10}$-Alkoxycarbonylrest, einen gegebenenfalls substituierten Arylrest, einen gegebenenfalls substituierten aliphatischen heterocyclischen Rest, einen gegebenenfalls substituierten aromatischen heterocyclischen Rest, oder $-C(=O)NR^6R^7$ darstellt (wobei $R^6$ und $R^7$ gleich oder verschieden sein können und jedes ein Wasserstoff oder einen gegebenenfalls substituierten $C_{1-10}$-Alkylrest darstellt),
$R^2$ ein Wasserstoffatom darstellt,
$R^3$ Formel (II) darstellt

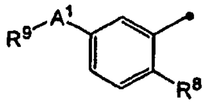

(II)

[wobei in Formel (II) $A^1$ Formel (III)

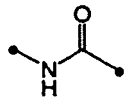

(III)

oder Formel (IV) darstellt

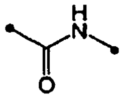

(IV)

,

$R^8$ ein Wasserstoffatom oder einen gegebenenfalls substituierten $C_{1-10}$-Alkylrest darstellt, und $R^9$ einen gegebenenfalls substituierten Arylrest oder einen gegebenenfalls substituierten aromatischen heterocyclischen Rest darstellt],
$R^4$ eine Hydroxygruppe oder einen gegebenenfalls substituierten $C_{1-10}$-Alkoxyrest darstellt, und $R^5$ einen gegebenenfalls substituierten Arylrest oder einen gegebenenfalls substituierten aromatischen heterocyclischen Rest darstellt}, oder
ein pharmazeutisch verträgliches Salz davon.

**2.** 2-Aminochinazolinderivat oder ein pharmazeutisch verträgliches Salz davon gemäß Anspruch 1, wobei $R^1$ ein Wasserstoffatom, einen gegebenenfalls substituierten $C_{1-10}$-Alkylrest, einen gegebenenfalls substituierten $C_{2-10}$-Alkenylrest, einen gegebenenfalls substituierten $C_{2-10}$-Alkinylrest, einen gegebenenfalls substituierten Cycloalkenylrest, einen gegebenenfalls substituierten $C_{2-11}$-Alkanoylrest, einen gegebenenfalls substituierten Cycloalkylcarbonylrest, einen gegebenenfalls substituierten $C_{1-10}$-Alkoxycarbonylrest oder $-C(=O)NR^6R^7$ darstellt (wobei $R^6$ und $R^7$ jeweils die gleiche Bedeutung wie vorstehend definiert haben).

**3.** 2-Aminochinazolinderivat oder ein pharmazeutisch verträgliches Salz davon gemäß Anspruch 1, wobei $R^1$ ein Wasserstoffatom ist.

**4.** 2-Aminochinazolinderivat oder ein pharmazeutisch verträgliches Salz davon gemäß Anspruch 1, wobei $R^1$ ein gegebenenfalls substituierter $C_{1-10}$-Alkylrest, ein gegebenenfalls substituierter $C_{2-11}$-Alkanoylrest, ein gegebenenfalls substituierter Cycloalkylcarbonylrest oder ein gegebenenfalls substituierter $C_{1-10}$-Alkoxycarbonylrest ist.

**5.** 2-Aminochinazolinderivat oder ein pharmazeutisch verträgliches Salz davon gemäß Anspruch 1, wobei $R^1$ ein gegebenenfalls substituierter Cycloalkylrest, ein gegebenenfalls substituierter Arylrest, ein gegebenenfalls substituierter aliphatischer heterocyclischer Rest oder ein gegebenenfalls substituierter aromatischer heterocyclischer Rest ist.

**6.** 2-Aminochinazolinderivat oder ein pharmazeutisch verträgliches Salz davon gemäß einem der Ansprüche 1 bis 5, wobei $R^4$ eine Hydroxy- oder Methoxygruppe ist.

**7.** 2-Aminochinazolinderivat oder ein pharmazeutisch verträgliches Salz davon gemäß einem der Ansprüche 1 bis 6, wobei $R^8$ eine Methylgruppe ist.

**8.** 2-Aminochinazolinderivat oder ein pharmazeutisch verträgliches Salz davon gemäß einem der Ansprüche 1 bis 7, wobei $R^9$ ein Rest gemäß Formel (V) ist

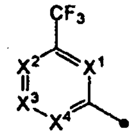 (V)

(wobei $X^1$, $X^2$, $X^3$ und $X^4$ gleich oder verschieden sein können, und jeweils CH, C-$A^2$ {wobei $A^2$ Halogen, einen gegebenenfalls substituierten $C_{1-10}$-Alkylrest, einen gegebenenfalls substituierten aliphatischen heterocyclischen Rest, einen gegebenenfalls substituierten aromatischen heterocyclischen Rest, einen gegebenenfalls substituierten $C_{1-10}$-Alkoxyrest, einen gegebenenfalls substituierten aliphatischen Heterocyclus-oxyrest, einen gegebenenfalls substituierten aromatischen Heterocyclus-oxyrest, einen gegebenenfalls substituierten aliphatischen Heterocyclus-methylrest, einen gegebenenfalls substituierten aromatischen Heterocyclus-methylrest, $CH_2NR^{10}R^{11}$ (wobei $R^{10}$ und $R^{11}$ gleich oder verschieden sein können und jeweils ein Wasserstoffatom oder einen gegebenenfalls substituierten $C_{1-10}$-Alkylrest darstellen) oder $NR^{12}R^{13}$ darstellt [worin $R^{12}$ ein Wasserstoffatom oder einen gegebenenfalls substituierten $C_{1-10}$-Alkylrest darstellt und $R^{13}$ ein Wasserstoffatom, einen gegebenenfalls substituierten $C_{1-10}$-Alkylrest, einen gegebenenfalls substituierten aliphatischen heterocyclischen Rest, einen gegebenenfalls substituierten aromatischen heterocyclischen Rest oder $NR^{10A}R^{11A}$ darstellt (worin $R^{10A}$ bzw. $R^{11A}$ jeweils die gleiche Bedeutung wie die vorstehend definierten $R^{10}$ bzw. $R^{11}$ haben)]} oder ein Stickstoffatom darstellen).

**9.** 2-Aminochinazolinderivat oder ein pharmazeutisch verträgliches Salz davon gemäß Anspruch 8, wobei $X^1$, $X^2$, $X^3$ und $X^4$ gleich oder verschieden sein können, und jedes CH oder ein Stickstoffatom ist.

**10.** 2-Aminochinazolinderivat oder ein pharmazeutisch verträgliches Salz davon gemäß Anspruch 8, wobei $A^2$ ein gegebenenfalls substituierter aliphatischer heterocyclischer Rest, $NR^{12}R^{13}$ (wobei $R^{12}$ bzw. $R^{13}$ die gleichen Bedeutungen wie vorstehend definiert haben), ein gegebenenfalls substituierter aliphatischer Heterocyclus-oxyrest, ein gegebenenfalls substituierter aliphatischer Heterocyclus-methylrest oder $CH_2NR^{10}R^{11}$ ist (wobei $R^{10}$ bzw. $R^{11}$ die gleiche Bedeutung wie vorstehend definiert haben).

**11.** 2-Aminochinazolinderivat oder ein pharmazeutisch verträgliches Salz davon gemäß Anspruch 8, wobei $A^2$ $NR^{12}R^{13}$ ist, wobei $R^{12}$ ein Wasserstoffatom ist und $R^{13}$ ein gegebenenfalls substituierter $C_{1-10}$-Alkylrest ist.

**12.** 2-Aminochinazolinderivat oder ein pharmazeutisch verträgliches Salz davon gemäß Anspruch 8, wobei einer der Reste $X^1$, $X^2$, $X^3$ und $X^4$ ein Stickstoffatom ist und die anderen gleich oder verschieden sein können und jeder CH oder C-$A^2$ ist (wobei $A^2$ die gleiche Bedeutung wie vorstehend definiert hat).

**13.** 2-Aminochinazolinderivat oder ein pharmazeutisch verträgliches Salz davon gemäß Anspruch 8, wobei unter $X^1$, $X^2$, $X^3$ und $X^4$ einer der Reste $X^1$, $X^2$ und $X^4$ ein Stickstoffatom ist und die anderen drei gleich oder verschieden sein können und jeder CH oder C-$A^2$ ist (wobei $A^2$ die gleiche Bedeutung wie vorstehend definiert hat).

**14.** 2-Aminochinazolinderivat oder ein pharmazeutisch verträgliches Salz davon gemäß Anspruch 8, wobei $X^1$, $X^2$ und $X^3$ gleich oder verschieden sein können und jeder CH oder C-$A^2$ ist (wobei $A^2$ die gleiche Bedeutung wie vorstehend definiert hat), und $X^4$ ein Stickstoffatom ist.

**15.** 2-Aminochinazolinderivat oder ein pharmazeutisch verträgliches Salz davon gemäß Anspruch 8, wobei unter $X^1$,

$X^2$, $X^3$ und $X^4$ jeweils zwei Stickstoffatome sind, und die anderen beiden gleich oder verschieden sein können und jedes CH oder C-$A^2$ ist (wobei $A^2$ die gleiche Bedeutung wie vorstehend definiert hat).

**16.** 2-Aminochinazolinderivat oder ein pharmazeutisch verträgliches Salz davon gemäß Anspruch 8, wobei unter $X^1$, $X^2$, $X^3$ und $X^4$, $X^1$ und $X^4$ oder $X^2$ und $X^4$ Stickstoffatome sind und die anderen beiden gleich oder verschieden sein können und jedes CH oder C$A^2$ ist (wobei $A^2$ die gleiche Bedeutung wie vorstehend definiert hat).

**17.** 2-Aminochinazolinderivat oder ein pharmazeutisch verträgliches Salz davon gemäß einem der Ansprüche 1 bis 16, wobei $A^1$ für Formel (III) steht.

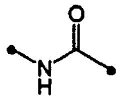
(III)

**18.** Verwendung des 2-Aminochinazolinderivats oder eines pharmazeutisch verträglichen Salzes davon gemäß einem der Ansprüche 1 bis 17 für die Herstellung eines Tie-2-Kinase-Inhibitors.

**19.** 2-Aminochinazolinderivat oder ein pharmazeutisch verträgliches Salz davon gemäß einem der Ansprüche 1 bis 17 zur Verwendung bei der Inhibition der Tie-2-Kinase.

**Revendications**

**1.** Dérivé de 2-aminoquinazoline représenté par la formule (I)

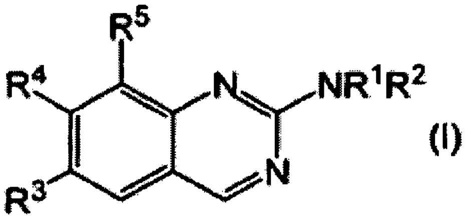
(I)

{où dans la formule (I), $R^1$ représente un atome d'hydrogène, un groupe alkyle en $C_{1-10}$ éventuellement substitué, alcényle en $C_{2-10}$ éventuellement substitué, alcynyle en $C_{2-10}$ éventuellement substitué, cycloalkyle éventuellement substitué, cycloalkyle éventuellement substitué, cycloalcényle éventuellement substitué, alcanoyle en $C_{2-11}$ éventuellement substitué, cycloalkylcarbonyle éventuellement substitué, alcoxycarbonyle en $C_{1-10}$ éventuellement substitué, aryle éventuellement substitué, un groupe hétérocyclique aliphatique éventuellement substitué, un groupe hétérocyclique aromatique éventuellement substitué, ou -C(=O)N$R^6R^7$ (où $R^6$ et $R^7$ peuvent être identiques ou différents et représentent chacun un atome d'hydrogène ou un groupe alkyle en $C_{1-10}$ éventuellement substitué),
$R^2$ représente un atome d'hydrogène,
$R^3$ représente la formule (II)

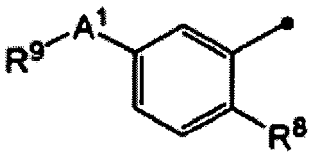
(II)

[où dans la formule (II), $A^1$ représente la formule (III)

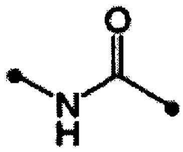

(III)

ou la formule (IV)

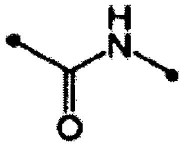

(IV)

,

$R^8$ représente un atome d'hydrogène ou un groupe alkyle en $C_{1-10}$ éventuellement substitué, et $R^9$ représente un groupe aryle éventuellement substitué ou un groupe hétérocyclique aromatique éventuellement substitué],
$R^4$ représente un groupe hydroxy, ou alcoxy en $C_{1-10}$ éventuellement substitué, et
$R^5$ représente un groupe aryle éventuellement substitué, ou un groupe hétérocyclique aromatique éventuellement substitué}, ou
sel pharmaceutiquement acceptable de celui-ci.

2. Dérivé de 2-aminoquinazoline ou sel pharmaceutiquement acceptable de celui-ci selon la revendication 1, dans lequel $R^1$ est un atome d'hydrogène, un groupe alkyle en $C_{1-10}$ éventuellement substitué, alcényle en $C_{2-10}$ éventuellement substitué, alcynyle en $C_{2-10}$ éventuellement substitué, cycloalcényle éventuellement substitué, alcanoyle en $C_{2-11}$ éventuellement substitué, cycloalkylcarbonyle éventuellement substitué, alcoxycarbonyle en $C_{1-10}$ éventuellement substitué ou -C(=O)NR$^6$R$^7$ (où $R^6$ et $R^7$ ont les mêmes significations que définies ci-dessus respectivement).

3. Dérivé de 2-aminoquinazoline ou sel pharmaceutiquement acceptable de celui-ci selon la revendication 1, dans lequel $R^1$ est un atome d'hydrogène.

4. Dérivé de 2-aminoquinazoline ou sel pharmaceutiquement acceptable de celui-ci selon la revendication 1, dans lequel $R^1$ est un groupe alkyle en $C_{1-10}$ éventuellement substitué, alcanoyle en $C_{2-11}$ éventuellement substitué, cycloalkylcarbonyle éventuellement substitué ou alcoxycarbonyle en $C_{1-10}$ éventuellement substitué.

5. Dérivé de 2-aminoquinazoline ou sel pharmaceutiquement acceptable de celui-ci selon la revendication 1, dans lequel $R^1$ est un groupe cycloalkyle éventuellement substitué, aryle éventuellement substitué, un groupe hétérocyclique aliphatique éventuellement substitué ou un groupe hétérocyclique aromatique éventuellement substitué.

6. Dérivé de 2-aminoquinazoline ou sel pharmaceutiquement acceptable de celui-ci selon l'une quelconque des revendications 1 à 5, dans lequel $R^4$ est un groupe hydroxy ou méthoxy.

7. Dérivé de 2-aminoquinazoline ou sel pharmaceutiquement acceptable de celui-ci selon l'une quelconque des revendications 1 à 6, dans lequel $R^8$ est le groupe méthyle.

8. Dérivé de 2-aminoquinazoline ou sel pharmaceutiquement acceptable de celui-ci selon l'une quelconque des revendications 1 à 7, dans lequel $R^9$ est un groupe représenté par la formule (V)

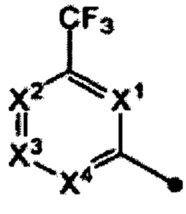

(V)

(où $X^1$, $X^2$, $X^3$, et $X^4$ peuvent être identiques ou différents, et représentent chacun CH, C-$A^2$ {où $A^2$ représente un atome d'halogène, un groupe alkyle en $C_{1-10}$ éventuellement substitué, un groupe hétérocyclique aliphatique éventuellement substitué, un groupe hétérocyclique aromatique éventuellement substitué, un groupe alcoxy en $C_{1-10}$ éventuellement substitué, un hétérocycle-oxy aliphatique éventuellement substitué, un hétérocycle-oxy aromatique éventuellement substitué, un hétérocycle-méthyle aliphatique éventuellement substitué, un hétérocycle-méthyle aromatique éventuellement substitué, $CH_2NR^{10}R^{11}$ (où $R^{10}$ et $R^{11}$ peuvent être identiques ou différents, et représentent chacun un atome d'hydrogène, ou un groupe alkyle en $C_{1-10}$ éventuellement substitué) ou $NR^{12}R^{13}$ [où $R^{12}$ représente un atome d'hydrogène ou un groupe alkyle en $C_{1-10}$ éventuellement substitué, et $R^{13}$ représente un atome d'hydrogène, un groupe alkyle en $C_{1-10}$ éventuellement substitué, un groupe hétérocyclique aliphatique éventuellement substitué, un groupe hétérocyclique aromatique éventuellement substitué ou -$NR^{10A}R^{11A}$ (où $R^{10A}$ et $R^{11A}$ ont les mêmes significations que $R^{10}$ et $R^{11}$ définis ci-dessus respectivement)]}, ou un atome d'azote).

**9.** Dérivé de 2-aminoquinazoline ou sel pharmaceutiquement acceptable de celui-ci selon la revendication 8, dans lequel $X^1$, $X^2$, $X^3$, et $X^4$ peuvent être identiques ou différents, et chacun est CH ou un atome d'azote.

**10.** Dérivé de 2-aminoquinazoline ou sel pharmaceutiquement acceptable de celui-ci selon la revendication 8, dans lequel $A^2$ est un groupe hétérocyclique aliphatique éventuellement substitué, $NR^{12}R^{13}$ (où $R^{12}$ et $R^{13}$ ont les mêmes significations que définies ci-dessus, respectivement), un hétérocycle-oxy aliphatique éventuellement substitué, un hétérocycle-méthyle aliphatique éventuellement substitué, ou $CH_2NR^{10}R^{11}$ (où $R^{10}$ et $R^{11}$ ont les mêmes significations que définies ci-dessus, respectivement).

**11.** Dérivé de 2-aminoquinazoline ou sel pharmaceutiquement acceptable de celui-ci selon la revendication 8, dans lequel $A^2$ est $NR^{12}R^{13}$, où ledit $R^{12}$ est un atome d'hydrogène, et ledit $R^{13}$ est un groupe alkyle en $C_{1-10}$ éventuellement substitué.

**12.** Dérivé de 2-aminoquinazoline ou sel pharmaceutiquement acceptable de celui-ci selon la revendication 8, dans lequel un de $X^1$, $X^2$, $X^3$, et $X^4$ est un atome d'azote, et les autres peuvent être identiques ou différents, et chacun est CH ou C-$A^2$ (où $A^2$ a la même signification que définie ci-dessus).

**13.** Dérivé de 2-aminoquinazoline ou sel pharmaceutiquement acceptable de celui-ci selon la revendication 8, dans lequel parmi $X^1$, $X^2$, $X^3$, et $X^4$, l'un de $X^1$, $X^2$ et $X^4$ est un atome d'azote, et les trois autres peuvent être identiques ou différents et chacun est CH ou C-$A^2$ (où $A^2$ a la même signification que définie ci-dessus).

**14.** Dérivé de 2-aminoquinazoline ou sel pharmaceutiquement acceptable de celui-ci selon la revendication 8, dans lequel $X^1$, $X^2$ , et $X^3$ peuvent être identiques ou différents, et chacun est CH ou C-$A^2$ (où $A^2$ a la même signification que définie ci-dessus), et $X^4$ est un atome d'azote.

**15.** Dérivé de 2-aminoquinazoline ou sel pharmaceutiquement acceptable de celui-ci selon la revendication 8, dans lequel parmi $X^1$, $X^2$, $X^3$, et $X^4$, deux quelconques d'entre eux sont des atomes d'azote, et les deux autres peuvent être identiques ou différents, et chacun est CH ou C-$A^2$ (où $A^2$ a la même signification que définie ci-dessus).

**16.** Dérivé de 2-aminoquinazoline ou sel pharmaceutiquement acceptable de celui-ci selon la revendication 8, dans lequel parmi $X^1$, $X^2$, $X^3$, et $X^4$, $X^1$ et $X^4$, ou $X^2$ et $X^4$, sont des atomes d'azote, et les deux autres peuvent être identiques ou différents, et chacun est CH ou C-$A^2$ (où $A^2$ a la même signification que définie ci-dessus).

**17.** Dérivé de 2-aminoquinazoline ou sel pharmaceutiquement acceptable de celui-ci selon l'une quelconque des revendications 1 à 16, dans lequel $A^1$ représente la formule (III).

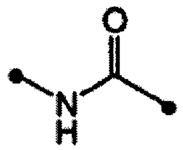
(III)

**18.** Utilisation du dérivé de 2-aminoquinazoline ou d'un sel pharmaceutiquement acceptable de celui-ci selon l'une quelconque des revendications 1 à 17 pour la fabrication d'un inhibiteur de kinase Tie-2.

19. Dérivé de 2-aminoquinazoline ou sel pharmaceutiquement acceptable de celui-ci selon l'une quelconque des revendications 1 à 17 pour une utilisation dans l'inhibition de kinase Tie-2.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 250501964 B
- WO 9307124 A
- WO 9322460 A
- WO 9850370 A
- US 6156758 A
- WO 200326667 A
- JP 6324437 A
- WO 2004098494 A
- WO 2005087742 A
- WO 2006015859 A
- WO 2006039718 A
- WO 0138315 A
- WO 2006071095 A
- WO 2006118256 A
- WO 2007117607 A
- WO 06039718 A
- WO 06021881 A
- WO 06021884 A
- WO 03093273 A


### Non-patent literature cited in the description


- *Nature Reviews Molecular Cell Biology,* 2001, vol. 2, 257
- *International Journal of Cancer,* 2002, vol. 99, 821
- *Bioorg. Med. Chem. Lett.,* 2007, vol. 17, 4756-4760
- **T. W. GREENE.** Protective Groups in Organic Synthesis. John Wiley & Sons Inc, 1999
- *Chemical Reviews,* 1990, vol. 90, 879
- *Jikken Kagaku Koza,* 1991, vol. 21, 30
- *Journal of Heterocyclic Chemistry,* 1997, vol. 34, 385
- *Journal of Organic Chemistry,* 1992, vol. 57, 2497
- Jikken Kagaku Koza. Japan Society for Analytical Chemistry, 1992, vol. 24
- Jikken Kagaku Koza. Maruzen, 1992, vol. 20, 279